# EUROPEAN PATENT SPECIFICATION

(11) **EP 2 314 712 B1**
(45) Date of publication and mention of the grant of the patent: **08.01.2014**
(21) Application number: 10180603.2
(22) Date of filing: 07.11.2002
(51) Int. Cl.: C12Q 1/37, A61K 39/00, C07K 14/18, C07K 14/47

(54) **Expression vectors encoding epitopes of antigens and methods for their design**
Für Epitope von Antigenen kodierende Expressionsvektoren sowie Verfahren zu deren Konzeption
Vecteurs d´expression codant pour des épitopes d´antigènes et procédés permittant leur conception

(30) Priority: 07.11.2001 US 336968 P
(43) Date of publication of application: 27.04.2011
(62) Divisional of application: 02806695.9
(73) Proprietor: Mannkind Corporation, Valencia, California 91355 (US)
(72) Inventor: Simard, John J.L., Austin, TX 78733 (US); Qui, Zhiyong, San Gabriel, CA 91776 (US); Diamond, David C., West Hills, CA 91304 (US); Lei, Xiang-Dong, West Hills, CA 91307 (US)
(74) Representative: Lasar, Andrea Gisela

(56) References cited:
- WO-A-01/82963
- WO-A2-01/55393
- CHEN J-L ET AL: "IDENTIFICATION OF NY-ESO-1 PEPTIDE ANALOGUES CAPABLE OF IMPROVED STIMULATION OF TUMOR-REACTIVE CTL", JOURNAL OF IMMUNOLOGY, AMERICAN ASSOCIATION OF IMMUNOLOGISTS, US, vol. 165, no. 2, 15 July 2000 (2000-07-15) , pages 948-955, XP001015752, ISSN: 0022-1767
- MOREL SANDRA ET AL: "Processing of some antigens by the standard proteasome but not by the immunoproteasome results in poor presentation by dendritic cells", IMMUNITY, CELL PRESS, US, vol. 12, no. 1, 1 January 2000 (2000-01-01), pages 107-117, XP002183661, ISSN: 1074-7613, DOI: DOI:10.1016/S1074-7613(00)80163-6

### SEQUENCE LISTING

<110> Simard, John J. L.
   Diamond, David C.
   Qiu, Zhiyong
   Lei, Xiang-Dong
<120> EXPRESSION VECTORS ENCODING EPITOPES OF
   TARGET-ASSOCIATED ANTIGENS AND METHODS FOR THEIR DESIGN
<130> M 9318
<140> EP 02806695.9
   <141> 2002-11-07
<150> 60/336,968
   <151> 2001-11-07
<160> 979
<170> FastSEQ for Windows Version 4.0
<210> 1
   <211> 10
   <212> PRT
   <213> Homo Sapien
<400> 1
   Glu Leu Ala Gly Ile Gly Ile Leu Thr Val
    1               5                  10
<210> 2
   <211> 118
   <212> PRT
   <213> Homo Sapien
<400> 2
   Met Pro Arg Glu Asp Ala His Phe Ile Tyr Gly Tyr Pro Lys Lys Gly
    1               5                  10                  15
   His Gly His Ser Tyr Thr Thr Ala Glu Glu Ala Ala Gly Ile Gly Ile
               20                  25                  30
   Leu Thr Val Ile Leu Gly Val Leu Leu Leu Ile Gly Cys Trp Tyr Cys
           35                  40                  45
   Arg Arg Arg Asn Gly Tyr Arg Ala Leu Met Asp Lys Ser Leu His Val
       50                  55                  60
   Gly Thr Gln Cys Ala Leu Thr Arg Arg Cys Pro Gln Glu Gly Phe Asp
   65                  70                  75                  80
   His Arg Asp Ser Lys Val Ser Leu Gln Glu Lys Asn Cys Glu Pro Val
                   85                  90                  95
   Val Pro Asn Ala Pro Pro Ala Tyr Glu Lys Leu Ser Ala Glu Gln Ser
               100                 105                 110
   Pro Pro Pro Tyr Ser Pro
           115
<210> 3
   <211> 529
   <212> PRT
   <213> Homo Sapien
<400> 3
<210> 4
   <211> 94
   <212> PRT
   <213> Artificial Sequence
<220>
   <223> pMA2M expression product
<400> 4
   Met Leu Leu Ala Val Leu Tyr Cys Leu Glu Leu Ala Gly Ile Gly Ile
    1               5                  10                  15
   Leu Thr Val Tyr Met Asp Gly Thr Met Ser Gln Val Gly Ile Leu Thr
               20                  25                  30
   Val Ile Leu Gly Val Leu Leu Leu Ile Gly Cys Trp Tyr Cys Arg Arg
           35                  40                  45
   Arg Asn Gly Tyr Arg Ala Leu Met Asp Lys Ser Leu His Val Gly Thr
       50                  55                  60
   Gln Cys Ala Leu Thr Arg Arg Cys Pro Gln Glu Gly Phe Asp His Arg
   65                  70                  75                  80
   Asp Ser Lys Val Ser Leu Gln Glu Lys Asn Cys Glu Pro Val
                   85                  90
<210> 5
   <211> 28
   <212> PRT
   <213> Artificial sequence
<220>
   <223> Epitope liberation sequence for SEQ ID NO. 1 from pMA2M
<400> 5
   Met Leu Leu Ala Val Leu Tyr Cys Leu Glu Leu Ala Gly Ile Gly Ile
    1               5                  10                  15
   Leu Thr Val Tyr Met Asp Gly Thr Met Ser Gln Val
               20                  25
<210> 6
   <211> 9
   <212> PRT
   <213> Homo Sapien
<400> 6
   Met Leu Leu Ala Val Leu Tyr Cys Leu
    1               5
<210> 7
   <211> 9
   <212> PRT
   <213> Homo Sapien
<400> 7
   Tyr Met Asp Gly Thr Met Ser Gln Val
    1               5
<210> 8
   <211> 10
   <212> PRT
   <213> Homo Sapien
<400> 8
   Glu Ala Ala Gly Ile Gly Ile Leu Thr Val
   1               5                   10
<210> 9
   <211> 307
   <212> DNA
   <213> Artificial Sequence
<220>
   <223> pMA2M insert coding region
<400> 9
   cttaagccac catgttacta gctgttttgt actgcctgga actagcaggg atcggcatat 60
   tgacagtgta tatggatgga acaatgtccc aggtaggaat tctgacagtg atcctgggag 120
   tcttactgct catcggctgt tggtattgta gaagacgaaa tggatacaga gccttgatgg 180
   ataaaagtct tcatgttggc actcaatgtg ccttaacaag aagatgccca caagaagggt 240
   ttgatcatcg ggacagcaaa gtgtctcttc aagagaaaaa ctgtgaacct gtgtagtgag 300
   cggccgc                                                           307
<210> 10
   <211> 85
   <212> PRT
   <213> Artificial sequence
<220>
   <223> Epitope array from pVAXM2 and pVAXM1
<400> 10
   Met Val Leu Tyr Cys Leu Glu Leu Ala Gly Ile Gly Ile Leu Thr Val
   1               5                   10                  15
   Tyr Met Asp Gly Thr Ala Val Leu Tyr Cys Leu Glu Leu Ala Gly Ile
               20                  25                  30
   Gly Ile Leu Thr Val Tyr Met Asp Gly Thr Met Leu Ala Val Leu Tyr
           35                  40                  45
   Cys Leu Glu Leu Ala Gly Ile Gly Ile Leu Thr Val Tyr Met Asp Gly
       50                  55                  60
   Thr Met Ser Leu Leu Ala Val Leu Tyr Cys Leu Glu Leu Ala Gly Ile
   65                  70                  75                  80
   Gly Ile Leu Thr Val
                   85
<210> 11
   <211> 180
   <212> PRT
   <213> Homo sapien
<400> 11
   Met Gln Ala Glu Gly Arg Gly Thr Gly Gly Ser Thr Gly Asp Ala Asp
   1               5                   10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Gly Leu Asn Gly Cys Cys Arg Cys Gly Ala
   65                  70                  75                  80
   Arg Gly Pro Glu Ser Arg Leu Leu Glu Phe Tyr Leu Ala Met Pro Phe
                   85                  90                  95
   Ala Thr Pro Met Glu Ala Glu Leu Ala Arg Arg Ser Leu Ala Gln Asp
               100                 105                 110
   Ala Pro Pro Leu Pro Val Pro Gly Val Leu Leu Lys Glu Phe Thr Val
           115                 120                 125
   Ser Gly Asn Ile Leu Thr Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Pro Pro Ser
                   165                 170                 175
   Gly Gln Arg Arg
               180
<210> 12
   <211> 9
   <212> PRT
   <213> Homo Sapien
<400> 12
   Ser Leu Leu Met Trp Ile Thr Gln Cys
   1               5
<210> 13
   <211> 9
   <212> PRT
   <213> Homo Sapien
<400> 13
   Lys Ala Ser Glu Lys Ile Phe Tyr Val
   1               5
<210> 14
   <211> 9
   <212> PRT
   <213> Homo Sapien
<400> 14
   Thr Gln Cys Phe Leu Pro Val Phe Leu
   1               5
<210> 15
   <211> 10
   <212> PRT
   <213> Homo Sapien
<400> 15
   Gly Leu Pro Ser Ile Pro Val His Pro Ile
   1 5 10
<210> 16
   <211> 6
   <212> PRT
   <213> Homo Sapien
<400> 16
   Ala Val Leu Tyr Cys Leu
   1 5
<210> 17
   <211> 123
   <212> PRT
   <213> Artificial Sequence
<220>
   <223> pN157 expression product
<400> 17
   Met Ser Leu Leu Met Trp Ile Thr Gln Cys Lys Ala Ser Glu Lys Ile
    1              5                  10                  15
   Phe Tyr Val Arg Cys Gly Ala Arg Gly Pro Glu Ser Arg Leu Leu Glu
               20                  25                  30
   Phe Tyr Leu Ala Met Pro Phe Ala Thr Pro Met Glu Ala Glu Leu Ala
           35                  40                  45
   Arg Arg Ser Leu Ala Gln Asp Ala Pro Pro Leu Pro Val Pro Gly Val
       50                  55                  60
   Leu Leu Lys Glu Phe Thr Val Ser Gly Asn Ile Leu Thr Ile Arg Leu
   65                  70                  75                  80
   Thr Ala Ala Asp His Arg Gln Leu Gln Leu Ser Ile Ser Ser Cys Leu
                   85                  90                  95
   Gln Gln Leu Ser Leu Leu Met Trp Ile Thr Gln Cys Phe Leu Pro Val
               100                 105                 110
   Phe Leu Ala Gln Pro Pro Ser Gly Gln Arg Arg
           115                 120
<210> 18
   <211> 19
   <212> PRT
   <213> Artificial Sequence
<220>
   <223> Epitope liberation sequence for SEQ ID No. 12 from pN157
<400> 18
   Met Ser Leu Leu Met Trp Ile Thr Gln Cys Lys Ala Ser Glu Lys Ile
    1              5                  10                  15
   Phe Tyr Val
<210> 19
   <211> 392
   <212> DNA
   <213> Artificial Sequence
<220>
   <223> pN157 insert coding region
<400> 19
   cttaagccac catgtccctg ttgatgtgga tcacgcagtg caaagcttcg gagaaaatct 60
   tctacgtacg gtgcggtgcc agggggccgg agagccgcct gcttgagttc tacctcgcca 120
   tgcctttcgc gacacccatg gaagcagagc tggcccgcag gagcctggcc caggatgccc 180
   caccgcttcc cgtgccaggg gtgcttctga aggagttcac tgtgtccggc aacatactga 240
   ctatccgact gactgctgca gaccaccgcc aactgcagct ctccatcagc tcctgtctcc 300
   agcagctttc cctgttgatg tggatcacgc agtgctttct gcccgtgttt ttggctcagc 360
   ctccctcagg gcagaggcgc tagtgagaat tc                               392
<210> 20
   <211> 179
   <212> PRT
   <213> Artificial Sequence
<220>
   <223> pBPL expression product

<400> 20
   Met Ser Leu Leu Met Trp Ile Thr Gln Cys Lys Ala Ser Glu Lys Ile
   1               5                   10                  15
   Phe Tyr Val Gly Leu Pro Ser Ile Pro Val His Pro Ile Gly Leu Pro
               20                  25                  30
   Ser Ile Pro Val His Pro Ile Lys Ala Ser Glu Lys Ile Phe Tyr Val
           35                  40                  45
   Ser Leu Leu Met Trp Ile Thr Gln Cys Lys Ala Ser Glu Lys Ile Phe
       50                  55                  60
   Tyr Val Lys Ala Ser Glu Lys Ile Phe Tyr Val Arg Cys Gly Ala Arg
   65                  70                  75                  80
   Gly Pro Glu Ser Arg Leu Leu Glu Phe Tyr Leu Ala Met Pro Phe Ala
                   85                  90                  95
   Thr Pro Met Glu Ala Glu Leu Ala Arg Arg Ser Leu Ala Gln Asp Ala
               100                 105                 110
   Pro Pro Leu Pro Val Pro Gly Val Leu Leu Lys Glu Phe Thr Val Ser
           115                 120                 125
   Gly Asn Ile Leu Thr Ile Arg Leu Thr Ala Ala Asp His Arg Gln Leu
       130                 135                 140
   Gln Leu Ser Ile Ser Ser Cys Leu Gln Gln Leu Ser Leu Leu Met Trp
   145                 150                 155                 160
   Ile Thr Gln Cys Phe Leu Pro Val Phe Leu Ala Gln Pro Pro Ser Gly
                   165                 170                 175
   Gln Arg Arg
<210> 21
   <211> 543
   <212> DNA
   <213> Artificial Sequence
<220>
   <223> pBPL insert coding region
<400> 21
   atgtccctgt tgatgtggat cacgcagtgc aaagcttcgg agaaaatctt ctatgtgggt 60
   cttccaagta ttcctgttca tccaattggt cttccaagta ttcctgttca tccaattaaa 120
   gcttcggaga aaatcttcta tgtgtccctg ttgatgtgga tcacgcagtg caaagcttcg 180
   gagaaaatct tctatgtgaa agcttcggag aaaatcttct acgtacggtg cggtgccagg 240
   gggccggaga gccgcctgct tgagttctac ctcgccatgc ctttcgcgac acccatggaa 300
   gcagagctgg cccgcaggag cctggcccag gatgccccac cgcttcccgt gccaggggtg 360
   cttctgaagg agttcactgt gtccggcaac atactgacta tccgactgac tgctgcagac 420
   caccgccaac tgcagctctc catcagctcc tgtctccagc agctttccct gttgatgtgg 480
   atcacgcagt gctttctgcc cgtgtttttg gctcagcctc cctcagggca gaggcgctag 540
   tga                                                               543
<210> 22
   <211> 29
   <212> PRT
   <213> Artificial Sequence
<220>
   <223> liberation sequence for SEQ ID NO. 22
<400> 22
   Ile Lys Ala Ser Glu Lys Ile Phe Tyr Val Ser Leu Leu Met Trp Ile
    1               5                  10                  15
   Thr Gln Cys Lys Ala Ser Glu Lys Ile Phe Tyr Val Lys
               20                  25
<210> 23
   <211> 9
   <212> PRT
   <213> Homo sapien
<400> 23
   Val Met Thr Lys Leu Gly Phe Lys Val
    1               5
<210> 24
   <211> 10
   <212> PRT
   <213> Homo Sapien
<400> 24
   Arg Gln Ile Tyr Val Ala Ala Phe Thr Val
    1               5                  10
<210> 25
   <211> 169
   <212> PRT
   <213> Homo Sapien
<400> 25
   Ala Gln Ile Pro Glu Lys Ile Gln Lys Ala Phe Asp Asp Ile Ala Lys
   1               5                   10                  15
   Tyr Phe Ser Lys Glu Glu Trp Glu Lys Met Lys Ala Ser Glu Lys Ile
               20                  25                  30
   Phe Tyr Val Tyr Met Lys Arg Lys Tyr Glu Ala Met Thr Lys Leu Gly
           35                  40                  45
   Phe Lys Ala Thr Leu Pro Pro Phe Met Cys Asn Lys Arg Ala Glu Asp
       50                  55                  60
   Phe Gln Gly Asn Asp Leu Asp Asn Asp Pro Asn Arg Gly Asn Gln Val
   65                  70                  75                  80
   Glu Arg Pro Gln Met Thr Phe Gly Arg Leu Gln Gly Ile Ser Pro Lys
                   85                  90                  95
   Ile Met Pro Lys Lys Pro Ala Glu Glu Gly Asn Asp Ser Glu Glu Val
               100                 105                 110
   Pro Glu Ala Ser Gly Pro Gln Asn Asp Gly Lys Glu Leu Cys Pro Pro
           115                 120                 125
   Gly Lys Pro Thr Thr Ser Glu Lys Ile His Glu Arg Ser Gly Pro Lys
       130                 135                 140
   Arg Gly Glu His Ala Trp Thr His Arg Leu Arg Glu Arg Lys Gln Leu
   145                 150                 155                 160
   Val Ile Tyr Glu Glu Ile Ser Asp Pro
                   165
<210> 26
   <211> 245
   <212> PRT
   <213> Artificial Sequence
<220>
   <223> CTLS1/pCBP expression product
<400> 26
<210> 27
   <211> 245
   <212> PRT
   <213> Artificial Sequence
<220>
   <223> CTL52 expression product
<400> 27
<210> 28
   <211> 208
   <212> PRT
   <213> Artificial Sequence
<220>
   <223> CTL53 expression product
<400> 28
   Met Val Met Thr Lys Leu Gly Phe Lys Val Lys Ala Ser Glu Lys Ile
   1               5                   10                  15
   Phe Tyr Val Arg Gln Ile Tyr Val Ala Ala Phe Thr Val Gly Leu Pro
               20                  25                  30
   Ser Ile Pro Val His Pro Ile Ala Gln Ile Pro Glu Lys Ile Gln Lys
           35                  40                  45
   Ala Phe Asp Asp Ile Ala Lys Tyr Phe Ser Lys Glu Glu Trp Glu Lys
       50                  55                  60
   Met Lys Ala Ser Glu Lys Ile Phe Tyr Val Tyr Met Lys Arg Lys Tyr
   65                  70                  75                  80
   Glu Ala Met Thr Lys Leu Gly Phe Lys Ala Thr Leu Pro Pro Phe Met
                   85                  90                  95
   Cys Asn Lys Arg Ala Glu Asp Phe Gln Gly Asn Asp Leu Asp Asn Asp
               100                 105                 110
   Pro Asn Arg Gly Asn Gln Val Glu Arg Pro Gln Met Thr Phe Gly Arg
           115                 120                 125
   Leu Gln Gly Ile Ser Pro Lys Ile Met Pro Lys Lys Pro Ala Glu Glu
       130                 135                 140
   Gly Asn Asp Ser Glu Glu Val Pro Glu Ala Ser Gly Pro Gln Asn Asp
   145                 150                 155                 160
   Gly Lys Glu Leu Cys Pro Pro Gly Lys Pro Thr Thr Ser Glu Lys Ile
                   165                 170                 175
   His Glu Arg Ser Gly Pro Lys Arg Gly Glu His Ala Trp Thr His Arg
               180                 185                 190
   Leu Arg Glu Arg Lys Gln Leu Val Ile Tyr Glu Glu Ile Ser Asp Pro
           195                 200                 205
<210> 29
   <211> 207
   <212> PRT
   <213> Artificial Sequence
<220>
   <223> CTL54 expression product
<400> 29
   Met Ala Gln Ile Pro Glu Lys Ile Gln Lys Ala Phe Asp Asp Ile Ala
   1               5                   10                  15
   Lys Tyr Phe Ser Lys Glu Glu Trp Glu Lys Met Lys Ala Ser Glu Lys
               20                  25                  30
   Ile Phe Tyr Val Tyr Met Lys Arg Lys Tyr Glu Ala Met Thr Lys Leu
           35                  40                  45
   Gly Phe Lys Ala Thr Leu Pro Pro Phe Met Cys Asn Lys Arg Ala Glu
       50                  55                  60
   Asp Phe Gln Gly Asn Asp Leu Asp Asn Asp Pro Asn Arg Gly Asn Gln
   65                  70                  75                  80
   Val Glu Arg Pro Gln Met Thr Phe Gly Arg Leu Gln Gly Ile Ser Pro
                   85                  90                  95
   Lys Ile Met Pro Lys Lys Pro Ala Glu Glu Gly Asn Asp Ser Glu Glu
               100                 105                 110
   Val Pro Glu Ala Ser Gly Pro Gln Asn Asp Gly Lys Glu Leu Cys Pro
           115                 120                 125
   Pro Gly Lys Pro Thr Thr Ser Glu Lys Ile His Glu Arg Ser Gly Pro
       130                 135                 140
   Lys Arg Gly Glu His Ala Trp Thr His Arg Leu Arg Glu Arg Lys Gln
   145                 150                 155                 160
   Leu Val Ile Tyr Glu Glu Ile Ser Asp Pro Thr Gln Cys Phe Leu Pro
                   165                 170                 175
   Val Phe Leu Val Met Thr Lys Leu Gly Phe Lys Val Arg Gln Ile Tyr
               180                 185                 190
   Val Ala Ala Phe Thr Val Lys Ala Ser Glu Lys Ile Phe Tyr Val
           195                 200                 205
<210> 30
   <211> 741
   <212> DNA
   <213> Artificial sequence
<220>
   <223> pCBP insert coding region
<400> 30
   atggtcatga ctaaactagg tttcaaggtc aaagcttcgg agaaaatctt ctatgtgaga 60
   cagatttatg ttgcagcctt cacagtgggt cttccaagta ttcctgttca tccaattacg 120
   cagtgctttc tgcccgtgtt tttggtcatg actaaactag gtttcaaggt cagacagatt 180
   tatgttgcag ccttcacagt gaaagcttcg gagaaaatct tctacgtagc tcaaatacca 240
   gagaagatcc aaaaggcctt cgatgatatt gccaaatact tctctaagga agagtgggaa 300
   aagatgaaag cctcggagaa aatcttctat gtgtatatga agagaaagta tgaggctatg 360
   actaaactag gtttcaaggc caccctccca cctttcatgt gtaataaacg ggccgaagac 420
   ttccagggga atgatttgga taatgaccct aaccgtggga atcaggttga acgtcctcag 480
   atgactttcg gcaggctcca gggaatctcc ccgaagatca tgcccaagaa gccagcagag 540
   gaaggaaatg attcggagga agtgccagaa gcatctggcc cacaaaatga tgggaaagag 600
   ctgtgccccc cgggaaaacc aactacctct gagaagattc acgagagatc tggacccaaa 660
   aggggggaac atgcctggac ccacagactg cgtgagagaa aacagctggt gatttatgaa 720
   gagatcagcg acccttagtg a                                            741
<210> 31
   <211> 28
   <212> PRT
   <213> Artificial Sequence
<220>
   <223> CTLS11-2 liberation/substrate sequence
<400> 31
   Arg Gln Ile Tyr Val Ala Ala Phe Thr Val Lys Ala Ser Glu Lys Ile
   1               5                   10                  15
   Phe Tyr Val Ala Gln Ile Pro Glu Lys Ile Gln Lys
               20                  25
<210> 32
   <211> 9
   <212> PRT
   <213> Homo Sapien
<400> 32
   Phe Leu Pro Trp His Arg Leu Phe Leu
   1               5
<210> 33
   <211> 104
   <212> PRT
   <213> Artificial Sequence
<220>
   <223> CTLT2/pMEL expression product
<400> 33
   Met Leu Leu Ala Val Leu Tyr Cys Leu Leu Trp Ser Phe Gln Thr Ser
   1               5                   10                  15
   Ala Phe Leu Pro Trp His Arg Leu Phe Leu Met Leu Leu Ala Val Leu
               20                  25                  30
   Tyr Cys Leu Leu Trp Ser Phe Gln Thr Ser Ala Phe Leu Pro Trp His
           35                  40                  45
   Arg Leu Phe Leu Met Leu Leu Ala Val Leu Tyr Cys Leu Leu Trp Ser
       50                  55                  60
   Phe Gln Thr Ser Ala Phe Leu Pro Trp His Arg Leu Phe Leu Met Leu
   65                  70                  75                  80
   Leu Ala Val Leu Tyr Cys Leu Leu Trp Ser Phe Gln Thr Ser Ala Phe
                   85                  90                  95
   Leu Pro Trp His Arg Leu Phe Leu
               100
<210> 34
   <211> 318
   <212> DNA
   <213> Artificial Sequence
<220>
   <223> CTLT2/pMEL insert coding region
<400> 34
   atgctcctgg ctgttttgta ctgcctgctg tggagtttcc agacctccgc ttttctgcct   60
   tggcatagac tcttcttgat gctcctggct gttttgtact gcctgctgtg gagtttccag  120
   acctccgctt ttctgccttg gcatagactc ttcttgatgc tcctggctgt tttgtactgc  180
   ctgctgtgga gtttccagac ctccgctttt ctgccttggc atagactctt cttgatgctc  240
   ctggctgttt tgtactgcct gctgtggagt ttccagacct ccgcttttct gccttggcat  300
   agactcttct tgtagtga                                                318
<210> 35
   <211> 1524
   <212> DNA
   <213> Homo Sapien
<400> 35
   agcagacaga ggactctcat taaggaaggt gtcctgtgcc ctgaccctac aagatgccaa 60
   gagaagatgc tcacttcatc tatggttacc ccaagaaggg gcacggccac tcttacacca 120
   cggctgaaga ggccgctggg atcggcatcc tgacagtgat cctgggagtc ttactgctca 180
   tcggctgttg gtattgtaga agacgaaatg gatacagagc cttgatggat aaaagtcttc 240
   atgttggcac tcaatgtgcc ttaacaagaa gatgcccaca agaagggttt gatcatcggg 300
   acagcaaagt gtctcttcaa gagaaaaact gtgaacctgt ggttcccaat gctccacctg 360
   cttatgagaa actctctgca gaacagtcac caccacctta ttcaccttaa gagccagcga 420
   gacacctgag acatgctgaa attatttctc tcacactttt gcttgaattt aatacagaca 480
   tctaatgttc tcctttggaa tggtgtagga aaaatgcaag ccatctctaa taataagtca 540
   gtgttaaaat tttagtaggt ccgctagcag tactaatcat gtgaggaaat gatgagaaat 600
   attaaattgg gaaaactcca tcaataaatg ttgcaatgca tgatactatc tgtgccagag 660
   gtaatgttag taaatccatg gtgttatttt ctgagagaca gaattcaagt gggtattctg 720
   gggccatcca atttctcttt acttgaaatt tggctaataa caaactagtc aggttttcga 780
   accttgaccg acatgaactg tacacagaat tgttccagta ctatggagtg ctcacaaagg 840
   atacttttac aggttaagac aaagggttga ctggcctatt tatctgatca agaacatgtc 900
   agcaatgtct ctttgtgctc taaaattcta ttatactaca ataatatatt gtaaagatcc 960
   tatagctctt tttttttgag atggagtttc gcttttgttg cccaggctgg agtgcaatgg 1020
   cgcgatcttg gctcaccata acctccgcct cccaggttca agcaattctc ctgccttagc 1080
   ctcctgagta gctgggatta caggcgtgcg ccactatgcc tgactaattt tgtagtttta 1140
   gtagagacgg ggtttctcca tgttggtcag gctggtctca aactcctgac ctcaggtgat 1200
   ctgcccgcct cagcctccca aagtgctgga attacaggcg tgagccacca cgcctggctg 1260
   gatcctatat cttaggtaag acatataacg cagtctaatt acatttcact tcaaggctca 1320
   atgctattct aactaatgac aagtattttc tactaaacca gaaattggta gaaggattta 1380
   aataagtaaa agctactatg tactgcctta gtgctgatgc ctgtgtactg ccttaaatgt 1440
   acctatggca atttagctct cttgggttcc caaatccctc tcacaagaat gtgcagaaga 1500
   aatcataaag gatcagagat tctg                                       1524
<210> 36
   <211> 1964
   <212> DNA
   <213> Homo Sapien
<400> 36
<210> 37
   <211> 752
   <212> DNA
   <213> Homo Sapien
<400> 37
   atcctcgtgg gccctgacct tctctctgag agccgggcag aggctccgga gccatgcagg 60
   ccgaaggccg gggcacaggg ggttcgacgg gcgatgctga tggcccagga ggccctggca 120
   ttcctgatgg cccagggggc aatgctggcg gcccaggaga ggcgggtgcc acgggcggca 180
   gaggtccccg gggcgcaggg gcagcaaggg cctcggggcc gggaggaggc gccccgcggg 240
   gtccgcatgg cggcgcggct tcagggctga atggatgctg cagatgcggg gccagggggc 300
   cggagagccg cctgcttgag ttctacctcg ccatgccttt cgcgacaccc atggaagcag 360
   agctggcccg caggagcctg gcccaggatg ccccaccgct tcccgtgcca ggggtgcttc 420
   tgaaggagtt cactgtgtcc ggcaacatac tgactatccg actgactgct gcagaccacc 480
   gccaactgca gctctccatc agctcctgtc tccagcagct ttccctgttg atgtggatca 540
   cgcagtgctt tctgcccgtg tttttggctc agcctccctc agggcagagg cgctaagccc 600
   agcctggcgc cccttcctag gtcatgcctc ctcccctagg gaatggtccc agcacgagtg 660
   gccagttcat tgtgggggcc tgattgtttg tcgctggagg aggacggctt acatgtttgt 720
   ttctgtagaa aataaaactg agctacgaaa aa                               752
<210> 38
   <211> 750
   <212> PRT
   <213> Homo Sapien
<400> 38
   Met Trp Asn Leu Leu His Glu Thr Asp Ser Ala Val Ala Thr Ala Arg
   1               5                   10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
           35                  40                  45
   Ala Thr Asn Ile Thr Pro Lys His Asn Met Lys Ala Phe Leu Asp Glu
       50                  55                  60
   Leu Lys Ala Glu Asn Ile Lys Lys Phe Leu Tyr Asn Phe Thr Gln Ile
   65                  70                  75                  80
   Pro His Leu Ala Gly Thr Glu Gln Asn Phe Gln Leu Ala Lys Gln Ile
                   85                  90                  95
   Gln Ser Gln Trp Lys Glu Phe Gly Leu Asp Ser Val Glu Leu Ala His
               100                 105                 110
   Tyr Asp Val Leu Leu Ser Tyr Pro Asn Lys Thr His Pro Asn Tyr Ile
           115                 120                 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
               180                 185                 190
   Lys Ile Asn Cys Ser Gly Lys Ile Val Ile Ala Arg Tyr Gly Lys Val
           195                 200                 205
   Phe Arg Gly Asn Lys Val Lys Asn Ala Gln Leu Ala Gly Ala Lys Gly
       210                 215                 220
   Val Ile Leu Tyr Ser Asp Pro Ala Asp Tyr Phe Ala Pro Gly Val Lys
   225                 230                 235                 240
   Ser Tyr Pro Asp Gly Trp Asn Leu Pro Gly Gly Gly Val Gln Arg Gly
                   245                 250                 255
   Asn Ile Leu Asn Leu Asn Gly Ala Gly Asp Pro Leu Thr Pro Gly Tyr
   260 265 270
   Pro Ala Asn Glu Tyr Ala Tyr Arg Arg Gly Ile Ala Glu Ala Val Gly
   275 280 285
   Leu Pro Ser Ile Pro Val His Pro Ile Gly Tyr Tyr Asp Ala Gln Lys
   290 295 300
   Leu Leu Glu Lys Met Gly Gly Ser Ala Pro Pro Asp Ser Ser Trp Arg
   305 310 315 320
   Gly Ser Leu Lys Val Pro Tyr Asn Val Gly Pro Gly Phe Thr Gly Asn
   325 330 335
   Phe Ser Thr Gln Lys Val Lys Met His Ile His Ser Thr Asn Glu Val
   340 345 350
   Thr Arg Ile Tyr Asn Val Ile Gly Thr Leu Arg Gly Ala Val Glu Pro
   355 360 365
   Asp Arg Tyr Val Ile Leu Gly Gly His Arg Asp Ser Trp Val Phe Gly
   370 375 380
   Gly Ile Asp Pro Gln Ser Gly Ala Ala Val Val His Glu Ile Val Arg
   385 390 395 400
   Ser Phe Gly Thr Leu Lys Lys Glu Gly Trp Arg Pro Arg Arg Thr Ile
   405 410 415
   Leu Phe Ala Ser Trp Asp Ala Glu Glu Phe Gly Leu Leu Gly Ser Thr
   420 425 430
   Glu Trp Ala Glu Glu Asn Ser Arg Leu Leu Gln Glu Arg Gly Val Ala
   435 440 445
   Tyr Ile Asn Ala Asp Ser Ser Ile Glu Gly Asn Tyr Thr Leu Arg Val
   450 455 460
   Asp Cys Thr Pro Leu Met Tyr Ser Leu Val His Asn Leu Thr Lys Glu
   465 470 475 480
   Leu Lys Ser Pro Asp Glu Gly Phe Glu Gly Lys Ser Leu Tyr Glu Ser
   485 490 495
   Trp Thr Lys Lys Ser Pro Ser Pro Glu Phe Ser Gly Met Pro Arg Ile
   500 505 510
   Ser Lys Leu Gly Ser Gly Asn Asp Phe Glu Val Phe Phe Gln Arg Leu
   515 520 525
   Gly Ile Ala Ser Gly Arg Ala Arg Tyr Thr Lys Asn Trp Glu Thr Asn
   530 535 540
   Lys Phe Ser Gly Tyr Pro Leu Tyr His Ser Val Tyr Glu Thr Tyr Glu
   545 550 555 560
   Leu Val Glu Lys Phe Tyr Asp Pro Met Phe Lys Tyr His Leu Thr Val
   565 570 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
   645 650 655
   Asn Pro Ile Val Leu Arg Met Met Asn Asp Gln Leu Met Phe Leu Glu
   660 665 670
   Arg Ala Phe Ile Asp Pro Leu Gly Leu Pro Asp Arg Pro Phe Tyr Arg
   675 680 685
   His Val Ile Tyr Ala Pro Ser Ser His Asn Lys Tyr Ala Gly Glu Ser
   690 695 700
   Phe Pro Gly Ile Tyr Asp Ala Leu Phe Asp Ile Glu Ser Lys Val Asp
   705 710 715 720
   Pro Ser Lys Ala Trp Gly Glu Val Lys Arg Gln Ile Tyr Val Ala Ala
   725 730 735
   Phe Thr Val Gln Ala Ala Ala Glu Thr Leu Ser Glu Val Ala
   740 745 750
<210> 39
   <211> 2653
   <212> DNA
   <213> Homo Sapien
<400> 39
   ctcaaaaggg gccggatttc cttctcctgg aggcagatgt tgcctctctc tctcgctcgg 60
   attggttcag tgcactctag aaacactgct gtggtggaga aactggaccc caggtctgga 120
   gcgaattcca gcctgcaggg ctgataagcg aggcattagt gagattgaga gagactttac 180
   cccgccgtgg tggttggagg gcgcgcagta gagcagcagc acaggcgcgg gtcccgggag 240
   gccggctctg ctcgcgccga gatgtggaat ctccttcacg aaaccgactc ggctgtggcc 300
   accgcgcgcc gcccgcgctg gctgtgcgct ggggcgctgg tgctggcggg tggcttcttt 360
   ctcctcggct tcctcttcgg gtggtttata aaatcctcca atgaagctac taacattact 420
   ccaaagcata atatgaaagc atttttggat gaattgaaag ctgagaacat caagaagttc 480
   ttatataatt ttacacagat accacattta gcaggaacag aacaaaactt tcagcttgca 540
   aagcaaattc aatcccagtg gaaagaattt ggcctggatt ctgttgagct agcacattat 600
   gatgtcctgt tgtcctaccc aaataagact catcccaact acatctcaat aattaatgaa 660
   gatggaaatg agattttcaa cacatcatta tttgaaccac ctcctccagg atatgaaaat 720
   gtttcggata ttgtaccacc tttcagtgct ttctctcctc aaggaatgcc agagggcgat 780
   ctagtgtatg ttaactatgc acgaactgaa gacttcttta aattggaacg ggacatgaaa 840
   atcaattgct ctgggaaaat tgtaattgcc agatatggga aagttttcag aggaaataag 900
   gttaaaaatg cccagctggc aggggccaaa ggagtcattc tctactccga ccctgctgac 960
   tactttgctc ctggggtgaa gtcctatcca gatggttgga atcttcctgg aggtggtgtc 1020
   cagcgtggaa atatcctaaa tctgaatggt gcaggagacc ctctcacacc aggttaccca 1080
   gcaaatgaat atgcttatag gcgtggaatt gcagaggctg ttggtcttcc aagtattcct 1140
   gttcatccaa ttggatacta tgatgcacag aagctcctag aaaaaatggg tggctcagca 1200
   ccaccagata gcagctggag aggaagtctc aaagtgccct acaatgttgg acctggcttt 1260
   actggaaact tttctacaca aaaagtcaag atgcacatcc actctaccaa tgaagtgaca 1320
   agaatttaca atgtgatagg tactctcaga ggagcagtgg aaccagacag atatgtcatt 1380
   ctgggaggtc accgggactc atgggtgttt ggtggtattg accctcagag tggagcagct 1440
   gttgttcatg aaattgtgag gagctttgga acactgaaaa aggaagggtg gagacctaga 1500
   agaacaattt tgtttgcaag ctgggatgca gaagaatttg gtcttcttgg ttctactgag 1560
   tgggcagagg agaattcaag actccttcaa gagcgtggcg tggcttatat taatgctgac 1620
   tcatctatag aaggaaacta cactctgaga gttgattgta caccgctgat gtacagcttg 1680
   gtacacaacc taacaaaaga gctgaaaagc cctgatgaag gctttgaagg caaatctctt 1740
   tatgaaagtt ggactaaaaa aagtccttcc ccagagttca gtggcatgcc caggataagc 1800
   aaattgggat ctggaaatga ttttgaggtg ttcttccaac gacttggaat tgcttcaggc 1860
   agagcacggt atactaaaaa ttgggaaaca aacaaattca gcggctatcc actgtatcac 1920
   agtgtctatg aaacatatga gttggtggaa aagttttatg atccaatgtt taaatatcac 1980
   ctcactgtgg cccaggttcg aggagggatg gtgtttgagc tagccaattc catagtgctc 2040
   ccttttgatt gtcgagatta tgctgtagtt ttaagaaagt atgctgacaa aatctacagt 2100
   atttctatga aacatccaca ggaaatgaag acatacagtg tatcatttga ttcacttttt 2160
   tctgcagtaa agaattttac agaaattgct tccaagttca gtgagagact ccaggacttt 2220
   gacaaaagca acccaatagt attaagaatg atgaatgatc aactcatgtt tctggaaaga 2280
   gcatttattg atccattagg gttaccagac aggccttttt ataggcatgt catctatgct 2340
   ccaagcagcc acaacaagta tgcaggggag tcattcccag gaatttatga tgctctgttt 2400
   gatattgaaa gcaaagtgga cccttccaag gcctggggag aagtgaagag acagatttat 2460
   gttgcagcct tcacagtgca ggcagctgca gagactttga gtgaagtagc ctaagaggat 2520
   tctttagaga atccgtattg aatttgtgtg gtatgtcact cagaaagaat cgtaatgggt 2580
   atattgataa attttaaaat tggtatattt gaaataaagt tgaatattat atataaaaaa 2640
   aaaaaaaaaa aaa 2653
<210> 40
   <211> 188
   <212> PRT
   <213> Homo Sapien
<400> 40
   Met Asn Gly Asp Asp Ala Phe Ala Arg Arg Pro Thr Val Gly Ala Gln
   1 5 10 15
   Ile Pro Glu Lys Ile Gln Lys Ala Phe Asp Asp Ile Ala Lys Tyr Phe
   20 25 30
   Ser Lys Glu Glu Trp Glu Lys Met Lys Ala Ser Glu Lys Ile Phe Tyr
   35 40 45
   Val Tyr Met Lys Arg Lys Tyr Glu Ala Met Thr Lys Leu Gly Phe Lys
   50 55 60
   Ala Thr Leu Pro Pro Phe Met Cys Asn Lys Arg Ala Glu Asp Phe Gln
   65 70 75 80
   Gly Asn Asp Leu Asp Asn Asp Pro Asn Arg Gly Asn Gln Val Glu Arg
   85 90 95
   Pro Gln Met Thr Phe Gly Arg Leu Gln Gly Ile Ser Pro Lys Ile Met
   100 105 110
   Pro Lys Lys Pro Ala Glu Glu Gly Asn Asp Ser Glu Glu Val Pro Glu
           115                 120                 125
   Ala Ser Gly Pro Gln Asn Asp Gly Lys Glu Leu Cys Pro Pro Gly Lys
       130                 135                 140
   Pro Thr Thr Ser Glu Lys Ile His Glu Arg Ser Gly Pro Lys Arg Gly
   145                 150                 155                 160
   Glu His Ala Trp Thr His Arg Leu Arg Glu Arg Lys Gln Leu Val Ile
                   165                 170                 175
   Tyr Glu Glu Ile Ser Asp Pro Glu Glu Asp Asp Glu
               180                 185
<210> 41
   <211> 766
   <212> DNA
   <213> Homo Sapien
<400> 41
   ctctctttcg attcttccat actcagagta cgcacggtct gattttctct ttggattctt 60
   ccaaaatcag agtcagactg ctcccggtgc catgaacgga gacgacgcct ttgcaaggag 120
   acccacggtt ggtgctcaaa taccagagaa gatccaaaag gccttcgatg atattgccaa 180
   atacttctct aaggaagagt gggaaaagat gaaagcctcg gagaaaatct tctatgtgta 240
   tatgaagaga aagtatgagg ctatgactaa actaggtttc aaggccaccc tcccaccttt 300
   catgtgtaat aaacgggccg aagacttcca ggggaatgat ttggataatg accctaaccg 360
   tgggaatcag gttgaacgtc ctcagatgac tttcggcagg ctccagggaa tctccccgaa 420
   gatcatgccc aagaagccag cagaggaagg aaatgattcg gaggaagtgc cagaagcatc 480
   tggcccacaa aatgatggga aagagctgtg ccccccggga aaaccaacta cctctgagaa 540
   gattcacgag agatctggac ccaaaagggg ggaacatgcc tggacccaca gactgcgtga 600
   gagaaaacag ctggtgattt atgaagagat cagcgaccct gaggaagatg acgagtaact 660
   cccctcaggg atacgacaca tgcccatgat gagaagcaga acgtggtgac ctttcacgaa 720
   catgggcatg gctgcggacc cctcgtcatc aggtgcatag caagtg 766
<210> 42
   <211> 903
   <212> DNA
   <213> Herpes Simplex Virus
<400> 42
   atgacctctc gccgctccgt gaagtcgggt ccgcgggagg ttccgcgcga tgagtacgag 60
   gatctgtact acaccccgtc ttcaggtatg gcgagtcccg atagtccgcc tgacacctcc 120
   cgccgtggcg ccctacagac acgctcgcgc cagaggggcg aggtccgttt cgtccagtac 180
   gacgagtcgg attatgccct ctacgggggc tcgtcatccg aagacgacga acacccggag 240
   gtcccccgga cgcggcgtcc cgtttccggg gcggttttgt ccggcccggg gcctgcgcgg 300
   gcgcctccgc cacccgctgg gtccggaggg gccggacgca cacccaccac cgccccccgg 360
   gccccccgaa cccagcgggt ggcgactaag gcccccgcgg ccccggcggc ggagaccacc 420
   cgcggcagga aatcggccca gccagaatcc gccgcactcc cagacgcccc cgcgtcgacg 480
   gcgccaaccc gatccaagac acccgcgcag gggctggcca gaaagctgca ctttagcacc 540
   gcccccccaa accccgacgc gccatggacc ccccgggtgg ccggctttaa caagcgcgtc 600
   ttctgcgccg cggtcgggcg cctggcggcc atgcatgccc ggatggcggc ggtccagctc 660
   tgggacatgt cgcgtccgcg cacagacgaa gacctcaacg aactccttgg catcaccacc 720
   atccgcgtga cggtctgcga gggcaaaaac ctgcttcagc gcgccaacga gttggtgaat 780
   ccagacgtgg tgcaggacgt cgacgcggcc acggcgactc gagggcgttc tgcggcgtcg 840
   cgccccaccg agcgacctcg agccccagcc cgctccgctt ctcgccccag acggcccgtc 900
   gag 903
<210> 43
   <211> 311
   <212> PRT
   <213> Herpes Simplex Virus
<400> 43
   Met Thr Ser Arg Arg Ser Val Lys Ser Gly Pro Arg Glu Val Pro Arg
   1               5                   10                  15
   Asp Glu Tyr Glu Asp Leu Tyr Tyr Thr Pro Ser Ser Gly Met Ala Ser
               20                  25                  30
   Pro Asp Ser Pro Pro Asp Thr Ser Arg Arg Gly Ala Leu Phe Thr Gln
           35                  40                  45
   Thr Arg Ser Arg Gln Arg Gly Glu Val Arg Phe Val Gln Tyr Asp Glu
       50                  55                  60
   Ser Asp Tyr Ala Leu Tyr Gly Gly Ser Ser Ser Glu Asp Asp Glu His
   65                  70                  75                  80
   Pro Glu Val Pro Arg Thr Arg Arg Pro Val Ser Gly Ala Val Leu Ser
                   85                  90                  95
   Gly Pro Gly Pro Ala Arg Ala Pro Pro Pro Phe Thr Pro Ala Gly Ser
               100                 105                 110
   Gly Gly Ala Gly Arg Thr Pro Thr Thr Ala Pro Arg Ala Pro Arg Thr
           115                 120                 125
   Gln Arg Val Ala Thr Lys Ala Pro Ala Ala Pro Ala Ala Glu Thr Thr
       130                 135                 140
   Arg Gly Arg Lys Ser Ala Gln Pro Glu Ser Ala Ala Leu Pro Asp Ala
   145                 150                 155                 160
   Pro Ala Ser Thr Ala Pro Thr Phe Thr Arg Ser Lys Thr Pro Ala Gln
                   165                 170                 175
   Gly Leu Ala Arg Lys Leu His Phe Ser Thr Ala Pro Pro Asn Pro Asp
               180                 185                 190
   Ala Pro Trp Thr Pro Arg Val Ala Gly Phe Asn Lys Arg Val Phe Cys
           195                 200                 205
   Ala Ala Val Gly Arg Leu Ala Ala Met His Ala Arg Met Ala Ala Val
       210                 215                 220
   Gln Leu Trp Asp Phe Thr Met Ser Arg Pro Arg Thr Asp Glu Asp Leu
   225                 230                 235                 240
   Asn Glu Leu Leu Gly Ile Thr Thr Ile Arg Val Thr Val Cys Glu Gly
                   245                 250                 255
   Lys Asn Leu Leu Gln Arg Ala Asn Glu Leu Val Asn Pro Asp Val Val
               260                 265                 270
   Gln Asp Val Asp Ala Ala Thr Ala Thr Arg Gly Arg Ser Ala Ala Ser
           275                 280                 285
   Arg Phe Thr Pro Thr Glu Arg Pro Arg Ala Pro Ala Arg Ser Ala Ser
       290                 295                 300
   Arg Pro Arg Arg Pro Val Glu
   305                 310
<210> 44
   <211> 9
   <212> PRT
   <213> Adenovirus 3
<400> 44
   Leu Ile Val Ile Gly Ile Leu Ile Leu
   1               5
<210> 45
   <211> 10
   <212> PRT
   <213> Adenovirus 5
<400> 45
   Ser Gly Pro Ser Asn Thr Pro Pro Glu Ile
   1               5                   10
<210> 46
   <211> 8
   <212> PRT
   <213> Adenovirus 5
<400> 46
   Val Asn Ile Arg Asn Cys Cys Tyr
   1 5
<210> 47
   <211> 10
   <212> PRT
   <213> Adenovirus 5
<400> 47
   Ser Gly Pro Ser Asn Ile Pro Pro Glu Ile
   1 5 10
<210> 48
   <211> 9
   <212> PRT
   <213> classical Swine Fever Virus
<400> 4R
<210> 49
   <211> 9
   <212> PRT
   <213> Dengue virus 4
<400> 49
   Thr Pro Glu Gly Ile Ile Pro Thr Leu
   1 5
<210> 50
   <211> 9
   <212> PRT
   <213> Epstein-Barr Virus
<400> 50
   Cys Leu Gly Gly Leu Leu Thr Met Val
   1 5
<210> 51
   <211> 9
   <212> PRT
   <213> Epstein-Barr Virus
<400> 51
   Asn Ile Ala Glu Gly Leu Arg Ala Leu
   1 5
<210> 52
   <211> 9
   <212> PRT
   <213> Epstein-Barr Virus
<400> 52
   Asn Leu Arg Arg Gly Thr Ala Leu Ala
   1 5
<210> 53
   <211> 9
   <212> PRT
   <213> Epstein-Barr Virus
<400> 53
   Ala Leu Ala Ile Pro Gln Cys Arg Leu
   1 5
<210> 54
   <211> 9
   <212> PRT
   <213> Epstein-Barr Virus
<400> 54
   Val Leu Lys Asp Ala Ile Lys Asp Leu
   1 5
<210> 55
   <211> 9
   <212> PRT
   <213> Epstein-Barr Virus
<400> 55
   Phe Met Val Phe Leu Gln Thr His Ile
   1 5
<210> 56
   <211> 9
   <212> PRT
   <213> Epstein-Barr Virus
<400> 56
   His Leu Ile Val Asp Thr Asp Ser Leu
   1 5
<210> 57
   <211> 9
   <212> PRT
   <213> Epstein-Barr Virus
<400> 57
   Ser Leu Gly Asn Pro Ser Leu Ser Val
   1 5
<210> 58
   <211> 9
   <212> PRT
   <213> Epstein-Barr Virus
<400> 58
   Pro Leu Ala Ser Ala Met Arg Met Leu
   1 5
<210> 59
   <211> 8
   <212> PRT
   <213> Epstein-Barr virus
<400> 59
   Arg Met Leu Trp Met Ala Asn Tyr
   1 5
<210> 60
   <211> 9
   <212> PRT
   <213> Epstein-Barr virus
<400> 60
   Met Leu Trp Met Ala Asn Tyr Ile Val
   1 5
<210> 61
   <211> 9
   <212> PRT
   <213> Epstein-Barr Virus
<400> 61
   Ile Leu Pro Gln Gly Pro Gln Thr Ala
   1 5
<210> 62
   <211> 9
   <212> PRT
   <213> Epstein-Barr Virus
<400> 62
   Pro Leu Arg Pro Thr Ala Pro Thr Ile
   1 5
<210> 63
   <211> 9
   <212> PRT
   <213> Epstein-Barr Virus
<400> 63
   Pro Leu Pro Pro Ala Thr Leu Thr Val
   1 5
<210> 64
   <211> 9
   <212> PRT
   <213> Epstein-Barr virus
<400> 64
   Arg Met His Leu Pro Val Leu His Val
   1 5
<210> 65
   <211> 9
   <212> PRT
   <213> Epstein-Barr Virus

<400> 65
   Pro Met Pro Leu Pro Pro Ser Gln Leu
   1 5
<210> 66
   <211> 9
   <212> PRT
   <213> Epstein-Barr Virus
<400> 66
   Gln Leu Pro Pro Pro Ala Ala Pro Ala
   1 5
<210> 67
   <211> 9
   <212> PRT
   <213> Epstein-Barr virus
<400> 67
   Ser Met Pro Glu Leu Ser Pro Val Leu
   1 5
<210> 68
   <211> 9
   <212> PRT
   <213> Epstein-Barr Virus
<400> 68
   Asp Leu Asp Glu Ser Trp Asp Tyr Ile
   1 5
<210> 69
   <211> 9
   <212> PRT
   <213> Epstein-Barr Virus
<400> 69
   Pro Leu Pro Cys Val Leu Trp Pro Val
   1 5
<210> 70
   <211> 9
   <212> PRT
   <213> Epstein-Barr Virus
<400> 70
   Ser Leu Glu Glu Cys Asp Ser Glu Leu
   1 5
<210> 71
   <211> 9
   <212> PRT
   <213> Epstein-Barr Virus
<400> 71
   Glu Ile Lys Arg Tyr Lys Asn Arg Val
   1 5
<210> 72
   <211> 9
   <212> PRT
   <213> Epstein-Barr virus
<400> 72
   Gln Leu Leu Gln His Tyr Arg Glu Val
   1 5
<210> 73
   <211> 9
   <212> PRT
   <213> Epstein-Barr Virus
<400> 73
   Leu Leu Gln His Tyr Arg Glu Val Ala
   1 5
<210> 74
   <211> 9
   <212> PRT
   <213> Epstein-Barr Virus
<400> 74
   Leu Leu Lys Gln Met Cys Pro Ser Leu
   1 5
<210> 75
   <211> 9
   <212> PRT
   <213> Epstein-Barr virus
<400> 75
   Ser Ile Ile Pro Arg Thr Pro Asp Val
   1 5
<210> 76
   <211> 10
   <212> PRT
   <213> Epstein-Barr Virus
<400> 76
   Leu Leu Asp Phe Val Arg Phe Met Gly Val
   1 5 10
<210> 77
   <211> 9
   <212> PRT
   <213> Epstein-Barr Virus
<400> 77
   Ser Val Arg Asp Arg Leu Ala Arg Leu
   1 5
<210> 78
   <211> 9
   <212> PRT
   <213> Epstein-Barr Virus
<400> 78
   Ile Val Thr Asp Phe Ser Val Ile Lys
   1 5
<210> 79
   <211> 10
   <212> PRT
   <213> Epstein-Barr virus
<400> 79
   Ala Val Phe Asp Arg Lys Ser Asp Ala Lys
   1 5 10
<210> 80
   <211> 8
   <212> PRT
   <213> Epstein-Barr Virus
<400> 80
   Arg Tyr Ser Ile Phe Phe Asp Tyr
   1 5
<210> 81
   <211> 9
   <212> PRT
   <213> Epstein-Barr virus
<400> 81
   Gln Pro Arg Ala Pro Ile Arg Pro Ile
   1 5
<210> 82
   <211> 9
   <212> PRT
   <213> Epstein-Barr virus
<400> 82
   Arg Pro Pro Ile Phe Ile Arg Arg Ile
   1 5
<210> 83
   <211> 9
   <212> PRT
   <213> Epstein-Barr Virus
<400> 83
   Glu Pro Asp Val Pro Pro Gly Ala Ile
   1 5
<210> 84
   <211> 9
   <212> PRT
   <213> Epstein-Barr Virus
<400> 84
   Ile Pro Gln Cys Arg Leu Thr Pro Leu
   1 5
<210> 85
   <211> 9
   <212> PRT
   <213> Epstein-sarr Virus
<400> 85
   Gly Pro Gly Pro Gln Pro Gly Pro Leu
   1 5
<210> 86
   <211> 9
   <212> PRT
   <213> Epstein-Barr Virus
<400> 86
   Gln Pro Gly Pro Leu Arg Glu Ser Ile
   1 5
<210> 87
   <211> 9
   <212> PRT
   <213> Epstein-Barr virus
<400> 87
   Arg Pro Gln Lys Arg Pro Ser Cys Ile
   1 5
<210> 88
   <211> 9
   <212> PRT
   <213> Epstein-Barr Virus
<400> 88
   Pro Pro Thr Pro Leu Leu Thr Val Leu
   1 5
<210> 89
   <211> 9
   <212> PRT
   <213> Epstein-Barr Virus
<400> 89
   Thr Pro Ser Pro Pro Arg Met His Leu
   1 5
<210> 90
   <211> 9
   <212> PRT
   <213> Epstein-Barr Virus
<400> 90
   Pro Pro Arg Met His Leu Pro Val Leu
   1 5
<210> 91
   <211> 9
   <212> PRT
   <213> Epstein-Barr Virus
<400> 91
   Val Pro Asp Gln Ser Met His Pro Leu
   1 5
<210> 92
   <211> 9
   <212> PRT
   <213> Epstein-Barr Virus
<400> 92
   Pro Pro Ser Ile Asp Pro Ala Asp Leu
   1 5
<210> 93
   <211> 9
   <212> PRT
   <213> Epstein-Barr Virus
<400> 93
   Leu Pro Cys Val Leu Trp Pro Val Leu
   1 5
<210> 94
   <211> 10
   <212> PRT
   <213> Epstein-Barr Virus
<400> 94
   Cys Pro Ser Leu Asp Val Asp Ser Ile Ile
   1 5 10
<210> 95
   <211> 9
   <212> PRT
   <213> Epstein-Barr Virus
<400> 95
   Thr Pro Asp Val Leu His Glu Asp Leu
   1 5
<210> 96
   <211> 9
   <212> PRT
   <213> Epstein-Barr Virus
<400> 96
   Phe Leu Arg Gly Arg Ala Tyr Gly Leu
   1 5
<210> 97
   <211> 9
   <212> PRT
   <213> Epstein-Barr virus
<400> 97
   Gln Ala Lys Trp Arg Leu Gln Thr Leu
   1 5
<210> 98
   <211> 9
   <212> PRT
   <213> Epstein-Barr Virus
<400> 98
   Ala Tyr Pro Leu His Glu Gln His Gly
   1 5
<210> 99
   <211> 9
   <212> PRT
   <213> Epstein-Barr virus
<400> 99
   Tyr Leu Lys Ser Phe Val Ser Asp Ala
   1 5
<210> 100
   <211> 9
   <212> PRT
   <213> Epstein-Barr virus
<400> 100
   Arg Arg Arg Trp Arg Arg Leu Thr Val
   1 5
<210> 101
   <211> 9
   <212> PRT
   <213> Epstein-Barr Virus
<400> 101
   Arg Arg Ile Tyr Asp Leu Ile Glu Leu
   1 5
<210> 102
   <211> 9
   <212> PRT
   <213> Epstein-Barr Virus
<400> 102
   Tyr Pro Leu His Glu Gln His Gly Met
   1 5
<210> 103
   <211> 9
   <212> PRT
   <213> Epstein-Barr Virus
<400> 103
   Tyr Pro Leu His Glu Gln His Gly Met
   1 5
<210> 104
   <211> 9
   <212> PRT
   <213> Hepatitis C Virus
<400> 104
   His Ser Lys Lys Lys Cys Asp Glu Leu
   1 5
<210> 105
   <211> 8
   <212> PRT
   <213> Hepatitis C virus
<400> 105
   Ala Ser Arg Cys Trp Val Ala Met
   1 5
<210> 106
   <211> 9
   <212> PRT
   <213> Hepatitis c Virus
<400> 106
   Gly Gln Ile Val Gly Gly Val Tyr Leu
   1 5
<210> 107
   <211> 10
   <212> PRT
   <213> Hepatitis C Virus
<400> 107
   Pro Pro Leu Thr Asp Phe Asp Gln Gly Trp
   1 5 10
<210> 108
   <211> 10
   <212> PRT
   <213> Hepatitis C Virus
<400> 108
   Leu Met Gly Tyr Ile Pro Leu Val Gly Ala
   1 5 10
<210> 109
   <211> 10
   <212> PRT
   <213> Hepatitis C Virus
<400> 109
   Ala Asp Leu Met Gly Tyr Ile Pro Leu Val
   1 5 10
<210> 110
   <211> 16
   <212> PRT
   <213> Hepatitis C Virus
<400> 110
   Met Ser Tyr Ser Trp Thr Gly Ala Leu Val Thr Pro Cys Ala Glu Glu
   1 5 10 15
<210> 111
   <211> 9
   <212> PRT
   <213> Hepatitis C Virus
<400> 111
   Lys His Pro Asp Ala Thr Tyr Ser Arg
   1 5
<210> 112
   <211> 10
   <212> PRT
   <213> Hepatitis C Virus 1
<400> 112
   Lys Leu Val Ala Leu Gly Ile Asn Ala Val
   1 5 10
<210> 113
   <211> 9
   <212> PRT
   <213> Hepatitis c Virus 1
<400> 113
   Gly Asp Phe Asp Ser Val Ile Asp Cys
   1 5
<210> 114
   <211> 9
   <212> PRT
   <213> Hepatitis c virus 1
<400> 114
   Gly Asn Ala Ser Arg Cys Trp Val Ala
   1 5
<210> 115
   <211> 9
   <212> PRT
   <213> Hepatitis C Virus 1
<400> 115
   Thr Arg Pro Pro Leu Gly Asn Trp Phe
   1 5
<210> 116
   <211> 9
   <212> PRT
   <213> Hepatitis c Virus 1
<400> 116
   Val Pro His Pro Asn Ile Glu Glu Val
   1 5
<210> 117
   <211> 9
   <212> PRT
   <213> Hepatitis C virus 1
<400> 117
   Tyr Thr Gly Asp Phe Asp Ser Val Ile
   1 5
<210> 118
   <211> 8
   <212> PRT
   <213> Hepatitis C Virus 1
<400> 118
   Ser Trp Ala Ile Lys Trp Glu Tyr
   1 5
<210> 119
   <211> 9
   <212> PRT
   <213> Hepatitis C Virus 1
<400> 119
   Lys His Pro Asp Ala Thr Tyr Ser Arg
   1 5
<210> 120
   <211> 9
   <212> PRT
   <213> Hepatitis c virus 1
<400> 120
   Gly Asp Phe Asp Ser Val Ile Asp Cys
   1 5
<210> 121
   <211> 9
   <212> PRT
   <213> Human immunodeficiency Virus
<400> 121
   Arg Tyr Leu Lys Asp Gln Gln Leu Leu
   1 5
<210> 122
   <211> 9
   <212> PRT
   <213> Human Immunodeficiency Virus
<400> 122
   Ile Val Gly Leu Asn Lys Ile Val Arg
   1 5
<210> 123
   <211> 9
   <212> PRT
   <213> Human Immunodeficiency Virus
<400> 123
   Glu Ile Tyr Lys Arg Trp Ile Ile Leu
   1 5
<210> 124
   <211> 8
   <212> PRT
   <213> Human Immunodeficiency virus
<400> 124
   Gly Glu Leu Tyr Lys Arg Trp Ile
   1 5
<210> 125
   <211> 9
   <212> PRT
   <213> Human Immunodeficiency Virus
<400> 125
   Glu Ile Lys Asp Thr Lys Glu Ala Leu
   1 5
<210> 126
   <211> 8
   <212> PRT
   <213> Human Immunodeficiency Virus
<400> 126
   Tyr Leu Lys Asp Gln Gln Leu Leu
   1 5
<210> 127
   <211> 11
   <212> PRT
   <213> Human Immunodeficiency virus
<400> 127
   Ile Leu Gly Leu Asn Lys Ile Val Arg Met Tyr
   1 5 10
<210> 128
   <211> 9
   <212> PRT
   <213> Human immunodeficiency virus
<400> 128
   Glu Arg Tyr Leu Lys Asp Gln Gln Leu
   1 5
<210> 129
   <211> 11
   <212> PRT
   <213> Human Immunodeficiency virus
<400> 129
   Tyr His Thr Gln Gly Tyr Phe Pro Gln Trp Gln
   1 5 10
<210> 130
   <211> 12
   <212> PRT
   <213> Human Immunodeficiency virus

<400> 130
   Thr Gln Gly Tyr Phe Pro Gln Trp Gln Asn Tyr Thr
   1 5 10
<210> 131
   <211> 9
   <212> PRT
   <213> Human immunodeficiency virus
<400> 131
   Gly Arg Ala Phe Val Thr Leu Gly Lys
   1 5
<210> 132
   <211> 9
   <212> PRT
   <213> Human Immunodeficiency virus
<400> 132
   Lys Arg Trp Ile Ile Leu Gly Leu Asn
   1 5
<210> 133
   <211> 10
   <212> PRT
   <213> Human Immunodeficiency Virus
<400> 133
   Gln Val Pro Leu Arg Pro Met Thr Tyr Lys
   1 5 10
<210> 134
   <211> 9
   <212> PRT
   <213> Human Immunodeficiency virus
<400> 134
   Thr Gln Gly Tyr Phe Pro Gln Trp Gln
   1 5
<210> 135
   <211> 9
   <212> PRT
   <213> Human Immunodeficiency virus
<400> 135
   His Gln Ala Ile Ser Pro Arg Thr Ile
   1 5
<210> 136
   <211> 12
   <212> PRT
   <213> Human Immunodeficiency virus
<400> 136
   Gln Met Val His Gln Ala Ile Ser Pro Arg Thr Leu
   1 5 10
<210> 137
   <211> 10
   <212> PRT
   <213> Human Immunodeficiency Virus
<400> 137
   Met Tyr Ala Pro Pro Ile Gly Gly Gln Ile
   1 5 10
<210> 138
   <211> 10
   <212> PRT
   <213> Human Immunodeficiency virus
<400> 138
   Arg Gly Pro Gly Arg Ala Phe Val Thr Ile
   1 5 10
<210> 139
   <211> 9
   <212> PRT
   <213> Human Immunocieficiency virus
<400> 139
   Met Pro Gly Arg Ala Phe Val Thr Ile
   1 5
<210> 140
   <211> 9
   <212> PRT
   <213> Human Immunodeficiency Virus 1
<400> 140
   Ile Leu Lys Glu Pro Val His Gly Val
   1 5
<210> 141
   <211> 9
   <212> PRT
   <213> Human Immunodeficiency Virus 1
<400> 141
   Ala Phe His His Val Ala Arg Glu Leu
   1 5
<210> 142
   <211> 9
   <212> PRT
   <213> Human Immunodeficiency virus 1
<400> 142
   Lys Leu Thr Pro Leu Cys Val Thr Leu
   1 5
<210> 143
   <211> 10
   <212> PRT
   <213> Human Immunodeficiency virus 1
<400> 143
   Ser Leu Leu Asn Ala Thr Asp Ile Ala Val
   1 5 10
<210> 144
   <211> 9
   <212> PRT
   <213> Human Immunodeficiency virus 1
<400> 144
   Val Ile Tyr Gln Tyr Met Asp Asp Leu
   1 5
<210> 145
   <211> 9
   <212> PRT
   <213> Human Immunodeficiency virus 1
<400> 145
   Ser Leu Tyr Asn Thr Val Ala Thr Leu
   1 5
<210> 146
   <211> 9
   <212> PRT
   <213> Human Immunodeficiency Virus 1
<400> 146
   Arg Gly Pro Gly Arg Ala Phe Val Thr
   1 5
<210> 147
   <211> 11
   <212> PRT
   <213> Human immunodeficiency Virus 1
<400> 147
   Arg Leu Arg Asp Leu Leu Leu Ile Val Thr Arg
   1 5 10
<210> 148
   <211> 10
   <212> PRT
   <213> Human Immunodeficiency Vi rus 1
<400> 148
   Gln Val Pro Leu Arg Pro Met Thr Tyr Lys
   1 5 10
<210> 149
   <211> 10
   <212> PRT
   <213> Human Immunodeficiency virus 1
<400> 149
   Thr Val Tyr Tyr Gly Val Pro Val Trp Lys
   1 5 10
<210> 150
   <211> 9
   <212> PRT
   <213> Human Immunodeficiency virus 1
<400> 150
   Arg Leu Arg Pro Gly Gly Lys Lys Lys
   1 5
<210> 151
   <211> 9
   <212> PRT
   <213> Human Immunodeficiency virus 1
<400> 151
   Val Tyr Tyr Gly Val Pro Val Trp Lys
   1 5
<210> 152
   <211> 9
   <212> PRT
   <213> Human Immunodeficiency virus 1
<400> 152
   Val Pro Leu Arg Pro Met Thr Tyr Lys
   1 5
<210> 153
   <211> 9
   <212> PRT
   <213> Human Immunodeficiency virus 1
<400> 153
   Ala Ile Phe Gln Ser Ser Met Thr Lys
   1 5
<210> 154
   <211> 10
   <212> PRT
   <213> Human Immunodeficiency virus 1
<400> 154
   Gln Val Pro Leu Arg Pro Met Thr Tyr Lys
   1 5 10
<210> 155
   <211> 12
   <212> PRT
   <213> Human Immunodeficiency virus 1
<400> 155
   Ala Ala Val Asp Leu Ser His Phe Leu Lys Glu Lys
   1               5                   10
<210> 156
   <211> 12
   <212> PRT
   <213> Human Immunodeficiency Virus 1
<400> 156
   Ala Cys Gln Gly Val Gly Gly Pro Gly Gly His Lys
   1               5                   10
<210> 157
   <211> 10
   <212> PRT
   <213> Human Immunodeficiency Virus 1
<400> 157
   Glu Thr Ile Asn Glu Glu Ala Ala Glu Trp
   1               5                   10
<210> 158
   <211> 10
   <212> PRT
   <213> Human Immunodeficiency virus 1
<400> 158
   Thr Pro Gly Pro Gly Val Arg Tyr Pro Leu
   1               5                   10
<210> 159
   <211> 8
   <212> PRT
   <213> Human Immunodeficiency virus 1
<400> 159
   Gly Gly Lys Lys Lys Tyr Lys Leu
   1               5
<210> 160
   <211> 9
   <212> PRT
   <213> Human Immunodeficiency virus 1
<400> 160
   Arg Val Lys Glu Lys Tyr Gln His Leu
   1               5
<210> 161
   <211> 9
   <212> PRT
   <213> Human Immunodeficiency virus 1
<400> 161
   Asp Arg Phe Tyr Lys Thr Leu Arg Ala
   1               5
<210> 162
   <211> 16
   <212> PRT
   <213> Human Immunodeficiency virus 1
<400> 162
   Gly Val Arg Tyr Pro Leu Thr Phe Gly Trp Cys Tyr Lys Leu Val Pro
   1               5                   10                  15
<210> 163
   <211> 10
   <212> PRT
   <213> Human Immunodeficiency virus 1
<400> 163
   Lys Arg Trp Ile Ile Leu Gly Leu Asn Lys
   1               5                   10
<210> 164
   <211> 9
   <212> PRT
   <213> Human Immunodeficiency Virus 1
<400> 164
   Ala Phe His His Val Ala Arg Glu Leu
   1               5
<210> 165
   <211> 9
   <212> PRT
   <213> Epstein-Barr virus
<400> 165
   Lys Glu His Val Ile Gln Asn Ala Phe
   1               5
<210> 166
   <211> 10
   <212> PRT
   <213> Epstein-Barr Virus
<400> 166
   Glu Glu Asn Leu Leu Asp Phe Val Arg Phe
   1 5 10
<210> 167
   <211> 10
   <212> PRT
   <213> Epstein-Barr Virus
<400> 167
   Asp Thr Pro Leu Ile Pro Leu Thr Ile Phe
   1 5 10
<210> 168
   <211> 10
   <212> PRT
   <213> Epstein-Barr Virus
<400> 168
   Gln Asn Gly Ala Leu Ala Ile Asn Thr Phe
   1 5 10
<210> 169
   <211> 9
   <212> PRT
   <213> Epstein-Barr Virus
<400> 169
   Arg Leu Arg Ala Glu Ala Gly Val Lys
   1 5
<210> 170
   <211> 10
   <212> PRT
   <213> Hepatitis B Virus
<400> 170
   Gly Leu Ser Pro Thr Val Trp Leu Ser Val
   1 5 10
<210> 171
   <211> 9
   <212> PRT
   <213> Hepatitis B virus
<400> 171
   Trp Leu Ser Leu Leu Val Pro Phe Val
   1 5
<210> 172
   <211> 10
   <212> PRT
   <213> Hepatitis B virus
<400> 172
   Phe Leu Pro Ser Asp Phe Phe Pro Ser Val
   1 5 10
<210> 173
   <211> 10
   <212> PRT
   <213> Hepatitis B Virus
<400> 173
   Phe Leu Pro Ser Asp Phe Phe Pro Ser Val
   1 5 10
<210> 174
   <211> 10
   <212> PRT
   <213> Hepatitis B Virus
<400> 174
   Phe Leu Pro Ser Asp Phe Phe Pro Ser Val
   1 5 10
<210> 175
   <211> 10
   <212> PRT
   <213> Hepatitis B Virus
<400> 175
   Phe Leu Pro Ser Asp Phe Phe Pro Ser Val
   1 5 10
<210> 176
   <211> 9
   <212> PRT
   <213> Hepatitis B Virus
<400> 176
   Phe Leu Leu Ser Leu Gly Ile His Leu
   1 5
<210> 177
   <211> 9
   <212> PRT
   <213> Hepatitis B virus
<400> 177
   Ser Leu Tyr Ala Asp Ser Pro Ser Val
   1 5
<210> 178
   <211> 9
   <212> PRT
   <213> Hepatitis B virus
<400> 178
   Gly Leu Ser Arg Tyr Val Ala Arg Leu
   1 5
<210> 179
   <211> 10
   <212> PRT
   <213> Hepatitis B virus
<400> 179
   Leu Leu Val Pro Phe Val Gln Trp Phe Val
   1 5 10
<210> 180
   <211> 9
   <212> PRT
   <213> Hepatitis B Virus
<400> 180
   Ala Leu Met Pro Leu Tyr Ala Cys Ile
   1 5
<210> 181
   <211> 10
   <212> PRT
   <213> Hepatitis B Virus
<400> 181
   Leu Leu Pro Ile Phe Phe Cys Leu Trp Val
   1 5 10
<210> 182
   <211> 9
   <212> PRT
   <213> Hepatitis B Virus
<400> 182
   Tyr Met Asp Asp Val Val Leu Gly Ala
   1 5
<210> 183
   <211> 9
   <212> PRT
   <213> Hepatitis B virus
<400> 183
   Leu Leu Leu Cys Leu Ile Phe Leu Leu
   1 5
<210> 184
   <211> 10
   <212> PRT
   <213> Hepatitis B virus
<400> 184
   Leu Leu Asp Tyr Gln Gly Met Leu Pro Val
   1 5 10
<210> 185
   <211> 10
   <212> PRT
   <213> Hepatitis B Virus
<400> 185
   Ser Ile Val Ser Pro Phe Ile Pro Leu Leu
   1 5 10
<210> 186
   <211> 9
   <212> PRT
   <213> Hepatitis B Virus
<400> 186
   Phe Leu Leu Thr Arg Ile Leu Thr Ile
   1 5
<210> 187
   <211> 9
   <212> PRT
   <213> Hepatitis B Virus
<400> 187
   Tyr Val Asn Val Asn Met Gly Leu Lys
   1 5
<210> 188
   <211> 11
   <212> PRT
   <213> Hepatitis B virus
<400> 188
   Ser Thr Leu Pro Glu Thr Thr Val Val Arg Arg
   1 5 10
<210> 189
   <211> 11
   <212> PRT
   <213> Hepatitis B Virus
<400> 189
   Ser Thr Leu Pro Glu Thr Thr Val Val Arg Arg
   1 5 10
<210> 190
   <211> 10
   <212> PRT
   <213> Hepatitis B Virus
<400> 190
   Phe Leu Pro Ser Asp Phe Phe Pro Ser Val
   1 5 10
<210> 191
   <211> 12
   <212> PRT
   <213> Hepatitis B virus

<400> 191
   Ile Pro Gln Ser Leu Asp Ser Trp Trp Thr Ser Leu
   1 5 10
<210> 192
   <211> 8
   <212> PRT
   <213> Hepatitis Virus
<400> 192
   Met Gly Leu Lys Phe Arg Gln Leu
   1 5
<210> 193
   <211> 9
   <212> PRT
   <213> Hepatitis B Virus
<220>
   <221> VARIANT
   <222> 4, 8
   <223> xaa = Any Amino Acid
<400> 193
   Ser Thr Asx Xaa Gln Ser Gly Xaa Gln
   1 5
<210> 194
   <211> 11
   <212> PRT
   <213> Human Cytomegalovirus
<400> 194
   Phe Ile Ala Gly Asn Ser Ala Tyr Glu Tyr Val
   1 5 10
<210> 195
   <211> 12
   <212> PRT
   <213> Human Cytomegalovirus
<400> 195
   Ser Asp Glu Glu Phe Ala Ile Val Ala Tyr Thr Leu
   1 5 10
<210> 196
   <211> 15
   <212> PRT
   <213> Human Cytomegalovirus
<400> 196
   Asp Asp Val Trp Thr Ser Gly Ser Asp Ser Asp Glu Glu Leu Val
   1 5 10 15
<210> 197
   <211> 9
   <212> PRT
   <213> Human Cytomegalovirus
<400> 197
   Ile Pro Ser Ile Asn Val His His Tyr
   1 5
<210> 198
   <211> 9
   <212> PRT
   <213> Human Cytomegalovirus
<400> 198
   Asn Leu Val Pro Met Val Ala Thr Val
   1 5
<210> 199
   <211> 9
   <212> PRT
   <213> Human cytomegalovirus
<400> 199
   Asp Leu Met Gly Tyr Ile Pro Leu Val
   1 5
<210> 200
   <211> 9
   <212> PRT
   <213> Hepatitis C virus
<400> 200
   Asp Leu Met Gly Tyr Ile Pro Leu Val
   1 5
<210> 201
   <211> 10
   <212> PRT
   <213> Hepatitis C Virus
<400> 201
   Leu Leu Ala Leu Leu Ser Cys Leu Thr Val
   1 5 10
<210> 202
   <211> 8
   <212> PRT
   <213> Hepatitis C Virus
<400> 202
   Ile Leu His Thr Pro Gly Cys Val
   1 5
<210> 203
   <211> 10
   <212> PRT
   <213> Hepatitis C virus
<400> 203
   Gln Leu Arg Arg His Ile Asp Leu Leu Val
   1 5 10
<210> 204
   <211> 9
   <212> PRT
   <213> Hepatitis C Virus
<400> 204
   Asp Leu Cys Gly Ser Val Phe Leu Val
   1 5
<210> 205
   <211> 9
   <212> PRT
   <213> Hepatitis C Virus
<400> 205
   Ser Met Val Gly Asn Trp Ala Lys Val
   1 5
<210> 206
   <211> 10
   <212> PRT
   <213> Hepatitis C Virus
<400> 206
   His Leu Ile Ile Gln Asn Ile Val Asp Val
   1 5 10
<210> 207
   <211> 9
   <212> PRT
   <213> Hepatitis C Virus
<400> 207
   Phe Leu Leu Leu Ala Asp Ala Arg Val
   1 5
<210> 208
   <211> 13
   <212> PRT
   <213> Hepatitis C Virus
<400> 208
   Gly Leu Arg Asp Leu Ala Val Ala Val Glu Pro Val Val
   1 5 10
<210> 209
   <211> 11
   <212> PRT
   <213> Hepatitis C Virus
<400> 209
   Ser Leu Leu Ala Pro Gly Ala Lys Gln Asn Val
   1 5 10
<210> 210
   <211> 10
   <212> PRT
   <213> Hepatitis C Virus
<400> 210
   Leu Leu Ala Pro Gly Ala Lys Gln Asn Val
   1 5 10
<210> 211
   <211> 10
   <212> PRT
   <213> Hepatitis C Virus
<400> 211
   Leu Leu Phe Asn Ile Leu Gly Gly Trp Val
   1 5 10
<210> 212
   <211> 9
   <212> PRT
   <213> Hepatitis C virus
<400> 212
   Tyr Leu Val Ala Tyr Gln Ala Thr Val
   1 5
<210> 213
   <211> 10
   <212> PRT
   <213> Hepatitis C Virus
<400> 213
   Tyr Leu Leu Pro Arg Arg Gly Pro Arg Leu
   1 5 10
<210> 214
   <211> 10
   <212> PRT
   <213> Hepatitis C virus
<400> 214
   Leu Leu Ala Leu Leu Ser Cys Leu Thr Ile
   1 5 10
<210> 215
   <211> 9
   <212> PRT
   <213> Hepatitis C Virus
<400> 215
   Ser Leu Met Ala Phe Thr Ala Ala Val
   1 5
<210> 216
   <211> 9
   <212> PRT
   <213> Hepatitis C virus
<400> 216
   Cys Ile Asn Gly Val Cys Trp Thr Val
   1 5
<210> 217
   <211> 9
   <212> PRT
   <213> Hepatitis C virus
<400> 217
   Leu Leu Cys Pro Ala Gly His Ala Val
   1 5
<210> 218
   <211> 9
   <212> PRT
   <213> Hepatitis C Virus
<400> 218
   Ile Leu Asp Ser Phe Asp Pro Leu Val
   1 5
<210> 219
   <211> 9
   <212> PRT
   <213> Hepatitis C Virus
<400> 219
   Ile Leu Ala Gly Tyr Gly Ala Gly Val
   1 5
<210> 220
   <211> 9
   <212> PRT
   <213> Hepatitis C Virus
<400> 220
   Gly Leu Gln Asp Cys Thr Met Leu Val
   1 5
<210> 221
   <211> 10
   <212> PRT
   <213> Hepatitis C Virus
<400> 221
   Thr Gly Ala Pro Val Thr Tyr Ser Thr Tyr
   1 5 10
<210> 222
   <211> 9
   <212> PRT
   <213> Hepatitis C Virus
<400> 222
   His Met Trp Asn Phe Ile Ser Gly Ile
   1 5
<210> 223
   <211> 9
   <212> PRT
   <213> Hepatitis C Virus
<400> 223
   Arg Val Cys Glu Lys Met Ala Leu Tyr
   1 5
<210> 224
   <211> 8
   <212> PRT
   <213> Hepatitis C Virus
<400> 224
   Thr Ile Asn Tyr Thr Ile Phe Lys
   1 5
<210> 225
   <211> 8
   <212> PRT
   <213> Hepatitis C virus
<400> 225
   Tyr Ile Ser Trp Cys Leu Trp Trp
   1 5
<210> 226
   <211> 9
   <212> PRT
   <213> Hepatitis C Virus
<400> 226
   Gly Pro Arg Leu Gly Val Arg Ala Thr
   1 5
<210> 227
   <211> 9
   <212> PRT
   <213> Human immunodeficiency Virus 1
<400> 227
   Ser Phe Asn Cys Gly Gly Glu Phe Phe
   1 5
<210> 228
   <211> 9
   <212> PRT
   <213> Human Immunodeficiency Virus 1
<400> 228
   Thr Glu Met Glu Lys Glu Gly Lys Ile
   1 5
<210> 229
   <211> 9
   <212> PRT
   <213> Human Immunodeficiency Virus 1
<400> 229
   Lys Ile Arg Leu Arg Pro Gly Gly Lys
   1 5
<210> 230
   <211> 10
   <212> PRT
   <213> Human immunodeficiency Virus 1
<400> 230
   Arg Leu Arg Pro Gly Gly Lys Lys Lys Tyr
   1 5 10
<210> 231
   <211> 9
   <212> PRT
   <213> Human Immunodeficiency Virus 1
<400> 231
   Ala Ile Phe Gln Ser Ser Met Thr Lys
   1 5
<210> 232
   <211> 9
   <212> PRT
   <213> Human Immunodeficiency virus 1
<400> 232
   Thr Leu Tyr Cys Val His Gln Arg Ile
   1 5
<210> 233
   <211> 10
   <212> PRT
   <213> Human Immunodeficiency virus 1
<400> 233
   Ile Tyr Gln Glu Pro Phe Lys Asn Leu Lys
   1 5 10
<210> 234
   <211> 9
   <212> PRT
   <213> Human Immunodeficiency Virus 1
<400> 234
   Lys Tyr Lys Leu Lys His Ile Val Trp
   1 5
<210> 235
   <211> 10
   <212> PRT
   <213> Human Immunodeficiency Virus 1
<400> 235
   Leu Phe Cys Ala Ser Asp Ala Lys Ala Tyr
   1 5 10
<210> 236
   <211> 11
   <212> PRT
   <213> Human Immunodeficiency Virus 1
<400> 236
   Gln Ala Ile Ser Pro Arg Thr Leu Asn Ala Trp
   1 5 10
<210> 237
   <211> 9
   <212> PRT
   <213> Human Immunodeficiency Virus 1
<400> 237
   Glu Val Ile Pro Met Phe Ser Ala Leu
   1 5
<210> 238
   <211> 11
   <212> PRT
   <213> Human Immunodeficiency Virus 1
<400> 238
   Glu Thr Phe Tyr Val Asp Gly Ala Ala Asn Arg
   1 5 10
<210> 239
   <211> 11
   <212> PRT
   <213> Human Immunodeficiency Virus 1
<400> 239
   Arg Leu Arg Asp Leu Leu Leu Ile Val Thr Arg
   1 5 10
<210> 240
   <211> 10
   <212> PRT
   <213> Human Immunodeficiency Virus 1
<400> 240
   Pro Ile Gln Lys Glu Thr Trp Glu Thr Trp
   1 5 10
<210> 241
   <211> 9
   <212> PRT
   <213> Human Immunodeficiency Virus 1
<400> 241
   Arg Ile Lys Gln Ile Ile Asn Met Trp
   1 5
<210> 242
   <211> 9
   <212> PRT
   <213> Human Immunodeficiency Virus 1
<400> 242
   Ile Thr Leu Trp Gln Arg Pro Leu Val
   1 5
<210> 243
   <211> 9
   <212> PRT
   <213> Human Immunodeficiency Virus 1
<400> 243
   Asp Thr Val Leu Glu Glu Met Asn Leu
   1 5
<210> 244
   <211> 9
   <212> PRT
   <213> Human Immunodeficiency Virus 1
<400> 244
   Ile Thr Leu Trp Gln Arg Pro Leu Val
   1 5
<210> 245
   <211> 9
   <212> PRT
   <213> Human Immunodeficiency virus 1
<400> 245
   Ser Pro Arg Thr Leu Asn Ala Trp Val
   1 5
<210> 246
   <211> 9
   <212> PRT
   <213> Human Immunodeficiency Virus 1
<400> 246
   Ala Thr Pro Gln Asp Leu Asn Thr Met
   1 5
<210> 247
   <211> 10
   <212> PRT
   <213> Human Immunodeficiency Virus 1
<400> 247
   Arg Pro Asn Asn Asn Thr Arg Lys Ser Ile
   1 5 10
<210> 248
   <211> 9
   <212> PRT
   <213> Human Immunodeficiency Virus 1
<400> 248
   Ile Pro Arg Arg Ile Arg Gln Gly Leu
   1 5
<210> 249
   <211> 9
   <212> PRT
   <213> Human Immunodeficiency Virus 1
<400> 249
   Glu Leu Arg Ser Leu Tyr Asn Thr Val
   1 5
<210> 250
   <211> 8
   <212> PRT
   <213> Human Immunodeficiency Virus 1
<400> 250
   Trp Pro Thr Val Arg Glu Arg Met
   1 5
<210> 251
   <211> 8
   <212> PRT
   <213> Human Immunodeficiency Virus 1
<400> 251
   Phe Leu Lys Glu Lys Gly Gly Leu
   1 5
<210> 252
   <211> 9
   <212> PRT
   <213> Human Immunodeficiency Virus 1
<400> 252
   Asp Leu Asn Thr Met Leu Asn Thr Val
   1 5
<210> 253
   <211> 10
   <212> PRT
   <213> Human Immunodeficiency virus 1
<400> 253
   Lys Ile Arg Leu Arg Pro Gly Gly Lys Lys
   1 5 10
<210> 254
   <211> 9
   <212> PRT
   <213> Human Immunodeficiency Virus 1
<400> 254
   Ile Arg Leu Arg Pro Gly Gly Lys Lys
   1 5
<210> 255
   <211> 10
   <212> PRT
   <213> Human Immunodeficiency Virus 1
<400> 255
   Gly Arg Arg Gly Trp Glu Ala Leu Lys Tyr
   1 5 10
<210> 256
   <211> 10
   <212> PRT
   <213> Human Immunodeficiency Virus 1
<400> 256
   Gln Val Pro Leu Arg Pro Met Thr Tyr Lys
   1 5 10
<210> 257
   <211> 8
   <212> PRT
   <213> Human Immunodeficiency Virus 1
<400> 257
   Arg Tyr Leu Lys Asp Gln Gln Leu
   1 5
<210> 258
   <211> 10
   <212> PRT
   <213> Human Immunodeficiency virus 1
<400> 258
   Arg Arg Gln Asp Ile Leu Asp Leu Trp Ile
   1 5 10
<210> 259
   <211> 8
   <212> PRT
   <213> Human Immunodeficiency Virus 1
<400> 259
   Arg Tyr Pro Leu Thr Phe Gly Trp
   1 5
<210> 260
   <211> 9
   <212> PRT
   <213> Human Immunodeficiency Virus 1
<400> 260
   Trp Ala Ser Arg Glu Leu Glu Arg Phe
   1 5
<210> 261
   <211> 9
   <212> PRT
   <213> Human Immunodeficiency Virus 1
<400> 261
   Thr Val Leu Asp Val Gly Asp Ala Tyr
   1 5
<210> 262
   <211> 11
   <212> PRT
   <213> Human Immunodeficiency Virus 1
<400> 262
   Val Pro Val Trp Lys Glu Ala Thr Thr Thr Leu
   1 5 10
<210> 263
   <211> 9
   <212> PRT
   <213> Human Immunodeficiency virus 1
<400> 263
   Asn Ser Ser Lys Val Ser Gln Asn Tyr
   1 5
<210> 264
   <211> 9
   <212> PRT
   <213> Human Immunodeficiency virus 1
<400> 264
   Pro Pro Ile Pro Val Gly Asp Ile Tyr
   1               5
<210> 265
   <211> 9
   <212> PRT
   <213> Human Immunodeficiency Virus 1
<400> 265
   His Pro Asp Ile Val Ile Tyr Gln Tyr
   1               5
<210> 266
   <211> 9
   <212> PRT
   <213> Human Immunodeficiency Virus 1
<400> 266
   Thr Ala Val Pro Trp Asn Ala Ser Trp
   1               5
<210> 267
   <211> 9
   <212> PRT
   <213> Human Immunodeficiency Virus 1

<400> 267
   Asn Pro Val Pro Val Gly Asn Leu Tyr
   1               5
<210> 268
   <211> 9
   <212> PRT
   <213> Human Immunodeficiency Virus 1
<400> 268
   Tyr Phe Pro Asp Trp Gln Asn Tyr Thr
   1               5
<210> 269
   <211> 9
   <212> PRT
   <213> Human Immunodeficiency Virus 1
<400> 269
   Gly His Gln Ala Ala Met Gln Met Leu
   1               5
<210> 270
   <211> 10
   <212> PRT
   <213> Human Immunodeficiency Virus 1
<400> 270
   Arg Leu Arg Pro Gly Gly Lys Lys Lys Tyr
   1 5 10
<210> 271
   <211> 9
   <212> PRT
   <213> Human Immunodeficiency Virus 1
<400> 271
   Tyr Pro Gly Ile Lys Val Arg Gln Leu
   1 5
<210> 272
   <211> 10
   <212> PRT
   <213> Human Immunodeficiency Virus 1
<400> 272
   Gly Ala Glu Thr Phe Tyr Val Asp Gly Ala
   1 5 10
<210> 273
   <211> 9
   <212> PRT
   <213> Human Immunodeficiency Virus 1
<400> 273
   Asn Ala Asn Pro Asp Cys Lys Thr Ile
   1 5
<210> 274
   <211> 8
   <212> PRT
   <213> Human Immunodeficiency Virus 1
<400> 274
   Arg Met Tyr Ser Pro Thr Ser Ile
   1 5
<210> 275
   <211> 10
   <212> PRT
   <213> Human Immunodeficiency Virus 1
<400> 275
   Val Pro Val Trp Lys Glu Ala Thr Thr Thr
   1 5 10
<210> 276
   <211> 9
   <212> PRT
   <213> Human Immunodeficiency Virus 1
<400> 276
   Ile Ser Pro Arg Thr Leu Asn Ala Trp
   1 5
<210> 277
   <211> 10
   <212> PRT
   <213> Human Immunodeficiency Virus 1
<400> 277
   Thr Ser Thr Leu Gln Glu Gln Ile Gly Trp
   1 5 10
<210> 278
   <211> 11
   <212> PRT
   <213> Human Immunodeficiency virus 1
<400> 278
   Lys Ala Phe Ser Pro Glu Val Ile Pro Met Phe
   1 5 10
<210> 279
   <211> 9
   <212> PRT
   <213> Human Immunodeficiency virus 1
<400> 279
   Gln Ala Ser Gln Glu Val Lys Asn Trp
   1 5
<210> 280
   <211> 9
   <212> PRT
   <213> Human Immunodeficiency virus 1
<400> 280
   Gln Ala Ser Gln Asp Val Lys Asn Trp
   1 5
<210> 281
   <211> 10
   <212> PRT
   <213> Human Immunodeficiency Virus 1
<400> 281
   His Thr Gln Gly Tyr Phe Pro Asp Trp Gln
   1 5 10
<210> 282
   <211> 9
   <212> PRT
   <213> Human Immunodeficiency Virus 1
<400> 282
   Tyr Phe Pro Asp Trp Gln Asn Tyr Thr
   1 5
<210> 283
   <211> 10
   <212> PRT
   <213> Human Immunodeficiency Virus 1
<400> 283
   Thr Ser Thr Leu Gln Glu Gln Ile Gly Trp
   1 5 10
<210> 284
   <211> 10
   <212> PRT
   <213> Human Immunodeficiency Virus 1
<400> 284
   Arg Leu Arg Pro Gly Gly Lys Lys Lys Tyr
   1 5 10
<210> 285
   <211> 9
   <212> PRT
   <213> Human Immunodeficiency Virus 1
<400> 285
   Leu Gly Leu Asn Lys Val Arg Met Tyr
   1 5
<210> 286
   <211> 12
   <212> PRT
   <213> Human Inmmunodeficiency Virus 1
<400> 286
   Leu Val Gly Lys Leu Asn Trp Ala Ser Gln Ile Tyr
   1 5 10
<210> 287
   <211> 10
   <212> PRT
   <213> Human Immunodeficiency Virus 1
<400> 287
   Ile Leu Lys Glu Pro Val His Gly Val Tyr
   1 5 10
<210> 288
   <211> 11
   <212> PRT
   <213> Human Immunodeficiency Virus 1
<400> 288
   Thr Gln Gly Tyr Phe Pro Asp Trp Gln Asn Tyr
   1 5 10
<210> 289
   <211> 8
   <212> PRT
   <213> Human Immunonodeficiency Virus 1
<400> 289
   Ala Val Asp Leu Ser His Phe Leu
   1 5
<210> 290
   <211> 8
   <212> PRT
   <213> Human Immunodeficiency Virus 1
<400> 290
   Val Ile Pro Met Phe Ser Ala Leu
   1 5
<210> 291
   <211> 9
   <212> PRT
   <213> Human Immunodeficiency Virus 1
<400> 291
   Phe Asn Cys Gly Gly Glu Phe Phe Tyr
   1 5
<210> 292
   <211> 9
   <212> PRT
   <213> Human Immunodeficiency Virus 1
<400> 292
   Ser Phe Asn Cys Gly Gly Glu Phe Phe
   1 5
<210> 293
   <211> 10
   <212> PRT
   <213> Human Immunodeficiency virus 1
<400> 293
   Pro Leu Thr Phe Gly Trp Cys Tyr Lys Leu
   1 5 10
<210> 294
   <211> 10
   <212> PRT
   <213> Human Immunodeficiency Virus 1
<400> 294
   Val Leu Glu Trp Arg Phe Asp Ser Arg Leu
   1 5 10
<210> 295
   <211> 10
   <212> PRT
   <213> Human Immunodeficiency Virus 1
<400> 295
   Phe Pro Val Thr Pro Gln Val Pro Leu Arg
   1 5 10
<210> 296
   <211> 10
   <212> PRT
   <213> Human Immunodeficiency Virus 1
<400> 296
   Thr Pro Gly Pro Gly Val Arg Tyr Pro Leu
   1 5 10
<210> 297
   <211> 9
   <212> PRT
   <213> Human Immunodeficiency Virus 1
<400> 297
   Gln Ala Ser Gln Glu Val Lys Asn Trp
   1 5
<210> 298
   <211> 10
   <212> PRT
   <213> Human Immunodeficiency Virus 1 IIIB
<400> 298
   Val Pro Leu Asp Glu Asp Phe Arg Lys Tyr
   1 5 10
<210> 299
   <211> 9
   <212> PRT
   <213> Human Immunodeficiency virus 1 IIIB
<400> 299
   Asn Pro Asp Ile Val Ile Tyr Gln Tyr
   1               5
<210> 300
   <211> 9
   <212> PRT
   <213> Human Immunodeficiency Virus 1 IIIB
<400> 300
   Arg Ala Ile Glu Ala Gln Ala His Leu
   1               5
<210> 301
   <211> 8
   <212> PRT
   <213> Human Immunodeficiency Virus 1 IIIB
<400> 301
   Thr Ala Phe Thr Ile Pro Ser Ile
   1               5
<210> 302
   <211> 10
   <212> PRT
   <213> Human Immunodeficiency Virus 1 IIIB
<400> 302
   Val His Pro Val His Ala Gly Pro Ile Ala
   1               5                   10
<210> 303
   <211> 10
   <212> PRT
   <213> Human Immunodeficiency Virus 1 IIIB
<400> 303
   Asn Cys Ser Phe Asn Ile Ser Thr Ser Ile
   1               5                   10
<210> 304
   <211> 9
   <212> PRT
   <213> Human Immunodeficiency virus 1 IIIB
<400> 304
   Cys Thr Asn Val Ser Thr Val Gln Cys
   1               5
<210> 305
   <211> 9
   <212> PRT
   <213> Human Immunodeficiency Virus 1 5F2
<400> 305
   Ile Gly Pro Gly Arg Ala Phe His Thr
   1 5
<210> 306
   <211> 9
   <212> PRT
   <213> Human Immunodeficiency Virus 1 5F2
<400> 306
   Asn Pro Asp Ile Val Ile Tyr Gln Tyr
   1 5
<210> 307
   <211> 10
   <212> PRT
   <213> Human Immunodeficiency Virus 1 5F2
<400> 307
   Glu Pro Ile Val Gly Ala Glu Thr Phe Tyr
   1 5 10
<210> 308
   <211> 9
   <212> PRT
   <213> Human Immunodeficiency Virus 1 5F2
<400> 308
   Glu Pro Ile Val Gly Ala Glu Thr Phe
   1 5
<210> 309
   <211> 9
   <212> PRT
   <213> Human Immunodeficiency Virus 1 5F2
<400> 309
   Ser Pro Ala Ile Phe Gln Ser Ser Met
   1 5
<210> 310
   <211> 10
   <212> PRT
   <213> Human Immunodeficiency Virus 1 5F2
<400> 310
   Val Pro Leu Asp Lys Asp Phe Arg Lys Tyr
   1 5 10
<210> 311
   <211> 9
   <212> PRT
   <213> Human Immunodeficiency Virus 1 5F2
<400> 311
   Ile Pro Leu Thr Glu Glu Ala Glu Leu
   1 5
<210> 312
   <211> 11
   <212> PRT
   <213> Human Immunodeficiency Virus 1 5F2
<400> 312
   Arg Pro Gln Val Pro Leu Arg Pro Met Thr Tyr
   1 5 10
<210> 313
   <211> 9
   <212> PRT
   <213> Human Immunodeficiency Virus 1 5F2
<400> 313
   Phe Pro Val Arg Pro Gln Val Pro Leu
   1 5
<210> 314
   <211> 9
   <212> PRT
   <213> Human Immunodeficiency Virus 1 5F2
<400> 314
   Asp Pro Asn Pro Gln Glu Val Val Leu
   1 5
<210> 315
   <211> 9
   <212> PRT
   <213> Human Immunodeficiency Virus 1 5F2
<400> 315
   Arg Pro Ile Val Ser Thr Gln Leu Leu
   1 5
<210> 316
   <211> 9
   <212> PRT
   <213> Human Immunodeficiency Virus 1 5F2
<400> 316
   Ile Pro Leu Thr Glu Glu Ala Glu Leu
   1 5
<210> 317
   <211> 9
   <212> PRT
   <213> Human Immunodeficiency Virus 1 5F2
<400> 317
   Asp Pro Asn Pro Gln Glu Val Val Leu
   1 5
<210> 318
   <211> 9
   <212> PRT
   <213> Human Immunodeficiency Virus 1 5F2
<400> 318
   Ala Met Gln Met Leu Lys Glu Thr Ile
   1 5
<210> 319
   <211> 9
   <212> PRT
   <213> Human Immunodeficiency Virus 2
<400> 319
   Thr Pro Tyr Asp Arg Asn Gln Met Leu
   1 5
<210> 320
   <211> 11
   <212> PRT
   <213> Human Immunodeficiency Virus 2
<400> 320
   Arg Arg Trp Ile Gln Leu Gly Leu Gln Lys Val
   1 5 10
<210> 321
   <211> 15
   <212> PRT
   <213> Human Immunodeficiency Virus 1 5F2
<400> 321
   Gly Ile Trp Gly Cys Ser Gly Lys Leu Ile Cys Thr Thr Ala Val
   1 5 10 15
<210> 322
   <211> 15
   <212> PRT
   <213> Human Immunodeficiency Virus 1 5F2
<400> 322
   Ala Leu Ile Trp Glu Asp Leu Arg Ser Leu Cys Leu Phe Ser Tyr
   1 5 10 15
<210> 323
   <211> 9
   <212> PRT
   <213> Human Papillomavirus 6b
<400> 323
   Gly Leu His Cys Tyr Glu Gln Leu Val
   1 5
<210> 324
   <211> 9
   <212> PRT
   <213> Human papillomavirus 6b
<400> 324
   Pro Leu Lys Gln His Phe Gln Ile Val
   1 5
<210> 325
   <211> 9
   <212> PRT
   <213> Human Papillomavirus 11
<400> 325
   Arg Leu Val Thr Leu Lys Asp Ile Val
   1 5
<210> 326
   <211> 9
   <212> PRT
   <213> Human Papillomavirus 16
<400> 326
   Thr Leu Gly Ile Val Cys Pro Ile Cys
   1 5
<210> 327
   <211> 9
   <212> PRT
   <213> Human papillomavirus 16
<400> 327
   Gly Thr Leu Gly Ile Val Cys Pro Ile
   1 5
<210> 328
   <211> 9
   <212> PRT
   <213> Human Papillomavirus 16
<400> 328
   Met Leu Asp Leu Gln Pro Glu Thr Thr
   1 5
<210> 329
   <211> 10
   <212> PRT
   <213> Human papillomavirus 16
<400> 329
   Tyr Met Leu Asp Leu Gln Pro Glu Thr Thr
   1 5 10
<210> 330
   <211> 8
   <212> PRT
   <213> Human Papillomavirus 16
<400> 330
   Arg Pro Arg Lys Leu Pro Gln Leu
   1 5
<210> 331
   <211> 9
   <212> PRT
   <213> Human papillomavirus 16
<400> 331
   Arg Ala His Tyr Asn Ile Val Thr Phe
   1 5
<210> 332
   <211> 8
   <212> PRT
   <213> Herpes simplex virus
<400> 332
   Ser Ser Ile Glu Phe Ala Arg Leu
   1 5
<210> 333
   <211> 9
   <212> PRT
   <213> Herpes Simplex Virus 1
<400> 333
   Gly Ile Gly Ile Gly Val Leu Ala Ala
   1 5
<210> 334
   <211> 9
   <212> PRT
   <213> Herpes simplex virus 1
<400> 334
   Asp Tyr Ala Thr Leu Gly Val Gly Val
   1 5
<210> 335
   <211> 11
   <212> PRT
   <213> Herpes simplex Virus 1
<400> 335
   Leu Tyr Arg Thr Phe Ala Gly Asn Pro Arg Ala
   1 5 10
<210> 336
   <211> 8
   <212> PRT
   <213> Herpes simplex Virus 1
<400> 336
   Gln Thr Phe Asp Phe Gly Arg Leu
   1 5
<210> 337
   <211> 9
   <212> PRT
   <213> Herpes simplex virus 2
<400> 337
   Gly Ala Gly Ile Gly Val Ala Val Leu
   1 5
<210> 338
   <211> 9
   <212> PRT
   <213> Human T-lymphotropic Virus 1
<400> 338
   Leu Leu Phe Gly Tyr Pro Val Tyr Val
   1 5
<210> 339
   <211> 9
   <212> PRT
   <213> Influenza
<400> 339
   Gly Ile Leu Gly Phe Val Phe Thr Leu
   1 5
<210> 340
   <211> 10
   <212> PRT
   <213> Influenza
<400> 340
   Ile Leu Gly Phe Val Phe Thr Leu Thr Val
   1 5 10
<210> 341
   <211> 9
   <212> PRT
   <213> Influenza
<400> 341
   Ile Leu Arg Gly Ser Val Ala His Lys
   1 5
<210> 342
   <211> 9
   <212> PRT
   <213> Influenza
<400> 342
   Lys Thr Gly Gly Pro Ile Tyr Lys Arg
   1 5
<210> 343
   <211> 9
   <212> PRT
   <213> Influenza
<400> 343
   Glu Leu Arg Ser Arg Tyr Trp Ala Ile
   1 5
<210> 344
   <211> 8
   <212> PRT
   <213> Influenza
<400> 344
   Leu Arg Ser Arg Tyr Trp Ala Ile
   1 5
<210> 345
   <211> 9
   <212> PRT
   <213> Influenza
<400> 345
   Glu Asp Leu Arg Val Leu Ser Phe Ile
   1 5
<210> 346
   <211> 9
   <212> PRT
   <213> Influenza
<400> 346
   Gly Glu Ile Ser Pro Leu Pro Ser Leu
   1 5
<210> 347
   <211> 9
   <212> PRT
   <213> Influenza
<400> 347
   Phe Glu Asp Leu Arg Val Leu Ser Phe
   1 5
<210> 348
   <211> 9
   <212> PRT
   <213> Influenza
<400> 348
   Gly Glu Ile Ser Pro Leu Pro Ser Leu
   1 5
<210> 349
   <211> 9
   <212> PRT
   <213> Influenza
<400> 349
   Phe Glu Asp Leu Arg Val Leu Ser Phe
   1 5
<210> 350
   <211> 9
   <212> PRT
   <213> Influenza
<400> 350
   Val Ser Asp Gly Gly Pro Lys Leu Tyr
   1 5
<210> 351
<271> 9
<212> PRT
   <213> Influenza A
<400> 351
<270> 352
<211> 9
   <212> PRT
   <213> Influenza
<400> 352
<210> 353
   <211> 10
   <212> PRT
   <213> Influenza A
<400> 353
   Ile Met Asp Lys Asn Ile Ile Leu Lys Ala
   1 5 10
<210> 354
   <211> 9
   <212> PRT
   <213> Influenza A
<400> 354
   Ser Arg Tyr Trp Ala Ile Arg Thr Arg
   1 5
<210> 355
   <211> 9
   <212> PRT
   <213> Influenza A
<400> 355
   Thr Tyr Gln Arg Thr Arg Ala Leu Val
   1 5
<210> 356
   <211> 10
   <212> PRT
   <213> Influenza A
<400> 356
   Thr Tyr Val Ser Val Ser Thr Ser Thr Leu
   1 5 10
<210> 357
   <271> 9
   <212> PRT
   <213> Influenza A
<400> 357
<210> 358
   <211> 8
   <212> PRT
   <213> Influenza A
<400> 358
   Phe Glu Ala Asn Gly Asn Leu Ile
   1 5
<210> 359
   <211> 9
   <212> PRT
   <213> Influenza A

<400> 359
   Ile Glu Gly Gly Trp Thr Gly Met Leu
   1 5
<210> 360
   <211> 8
   <212> PRT
   <213> Influenza A
<400> 360
   Ser Asp Tyr Glu Gly Arg Leu Ile
   1 5
<210> 361
   <211> 9
   <212> PRT
   <213> Influenza A
<400> 361
   Glu Glu Gly Ala Ile Val Gly Glu Ile
   1 5
<210> 362
   <211> 9
   <212> PRT
   <213> Influenza A34
<400> 362
   Ala Ser Asn Glu Asn Met Glu Thr Met
   1 5
<210> 363
   <211> 9
   <212> PRT
   <213> Influenza A68
<400> 363
   Ala Ser Asn Glu Asn Met Asp Ala Met
   1 5
<210> 364
   <211> 10
   <212> PRT
   <213> Influenza B
<400> 364
   Lys Leu Gly Glu Phe Tyr Asn Gln Met Met
   1 5 10
<210> 365
   <211> 10
   <212> PRT
   <213> Influenza B
<400> 365
   Lys Ala Gly Glu Phe Tyr Asn Gln Met Met
   1 5 10
<210> 366
   <211> 9
   <212> PRT
   <213> Influenza JAP
<400> 366
   Leu Tyr Gln Asn Val Gly Thr Tyr Val
   1 5
<210> 367
   <211> 10
   <212> PRT
   <213> Influenza JAP
<400> 367
   Thr Tyr Val Ser Val Gly Thr Ser Thr Leu
   1 5 10
<210> 368
   <211> 9
   <212> PRT
   <213> Influenza JAP
<400> 368
   Val Tyr Gln Ile Leu Ala Thr Tyr Ala
   1 5
<210> 369
   <211> 9
   <212> PRT
   <213> Influenza JAP
<400> 369
   Ile Tyr Ala Thr Val Ala Gly Ser Leu
   1 5
<210> 370
   <211> 10
   <212> PRT
   <213> Influenza JAP
<400> 370
   Thr Tyr Val Ser Val Gly Thr Ser Thr Ile
   1 5 10
<210> 371
   <211> 8
   <212> PRT
   <213> Influenza JAP
<400> 371
   Phe Glu Ser Thr Gly Asn Leu Ile
   1 5
<210> 372
   <211> 9
   <212> PRT
   <213> Mouse Hepatitis Virus Strain JHM
<400> 372
   Ala Pro Thr Ala Gly Ala Phe Phe Phe
   1 5
<210> 373
   <211> 9
   <212> PRT
   <213> Lymphocytic Choriomeningitis virus
<400> 373
   Arg Pro Gln Ala Ser Gly Val Tyr Met
   1 5
<210> 374
   <211> 9
   <212> PRT
   <213> Lymphocytic Choriomeningitis virus
<400> 374
   Phe Gln Pro Gln Asn Gly Gln Phe Ile
   1 5
<210> 375
   <211> 11
   <212> PRT
   <213> Lymphocytic choriomeningitis virus
<400> 375
   Ser Gly Val Glu Asn Pro Gly Gly Tyr Cys Leu
   1 5 10
<210> 376
   <211> 10
   <212> PRT
   <213> Lymphocytic Choriomeningitis virus
<400> 376
   Lys Ala Val Tyr Asn Phe Ala Thr Cys Gly
   1 5 10
<210> 377
   <211> 9
   <212> PRT
   <213> Murine cytomegalovirus
<400> 377
   Tyr Pro His Phe Met Pro Thr Asn Leu
   1 5
<210> 378
   <211> 9
   <212> PRT
   <213> Mouse Hepatitis virus
<400> 378
   Cys Leu Ser Trp Asn Gly Pro His Leu
   1 5
<210> 379
   <211> 9
   <212> PRT
   <213> Mouse Mammarytumor Virus
<400> 379
   Ser Phe Ala Val Ala Thr Thr Ala Leu
   1 5
<210> 380
   <211> 9
   <212> PRT
   <213> Mouse Mammarytumor virus
<400> 380
   Ser Tyr Glu Thr Phe Ile Ser Arg Leu
   1 5
<210> 381
   <211> 8
   <212> PRT
   <213> Mouse Mammarytumor virus
<400> 381
   Ala Asn Tyr Asp Phe Ile Cys Val
   1 5
<210> 382
   <211> 8
   <212> PRT
   <213> Murine Leukemia virus
<400> 382
   Lys Ser Pro Trp Phe Thr Thr Leu
   1 5
<210> 383
   <211> 8
   <212> PRT
   <213> Murine Leukemia virus
<400> 383
   Ser Ser Trp Asp Phe Ile Thr Val
   1 5
<210> 384
   <211> 9
   <212> PRT
   <213> Murine Leukemia Virus
<400> 384
   Cys Cys Leu Cys Leu Thr Val Phe Leu
   1 5
<210> 385
   <211> 9
   <212> PRT
   <213> Murine Leukemia Virus
<400> 385
   Ser Pro Ser Tyr Val Tyr His Gln Phe
   1 5
<210> 386
   <211> 11
   <212> PRT
   <213> Measles Virus
<400> 386
   Ser Arg Arg Tyr Pro Asp Ala Val Tyr Leu His
   1 5 10
<210> 387
   <211> 9
   <212> PRT
   <213> Measles Virus
<400> 387
   Arg Arg Tyr Pro Asp Ala Val Tyr Leu
   1 5
<210> 388
   <211> 9
   <212> PRT
   <213> Measles Virus
<400> 388
   Tyr Pro Ala Leu Gly Leu His Glu Phe
   1 5
<210> 389
   <211> 9
   <212> PRT
   <213> Measles Virus
<400> 389
   Asp Pro Val Ile Asp Arg Leu Tyr Leu
   1 5
<210> 390
   <211> 9
   <212> PRT
   <213> Measles Virus
<400> 390
   Ser Pro Gly Arg Ser Phe Ser Tyr Phe
   1 5
<210> 391
   <211> 8
   <212> PRT
   <213> Poliovirus
<400> 391
   Thr Tyr Lys Asp Thr Val Gln Leu
   1 5
<210> 392
   <211> 10
   <212> PRT
   <213> poliovirus
<400> 392
   Phe Tyr Asp Gly Phe Ser Lys Val Pro Leu
   1 5 10
<210> 393
   <211> 9
   <212> PRT
   <213> Pseudorabies virus gp
<400> 393
   Ile Ala Gly Ile Gly Ile Leu Ala Ile
   1 5
<210> 394
   <211> 9
   <212> PRT
   <213> Rabiesvirus
<400> 394
   Val Glu Ala Glu Ile Ala His Gln Ile
   1 5
<210> 395
   <211> 8
   <212> PRT
   <213> Rotavirus
<400> 395
   Leu Leu Tyr Arg Phe Leu Leu Leu
   1 5
<210> 396
   <211> 9
   <212> PRT
   <213> Rotavirus
<400> 396
   Val Gly Pro Val Phe Pro Pro Gly Met
   1 5
<210> 397
   <211> 9
   <212> PRT
   <213> Rotavirus
<400> 397
   Tyr Ser Gly Tyr Ile Phe Arg Asp Leu
   1               5
<210> 398
   <211> 9
   <212> PRT
   <213> Respiratory Syncytial virus
<400> 398
   Ser Tyr Ile Gly Ser Ile Asn Asn Ile
   1               5
<210> 399
   <211> 12
   <212> PRT
   <213> simian Immunodeficiency virus
<400> 399
   Glu Gly Cys Thr Pro Tyr Asp Thr Asn Gln Met Leu
   1               5                   10
<210> 400
   <211> 9
   <212> PRT
   <213> Sendai Virus
<400> 400
   Phe Ala Pro Gly Asn Tyr Pro Ala Leu
   1               5
<210> 401
   <211> 10
   <212> PRT
   <213> sendai virus
<400> 401
   Phe Ala Pro Cys Thr Asn Tyr Pro Ala Leu
   1               5                   10
<210> 402
   <211> 8
   <212> PRT
   <213> Sendai Virus 40
<400> 402
   Val Val Tyr Asp Phe Leu Lys Cys
   1               5
<210> 403
   <211> 10
   <212> PRT
   <213> Sendai Virus 40
<400> 403
   Ser Ala Ile Asn Asn Tyr Ala Gln Lys Leu
   1 5 10
<210> 404
   <211> 9
   <212> PRT
   <213> Sendai Virus 40
<400> 404
   Cys Lys Gly Val Asn Lys Glu Tyr Leu
   1 5
<210> 405
   <211> 9
   <212> PRT
   <213> Sendai Virus 40
<400> 405
   Gln Gly Ile Asn Asn Leu Asp Asn Leu
   1 5
<210> 406
   <211> 10
   <212> PRT
   <213> Sendai Virus 40
<400> 406
   Asn Asn Leu Asp Asn Leu Arg Asp Tyr Leu
   1 5 10
<210> 407
   <211> 9
   <212> PRT
   <213> Sendai virus 40
<400> 407
   Ser Glu Phe Leu Leu Glu Lys Arg Ile
   1 5
<210> 408
   <211> 8
   <212> PRT
   <213> Vesicular Stomatitis Virus
<400> 408
   Arg Gly Tyr Val Tyr Gln Gly Leu
   1 5
<210> 409
   <211> 9
   <212> PRT
   <213> Homo Sapiens
<400> 409
   Glu Ala Asp Pro Thr Gly His Ser Tyr
   1               5
<210> 410
   <211> 9
   <212> PRT
   <213> Influenza
<400> 410
   Val Ser Asp Gly Gly Pro Asn Leu Tyr
   1               5
<210> 411
   <211> 9
   <212> PRT
   <213> Influenza
<400> 411
   Cys Thr Glu Leu Lys Leu Ser Asp Tyr
   1               5
<210> 412
   <211> 9
   <212> PRT
   <213> Homo Sapiens
<400> 412
   Glu Val Asp Pro Ile Gly His Leu Tyr
   1               5
<210> 413
   <211> 10
   <212> PRT
   <213> Homo Sapien (calreticulin)
<400> 413
   Met Leu Leu Ser Val Pro Leu Leu Leu Gly
   1               5                   10
<210> 414
   <211> 9
   <212> PRT
   <213> Hepatitis B Virus
<220>
   <221> VARIANT
   <222> 4, 8
   <223> Xaa = Any Amino acid
<400> 414
   Ser Thr Asx Xaa Gln Ser Gly Xaa Gln
   1 5
<210> 415
   <211> 9
   <212> PRT
   <213> Homo Sapiens
<400> 415
   Tyr Met Asp Gly Thr Met Ser Gln Val
   1 5
<210> 416
   <211> 9
   <212> PRT
   <213> Human immunodeficiency Virus 1
<400> 416
   Ile Leu Lys Glu Pro Val His Gly Val
   1 5
<210> 417
   <211> 10
   <212> PRT
   <213> Influenza MP
<400> 417
   Leu Leu Gly Phe Val Phe Thr Leu Thr Val
   1 5 10
<210> 418
   <211> 10
   <212> PRT
   <213> Human T-lymphotropic Virus 1
<400> 418
   Leu Leu Phe Gly Tyr Pro Val Tyr Val Val
   1 5 10
<210> 419
   <211> 10
   <212> PRT
   <213> Hepatitis B Virus
<400> 419
   Gly Leu Ser Pro Thr Val Trp Leu Ser Val
   1 5 10
<210> 420
   <211> 9
   <212> PRT
   <213> Hepatitis B virus
<400> 420
   Trp Leu Ser Leu Leu Val Pro Phe Val
   1 5
<210> 421
   <211> 10
   <212> PRT
   <213> Hepatitis B virus
<400> 421
   Phe Leu Pro Ser Asp Phe Phe Pro Ser Val
   1 5 10
<210> 422
   <211> 8
   <212> PRT
   <213> Epstein-Barr Virus
<400> 422
   Cys Leu Gly Leu Leu Thr Met Val
   1 5
<210> 423
   <211> 11
   <212> PRT
   <213> Human cytomegalovirus
<400> 423
   Phe Leu Ala Gly Asn Ser Ala Tyr Glu Tyr Val
   1 5 10
<210> 424
   <211> 10
   <212> PRT
   <213> Influenza
<400> 424
   Lys Leu Gly Glu Phe Tyr Asn Gln Met Met
   1 5 10
<210> 425
   <211> 10
   <212> PRT
   <213> Hepatitis C Virus 1
<400> 425
   Lys Leu Val Ala Leu Gly Ile Asn Ala Val
   1 5 10
<210> 426
   <211> 9
   <212> PRT
   <213> Hepatitis C Virus
<400> 426
   Asp Leu Met Gly Tyr Ile Pro Leu Val
   1 5
<210> 427
   <211> 9
   <212> PRT
   <213> Human Papillomavirus
<400> 427
   Arg Leu Val Thr Leu Lys Asp Ile Val
   1 5
<210> 428
   <211> 9
   <212> PRT
   <213> Homo Sapien
<400> 428
   Met Leu Leu Ala Val Leu Tyr Cys Leu
   1 5
<210> 429
   <211> 9
   <212> PRT
   <213> Homo Sapien
<400> 429
   Ala Ala Gly Ile Gly Ile Leu Thr Val
   1 5
<210> 430
   <211> 9
   <212> PRT
   <213> Homo Sapien
<400> 430
   Tyr Leu Glu Pro Gly Pro Val Thr Ala
   1 5
<210> 431
   <211> 10
   <212> PRT
   <213> Homo Sapien
<400> 431
   Ile Leu Asp Gly Thr Ala Thr Leu Arg Leu
   1 5 10
<210> 432
   <211> 10
   <212> PRT
   <213> Homo Sapien
<400> 432
   Leu Leu Asp Gly Thr Ala Thr Leu Arg Leu
   1 5 10
<210> 433
   <211> 9
   <212> PRT
   <213> Homo Sapien
<400> 433
   Ile Thr Asp Gln Val Pro Phe Ser Val
   1 5
<210> 434
   <211> 9
   <212> PRT
   <213> Homo Sapien
<400> 434
   Lys Thr Trp Gly Gln Tyr Trp Gln Val
   1 5
<210> 435
   <211> 10
   <212> PRT
   <213> Homo Sapien
<400> 435
   Thr Ile Thr Asp Gln Val Pro Phe Ser Val
   1 5 10
<210> 436
   <211> 10
   <212> PRT
   <213> Human Immunodeviciency Virus 1
<400> 436
   Ala Phe His Ile Ile Val Ala Arg Glu Leu
   1 5 10
<210> 437
   <211> 9
   <212> PRT
   <213> Plasmodium Falciparum
<400> 437
   Tyr Leu Asn Lys Ile Gln Asn Ser Leu
   1 5
<210> 438
   <211> 10
   <212> PRT
   <213> Plasmodium Falciparum
<400> 438
   Met Met Arg Lys Leu Ala Ile Leu Ser Val
   1 5 10
<210> 439
   <211> 10
   <212> PRT
   <213> Influenza
<400> 439
   Lys Ala Gly Glu Phe Tyr Asn Gln Met Met
   1 5 10
<210> 440
   <211> 9
   <212> PRT
   <213> Epstein-Barr virus (EBNA)
<400> 440
   Asn Ile Ala Glu Gly Leu Arg Ala Leu
   1 5
<210> 441
   <211> 9
   <212> PRT
   <213> Epstein-Barr Virus (EBN_{A})
<400> 441
   Asn Leu Arg Arg Gly Thr Ala Leu Ala
   1 5
<210> 442
   <211> 9
   <212> PRT
   <213> Epstein-Barr virus (EBNA)
<400> 442
   Ala Leu Ala Ile Pro Gln Cys Arg Leu
   1 5
<210> 443
   <211> 9
   <212> PRT
   <213> Epstein-Barr Virus (EBNA)
<400> 443
   Val Leu Lys Asp Ala Ile Lys Asp Leu
   1 5
<210> 444
   <211> 9
   <212> PRT
   <213> Epstein-Barr Virus (EBNA)
<400> 444
   Phe Met Val Phe Leu Gln Thr His Ile
   1 5
<210> 445
   <211> 9
   <212> PRT
   <213> Epstein-Barr Virus (EBNA)
<400> 445
   His Leu Ile Val Asp Thr Asp Ser Leu
   1 5
<210> 446
   <211> 9
   <212> PRT
   <213> Epstein-Barr Virus (EBNA)
<400> 446
   Ser Leu Gly Asn Pro Ser Leu Ser Val
   1 5
<210> 447
   <211> 9
   <212> PRT
   <213> Epstein-Barr Virus (EBNA)

<400> 447
   Pro Leu Ala Ser Ala Met Arg Met Leu
   1 5
<210> 448
   <211> 9
   <212> PRT
   <213> Epstein-Barr Virus (EBNA)
<400> 448
   Arg Met Leu Trp Met Ala Asn Tyr Ile
   1               5
<210> 449
   <211> 9
   <212> PRT
   <213> Epstein-Barr Virus (EBNA)
<400> 449
   Met Leu Trp Met Ala Asn Tyr Ile Val
   1               5
<210> 450
   <211> 9
   <212> PRT
   <213> Epstein-Barr Virus (EBNA)
<400> 450
   Ile Leu Pro Gln Gly Pro Gln Thr Ala
   1               5
<210> 451
   <211> 10
   <212> PRT
   <213> Epstein-Barr Virus (EBNA)
<400> 451
   Pro Leu Arg Pro Thr Ala Pro Thr Thr Ile
   1               5                   10
<210> 452
   <211> 9
   <212> PRT
   <213> Epstein-Barr Virus (EBNA)
<400> 452
   Pro Leu Pro Pro Ala Thr Leu Thr Val
   1               5
<210> 453
   <211> 9
   <212> PRT
   <213> Epstein-Barr Virus (EBNA)
<400> 453
   Arg Met His Leu Pro Val Leu His Val
   1               5
<210> 454
   <211> 9
   <212> PRT
   <213> Epstein-Barr Virus (EBNA)
<400> 454
   Pro Met Pro Leu Pro Pro Ser Gln Leu
   1 5
<210> 455
   <211> 9
   <212> PRT
   <213> Epstein-Barr Virus (EBNA)
<400> 455
   Gln Leu Pro Pro Pro Ala Ala Pro Ala
   1 5
<210> 456
   <211> 9
   <212> PRT
   <213> Epstein-Barr Virus (EBNA)
<400> 456
   Ser Met Pro Glu Leu Ser Pro Val Leu
   1 5
<210> 457
   <211> 9
   <212> PRT
   <213> Epstein-Barr Virus (EBNA)
<400> 457
   Asp Leu Asp Glu Ser Trp Asp Tyr Leu
   1 5
<210> 458
   <211> 10
   <212> PRT
   <213> Epstein-Barr Virus (EBNA)
<400> 458
   Pro Leu Pro Cys Val Leu Trp Pro Val Val
   1 5 10
<210> 459
   <211> 9
   <212> PRT
   <213> Epstain-Barr Virus (EBNA)
<400> 459
   Ser Leu Glu Glu Cys Asp Ser Glu Leu
   1 5
<210> 460
   <211> 9
   <212> PRT
   <213> Epstein-Barr Virus (EBNA)
<400> 460
   Glu Ile Lys Arg Tyr Lys Asn Arg Val
   1 5
<210> 461
   <211> 10
   <212> PRT
   <213> Epstein-Barr Virus (EBNA)
<400> 461
   Gln Leu Leu Gln Phe Ile Tyr Arg Glu Val
   1 5 10
<210> 462
   <211> 9
   <212> PRT
   <213> Epstein-Barr Virus (EBNA)
<400> 462
   Leu Leu Gln His Tyr Arg Glu Val Ala
   1 5
<210> 463
   <211> 9
   <212> PRT
   <213> Epstein-Barr Virus (EBNA)
<400> 463
   Leu Leu Lys Gln Met Cys Pro Ser Leu
   1 5
<210> 464
   <211> 9
   <212> PRT
   <213> Epstein-Barr Virus (EBNA)
<400> 464
   Ser Ile Ile Pro Arg Thr Pro Asp Val
   1 5
<210> 465
   <211> 9
   <212> PRT
   <213> Influenza A
<400> 465
   Ala Ile Met Asp Lys Asn Ile Ile Leu
   1 5
<210> 466
   <211> 10
   <212> PRT
   <213> Influenza A
<400> 466
   Ile Met Asp Lys Asn Ile Ile Leu Lys Ala
   1               5                   10
<210> 467
   <211> 10
   <212> PRT
   <213> Hepatitis C Virus
<400> 467
   Leu Leu Ala Leu Leu Ser Cys Leu Thr Val
   1               5                   10
<210> 468
   <211> 8
   <212> PRT
   <213> Hepatitis C Virus
<400> 468
   Ile Leu His Thr Pro Gly Cys Val
   1               5
<210> 469
   <211> 10
   <212> PRT
   <213> Hepatitis C Virus
<400> 469
   Gln Leu Arg Arg His Ile Asp Leu Leu Val
   1               5                   10
<210> 470
   <211> 9
   <212> PRT
   <213> Hepatites C Virus
<400> 470
   Asp Leu Cys Gly Ser Val Phe Leu Val
   1               5
<210> 471
   <211> 9
   <212> PRT
   <213> Hepatitis C Virus
<400> 471
   Ser Met Val Gly Asn Trp Ala Lys Val
   1               5
<210> 472
   <211> 9
   <212> PRT
   <213> Hepatitis C Virus
<400> 472
   His Leu His Gln Asn Ile Val Asp Val
   1 5
<210> 473
   <211> 9
   <212> PRT
   <213> Hepatitis C Virus
<400> 473
   Phe Leu Leu Leu Ala Asp Ala Arg Val
   1 5
<210> 474
   <211> 13
   <212> PRT
   <213> Hepatitis C Virus
<400> 474
   Gly Leu Arg Asp Leu Ala Val Ala Val Glu Pro Val Val
   1 5 10
<210> 475
   <211> 11
   <212> PRT
   <213> Hepatitis C Virus
<400> 475
   Ser Leu Leu Ala Pro Gly Ala Lys Gln Asn Val
   1 5 10
<210> 476
   <211> 10
   <212> PRT
   <213> Hepatitis C Virus
<400> 476
   Leu Leu Ala Pro Gly Ala Lys Gln Asn Val
   1 5 10
<210> 477
   <211> 9
   <212> PRT
   <213> Hepatitis B Virus
<400> 477
   Phe Leu Leu Ser Leu Gly Ile His Leu
   1 5
<210> 478
   <211> 9
   <212> PRT
   <213> Hepatitis B Virus
<400> 478
   Ser Leu Tyr Ala Asp Ser Pro Ser Val
   1 5
<210> 479
   <211> 9
   <212> PRT
   <213> Hepatitis B Virus
<400> 479
   Gly Leu Ser Arg Tyr Val Ala Arg Leu
   1 5
<210> 480
   <211> 9
   <212> PRT
   <213> Homo Sapien
<400> 480
   Lys Ile Phe Gly Ser Leu Ala Phe Leu
   1 5
<210> 481
   <211> 9
   <212> PRT
   <213> Homo Sapien
<400> 481
   Glu Leu Val Ser Glu Phe Ser Arg Met
   1 5
<210> 482
   <211> 9
   <212> PRT
   <213> Human immunodeficiency Virus 1
<400> 482
   Lys Leu Thr Pro Leu Cys Val Thr Leu
   1 5
<210> 483
   <211> 10
   <212> PRT
   <213> Human Immunodeficiency Virus 1
<400> 483
   Ser Leu Leu Asn Ala Thr Asp Ile Ala Val
   1 5 10
<210> 484
   <211> 10
   <212> PRT
   <213> Homo Sapien
<400> 484
   Val Leu Tyr Arg Tyr Gly Ser Phe Ser Val
   1 5 10
<210> 485
   <211> 9
   <212> PRT
   <213> Homo Sapien
<400> 485
   Tyr Ile Gly Glu Val Leu Val Ser Val
   1 5
<210> 486
   <211> 10
   <212> PRT
   <213> Hepatitis C virus
<400> 486
   Leu Leu Phe Asn Ile Leu Gly Gly Trp Val
   1 5 10
<210> 487
   <211> 10
   <212> PRT
   <213> Hepatitis B Virus
<400> 487
   Leu Leu Val Pro Phe Val Gln Trp Phe Trp
   1 5 10
<210> 488
   <211> 9
   <212> PRT
   <213> Hepatitis B virus
<400> 488
   Ala Leu Met Pro Leu Tyr Ala Cys Ile
   1 5
<210> 489
   <211> 9
   <212> PRT
   <213> Hepatitis C Virus
<400> 489
   Tyr Leu Val Ala Tyr Gln Ala Thr Val
   1 5
<210> 490
   <211> 9
   <212> PRT
   <213> Himetobi P Virus (HiPV)
<400> 490
   Thr Leu Gly Ile Val Cys Pro Ile Cys
   1 5
<210> 491
   <211> 10
   <212> PRT
   <213> Hepatitis C Virus
<400> 491
   Tyr Leu Leu Pro Arg Arg Gly Pro Arg Leu
   1 5 10
<210> 492
   <211> 10
   <212> PRT
   <213> Hepatitis B Virus
<400> 492
   Leu Leu Pro Ile Phe Phe Cys Leu Trp Val
   1 5 10
<210> 493
   <211> 9
   <212> PRT
   <213> Hepatites B virus
<400> 493
   Tyr Met Asp Asp Val Val Leu Gly Ala
   1 5
<210> 494
   <211> 9
   <212> PRT
   <213> Human papillomavirus
<400> 494
   Gly Thr Leu Gly Ile Val Cys Pro Ile
   1 5
<210> 495
   <211> 10
   <212> PRT
   <213> Hepatitis C Virus
<400> 495
   Leu Leu Ala Leu Leu Ser Cys Leu Thr Ile
   1               5                   10
<210> 496
   <211> 9
   <212> PRT
   <213> Human Papillomavirus
<400> 496
   Met Leu Asp Leu Gln Pro Glu Thr Thr
   1               5
<210> 497
   <211> 9
   <212> PRT
   <213> Hepatitis C virus
<400> 497
   Ser Leu Met Ala Phe Thr Ala Ala Val
   1               5
<210> 498
   <211> 9
   <212> PRT
   <213> Hepatitis C virus
<400> 498
   Cys Ile Asn Gly Val Cys Trp Thr Val
   1               5
<210> 499
   <211> 10
   <212> PRT
   <213> Homo Sapien
<400> 499
   Val Met Asn Ile Leu Leu Gln Tyr Val Val
   1               5                   10
<210> 500
   <211> 9
   <212> PRT
   <213> Homo Sapien
<400> 500
   Ile Leu Thr Val Ile Leu Gly Val Leu
   1 5
<210> 501
   <211> 9
   <212> PRT
   <213> Homo Sapien
<400> 501
   Phe Leu Trp Gly Pro Arg Ala Leu Val
   1 5
<210> 502
   <211> 9
   <212> PRT
   <213> Hepatitis C virus
<400> 502
   Leu Leu Cys Pro Ala Gly His Ala Val
   1 5
<210> 503
   <211> 9
   <212> PRT
   <213> Hepatitis C virus
<400> 503
   Ile Leu Asp Ser Phe Asp Pro Leu Val
   1 5
<210> 504
   <211> 9
   <212> PRT
   <213> Hepatitis B Virus
<400> 504
   Leu Leu Leu Cys Leu Ile Phe Leu Leu
   1 5
<210> 505
   <211> 10
   <212> PRT
   <213> Hepatitis B virus
<400> 505
   Leu Ile Asp Tyr Gln Gly Met Leu Pro Val
   1 5 10
<210> 506
   <211> 10
   <212> PRT
   <213> Hepatitis B Virus
<400> 506
   Ser Ile Val Ser Pro Phe Ile Pro Leu Leu
   1 5 10
<210> 507
   <211> 9
   <212> PRT
   <213> Hepatitis B Virus
<400> 507
   Phe Leu Leu Thr Arg Ile Leu Thr Ile
   1 5
<210> 508
   <211> 9
   <212> PRT
   <213> Plasmodium Faciparum
<400> 508
   His Leu Gly Asn Val Lys Tyr Leu Val
   1 5
<210> 509
   <211> 9
   <212> PRT
   <213> Plasmodium Faciparum
<400> 509
   Gly Ile Ala Gly Gly Leu Ala Leu Leu
   1 5
<210> 510
   <211> 9
   <212> PRT
   <213> Hepatitis C Virus
<400> 510
   Ile Leu Ala Gly Tyr Gly Ala Gly Val
   1 5
<210> 511
   <211> 9
   <212> PRT
   <213> Hepatitis C Virus

<400> 511
   Gly Leu Gln Asp Cys Thr Met Leu Val
   1 5
<210> 512
   <211> 10
   <212> PRT
   <213> Hepatitis C Virus
<400> 512
   Thr Gly Ala Pro Val Thr Tyr Ser Thr Tyr
   1 5 10
<210> 513
   <211> 10
   <212> PRT
   <213> Human Immunodeficiency Virus 1
<400> 513
   Val Ile Tyr Gln Tyr Met Asp Asp Leu Val
   1 5 10
<210> 514
   <211> 10
   <212> PRT
   <213> Homo Sapien
<400> 514
   Val Leu Pro Asp Val Phe Ile Arg Cys Val
   1 5 10
<210> 515
   <211> 9
   <212> PRT
   <213> Homo Sapien
<400> 515
   Val Leu Pro Asp Val Phe Ile Arg Cys
   1 5
<210> 516
   <211> 9
   <212> PRT
   <213> Homo Sapien
<400> 516
   Ala Val Gly Ile Gly Ile Ala Val Val
   1 5
<210> 517
   <211> 9
   <212> PRT
   <213> Homo Sapien
<400> 517
   Leu Val Val Leu Gly Leu Leu Ala Val
   1 5
<210> 518
   <211> 9
   <212> PRT
   <213> Homo Sapien
<400> 518
   Ala Leu Gly Leu Gly Leu Leu Pro Val
   1 5
<210> 519
   <211> 9
   <212> PRT
   <213> Herpes Simplex Virus I
<400> 519
   Gly Ile Gly Ile Gly Val Leu Ala Ala
   1 5
<210> 520
   <211> 9
   <212> PRT
   <213> Herpes Simplex Virus 2
<400> 520
   Gly Ala Gly Ile Gly Val Ala Val Leu
   1 5
<210> 521
   <211> 9
   <212> PRT
   <213> Pseudorabies Virus
<400> 521
   Ile Ala Gly Ile Gly Ile Leu Ala Ile
   1 5
<210> 522
   <211> 9
   <212> PRT
   <213> Adenovirus
<400> 522
   Leu Ile Val Ile Gly Ile Leu Ile Leu
   1 5
<210> 523
   <211> 9
   <212> PRT
   <213> Streptomyces Lincolnensis
<400> 523
   Leu Ala Gly Ile Gly Leu Ile Ala Ala
   1 5
<210> 524
   <211> 9
   <212> PRT
   <213> Yeast (YSA-1)
<400> 524
   Val Asp Gly Ile Gly Ile Leu Thr Ile
   1 5
<210> 525
   <211> 9
   <212> PRT
   <213> Bacillus Polymyxa
<400> 525
   Gly Ala Gly Ile Gly Val Leu Thr Ala
   1 5
<210> 526
   <211> 9
   <212> PRT
   <213> Escherichia coli
<400> 526
   Ala Ala Gly Ile Gly Ile Ile Gln Ile
   1 5
<210> 527
   <211> 9
   <212> PRT
   <213> Escherichia coli
<400> 527
   Gln Ala Gly Ile Gly Ile Leu Leu Ala
   1 5
<210> 528
   <211> 11
   <212> PRT
   <213> Homo Sapien
<400> 528
   Lys Ala Arg Asp Pro His Ser Gly His Phe Val
   1 5 10
<210> 529
   <211> 13
   <212> PRT
   <213> Homo Sapien
<400> 529
   Lys Ala Cys Asp Pro Ile Ile Ser Gly Ile Ile Phe Val
   1               5                   10
<210> 530
   <211> 11
   <212> PRT
   <213> Homo Sapien
<400> 530
   Ala Cys Asp Pro Phe Ile Ser Gly His Phe Val
   1               5                   10
<210> 531
   <211> 9
   <212> PRT
   <213> Human Immunodeficiency Virus I
<400> 531
   Ser Leu Tyr Asn Thr Val Ala Thr Leu
   1               5
<210> 532
   <211> 9
   <212> PRT
   <213> Homo Sapien
<400> 532
   Glu Leu Val Ser Glu Phe Ser Arg Val
   1               5
<210> 533
   <211> 10
   <212> PRT
   <213> Human Immunodeficiency Virus I
<400> 533
   Arg Gly Pro Gly Arg Ala Phe Val Thr Ile
   1               5                   10
<210> 534
   <211> 9
   <212> PRT
   <213> Hepatitis C Virus
<400> 534
   His Met Trp Asn Phe Ile Ser Gly Ile
   1               5
<210> 535
   <211> 10
   <212> PRT
   <213> Human Cytomegalovirus
<400> 535
   Asn Leu Val Pro Met Val Ala Thr Val Gln
   1               5                   10
<210> 536
   <211> 9
   <212> PRT
   <213> Human Papillomavirus
<400> 536
   Gly Leu His Cys Tyr Glu Gln Leu Val
   1               5
<210> 537
   <211> 9
   <212> PRT
   <213> Human Papillomavirus
<400> 537
   Pro Leu Lys Gln His Phe Gln Ile Val
   1               5
<210> 538
   <211> 10
   <212> PRT
   <213> Epstein-Barr Virus (EBNA)
<400> 538
   Leu Leu Asp Phe Val Arg Phe Met Gly Val
   1               5                   10
<210> 539
   <211> 9
   <212> PRT
   <213> Influenza
<400> 539
   Ala Ile Met Glu Lys Asn Ile Met Leu
   1               5
<210> 540
   <211> 9
   <212> PRT
   <213> Plasmodium Falciparum
<400> 540
   Tyr Leu Lys Thr Ile Gln Asn Ser Leu
   1               5
<210> 541
   <211> 9
   <212> PRT
   <213> Plasmodium Falciparum
<400> 541
   Tyr Leu Asn Lys Ile Gln Asn Ser Leu
   1 5
<210> 542
   <211> 10
   <212> PRT
   <213> Human Papillomavirus
<400> 542
   Tyr Met Leu Asp Leu Gln Pro Glu Thr Thr
   1 5 10
<210> 543
   <211> 9
   <212> PRT
   <213> Human Papillomavirus
<400> 543
   Leu Leu Met Gly Thr Leu Gly Ile Val
   1 5
<210> 544
   <211> 8
   <212> PRT
   <213> Human Papillomavirus
<400> 544
   Thr Leu Gly Ile Val Cys Pro Ile
   1 5
<210> 545
   <211> 9
   <212> PRT
   <213> Human Immunodeficiency Virus 1
<400> 545
   Thr Leu Thr Ser Cys Asn Thr Ser Val
   1 5
<210> 546
   <211> 9
   <212> PRT
   <213> Human Papillomavirus
<400> 546
   Lys Leu Pro Gln Leu Cys Thr Glu Leu
   1 5
<210> 547
   <211> 9
   <212> PRT
   <213> Human Papillomavirus 16
<400> 547
   Thr Ile His Asp Ile Ile Leu Glu Cys
   1 5
<210> 548
   <211> 9
   <212> PRT
   <213> Human Papillomavirus 16
<400> 548
   Leu Gly Ile Val Cys Pro Ile Cys Ser
   1 5
<210> 549
   <211> 9
   <212> PRT
   <213> Homo Sapien
<400> 549
   Val Ile Leu Gly Val Leu Leu Leu Ile
   1 5
<210> 550
   <211> 9
   <212> PRT
   <213> Homo Sapien
<400> 550
   Ala Leu Met Asp Lys Ser Leu His Val
   1 5
<210> 551
   <211> 9
   <212> PRT
   <213> Homo Sapien
<400> 551
   Gly Ile Leu Thr Val Ile Leu Gly Val
   1 5
<210> 552
   <211> 9
   <212> PRT
   <213> Plasmodium Falciparum
<400> 552
   Met Ile Asn Ala Tyr Leu Asp Lys Leu
   1 5
<210> 553
   <211> 9
   <212> PRT
   <213> Homo Sapien
<400> 553
   Ala Ala Gly Ile Gly Ile Leu Thr Val
   1 5
<210> 554
   <211> 10
   <212> PRT
   <213> Hepatitis B Virus
<400> 554
   Phe Leu Pro Ser Asp Phe Phe Pro Ser Val
   1 5 10
<210> 555
   <211> 9
   <212> PRT
   <213> Epstein-Barr Virus (EBNA)
<400> 555
   Ser Val Arg Asp Arg Leu Ala Arg Leu
   1 5
<210> 556
   <211> 9
   <212> PRT
   <213> Homo Sapien
<400> 556
   Ala Ala Gly Ile Gly Ile Leu Thr Val
   1 5
<210> 557
   <211> 9
   <212> PRT
   <213> Homo Sapien
<400> 557
   Phe Ala Tyr Asp Gly Lys Asp Tyr Ile
   1 5
<210> 558
   <211> 9
   <212> PRT
   <213> Homo Sapien
<400> 558
   Ala Ala Gly Ile Gly Ile Leu Thr Val
   1 5
<210> 559
   <211> 10
   <212> PRT
   <213> Hepatitis B Virus
<400> 559
   Phe Leu Pro Ser Asp Phe Phe Pro Ser Val
   1 5 10
<210> 560
   <211> 9
   <212> PRT
   <213> Homo Sapien
<400> 560
   Ala Ala Gly Ile Gly Ile Leu Thr Val
   1 5
<210> 561
   <211> 10
   <212> PRT
   <213> Hepatitis B Virus
<400> 561
   Phe Leu Pro Ser Asp Phe Phe Pro Ser Val
   1 5 10
<210> 562
   <211> 9
   <212> PRT
   <213> Homo Sapien
<400> 562
   Ala Ala Gly Ile Gly Ile Leu Thr Val
   1 5
<210> 563
   <211> 9
   <212> PRT
   <213> Homo Sapien
<400> 563
   Ala Leu Leu Ala Val Gly Ala Thr Lys
   1 5
<210> 564
   <211> 11
   <212> PRT
   <213> Human Immunodeficiency Virus 1
<400> 564
   Arg Leu Arg Asp Leu Leu Leu Ile Val Thr Arg
   1 5 10
<210> 565
   <211> 10
   <212> PRT
   <213> Human Immunodeficiency Virus 1
<400> 565
   Gln Val Pro Leu Arg Pro Met Thr Tyr Lys
   1 5 10
<210> 566
   <211> 10
   <212> PRT
   <213> Human Immunodeficiency Virus 1
<400> 566
   Thr Val Tyr Tyr Gly Val Pro Val Trp Lys
   1 5 10
<210> 567
   <211> 9
   <212> PRT
   <213> Human Immunodeficiency Virus 1
<400> 567
   Arg Leu Arg Pro Gly Gly Lys Lys Lys
   1 5
<210> 568
   <211> 9
   <212> PRT
   <213> Influenza
<400> 568
   Ile Leu Arg Gly Ser Val Ala His Lys
   1 5
<210> 569
   <211> 9
   <212> PRT
   <213> Epstein-Barr Virus (EBNA)
<400> 569
   Arg Leu Arg Ala Glu Ala Gly Val Lys
   1 5
<210> 570
   <211> 11
   <212> PRT
   <213> Human Immunodeficiency virus 1
<400> 570
   Arg Leu Arg Asp Leu Leu Leu Ile Val Thr Arg
   1 5 10
<210> 571
   <211> 9
   <212> PRT
   <213> Human Immunodeficiency Virus 1
<400> 571
   Val Tyr Tyr Gly Val Pro Val Trp Lys
   1 5
<210> 572
   <211> 9
   <212> PRT
   <213> Hepatitis C Virus
<400> 572
   Arg Val Cys Glu Lys Met Ala Leu Tyr
   1 5
<210> 573
   <211> 9
   <212> PRT
   <213> Homo Sapien
<400> 573
   Lys Ile Phe Ser Glu Val Thr Leu Lys
   1 5
<210> 574
   <211> 9
   <212> PRT
   <213> Hepatitis B Virus
<400> 574
   Tyr Val Asn Val Asn Met Gly Leu Lys
   1 5
<210> 575
   <211> 9
   <212> PRT
   <213> Epstein-Barr Virus (EBNA)
<400> 575
   Ile Val Thr Asp Phe Ser Val Ile Lys
   1 5
<210> 576
   <211> 9
   <212> PRT
   <213> Homo Sapien
<400> 576
   Glu Leu Asn Glu Ala Leu Glu Leu Lys
   1 5
<210> 577
   <211> 9
   <212> PRT
   <213> Human Immunodeficiency virus 1
<400> 577
   Val Pro Leu Arg Pro Met Thr Tyr Lys
   1 5
<210> 578
   <211> 9
   <212> PRT
   <213> Human Immunodeficiency Virus 1
<400> 578
   Ala Ile Phe Gln Ser Ser Met Thr Lys
   1 5
<210> 579
   <211> 10
   <212> PRT
   <213> Human Immunodeficiency Virus 1
<400> 579
   Gln Val Pro Leu Arg Pro Met Thr Tyr Lys
   1 5 10
<210> 580
   <211> 11
   <212> PRT
   <213> Influenza
<400> 580
   Thr Ile Asn Tyr Thr Ile Phe Lys His Cys Val
   1 5 10
<210> 581
   <211> 12
   <212> PRT
   <213> Human Immunodeficiency Virus 1
<400> 581
   Ala Ala Val Asp Leu Ser His Phe Leu Lys Glu Lys
   1 5 10
<210> 582
   <211> 12
   <212> PRT
   <213> Human Immunodeficiency Virus 1
<400> 582
   Ala Cys Gln Gly Val Gly Gly Pro Gly Gly His Lys
   1 5 10
<210> 583
   <211> 9
   <212> PRT
   <213> Homo Sapien
<400> 583
   Ser Tyr Leu Asp Ser Gly Ile His Phe
   1 5
<210> 584
   <211> 9
   <212> PRT
   <213> Human Immunodeficiency virus 1
<400> 584
   Arg Tyr Leu Lys Asp Gln Gln Leu Leu
   1 5
<210> 585
   <211> 9
   <212> PRT
   <213> Homo Sapien
<400> 585
   Ala Tyr Gly Leu Asp Phe Tyr Ile Leu
   1 5
<210> 586
   <211> 10
   <212> PRT
   <213> Homo Sapien
<400> 586
   Ala Phe Leu Pro Trp His Arg Leu Phe Leu
   1 5 10
<210> 587
   <211> 9
   <212> PRT
   <213> Homo Sapien
<400> 587
   Ala Phe Leu Pro Trp His Arg Leu Phe
   1 5
<210> 588
   <211> 8
   <212> PRT
   <213> Epstein-Barr Virus (EBNA)
<400> 588
   Arg Tyr Ser Ile Phe Phe Asp Tyr
   1 5
<210> 589
   <211> 10
   <212> PRT
   <213> Human Immunodeficiency Virus 1
<400> 589
   Glu Thr Ile Asn Glu Glu Ala Ala Glu Trp
   1 5 10
<210> 590
   <211> 11
   <212> PRT
   <213> Hepatitis B Virus
<400> 590
   Ser Thr Leu Pro Glu Thr Thr Val Val Arg Arg
   1 5 10
<210> 591
   <211> 9
   <212> PRT
   <213> Homo Sapien
<400> 591
   Met Ser Leu Gln Arg Gln Phe Leu Arg
   1 5
<210> 592
   <211> 9
   <212> PRT
   <213> Homo Sapien
<400> 592
   Leu Leu Pro Gly Gly Arg Pro Tyr Arg
   1               5
<210> 593
   <211> 9
   <212> PRT
   <213> Human Immunodeficiency virus
<400> 593
   Ile Val Gly Leu Asn Lys Ile Val Arg
   1               5
<210> 594
   <211> 9
   <212> PRT
   <213> Homo Sapien
<400> 594
   Ala Ala Gly Ile Gly Ile Leu Thr Val
   1               5
<210> 595
   <211> 9
   <212> PRT
   <213> Homo Sapien
<400> 595
   Glu Val Asp Pro Thr Ser Asn Thr Tyr
   1               5
<210> 596
   <211> 9
   <212> PRT
   <213> Homo Sapien
<400> 596
   Glu Ala Asp Pro Thr Ser Asn Thr Tyr
   1               5
<210> 597
   <211> 9
   <212> PRT
   <213> Homo Sapien
<400> 597
   Glu Ala Asp Pro Thr Ser Asn Thr Tyr
   1               5
<210> 598
   <211> 9
   <212> PRT
   <213> Homo Sapien
<400> 598
   Glu Val Asp Pro Ile Gly His Val Tyr
   1               5
<210> 599
   <211> 10
   <212> PRT
   <213> Homo Sapien
<400> 599
   Met Leu Leu Ala Val Leu Tyr Cys Leu Leu
   1               5                   10
<210> 600
   <211> 9
   <212> PRT
   <213> Homo Sapien
<400> 600
   Met Leu Leu Ala Val Leu Tyr Cys Leu
   1               5
<210> 601
   <211> 10
   <212> PRT
   <213> Homo Sapien
<400> 601
   Ser Glu Ile Trp Arg Asp Ile Asp Phe Ala
   1               5                   10
<210> 602
   <211> 9
   <212> PRT
   <213> Homo Sapien
<400> 602
   Ser Glu Ile Trp Arg Asp Ile Asp Phe
   1               5
<210> 603
   <211> 9
   <212> PRT
   <213> Homo Sapien
<220>
   <221> VARIANT
   <222> 1
   <223> Xaa = Any Amino Acid
<400> 603
   Xaa Glu Ile Trp Arg Asp Ile Asp Phe
   1 5
<210> 604
   <211> 10
   <212> PRT
   <213> Homo Sapien
<400> 604
   Ser Thr Leu Val Glu Val Thr Leu Gly Val
   1 5 10
<210> 605
   <211> 9
   <212> PRT
   <213> Homo Sapien
<400> 605
   Leu Val Glu Val Thr Leu Gly Glu Val
   1 5
<210> 606
   <211> 10
   <212> PRT
   <213> Homo Sapien
<400> 606
   Val Ile Phe Ser Lys Ala Ser Glu Tyr Leu
   1 5 10
<210> 607
   <211> 9
   <212> PRT
   <213> Homo Sapien

<400> 607
   Ile Ile Val Leu Ala Ile Ile Ala Ile
   1 5
<210> 608
   <211> 11
   <212> PRT
   <213> Homo Sapien
<400> 608
   Lys Ile Trp Glu Glu Leu Ser Met Leu Glu Tyr
   1 5 10
<210> 609
   <211> 9
   <212> PRT
   <213> Homo Sapien
<400> 609
   Leu Ile Glu Thr Ser Tyr Val Lys Val
   1 5
<210> 610
   <211> 9
   <212> PRT
   <213> Homo Sapien
<400> 610
   Phe Leu Trp Gly Pro Arg Ala Leu Val
   1 5
<210> 611
   <211> 10
   <212> PRT
   <213> Homo Sapien
<400> 611
   Thr Leu Val Glu Val Thr Leu Gly Glu Val
   1 5 10
<210> 612
   <211> 10
   <212> PRT
   <213> Homo Sapien
<400> 612
   Ala Leu Val Glu Thr Ser Tyr Val Lys Val
   1 5 10
<210> 613
   <211> 9
   <212> PRT
   <213> Homo Sapien
<400> 613
   Lys Ile Trp Glu Glu Leu Ser Val Leu
   1 5
<210> 614
   <211> 9
   <212> PRT
   <213> Homo Sapien
<400> 614
   Glu Val Asp Pro Ile Gly His Leu Tyr
   1 5
<210> 615
   <211> 9
   <212> PRT
   <213> Homo Sapien
<220>
   <221> VARIANT
   <222> 2, 4, 5, 6, 7, 8
   <223> Xaa = Any Amino Acid
<400> 615
   Glu Xaa Asp Xaa Xaa Xaa Xaa Xaa Tyr
   1 5
<210> 616
   <211> 9
   <212> PRT
   <213> Homo Sapien
<400> 616
   Glu Ala Asp Pro Thr Gly His Ser Tyr
   1 5
<210> 617
   <211> 9
   <212> PRT
   <213> Homo Sapien
<220>
   <221> VARIANT
   <222> 4, 5, 6, 7, 8
   <223> Xaa = Any Amino Acid
<221> VARIANT
   <222> 2
   <223> Xaa = A or V
<400> 617
   Glu Xaa Asp Xaa Xaa Xaa Xaa Xaa Tyr
   1 5
<210> 618
   <211> 9
   <212> PRT
   <213> Homo Sapien
<220>
   <221> VARIANT
   <222> 5, 6, 7, 8
   <223> Xaa = Any Amino Acid
<221> VARIANT
   <222> 2
   <223> Xaa = A or V
<400> 618
   Glu Xaa Asp Pro Xaa Xaa Xaa Xaa Tyr
   1 5
<210> 619
   <211> 9
   <212> PRT
   <213> Homo Sapien
<220>
   <221> VARIANT
   <222> 6, 7, 8
   <223> Xaa = Any Amino Acid
<221> VARIANT
   <222> 2
   <223> Xaa = A or V
<221> VARIANT
   <222> 5
   <223> Xaa = I or A or T
<400> 619
   Glu Xaa Asp Pro Xaa Xaa Xaa Xaa Tyr
   1 5
<210> 620
   <211> 9
   <212> PRT
   <213> Homo Sapien
<220>
   <221> VARIANT
   <222> 7, 8
   <223> Xaa = Any Amino Acid
<221> VARIANT
   <222> 2
   <223> Xaa = A or V
<221> VARIANT
   <222> 5
   <223> Xaa = I or A or T
<221> VARIANT
   <222> 6
   <223> Xaa = G or S
<400> 620
   Glu Xaa Asp Pro Xaa Xaa Xaa Xaa Tyr
   1 5
<210> 621
   <211> 9
   <212> PRT
   <213> Homo Sapien
<220>
   <221> VARIANT
   <222> 8
   <223> Xaa = Any Amino Acid
<221> VARIANT
   <222> 2
   <223> Xaa = A or V
<221> VARIANT
   <222> 5
   <223> Xaa = I or A or T
<221> VARIANT
   <222> 6
   <223> Xaa = G or S
<221> VARIANT
   <222> 7
   <223> Xaa = H or N

<400> 621
   Glu Xaa Asp Pro Xaa Xaa Xaa Xaa Tyr
   1               5
<210> 622
   <211> 9
   <212> PRT
   <213> Homo Sapien
<220>
   <221> VARIANT
   <222> 2
   <223> Xaa = A or V
<221> VARIANT
   <222> 5
   <223> Xaa = I or A or T
<221> VARIANT
   <222> 6
   <223> Xaa = G or S
<221> VARIANT
   <222> 7
   <223> Xaa = H or N
<221> VARIANT
   <222> 8
   <223> Xaa = L or T or V
<400> 622
   Glu Xaa Asp Pro Xaa Xaa Xaa Xaa Tyr
   1               5
<210> 623
   <211> 16
   <212> PRT
   <213> Homo Sapien
<400> 623
   Glu Leu His Ser Ala Tyr Gly Glu Pro Arg Lys Leu Leu Thr Gln Asp
   1 5 10 15
<210> 624
   <211> 12
   <212> PRT
   <213> Homo sapien
<400> 624
   Glu His Ser Ala Tyr Gly Glu Pro Arg Lys Leu Leu
   1 5 10
<210> 625
   <211> 9
   <212> PRT
   <213> Homo Sapien
<400> 625
   Ser Ala Tyr Gly Glu Pro Arg Lys Leu
   1 5
<210> 626
   <211> 9
   <212> PRT
   <213> Homo Sapien
<400> 626
   Glu Ala Asp Pro Thr Gly His Ser Tyr
   1 5
<210> 627
   <211> 22
   <212> PRT
   <213> Homo Sapien
<400> 627
   Met Ala Ala Arg Ala Val Phe Leu Ala Leu Ser Ala Gln Leu Leu Gln
   1 5 10 15
   Ala Arg Leu Met Lys Glu
   20
<210> 628
   <211> 16
   <212> PRT
   <213> Homo Sapien
<400> 628
   Met Ala Ala Arg Ala Val Phe Leu Ala Leu Ser Ala Gln Leu Leu Gln
   1               5                   10                  15
<210> 629
   <211> 9
   <212> PRT
   <213> Homo Sapien
<400> 629
   Ala Ala Arg Ala Val Phe Leu Ala Leu
   1               5
<210> 630
   <211> 8
   <212> PRT
   <213> Homo Sapien
<400> 630
   Tyr Arg Pro Arg Pro Arg Arg Tyr
   1               5
<210> 631
   <211> 9
   <212> PRT
   <213> Artificial Sequence
<220>
   <223> Synthetic HLA-A2 binding nonamer peptide
<400> 631
   Ala Leu Phe Ala Ala Ala Ala Ala Val
   1               5
<210> 632
   <211> 9
   <212> PRT
   <213> Artificial sequence
<220>
   <223> Synthetic HLA-A2 binding nonamer peptide
<400> 632
   Gly Ile Phe Gly Gly Val Gly Gly Val
   1               5
<210> 633
   <211> 8
   <212> PRT
   <213> Artificial Sequence
<220>
   <223> Synthetic HLA-A2 binding nonamer peptide
<400> 633
   Gly Leu Asp Lys Gly Gly Gly Val
   1 5
<210> 634
   <211> 9
   <212> PRT
   <213> Artificial Sequence
<220>
   <223> Synthetic HLA-A2 binding nonamer peptide
<400> 634
   Gly Leu Phe Gly Gly Phe Gly Gly Val
   1 5
<210> 635
   <211> 9
   <212> PRT
   <213> Artificial Sequence
<220>
   <223> Synthetic HLA-A2 binding nonamer peptide
<400> 635
   Gly Leu Phe Gly Gly Gly Ala Gly Val
   1 5
<210> 636
   <211> 9
   <212> PRT
   <213> Artificial Sequence
<220>
   <223> Synthetic HLA-A2 binding nonamer peptide
<400> 636
   Gly Leu Phe Gly Gly Gly Glu Gly Val
   1 5
<210> 637
   <211> 9
   <212> PRT
   <213> Artificial Sequence
<220>
   <223> Synthetic HLA-A2 binding nonamer peptide
<400> 637
   Gly Leu Phe Gly Gly Gly Phe Gly Val
   1 5
<210> 638
   <211> 9
   <212> PRT
   <213> Artificial Sequence
<220>
   <223> Synthetic HLA-A2 binding nonamer peptide
<400> 638
   Gly Leu Phe Gly Gly Gly Gly Gly Leu
   1 5
<210> 639
   <211> 9
   <212> PRT
   <213> Artificial Sequence
<220>
   <223> Synthetic HLA-A2 binding nonamer peptide
<400> 639
   Gly Leu Phe Gly Gly Gly Gly Gly Val
   1 5
<210> 640
   <211> 9
   <212> PRT
   <213> Artificial Sequence
<220>
   <223> Synthetic HLA-A2 binding nonamer peptide
<400> 640
   Gly Leu Phe Gly Gly Gly Val Gly Val
   1 5
<210> 641
   <211> 9
   <212> PRT
   <213> Artificial Sequence
<220>
   <223> Synthetic HLA-A2 binding nonamer peptide
<400> 641
   Gly Leu Phe Gly Gly Val Gly Gly Val
   1 5
<210> 642
   <211> 9
   <212> PRT
   <213> Artificial Sequence
<220>
   <223> Synthetic HLA-A2 binding nonamer peptide
<400> 642
   Gly Leu Phe Gly Gly Val Gly Lys Val
   1 5
<210> 643
   <211> 9
   <212> PRT
   <213> Artificial Sequence
<220>
   <223> Synthetic HLA-A2 binding nonamer peptide
<400> 643
   Gly Leu Phe Lys Gly Val Gly Gly Val
   1 5
<210> 644
   <211> 9
   <212> PRT
   <213> Artificial Sequence
<220>
   <223> synthetic HLA-A2 binding nonamer peptide
<400> 644
   Gly Leu Gly Gly Gly Gly Phe Gly Val
   1 5
<210> 645
   <211> 9
   <212> PRT
   <213> Artificial Sequence
<220>
   <223> Synthetic HLA-A2 binding nonamer peptide
<400> 645
   Gly Leu Leu Gly Gly Gly Val Gly Val
   1 5
<210> 646
   <211> 9
   <212> PRT
   <213> Artificial Sequence
<220>
   <223> synthetic HLA-A2 binding nonamer peptide
<400> 646
   Gly Leu Tyr Gly Gly Gly Gly Gly Val
   1 5
<210> 647
   <211> 9
   <212> PRT
   <213> Artificial Sequence
<220>
   <223> Synthetic HLA-A2 binding nonamer peptide
<400> 647
   Gly Met Phe Gly Gly Gly Gly Gly Val
   1               5
<210> 648
   <211> 9
   <212> PRT
   <213> Artificial Sequence
<220>
   <223> Synthetic HLA-A2 binding nonamer peptide
<400> 648
   Gly Met Phe Gly Gly Val Gly Gly Val
   1               5
<210> 649
   <211> 9
   <212> PRT
   <213> Artificial Sequence
<220>
   <223> synthetic HLA-A2 binding nonamer peptide
<400> 649
   Gly Gln Phe Gly Gly Val Gly Gly Val
   1               5
<210> 650
   <211> 9
   <212> PRT
   <213> Artificial Sequence
<220>
   <223> synthetic HLA-A2 binding nonamer peptide
<400> 650
   Gly Val Phe Gly Gly Val Gly Gly Val
   1               5
<210> 651
   <211> 9
   <212> PRT
   <213> Artificial Sequence
<220>
   <223> Synthetic HLA-A2 binding nonamer peptide
<400> 651
   Lys Leu Phe Gly Gly Gly Gly Gly Val
   1 5
<210> 652
   <211> 9
   <212> PRT
   <213> Artificial Sequence
<220>
   <223> Synthetic HLA-A2 binding nonamer peptide
<400> 652
   Lys Leu Phe Gly Gly Val Gly Gly Val
   1 5
<210> 653
   <211> 9
   <212> PRT
   <213> Artificial Sequence
<220>
   <223> synthetic HLA-A2 binding nonamer peptide
<400> 653
   Ala Ile Leu Gly Phe Val Phe Thr Leu
   1 5
<210> 654
   <211> 9
   <212> PRT
   <213> Artificial sequence
<220>
   <223> Synthetic HLA-A2 binding nonamer peptide
<400> 654
   Gly Ala Ile Gly Phe Val Phe Thr Leu
   1 5
<210> 655
   <211> 9
   <212> PRT
   <213> Artificial Sequence
<220>
   <223> Synthetic HLA-A2 binding nonamer peptide

<400> 655
   Gly Ala Leu Gly Phe Val Phe Thr Leu
   1 5
<210> 656
   <211> 9
   <212> PRT
   <213> Artificial sequence
*<220>*
   <223> Synthetic HLA-A2 binding nonamer peptide
<400> 656
   Gly Glu Leu Gly Phe Val Phe Thr Leu
   1 5
<210> 657
   <211> 9
   <212> PRT
   <213> Artificial Sequence
<220>
   <223> Synthetic HLA-A2 binding nonamer peptide
<400> 657
   Gly Ile Ala Gly Phe Val Phe Thr Leu
   1 5
<210> 658
   <211> 9
   <212> PRT
   <213> Artificial sequence
<220>
   <223> Synthetic HLA-A2 binding nonamer peptide
<400> 658
   Gly Ile Glu Gly Phe Val Phe Thr Leu
   1 5
<210> 659
   <211> 9
   <212> PRT
   <213> Artificial sequence
<220>
   <223> Synthetic HLA-A2 binding nonamer peptide
<400> 659
   Gly Ile Leu Ala Phe Val Phe Thr Leu
   1 5
<210> 660
   <211> 9
   <212> PRT
   <213> Artificial Sequence
<220>
   <223> synthetic HLA-A2 binding nonamer peptide
<400> 660
   Gly Ile Leu Gly Ala Val Phe Thr Leu
   1               5
<210> 661
   <211> 9
   <212> PRT
   <213> Artificial Sequence
<220>
   <223> Synthetic HLA-A2 binding nonamer peptide
<400> 661
   Gly Ile Leu Gly Glu Val Phe Thr Leu
   1               5
<210> 662
   <211> 9
   <212> PRT
   <213> Artificial Sequence
<220>
   <223> Synthetic HLA-A2 binding nonamer peptide
<400> 662
   Gly Ile Leu Phe Gly Ala Phe Thr Leu
   1               5
<210> 663
   <211> 9
   <212> PRT
   <213> Artificial Sequence
<220>
   <223> Synthetic HLA-A2 binding nonamer peptide
<400> 663
   Gly Ile Leu Gly Phe Glu Phe Thr Leu
   1               5
<210> 664
   <211> 9
   <212> PRT
   <213> Artificial Sequence
<220>
   <223> Synthetic HLA-A2 binding nonamer peptide
<400> 664
   Gly Ile Leu Gly Phe Lys Phe Thr Leu
   1               5
<210> 665
   <211> 9
   <212> PRT
   <213> Artificial sequence
<220>
   <223> Synthetic HLA-A2 binding nonamer peptide
<400> 665
   Gly Ile Leu Gly Phe Val Ala Thr Leu
   1               5
<210> 666
   <211> 9
   <212> PRT
   <213> Artificial Sequence
<220>
   <223> Synthetic HLA-A2 binding nonamer peptide
<400> 666
   Gly Ile Leu Gly Phe Val Glu Thr Leu
   1               5
<210> 667
   <211> 9
   <212> PRT
   <213> Artificial Sequence
<220>
   <223> synthetic HLA-A2 binding nonamer peptide
<400> 667
   Gly Ile Leu Gly Phe Val Phe Ala Leu
   1               5
<210> 668
   <211> 9
   <212> PRT
   <213> Artificial Sequence
<220>
   <223> Synthetic HLA-A2 binding nonamer peptide
<400> 668
   Gly Ile Leu Gly Phe Val Phe Glu Leu
   1               5
<210> 669
   <211> 9
   <212> PRT
   <213> Artificial Sequence
<220>
   <223> Synthetic HLA-A2 binding nonamer peptide
<400> 669
   Gly Ile Leu Gly Phe Val Phe Lys Leu
   1 5
<210> 670
   <211> 9
   <212> PRT
   <213> Artificial Sequence
<220>
   <223> synthetic HLA-A2 binding nonamer peptide
<400> 670
   Gly Ile Leu Gly Phe Val Phe Thr Ala
   1 5
<210> 671
   <211> 9
   <212> PRT
   <213> Artificial sequence
<220>
   <223> synthetic HLA-A2 binding nonamer peptide
<400> 671
   Gly Ile Leu Gly Phe Val Phe Thr Leu
   1 5
<210> 672
   <211> 9
   <212> PRT
   <213> Artificial sequence
<220>
   <223> Synthetic HLA-A2 binding nonamer peptide
<400> 672
   Gly Ile Leu Gly Phe Val Phe Val Leu
   1 5
<210> 673
   <211> 9
   <212> PRT
   <213> Artificial Sequence
<220>
   <223> Synthetic HLA-A2 binding nonamer peptide
<400> 673
   Gly Ile Leu Gly Phe Val Lys Thr Leu
   1 5
<210> 674
   <211> 9
   <212> PRT
   <213> Artificial Sequence
<220>
   <223> Synthetic HLA-A2 binding nonamer peptide
<400> 674
   Gly Ile Leu Gly Lys Val Phe Thr Leu
   1 5
<210> 675
   <211> 9
   <212> PRT
   <213> Artificial Sequence
<220>
   <223> Synthetic HLA-A2 binding nonamer peptide
<400> 675
   Gly Ile Leu Lys Phe Val Phe Thr Leu
   1 5
<210> 676
   <211> 9
   <212> PRT
   <213> Artificial Sequence
<220>
   <223> synthetic HLA-A2 binding nonamer peptide
<400> 676
   Gly Ile Leu Pro Phe Val Phe Thr Leu
   1 5
<210> 677
   <211> 9
   <212> PRT
   <213> Artificial sequence
<220>
   <223> Synthetic HLA-A2 binding nonamer peptide
<400> 677
   Gly Ile Val Gly Phe Val Phe Thr Leu
   1 5
<210> 678
   <211> 9
   <212> PRT
   <213> Artificial Sequence
<220>
   <223> Synthetic HLA-A2 binding nonamer peptide
<400> 678
   Gly Lys Leu Gly Phe Val Phe Thr Leu
   1 5
<210> 679
   <211> 9
   <212> PRT
   <213> Artificial Sequence
<220>
   <223> synthetic HLA-A2 binding nonamer peptide
<400> 679
   Gly Leu Leu Gly Phe Val Phe Thr Leu
   1 5
<210> 680
   <211> 9
   <212> PRT
   <213> Artificial sequence
<220>
   <223> Synthetic HLA-A2 binding nonamer peptide
<400> 680
   Gly Gln Leu Gly Phe Val Phe Thr Leu
   1 5
<210> 681
   <211> 9
   <212> PRT
   <213> Artificial sequence
<220>
   <223> Synthetic HLA-A2 binding nonamer peptide
<400> 681
   Lys Ala Leu Gly Phe Val Phe Thr Leu
   1 5
<210> 682
   <211> 9
   <212> PRT
   <213> Artificial Sequence
<220>
   <223> Synthetic HLA-A2 binding nonamer peptide
<400> 682
   Lys Ile Leu Gly Phe Val Phe Thr Leu
   1 5
<210> 683
   <211> 9
   <212> PRT
   <213> Artificial Sequence
<220>
   <223> Synthetic HLA-A2 binding nonamer peptide
<400> 683
   Lys Ile Leu Gly Lys Val Phe Thr Leu
   1 5
<210> 684
   <211> 9
   <212> PRT
   <213> Artificial Sequence
<220>
   <223> Synthetic HLA-A2 binding nonamer peptide
<400> 684
   Ala Ile Leu Leu Gly Val Phe Met Leu
   1 5
<210> 685
   <211> 9
   <212> PRT
   <213> Artificial Sequence
<220>
   <223> Synthetic HLA-A2 binding nonamer peptide
<400> 685
   Ala Ile Tyr Lys Arg Trp Ile Ile Leu
   1 5
<210> 686
   <211> 9
   <212> PRT
   <213> Artificial Sequence
<220>
   <223> synthetic HLA-A2 binding nonamer peptide
<400> 686
   Ala Leu Phe Phe Phe Asp Ile Asp Leu
   1 5
<210> 687
   <211> 9
   <212> PRT
   <213> Artificial Sequence
<220>
   <223> synthetic HLA-A2 binding nonamer peptide
<400> 687
   Ala Thr Val Glu Leu Leu Ser Glu Leu
   1 5
<210> 688
   <211> 9
   <212> PRT
   <213> Artificial Sequence
<220>
   <223> Synthetic HLA-A2 binding nonamer peptide
<400> 688
   Cys Leu Phe Gly Tyr Pro Val Tyr Val
   1 5
<210> 689
   <211> 9
   <212> PRT
   <213> Artificial sequence
<220>
   <223> Synthetic HLA-A2 binding nonamer peptide
<400> 689
   Phe Ile Phe Pro Asn Tyr Thr Ile Val
   1 5
<210> 690
   <211> 9
   <212> PRT
   <213> Artificial Sequence
<220>
   <223> Synthetic HLA-A2 binding nonamer peptide
<400> 690
   Ile Ile Ser Leu Trp Asp Ser Gln Leu
   1 5
<210> 691
   <211> 9
   <212> PRT
   <213> Artificial Sequence
<220>
   <223> Synthetic HLA-A2 binding nonamer peptide
<400> 691
   Ile Leu Ala Ser Leu Phe Ala Ala Val
   1 5
<210> 692
   <211> 9
   <212> PRT
   <213> Artificial sequence
<220>
   <223> Synthetic HLA-A2 binding nanamer peptide
<400> 692
   Ile Leu Glu Ser Leu Phe Ala Ala Val
   1 5
<210> 693
   <211> 9
   <212> PRT
   <213> Artificial Sequence
<220>
   <223> Synthetic HLA-A2 binding nonamer peptide
<400> 693
   Lys Leu Gly Glu Phe Phe Asn Gln Met
   1 5
<210> 694
   <211> 9
   <212> PRT
   <213> Artificial Sequence
<220>
   <223> Synthetic HLA-A2 binding nonamer peptide
<400> 694
   Lys Leu Gly Glu Phe Tyr Asn Gln Met
   1 5
<210> 695
   <211> 9
   <212> PRT
   <213> Artificial Sequence
<220>
   <223> Synthetic HLA-A2 binding nonamer peptide
<400> 695
   Leu Leu Phe Gly Tyr Pro Val Tyr Val
   1 5
<210> 696
   <211> 9
   <212> PRT
   <213> Artificial Sequence

<220>
   <223> Synthetic HLA-A2 binding nonamer peptide
<400> 696
   Leu Leu Trp Lys Gly Glu Gly Ala Val
   1 5
<210> 697
   <211> 9
   <212> PRT
   <213> Artificial Sequence
<220>
   <223> Synthetic HLA-A2 binding nonamer peptide
<400> 697
   Leu Met Phe Gly Tyr Pro Val Tyr Val
   1 5
<210> 698
   <211> 9
   <212> PRT
   <213> Artificial sequence
<220>
   <223> Synthetic HLA-A2 binding nonamer peptide
<400> 698
   Leu Asn Phe Gly Tyr Pro Val Tyr Val
   1 5
<210> 699
   <211> 9
   <212> PRT
   <213> Artificial Sequence
<220>
   <223> Synthetic HLA-A2 binding nonamer peptide
<400> 699
   Leu Gln Phe Gly Tyr Pro Val Tyr Val
   1 5
<210> 700
   <211> 9
   <212> PRT
   <213> Artificial sequence
<220>
   <223> Synthetic HLA-A2 binding nonamer peptide
<400> 700
   Asn Ile Val Ala His Thr Phe Lys Val
   1 5
<210> 701
   <211> 8
   <212> PRT
   <213> Artificial sequence
<220>
   <223> Synthetic HLA-A2 binding nonamer peptide
<400> 701
   Asn Leu Pro Met Val Ala Thr Val
   1 5
<210> 702
   <211> 9
   <212> PRT
   <213> Artificial Sequence
<220>
   <223> Synthetic HLA-A2 binding nonamer peptide
<400> 702
   Gln Met Leu Leu Ala Ile Ala Arg Leu
   1 5
<210> 703
   <211> 9
   <212> PRT
   <213> Artificial Sequence
<220>
   <223> synthetic HLA-A2 binding nonamer peptide
<400> 703
   Gln Met Trp Gln Ala Arg Leu Thr Val
   1 5
<210> 704
   <211> 9
   <212> PRT
   <213> Artificial Sequence
<220>
   <223> Synthetic HLA-A2 binding nonamer peptide
<400> 704
   Arg Leu Leu Gln Thr Gly Ile His Val
   1 5
<210> 705
   <211> 9
   <212> PRT
   <213> Artificial Sequence
<220>
   <223> Synthetic HLA-A2 binding nonamer peptide
<400> 705
   Arg Leu Val Asn Gly Ser Leu Ala Leu
   1 5
<210> 706
   <211> 9
   <212> PRT
   <213> Artificial Sequence
<220>
   <223> Synthetic HLA-A2 binding nonamer peptide
<400> 706
   Ser Leu Tyr Asn Thr Val Ala Thr Leu
   1 5
<210> 707
   <211> 9
   <212> PRT
   <213> Artificial Sequence
<220>
   <223> Synthetic HLA-A2 binding nonamer peptide
<400> 707
   Thr Leu Asn Ala Trp Val Lys Val Val
   1 5
<210> 708
   <211> 9
   <212> PRT
   <213> Artificial Sequence
<220>
   <223> Syntheti HLA-A2 binding nonamer peptide
<400> 708
   Trp Leu Tyr Arg Glu Thr Cys Asn Leu
   1 5
<210> 709
   <211> 9
   <212> PRT
   <213> Artificial Sequence
<220>
   <223> Synthetic HLA-A2 binding nonamer peptide
<400> 709
   Tyr Leu Phe Lys Arg Met Ile Asp Leu
   1 5
<210> 710
   <211> 9
   <212> PRT
   <213> Artificial Sequence
<220>
   <223> Synthetic HLA-A2 binding nonamer peptide
<400> 710
   Gly Ala Phe Gly Gly Val Gly Gly Val
   1 5
<210> 711
   <211> 9
   <212> PRT
   <213> Artificial Sequence
<220>
   <223> Synthetic HLA-A2 binding nonamer peptide
<400> 711
   Gly Ala Phe Gly Gly Val Gly Gly Tyr
   1 5
<210> 712
   <211> 9
   <212> PRT
   <213> Artificial Sequence
<220>
   <223> synthetic HLA-A2 binding nonamer peptide
<400> 712
   Gly Glu Phe Gly Gly Val Gly Gly Val
   1 5
<210> 713
   <211> 9
   <212> PRT
   <213> Artificial Sequence
<220>
   <223> Synthetic HLA-A2 binding nonamer peptide
<400> 713
   Gly Gly Phe Gly Gly Val Gly Gly Val
   1 5
<210> 714
   <211> 9
   <212> PRT
   <213> Artificial Sequence
<220>
   <223> Synthetic HLA-A2 binding nonamer peptide
<400> 714
   Gly Ile Phe Gly Gly Gly Gly Gly Val
   1 5
<210> 715
   <211> 9
   <212> PRT
   <213> Artificial Sequence
<220>
   <223> Synthetic HLA-A2 binding nonamer peptide
<400> 715
   Gly Ile Gly Gly Phe Gly Gly Gly Leu
   1 5
<210> 716
   <211> 9
   <212> PRT
   <213> Artificial Sequence
<220>
   <223> Synthetic HLA-A2 binding nonamer peptide
<400> 716
   Gly Ile Gly Gly Gly Gly Gly Gly Leu
   1 5
<210> 717
   <211> 9
   <212> PRT
   <213> Artificial sequence
<220>
   <223> synthetic HLA-A2 binding nonamer peptide
<400> 717
   Gly Leu Asp Gly Gly Gly Gly Gly Val
   1 5
<210> 718
   <211> 9
   <212> PRT
   <213> Artificial Sequence
<220>
   <223> Synthetic HLA-A2 binding nonamer peptide
<400> 718
   Gly Leu Asp Gly Lys Gly Gly Gly Val
   1 5
<210> 719
   <211> 9
   <212> PRT
   <213> Artificial Sequence
<220>
   <223> Synthetic. HLA-A2 binding nonamer peptide
<400> 719
   Gly Leu Asp Lys Lys Gly Gly Gly Val
   1 5
<210> 720
   <211> 9
   <212> PRT
   <213> Artificial Sequence
<220>
   <223> Synthetic HLA-A2 binding nonamer peptide
<400> 720
   Gly Leu Phe Gly Gly Gly Phe Gly Phe
   1 5
<210> 721
   <211> 9
   <212> PRT
   <213> Artificial Sequence
<220>
   <223> synthetic HLA-A2 binding nonamer peptide
<400> 721
   Gly Leu Phe Gly Gly Gly Phe Gly Gly
   1 5
<210> 722
   <211> 9
   <212> PRT
   <213> Artificial Sequence
<220>
   <223> synthetic HLA-A2 binding nonamer peptide
<400> 722
   Gly Leu Phe Gly Gly Gly Phe Gly Asn
   1 5
<210> 723
   <211> 9
   <212> PRT
   <213> Artificial Sequence
<220>
   <223> synthetic HLA-A2 binding nonamer peptide
<400> 723
   Gly Leu Phe Gly Gly Gly Phe Gly Ser
   1 5
<210> 724
   <211> 9
   <212> PRT
   <213> Artificial Sequence
<220>
   <223> synthetic HLA-A2 binding nonamer peptide
<400> 724
   Gly Leu Phe Gly Gly Gly Gly Gly Ile
   1 5
<210> 725
   <211> 9
   <212> PRT
   <213> Artificial Sequence
<220>
   <223> Synthetic HLA-A2 binding nonamer peptide
<400> 725
   Gly Leu Phe Gly Gly Gly Gly Gly Met
   1 5
<210> 726
   <211> 9
   <212> PRT
   <213> Artificial Sequence
<220>
   <223> Synthetic HLA-A2 binding nonamer peptide
<400> 726 Gly Leu Phe Gly Gly Gly Gly Gly Thr 1 5
<210> 727
   <211> 9
   <212> PRT
   <213> Artificial Sequence
<220>
   <223> Synthetic HLA-A2 binding nonamer peptide
<400> 727
   Gly Leu Phe Gly Gly Gly Gly Gly Tyr
   1 5
<210> 728
   <211> 9
   <212> PRT
   <213> Artificial Sequence
<220>
   <223> synthetic HLA-A2 binding nonamer peptide
<400> 728
   Gly Leu Gly Phe Gly Gly Gly Gly Val
   1 5
<210> 729
   <211> 9
   <212> PRT
   <213> Artificial Sequence
<220>
   <223> Synthetic HLA-A2 binding nonamer peptide
<400> 729
   Gly Leu Gly Gly Phe Gly Gly Gly Val
   1 5
<210> 730
   <211> 9
   <212> PRT
   <213> Artificial Sequence
<220>
   <223> Synthetic HLA-A2 binding nonamer peptide
<400> 730
   Gly Leu Gly Gly Gly Phe Gly Gly Val
   1 5
<210> 731
   <211> 9
   <212> PRT
   <213> Artificial Sequence
<220>
   <223> synthetic HLA-A2 binding nonamer peptide
<400> 731
   Gly Leu Gly Gly Gly Gly Gly Phe Val
   1 5
<210> 732
   <211> 9
   <212> PRT
   <213> Artificial Sequence
<220>
   <223> Synthetic HLA-A2 binding nonamer peptide
<400> 732
   Gly Leu Gly Gly Gly Gly Gly Gly Tyr
   1               5
<210> 733
   <211> 9
   <212> PRT
   <213> Artificial Sequence
<220>
   <223> Synthetic HLA-A2 binding nonamer peptide
<400> 733
   Gly Leu Gly Gly Gly Val Gly Gly Val
   1               5
<210> 734
   <211> 9
   <212> PRT
   <213> Artificial Sequence
<220>
   <223> Synthetic HLA-A2 binding nonamer peptide
<400> 734
   Gly Leu Leu Gly Gly Gly Gly Gly Val
   1               5
<210> 735
   <211> 9
   <212> PRT
   <213> Artificial Sequence
<220>
   <223> Synthetic HLA-A2 binding nonamer peptide

<400> 735
   Gly Leu Pro Gly Gly Gly Gly Gly Val
   1               5
<210> 736
   <211> 9
   <212> PRT
   <213> Artificial Sequence
<220>
   <223> Synthetic III-A A2 binding nonamer peptide
<400> 736
   Gly Asn Phe Gly Gly Val Gly Gly Val
   1               5
<210> 737
   <211> 9
   <212> PRT
   <213> Artificial Sequence
<220>
   <223> Synthetic HLA-A2 binding nonamer peptide
<400> 737
   Gly Ser Phe Gly Gly Val Gly Gly Val
   1 5
<210> 738
   <221> 9
   <212> PRT
   <213> Artificial Sequence
<220>
   <223> Synthetic HLA-A2 binding nonamer peptide
<400> 738
<210> 739
   <211> 9
   <212> PRT
   <213> Artificial Sequence
<220>
   <223> Synthetic HLA-A2 binding nonamer peptide
<400> 739
   Ala Gly Asn Ser Ala Tyr Glu Tyr Val
   1 5
<210> 740
   <211> 9
   <212> PRT
   <213> Artificial Sequence
<220>
   <223> Synthetic HLA-A2 binding nonamer peptide
<400> 740
   Gly Leu Phe Pro Gly Gln Phe Ala Tyr
   1 5
<210> 741
   <211> 9
   <212> PRT
   <213> Artificial Sequence
<220>
   <223> Synthetic HLA-A2 binding nonamer peptide
<400> 741
   His Ile Leu Leu Gly Val Phe Met Leu
   1               5
<210> 742
   <211> 9
   <212> PRT
   <213> Artificial Sequence
<220>
   <223> Synthetic HLA-A2 binding nonamer peptide
<400> 742
   Ile Leu Glu Ser Leu Phe Arg Ala Val
   1               5
<210> 743
   <211> 9
   <212> PRT
   <213> Artificial sequence
<220>
   <223> Synthetic HLA-A2 binding nonamer peptide
<400> 743
   Lys Lys Lys Tyr Lys Leu Lys His Ile
   1               5
<210> 744
   <211> 9
   <212> PRT
   <213> Artificial Sequence
<220>
   <223> synthetic HLA-A2 binding nonamer peptide
<400> 744
   Met Leu Ala Ser Ile Asp Leu Lys Tyr
   1               5
<210> 745
   <211> 9
   <212> PRT
   <213> Artificial Sequence
<220>
   <223> Synthetic HLA-A2 binding nonamer peptide
<400> 745
   Met Leu Glu Arg Glu Leu Val Arg Lys
   1               5
<210> 746
   <211> 9
   <212> PRT
   <213> Artificial Sequence
<220>
   <223> Synthetic HLA-A2 binding nonamer peptide.
<400> 746
   Lys Leu Phe Gly Phe Val Phe Thr Val
   1 5
<210> 747
   <211> 9
   <212> PRT
   <213> Artificial Sequence
<220>
   <223> Synthetic HLA-A2 binding nonamer peptide
<400> 747
   Ile Leu Asp Lys Lys Val Glu Lys Val
   1 5
<210> 748
   <211> 9
   <212> PRT
   <213> Artificial Sequence
<220>
   <223> Synthetic HLA-A2 binding nonamer peptide
<400> 748
   Ile Leu Lys Glu Pro Val His Gly Val
   1 5
<210> 749
   <211> 9
   <212> PRT
   <213> Artificial Sequence
<220>
   <223> Synthetic HLA-A2 binding nonamer peptide
<400> 749
   Ala Leu Phe Ala Ala Ala Ala Ala Tyr
   1 5
<210> 750
   <211> 9
   <212> PRT
   <213> Artificial sequence
<220>
   <223> synthetic HLA-A2 binding nonamer peptide
<400> 750
   Gly Ile Gly Phe Gly Gly Gly Gly Leu
   1 5
<210> 751
   <211> 9
   <212> PRT
   <213> Artificial Sequence
<220>
   <223> Synthetic HLA-A2 binding nonamer peptide
<400> 751
   Gly Lys Phe Gly Gly Val Gly Gly Val
   1 5
<210> 752
   <211> 9
   <212> PRT
   <213> Artificial Sequence
<220>
   <223> Synthetic HLA-A2 binding nonamer peptide
<400> 752
   Gly Leu Phe Gly Gly Gly Gly Gly Lys
   1 5
<210> 753
   <211> 9
   <212> PRT
   <213> Artificial Sequence
<220>
   <223> Synthetic HLA-A2 binding nonamer peptide
<400> 753
   Glu Ile Leu Gly Phe Val Phe Thr Leu
   1 5
<210> 754
   <211> 9
   <212> PRT
   <213> Artificial Sequence
<220>
   <223> Synthetic HLA-A2 binding nonamer peptide
<400> 754
   Gly Ile Lys Gly Phe Val Phe Thr Leu
   1 5
<210> 755
   <211> 9
   <212> PRT
   <213> Artificial Sequence
<220>
   <223> Synthetic HLA-A2 binding nonamer peptide
<400> 755
   Gly Gln Leu Gly Phe Val Phe Thr Lys
   1 5
<210> 756
   <211> 9
   <212> PRT
   <213> Artificial Sequence
<220>
   <223> Synthetic HLA-A2 binding nonamer peptide
<400> 756
   Ile Leu Gly Phe Val Phe Thr Leu Thr
   1 5
<210> 757
   <211> 9
   <212> PRT
   <213> Artificial Sequence
<220>
   <223> Synthetic HLA-A2 binding nonamer peptide
<400> 757
   Lys Ile Leu Gly Phe Val Phe Thr Lys
   1 5
<210> 758
   <211> 9
   <212> PRT
   <213> Artificial Sequence
<220>
   <223> Synthetic HLA-A2 binding nonamer peptide
<400> 758
   Lys Lys Leu Gly Phe Val Phe Thr Leu
   1 5
<210> 759
   <211> 9
   <212> PRT
   <213> Artificial Sequence
<220>
   <223> Synthetic HLA-A2 binding nonamer peptide
<400> 759
   Lys Leu Phe Glu Lys Val Tyr Asn Tyr
   1 5
<210> 760
   <211> 9
   <212> PRT
   <213> Artificial sequence
<220>
   <223> Synthetic HLA-A2 binding nonamer peptide
<400> 760
   Leu Arg Phe Gly Tyr Pro Val Tyr Val
   1 5
<210> 761
   <211> 9
   <212> PRT
   <213> Homo Sapien
<400> 761
   Ile Arg Arg Gly Val Met Leu Ala Val
   1 5
<210> 762
   <211> 9
   <212> PRT
   <213> Homo Sapien
<400> 762
   Lys Arg Ile Gln Glu Ile Ile Glu Gln
   1 5
<210> 763
   <211> 9
   <212> PRT
   <213> Homo Sapien
<400> 763
   Lys Arg Thr Leu Lys Ile Pro Ala Met
   1 5
<210> 764
   <211> 9
   <212> PRT
   <213> Yersenia pestis
<400> 764
   Gly Arg Asn Val Val Leu Asp Lys Ser
   1 5
<210> 765
   <211> 9
   <212> PRT
   <213> Yersenia Pestis
<400> 765
   Lys Arg Gly Ile Asp Lys Ala Val Ile
   1 5
<210> 766
   <211> 9
   <212> PRT
   <213> Yersenia Pestis
<400> 766
   Ile Arg Ala Ala Ser Ala Ile Thr Ala
   1 5
<210> 767
   <211> 9
   <212> PRT
   <213> Yersenia Pestis
<400> 767
   Arg Arg Lys Ala Met Phe Glu Asp Ile
   1 5
<210> 768
   <211> 8
   <212> PRT
   <213> Plasmodium Falciparum
<400> 768
   Lys Pro Lys Asp Glu Leu Asp Tyr
   1 5
<210> 769
   <211> 9
   <212> PRT
   <213> influenza
<400> 769
   Leu Glu Leu Arg Ser Arg Tyr Trp Ala
   1 5
<210> 770
   <211> 10
   <212> PRT
   <213> Homo Sapien
<400> 770
   Gly Pro Pro His Ser Asn Asn Phe Gly Tyr
   1 5 10
<210> 771
   <211> 8
   <212> PRT
   <213> Rotavirus
<400> 771
   Ile Ile Tyr Arg Phe Leu Leu Ile
   1 5
<210> 772
   <211> 9
   <212> PRT
   <213> Homo sapien
<400> 772
   Gln Leu Ser Pro Tyr Pro Phe Asp Leu
   1 5
<210> 773
   <211> 8
   <212> PRT
   <213> Homo sapien
<400> 773
   Val Tyr Asp Phe Phe Val Trp Leu
   1 5
<210> 774
   <211> 8
   <212> PRT
   <213> Homo Sapien
<400> 774
   Ser Asn Phe Val Phe Ala Gly Ile
   1 5
<210> 775
   <211> 8
   <212> PRT
   <213> Rotavirus
<400> 775
   Ser Val Val Glu Phe Ser Ser Leu
   1 5
<210> 776
   <211> 8
   <212> PRT
   <213> Artificial sequence
<220>
   <223> Epitope mimic of natural tumor Ag
<400> 776
   Ala His Tyr Leu Phe Arg Asn Leu
   1 5
<210> 777
   <211> 8
   <212> PRT
   <213> Artificial sequence
<220>
   <223> Epitope mimic of natural tumor Ag
<400> 777
   Thr His Tyr Leu Phe Arg Asn Leu
   1 5
<210> 778
   <211> 8
   <212> PRT
   <213> Artificial Sequence
<220>
   <223> Epitope Mimic of H-3 miHAg
<400> 778
   Leu Ile Val Ile Tyr Asn Thr Leu
   1 5
<210> 779
   <211> 8
   <212> PRT
   <213> Artificial Sequence
<220>
   <223> Epitope Mimic of H-3 miHAg
<400> 779
   Leu Ile Tyr Glu Phe Asn Thr Leu
   1 5
<210> 780
   <211> 8
   <212> PRT
   <213> Artificial sequence
<220>
   <223> Epitope Mimic of H-3 miHAg
<400> 780
   Ile Pro Tyr Ile Tyr Asn Thr Leu
   1               5
<210> 781
   <211> 8
   <212> PRT
   <213> Artificial Sequence
<220>
   <223> Epitope Mimic of H-3 miHAg

<400> 781
   Ile Ile Tyr Ile Tyr His Arg Leu
   1               5
<210> 782
   <211> 8
   <212> PRT
   <213> Artificial Sequence
<220>
   <223> Epitope Mimic of H-3 miHAg
<400> 782
   Leu Ile Tyr Ile Phe Asn Thr Leu
   1               5
<210> 783
   <211> 8
   <212> PRT
   <213> Hepatitis B Virus
<400> 783
   Met Gly Leu Lys Phe Arg Gln Leu
   1               5
<210> 784
   <211> 9
   <212> PRT
   <213> Homo Sapien
<400> 784
   Ile Met Ile Lys Phe Arg Asn Arg Leu
   1               5
<210> 785
   <211> 9
   <212> PRT
   <213> Mus Musculus
<400> 785
   Trp Met His His Asn Met Asp Leu Ile
   1 5
<210> 786
   <211> 9
   <212> PRT
   <213> Mus Musculus
<400> 786
   Lys Tyr Met Cys Asn Ser Ser Cys Met
   1 5
<210> 787
   <211> 9
   <212> PRT
   <213> Mus Musculus
<400> 787
   Gly Arg Pro Lys Asn Gly Cys Ile Val
   1 5
<210> 788
   <211> 9
   <212> PRT
   <213> Artificial Sequence
<220>
   <223> Epitope mimic of natural tumor Ag
<400> 788
   Ala Gln His Pro Asn Ala Glu Leu Leu
   1 5
<210> 789
   <211> 9
   <212> PRT
   <273> Murine Leukemia virus
<400> 789
   Cys Cys Leu Cys Leu Thr Val Phe Leu
   1 5
<210> 790
   <211> 8
   <212> PRT
   <213> plasmodium Falciparum
<400> 790
   Tyr Glu Asn Asp Ile Glu Lys Lys
   1 5
<210> 791
   <211> 9
   <212> PRT
   <213> Plasmodium Falciparum
<400> 791
   Asp Glu Leu Asp Tyr Glu Asn Asp Ile
   1 5
<210> 792
   <211> 9
   <212> PRT
   <213> Human Immunodeficiency Virus
<400> 792
   Thr Glu Met Glu Lys Glu Gly Lys Ile
   1 5
<210> 793
   <211> 9
   <212> PRT
   <213> Rabies
<400> 793
   Val Glu Ala Glu Ile Ala His Gln Ile
   1 5
<210> 794
   <211> 9
   <212> PRT
   <213> Influenza A
<400> 794
   Glu Glu Gly Ala Ile Val Gly Glu Ile
   1 5
<210> 795
   <211> 8
   <212> PRT
   <213> Mus Musculus
<400> 795
   Thr Glu Asn Ser Gly Lys Asp Ile
   1 5
<210> 796
   <211> 9
   <212> PRT
   <213> Artificial Sequence
<220>
   <223> MHC Class I Leader
<400> 796
   Ala Met Ala Pro Arg Thr Leu Leu Leu
   1 5
<210> 797
   <211> 11
   <212> PRT
   <213> Homo Sapien
<400> 797
   Phe Phe Ile Asn Ile Leu Thr Leu Leu Val Pro
   1 5 10
<210> 798
   <211> 11
   <212> PRT
   <213> Homo Sapien
<400> 798
   Phe Phe Ile Asn Ile Leu Thr Leu Leu Val Pro
   1 5 10
<210> 799
   <211> 16
   <212> PRT
   <213> Homo Sapien
<400> 799
   Phe Phe Ile Asn Ile Leu Thr Leu Leu Val Pro Ile Leu Ile Ala Met
   1 5 10 15
<210> 800
   <211> 16
   <212> PRT
   <213> Homo Sapien
<400> 800
   Phe Phe Ile Asn Ala Leu Thr Leu Leu Val Pro Ile Leu Ile Ala Met
   1 5 10 15
<210> 801
   <211> 5
   <212> PRT
   <213> Homo Sapien
<400> 801
   Phe Ile Asn Arg Trp
   1 5
<210> 802
   <211> 5
   <212> PRT
   <213> Listeria Monocytogenes
<400> 802
   Ile Gly Trp Ile Ile
   1 5
<210> 803
   <211> 12
   <212> PRT
   <213> simian Immunodeficiency Virus
<400> 803
   Glu Gly Cys Thr Pro Tyr Asp Ile Asn Gln Met Leu
   1 5 10
<210> 804
   <211> 9
   <212> PRT
   <213> Homo Sapien
<400> 804
   Ala Leu Ser Arg Lys Val Ala Glu Leu
   1 5
<210> 805
   <211> 9
   <212> PRT
   <213> Homo sapien
<400> 805
   Ile Met Pro Lys Ala Gly Leu Leu Ile
   1 5
<210> 806
   <211> 9
   <212> PRT
   <213> Homo Sapien
<400> 806
   Lys Ile Trp Glu Glu Leu Ser Val Leu
   1 5
<210> 807
   <211> 10
   <212> PRT
   <213> Homo Sapien
<400> 807
   Ala Leu Val Glu Thr Ser Tyr Val Lys Val
   1 5 10
<210> 808
   <211> 10
   <212> PRT
   <213> Homo Sapien
<400> 808
   Thr Leu Val Glu Val Thr Leu Gly Glu Val
   1 5 10
<210> 809
   <211> 9
   <212> PRT
   <213> Homo Sapien
<400> 809
   Ala Leu Ser Arg Lys Val Ala Glu Leu
   1 5
<210> 810
   <211> 9
   <212> PRT
   <213> Homo Sapien
<400> 810
   Ile Met Pro Lys Ala Gly Leu Leu Ile
   1 5
<210> 811
   <211> 9
   <212> PRT
   <213> Homo Sapien
<400> 811
   Lys Ile Trp Glu Glu Leu Ser Val Leu
   1 5
<210> 812
   <211> 10
   <212> PRT
   <213> Homo sapien
<400> 812
   Ala Leu Val Glu Thr Ser Tyr Val Lys Val
   1 5 10
<210> 813
   <211> 12
   <212> PRT
   <213> Homo Sapien
<400> 813
   Lys Gly Ile Leu Gly Phe Val Phe Thr Leu Thr Val
   1 5 10
<210> 814
   <211> 9
   <212> PRT
   <213> Homo Sapien
<400> 814
   Gly Ile Ile Gly Phe Val Phe Thr Ile
   1 5
<210> 815
   <211> 9
   <212> PRT
   <213> Homo Sapien
<400> 815
   Gly Ile Ile Gly Phe Val Phe Thr Leu
   1 5
<210> 816
   <211> 9
   <212> PRT
   <213> Homo Sapien
<400> 816
   Gly Ile Leu Gly Phe Val Phe Thr Leu
   1 5
<210> 817
   <211> 9
   <212> PRT
   <213> Homo Sapien
<400> 817
   Gly Leu Leu Gly Phe Val Phe Thr Leu
   1 5
<210> 818
   <211> 9
   <212> PRT
   <213> Homo Sapien
<220>
   <221> VARIANT
   <222> 1, 2, 5, 6, 9
   <223> Xaa = Any Amino Acid
<400> 818
   Xaa Xaa Thr Val Xaa Xaa Gly Val Xaa
   1 5
<210> 819
   <211> 9
   <212> PRT
   <213> Homo Sapien
<400> 819
   Ile Leu Thr Val Ile Leu Gly Val Leu
   1 5
<210> 820
   <211> 9
   <212> PRT
   <213> Homo Sapien
<400> 820
   Tyr Leu Glu Pro Gly Pro Val Thr Ala
   1 5
<210> 821
   <211> 10
   <212> PRT
   <213> Homo Sapien
<400> 821
   Gln Val Pro Leu Arg Pro Met Thr Tyr Lys
   1 5 10
<210> 822
   <211> 11
   <212> PRT
   <213> Homo Sapien
<400> 822
   Asp Gly Leu Ala Pro Pro Gln His Leu Ile Arg
   1 5 10
<210> 823
   <211> 9
   <212> PRT
   <213> Homo Sapien
<400> 823
   Leu Leu Gly Arg Asn Ser Phe Glu Val
   1 5
<210> 824
   <211> 16
   <212> PRT
   <213> Homo Sapien
<400> 824
   Glu His Ser Ala Tyr Gly Glu Pro Arg Lys Leu Leu Thr Gln Asp Leu
   1 5 10 15
<210> 825
   <211> 12
   <212> PRT
   <213> Homo Sapien
<400> 825
   Glu His Ser Ala Tyr Gly Glu Pro Arg Lys Leu Leu
   1 5 10
<210> 826
   <211> 9
   <212> PRT
   <213> Homo Sapien
<400> 826
   Ser Ala Tyr Gly Glu Pro Arg Lys Leu
   1 5
<210> 827
   <211> 8
   <212> PRT
   <213> Homo Sapien
<400> 827
   Tyr Arg Pro Arg Pro Arg Arg Tyr
   1 5
<210> 828
   <211> 9
   <212> PRT
   <213> Homo Sapien
<400> 828
   Thr Tyr Arg Pro Arg Pro Arg Arg Tyr
   1 5
<210> 829
   <211> 9
   <212> PRT
   <213> Homo Sapien
<400> 829
   Tyr Arg Pro Arg Pro Arg Arg Tyr Val
   1 5
<210> 830
   <211> 10
   <212> PRT
   <213> Homo Sapien
<400> 830
   Thr Tyr Arg Pro Arg Pro Arg Arg Tyr Val
   1 5 10
<210> 831
   <211> 9
   <212> PRT
   <213> Homo Sapien
<400> 831
   Arg Pro Arg Pro Arg Arg Tyr Val Glu
   1 5
<210> 832
   <211> 15
   <212> PRT
   <213> Homo Sapien
<400> 832
   Met Ser Trp Arg Gly Arg Ser Thr Tyr Arg Pro Arg Pro Arg Arg
   1 5 10 15
<210> 833
   <211> 16
   <212> PRT
   <213> Homo Sapien
<400> 833
   Thr Tyr Arg Pro Arg Pro Arg Arg Tyr Val Glu Pro Pro Glu Met Ile
   1 5 10 15
<210> 834
   <211> 3
   <212> PRT
   <213> Homo Sapien
<400> 834 Glu Asp Tyr 1
<210> 835
   <211> 9
   <212> PRT
   <213> Homo Sapien
<400> 835
   Glu Val Val Pro Ile Ser His Leu Tyr
   1 5
<210> 836
   <211> 9
   <212> PRT
   <213> Homo Sapien
<400> 836
   Glu Val Val Arg Ile Gly His Leu Tyr
   1 5
<210> 837
   <211> 9
   <212> PRT
   <213> Homo Sapien
<400> 837
   Glu Val Asp Pro Ile Gly His Leu Tyr
   1 5
<210> 838
   <211> 9
   <212> PRT
   <213> Homo Sapien
<400> 838
   Glu Val Asp Pro Ala Ser Asn Thr Tyr
   1 5
<210> 839
   <211> 9
   <212> PRT
   <213> Homo Sapien
<400> 839
   Glu Val Asp Pro Thr Ser Asn Thr Tyr
   1 5
<210> 840
   <211> 9
   <212> PRT
   <213> Homo Sapien
<400> 840
   Glu Ala Asp Pro Thr Ser Asn Thr Tyr
   1 5
<210> 841
   <211> 9
   <212> PRT
   <213> Homo Sapien
<400> 841
   Glu Val Asp Pro Ile Gly His Val Tyr
   1 5
<210> 842
   <211> 27
   <212> DNA
   <213> Homo Sapien
<400> 842
   gaagtggtcc ccatcagcca cttgtac 27
<210> 843
   <211> 27
   <212> DNA
   <213> Homo Sapien
<400> 843
   gaagtggtcc gcatcggcca cttgtac 27
<210> 844
   <211> 27
   <212> DNA
   <213> Homo Sapien
<400> 844
   gaagtggacc ccatcggcca cttgtac 27
<210> 845
   <211> 27
   <212> DNA
   <213> Homo Sapien
<400> 845
   gaagtggacc ccgccagcaa cacctac 27
<210> 846
   <211> 27
   <212> DNA
   <213> Homo Sapien
<400> 846
   gaagtggacc ccaccagcaa cacctac 27
<210> 847
   <211> 27
   <212> DNA
   <213> Homo Sapien
<400> 847
   gaagcggacc ccaccagcaa cacctac 27
<210> 848
   <211> 27
   <212> DNA
   <213> Homo sapien
<400> 848
   gaagcggacc ccaccagcaa cacctac 27

<210> 849
   <211> 27
   <212> DNA
   <213> Homo sapien
<400> 849
   gaagtggacc ccatcggcca cgtgtac 27
<210> 850
   <211> 8
   <212> PRT
   <213> Homo Sapien
<400> 850
   Glu Ala Asp Pro Thr Gly His Ser
   1 5
<210> 851
   <211> 8
   <212> PRT
   <213> Homo Sapien
<400> 851
   Ala Asp Pro Trp Gly His Ser Tyr
   1 5
<210> 852
   <211> 11
   <212> PRT
   <213> Homo sapien
<400> 852
   Ser Thr Leu Val Glu Val Thr Leu Gly Glu Val
   1 5 10
<210> 853
   <211> 9
   <212> PRT
   <213> Homo Sapien
<400> 853
   Leu Val Glu Val Thr Leu Gly Glu Val
   1 5
<210> 854
   <211> 9
   <212> PRT
   <213> Homo Sapien
<400> 854
   Lys Met Val Glu Leu Val His Phe Leu
   1 5
<210> 855
   <211> 10
   <212> PRT
   <213> Homo Sapien
<400> 855
   Val Ile Phe Ser Lys Ala Ser Glu Tyr Leu
   1 5 10
<210> 856
   <211> 10
   <212> PRT
   <213> Homo Sapien
<400> 856
   Tyr Leu Gln Leu Val Phe Gly Ile Glu Val
   1               5                   10
<210> 857
   <211> 9
   <212> PRT
   <213> Homo Sapien
<400> 857
   Gln Leu Val Phe Gly Ile Glu Val Val
   1               5
<210> 858
   <211> 11
   <212> PRT
   <213> Homo Sapien
<400> 858
   Gln Leu Val Phe Gly Ile Glu Val Val Glu Val
   1               5                   10
<210> 859
   <211> 9
   <212> PRT
   <213> Homo Sapien
<400> 859
   Ile Ile Val Leu Ala Ile Ile Ala Ile
   1               5
<210> 860
   <211> 11
   <212> PRT
   <213> Homo Sapien
<400> 860
   Lys Ile Trp Glu Glu Leu Ser Met Leu Glu Val
   1               5                   10
<210> 861
   <211> 10
   <212> PRT
   <213> Homo Sapien
<400> 861
   Ala Leu Ile Glu Thr Ser Tyr Val Lys Val
   1               5                   10
<210> 862
   <211> 9
   <212> PRT
   <213> Homo Sapien
<400> 862
   Leu Ile Glu Thr Ser Tyr Val Lys Val
   1               5
<210> 863
   <211> 11
   <212> PRT
   <213> Homo Sapien
<400> 863
   Gly Leu Glu Ala Arg Gly Glu Ala Leu Gly Leu
   1               5                   10
<210> 864
   <211> 9
   <212> PRT
   <213> Homo Sapien
<400> 864
   Gly Leu Glu Ala Arg Gly Glu Ala Leu
   1               5
<210> 865
   <211> 9
   <212> PRT
   <213> Homo Sapien
<400> 865
   Ala Leu Gly Leu Val Gly Ala Gln Ala
   1               5
<210> 866
   <211> 9
   <212> PRT
   <213> Homo sapien
<400> 866
   Gly Leu Val Gly Ala Gln Ala Pro Ala
   1               5
<210> 867
   <211> 9
   <212> PRT
   <213> Homo Sapien
<400> 867
   Asp Leu Glu Ser Glu Phe Gln Ala Ala
   1 5
<210> 868
   <211> 10
   <212> PRT
   <213> Homo Sapien
<400> 868
   Asp Leu Glu Ser Glu Phe Gln Ala Ala Ile
   1 5 10
<210> 869
   <211> 10
   <212> PRT
   <213> Homo Sapien
<400> 869
   Ala Ile Ser Arg Lys Met Val Glu Leu Val
   1 5 10
<210> 870
   <211> 9
   <212> PRT
   <213> Homo Sapien
<400> 870
   Ala Ile Ser Arg Lys Met Val Glu Leu
   1 5
<210> 871
   <211> 10
   <212> PRT
   <213> Homo Sapien
<400> 871
   Lys Met Val Glu Leu Val His Phe Leu Leu
   1 5 10
<210> 872
   <211> 11
   <212> PRT
   <213> Homo Sapien
<400> 872
   Lys Met Val Glu Leu Val His Phe Leu Leu Leu
   1 5 10
<210> 873
   <211> 11
   <212> PRT
   <213> Homo Sapien
<400> 873
   Leu Leu Leu Lys Tyr Arg Ala Arg Glu Pro Val
   1 5 10
<210> 874
   <211> 10
   <212> PRT
   <213> Homo Sapien
<400> 874
   Leu Leu Lys Tyr Arg Ala Arg Glu Pro Val
   1 5 10
<210> 875
   <211> 11
   <212> PRT
   <213> Homo Sapien
<400> 875
   Val Leu Arg Asn Cys Gln Asp Phe Phe Pro Val
   1 5 10
<210> 876
   <211> 11
   <212> PRT
   <213> Homo Sapien
<400> 876
   Tyr Leu Gln Leu Val Phe Gly Ile Glu Val Val
   1 5 10
<210> 877
   <211> 10
   <212> PRT
   <213> Homo Sapien
<400> 877
   Gly Ile Glu Val Val Glu Val Val Pro Ile
   1 5 10
<210> 878
   <211> 9
   <212> PRT
   <213> Homo Sapien
<400> 878
   Pro Ile Ser His Leu Tyr Ile Leu Val
   1 5
<210> 879
   <211> 9
   <212> PRT
   <213> Homo Sapien
<400> 879
   His Leu Tyr Ile Leu Val Thr Cys Leu
   1 5
<210> 880
   <211> 11
   <212> PRT
   <213> Homo Sapien
<400> 880
   His Leu Tyr Ile Leu Val Thr Cys Leu Gly Leu
   1 5 10
<210> 881
   <211> 9
   <212> PRT
   <213> Homo Sapien
<400> 881
   Tyr Ile Leu Val Thr Cys Leu Gly Leu
   1 5
<210> 882
   <211> 9
   <212> PRT
   <213> Homo Sapien
<400> 882
   Cys Leu Gly Leu Ser Tyr Asp Gly Leu
   1 5
<210> 883
   <211> 10
   <212> PRT
   <213> Homo Sapien
<400> 883
   Cys Leu Gly Leu Ser Tyr Asp Gly Leu Leu
   1 5 10
<210> 884
   <211> 9
   <212> PRT
   <213> Homo Sapien
<400> 884
   Val Met Pro Lys Thr Gly Leu Leu Ile
   1 5
<210> 885
   <211> 10
   <212> PRT
   <213> Homo Sapien
<400> 885
   Val Met Pro Lys Thr Gly Leu Leu Ile Ile
   1 5 10
<210> 886
   <211> 11
   <212> PRT
   <213> Homo Sapien
<400> 886
   Val Met Pro Lys Thr Gly Leu Leu Ile Ile Val
   1 5 10
<210> 887
   <211> 9
   <212> PRT
   <213> Homo Sapien
<400> 887
   Gly Leu Leu Ile Ile Val Leu Ala Ile
   1 5
<210> 888
   <211> 10
   <212> PRT
   <213> Homo Sapien
<400> 888
   Gly Leu Leu Ile Ile Val Leu Ala Ile Ile
   1 5 10
<210> 889
   <211> 11
   <212> PRT
   <213> Homo Sapien
<400> 889
   Gly Leu Leu Ile Ile Val Leu Ala Ile Ile Ala
   1 5 10
<210> 890
   <211> 9
   <212> PRT
   <213> Homo Sapien
<400> 890
   Leu Leu Ile Ile Val Leu Ala Ile Ile
   1               5
<210> 891
   <211> 10
   <212> PRT
   <213> Homo Sapien
<400> 891
   Leu Leu Ile Ile Val Leu Ala Ile Ile Ala
   1               5                   10
<210> 892
   <211> 11
   <212> PRT
   <213> Homo Sapien
<400> 892
   Leu Leu Ile Ile Val Leu Ala Ile Ile Ala Ile
   1               5                   10
<210> 893
   <211> 9
   <212> PRT
   <213> Homo Sapien
<400> 893
   Leu Ile Ile Val Leu Ala Ile Ile Ala
   1               5
<210> 894
   <211> 10
   <212> PRT
   <213> Homo Sapien
<400> 894
   Leu Ile Ile Val Leu Ala Ile Ile Ala Ile
   1               5                   10
<210> 895
   <211> 9
   <212> PRT
   <213> Homo Sapien
<400> 895
   Ile Ile Ala Ile Glu Gly Asp Cys Ala
   1               5
<210> 896
   <211> 9
   <212> PRT
   <213> Homo Sapien
<400> 896
   Lys Ile Trp Glu Glu Leu Ser Met Leu
   1               5
<210> 897
   <211> 11
   <212> PRT
   <213> Homo Sapien
<400> 897
   Leu Met Gln Asp Leu Val Gln Glu Asn Tyr Leu
   1               5                   10
<210> 898
   <211> 9
   <212> PRT
   <213> Homo Sapien

<400> 898
   Phe Leu Trp Gly Pro Arg Ala Leu Ile
   1               5
<210> 899
   <211> 9
   <212> PRT
   <213> Homo Sapien
<400> 899
   Leu Ile Glu Thr Ser Tyr Val Lys Val
   1               5
<210> 900
   <211> 11
   <212> PRT
   <213> Homo Sapien
<400> 900
   Ala Leu Ile Glu Thr Ser Tyr Val Lys Val Leu
   1               5                   10
<210> 901
   <211> 10
   <212> PRT
   <213> Homo Sapien
<400> 901
   Thr Leu Lys Ile Gly Gly Glu Pro His Ile
   1               5                   10
<210> 902
   <211> 11
   <212> PRT
   <213> Homo Sapien
<400> 902.
   His Ile Ser Tyr Pro Pro Leu His Glu Arg Ala
   1 5 10
<210> 903
   <211> 9
   <212> PRT
   <213> Homo Sapien
<400> 903
   Gln Thr Ala Ser Ser Ser Ser Thr Leu
   1 5
<210> 904
   <211> 10
   <212> PRT
   <213> Homo Sapien
<400> 904
   Gln Thr Ala Ser Ser Ser Ser Thr Leu Val
   1 5 10
<210> 905
   <211> 9
   <212> PRT
   <213> Homo Sapien
<400> 905
   Val Thr Leu Gly Glu Val Pro Ala Ala
   1 5
<210> 906
   <211> 10
   <212> PRT
   <213> Homo Sapien
<400> 906
   Val Thr Lys Ala Glu Met Leu Glu Ser Val
   1 5 10
<210> 907
   <211> 11
   <212> PRT
   <213> Homo Sapien
<400> 907
   Val Thr Lys Ala Glu Met Leu Glu Ser Val Leu
   1 5 10
<210> 908
   <211> 11
   <212> PRT
   <213> Homo Sapien
<400> 908
   Val Thr Cys Leu Gly Leu Ser Tyr Asp Gly Leu
   1 5 10
<210> 909
   <211> 9
   <212> PRT
   <213> Homo Sapien
<400> 909
   Lys Thr Gly Leu Leu Ile Ile Val Leu
   1 5
<210> 910
   <211> 10
   <212> PRT
   <213> Homo Sapien
<400> 910
   Lys Thr Gly Leu Leu Ile Ile Val Leu Ala
   1 5 10
<210> 911
   <211> 11
   <212> PRT
   <213> Homo Sapien
<400> 911
   Lys Thr Gly Leu Leu Ile Ile Val Leu Ala Ile
   1 5 10
<210> 912
   <211> 11
   <212> PRT
   <213> Homo Sapien
<400> 912
   His Thr Leu Lys Ile Gly Gly Glu Pro His Ile
   1 5 10
<210> 913
   <211> 9
   <212> PRT
   <213> Homo Sapien
<400> 913
   Met Leu Asp Leu Gln Pro Glu Thr Thr
   1 5
<210> 914
   <211> 9
   <212> PRT
   <213> Homo Sapien
<400> 914
   Gly Leu Glu Ala Arg Gly Glu Ala Leu
   1 5
<210> 915
   <211> 9
   <212> PRT
   <213> Homo sapien
<400> 915
   Ala Leu Ser Arg Lys Val Ala Glu Leu
   1 5
<210> 916
   <211> 9
   <212> PRT
   <213> Homo Sapien
<400> 916
   Phe Leu Trp Gly Pro Arg Ala Leu Val
   1 5
<210> 917
   <211> 10
   <212> PRT
   <213> Homo Sapien
<400> 917
   Thr Leu Val Glu Val Thr Leu Gly Glu Val
   1 5 10
<210> 918
   <211> 10
   <212> PRT
   <213> Homo Sapien
<400> 918
   Ala Leu Ser Arg Lys Val Ala Glu Leu Val
   1 5 10
<210> 919
   <211> 10
   <212> PRT
   <213> Homo Sapien
<400> 919
   Ala Leu Val Glu Thr Ser Tyr Val Lys Val
   1 5 10
<210> 920
   <211> 9
   <212> PRT
   <213> Homo Sapien
<400> 920
   Tyr Met Asn Gly Thr Met Ser Gln Val
   1 5
<210> 921
   <211> 10
   <212> PRT
   <213> Homo Sapien
<400> 921
   Met Leu Leu Ala Val Leu Tyr Cys Leu Leu
   1 5 10
<210> 922
   <211> 9
   <212> PRT
   <213> Homo Sapien
<400> 922
   Met Leu Leu Ala Val Leu Tyr Cys Leu
   1 5
<210> 923
   <211> 9
   <212> PRT
   <213> Homo Sapien
<400> 923
   Leu Leu Ala Val Leu Tyr Cys Leu Leu
   1 5
<210> 924
   <211> 13
   <212> PRT
   <213> Homo Sapien
<400> 924
   Ser Glu Ile Trp Arg Asp Ile Asp Phe Ala His Glu Ala
   1 5 10
<210> 925
   <211> 9
   <212> PRT
   <213> Homo Sapien
<400> 925
   Ser Glu Ile Trp Arg Asp Ile Asp Phe
   1 5
<210> 926
   <211> 10
   <212> PRT
   <213> Homo Sapien
<400> 926
   Glu Glu Asn Leu Leu Asp Phe Val Arg Phe
   1 5 10
<210> 927
   <211> 10
   <212> PRT
   <213> Homo Sapien
<400> 927
   Glu Ala Ala Gly Ile Gly Ile Leu Thr Val
   1 5 10
<210> 928
   <211> 9
   <212> PRT
   <213> Homo Sapien
<400> 928
   Met Leu Leu Ala Val Leu Tyr Cys Leu
   1 5
<210> 929
   <211> 9
   <212> PRT
   <213> Homo sapien
<400> 929
   Tyr Met Asp Gly Thr Met Ser Gln Val
   1 5
<210> 930
   <211> 9
   <212> PRT
   <213> Homo Sapien
<400> 930
   Tyr Leu Glu Pro Gly Pro Val Thr Ala
   1 5
<210> 931
   <211> 10
   <212> PRT
   <213> Homo Sapien
<400> 931
   Leu Leu Asp Gly Thr Ala Thr Leu Arg Leu
   1 5 10
<210> 932
   <211> 9
   <212> PRT
   <213> Homo Sapien
<400> 932
   Gly Ile Leu Gly Phe Val Phe Thr Leu
   1 5
<210> 933
   <211> 9
   <212> PRT
   <213> Homo Sapien
<400> 933
   Glu Ala Asp Pro Thr Gly His Ser Tyr
   1 5
<210> 934
   <211> 9
   <212> PRT
   <213> Homo Sapien
<400> 934
   Glu Ala Asp Pro Thr Gly His Ser Tyr
   1 5
<210> 935
   <211> 22
   <212> PRT
   <213> Homo Sapien
<400> 935
   Met Ala Ala Arg Ala Val Phe Leu Ala Leu Ser Ala Gln Leu Leu Gln
   1 5 10 15
   Ala Arg Leu Met Lys Glu
   20
<210> 936
   <211> 16
   <212> PRT
   <213> Homo Sapien
<400> 936
   Met Ala Ala Arg Ala Val Phe Leu Ala Leu Ser Ala Gln Leu Leu Gln
   1 5 10 15
<210> 937
   <211> 9
   <212> PRT
   <213> Homo Sapien
<400> 937
   Ala Ala Arg Ala Val Phe Leu Ala Leu
   1 5
<210> 938
   <211> 9
   <212> PRT
   <213> Influenza
<400> 938
   Ile Tyr Gln Arg Ile Arg Ala Leu Val
   1 5
<210> 939
   <211> 9
   <212> PRT
   <213> Homo Sapien
<400> 939
   Ser Tyr Phe Pro Glu Ile Thr His Ile
   1 5
<210> 940
   <211> 9
   <212> PRT
   <213> Influenza
<400> 940
   Ile Tyr Ala Thr Val Ala Gly Ser Leu
   1 5
<210> 941
   <211> 9
   <212> PRT
   <213> Influenza
<400> 941
   Val Tyr Gln Ile Leu Ala Ile Tyr Ala
   1 5
<210> 942
   <211> 9
   <212> PRT
   <213> Influenza
<400> 942
   Ile Tyr Ser Thr Val Ala Ser Ser Leu
   1 5
<210> 943
   <211> 9
   <212> PRT
   <213> Influenza
<400> 943
   Leu Tyr Gln Asn Val Gly Thr Tyr Val
   1 5
<210> 944
   <211> 9
   <212> PRT
   <213> Homo Sapien
<400> 944
   Arg Tyr Leu Glu Asn Gln Lys Arg Thr
   1 5
<210> 945
   <211> 9
   <212> PRT
   <213> Homo Sapien
<400> 945
   Arg Tyr Leu Lys Asn Gly Lys Glu Thr
   1 5
<210> 946
   <211> 9
   <212> PRT
   <213> Homo Sapien
<400> 946
   Lys Tyr Gln Ala Val Thr Thr Thr Leu
   1 5
<210> 947
   <211> 9
   <212> PRT
   <213> Plasmodium Berghei
<400> 947
   Ser Tyr Ile Pro Ser Ala Glu Lys Ile
   1 5
<210> 948
   <211> 9
   <212> PRT
   <213> Plasmodium Yoelii
<400> 948
   Ser Tyr Val Pro Ser Ala Phe Gln Ile
   1 5
<210> 949
   <211> 8
   <212> PRT
   <213> Vesicular Stomatitis virus
<400> 949
   Arg Gly Tyr Val Tyr Gln Gly Leu
   1 5
<210> 950
   <211> 8
   <212> PRT
   <213> Gallus Domesticus
<400> 950
   Ser Ile Ile Asn Phe Glu Lys Leu
   1 5
<210> 951
   <211> 8
   <212> PRT
   <213> Sendai virus
<400> 951
   Ala Pro Gly Asn Tyr Pro Ala Leu
   1 5
<210> 952
   <211> 9
   <212> PRT
   <213> Homo Sapien
<400> 952
   Val Pro Tyr Gly Ser Phe Lys His Val
   1 5
<210> 953
   <211> 9
   <212> PRT
   <213> Influenza
<400> 953
   Thr Tyr Gln Arg Thr Arg Ala Leu Val
   1 5
<210> 954
   <211> 9
   <212> PRT
   <213> Homo Sapien
<400> 954
   Ser Tyr Phe Pro Glu Ile Thr His Ile
   1 5
<210> 955
   <211> 9
   <212> PRT
   <213> Influenza
<400> 955
   Ile Tyr Ala Thr Val Ala Gly Ser Leu
   1 5
<210> 956
   <211> 9
   <212> PRT
   <213> Influenza
<400> 956
   Val Tyr Gln Ile Leu Ala Ile Tyr Ala
   1 5
<210> 957
   <211> 9
   <212> PRT
   <213> Influenza
<400> 957
   Ile Tyr Ser Thr Val Ala Ser Ser Leu
   1 5
<210> 958
   <211> 9
   <212> PRT
   <213> Influenza
<400> 958
   Leu Tyr Gln Asn Val Gly Thr Tyr Val
   1 5
<210> 959
   <211> 10
   <212> PRT
   <213> Homo sapien
<400> 959
   Arg Tyr Leu Glu Asn Gly Lys Glu Thr Leu
   1 5 10
<210> 960
   <211> 10
   <212> PRT
   <213> Homo Sapien
<400> 960
   Arg Tyr Leu Lys Asn Gly Lys Glu Thr Leu
   1 5 10
<210> 961
   <211> 9
   <212> PRT
   <213> Homo Sapien
<400> 961
   Lys Tyr Gln Ala Val Thr Thr Thr Leu
   1 5
<210> 962
   <211> 9
   <212> PRT
   <213> Plasmodium Berghei
<400> 962
   Ser Tyr Ile Pro Ser Ala Glu Lys Ile
   1 5
<210> 963
   <211> 9
   <212> PRT
   <213> Plasmodiuin Yoelii
<400> 963
   Ser Tyr Val Pro Ser Ala Glu Gln Ile
   1 5
<210> 964
   <211> 9
   <212> PRT
   <213> Influenza
<400> 964
   Ala Ser Asn Glu Asn Met Glu Thr Met
   1 5
<210> 965
   <211> 10
   <212> PRT
   <213> Adenovirus
<400> 965
   Ser Gly Pro Ser Asn Thr Pro Pro Glu Ile
   1 5 10
<210> 966
   <211> 11
   <212> PRT
   <213> Lymphocytic choriomeningitis virus
<400> 966
   Ser Gly Val Glu Asn Pro Gly Gly Tyr Cys Leu
   1 5 10
<210> 967
   <211> 9
   <212> PRT
   <213> Simian virus
<220>
   <221> VARIANT
   <222> 7, 8, 9
   <223> Xaa = Any Amino Acid
<400> 967
   Ser Ala Ile Asn Asn Tyr Xaa Xaa Xaa
   1 5
<210> 968
   <211> 9
   <212> PRT
   <213> Human Immunodeficiency virus
<400> 968
   Ile Leu Lys Glu Pro Val His Gly Val
   1 5
<210> 969
   <211> 9
   <212> PRT
   <213> Influenza

<400> 969
   Gly Ile Leu Gly Phe Val Phe Thr Leu
   1 5
<210> 970
   <211> 10
   <212> PRT
   <213> Influenza
<400> 970
   Ile Leu Gly Phe Val Phe Thr Leu Thr Val
   1 5 10
<210> 971
   <211> 9
   <212> PRT
   <213> Human Immunodeficiency Virus
<400> 971
   Phe Leu Gln Ser Arg Pro Glu Pro Thr
   1 5
<210> 972
   <211> 9
   <212> PRT
   <213> Human immunodeficiency virus
<220>
   <221> VARIANT
   <222> 8, 9
   <223> Xaa = Any Amino Acid
<400> 972
   Ala Met Gln Met Leu Lys Glu Xaa Xaa
   1 5
<210> 973
   <211> 9
   <212> PRT
   <213> Human Immunodeficiency virus
<400> 973
   Pro Ile Ala Pro Gly Gln Met Arg Glu
   1 5
<210> 974
   <211> 9
   <212> PRT
   <213> Human Immunodeficiency Virus
<400> 974
   Gln Met Lys Asp Cys Thr Glu Arg Gln
   1 5
<210> 975
   <211> 7
   <212> PRT
   <213> Human Immunodeficiency virus
<400> 975
   Val Tyr Gly Val Ile Gln Lys
   1 5
<210> 976
   <211> 12
   <212> PRT
   <213> Artificial sequence
<220>
   <223> Synthetic HLA-A2 binding nonamer peptide
<400> 976
   Ser Asp Leu Arg Gly Tyr Val Tyr Gln Gly Leu Lys
   1               5                   10
<210> 977
   <211> 15
   <212> PRT
   <213> Artificial sequence
<220>
   <223> Epitope mimic of natural tumor Ag
<400> 977
   Arg Pro Gln Ala Ser Gly Val Tyr Met Gly Asn Leu Thr Thr Gln
   1               5                   10                  15
<210> 978
   <211> 9
   <212> PRT
   <213> Artificial Sequence
<220>
   <223> Epitope mimic of natural tumor Ag
<400> 978
   Lys Ala Val Tyr Asn Phe Ala Thr Cys
   1               5
<210> 979
   <211> 9
   <212> PRT
   <213> Homo sapien
<400> 979
   Glu Val Asp Pro Ala Ser Asn Thr Tyr
   1               5

## Description

### Background of the Invention

### Field of the Invention

The invention disclosed herein is directed to methods for the design of epitope-encoding vectors for use in compositions, including for example, pharmaceutical compositions capable of inducing an immune response in a subject to whom the compositions are administered. The invention is further directed to the vectors themselves. The epitope(s) expressed using such vectors can stimulate a cellular immune response against a target cell displaying the epitope(s),

### Description of the Related Art

The immune system can be categorized into two discrete effector arms. The first is innate immunity, which involves numerous cellular components and soluble factors that respond to all infectious challenges. The other is the adaptive immune response, which is customized to respond specifically to precise epitopes from infectious agents. The adaptive immune response is further broken down into two effector arms known as the humoral and cellular immune systems. The humoral arm is centered on the production, of antibodies by B-lymphocytes while the cellular arm involves the killer cell activity of cytotoxic T Lymphocytes.

Cytotoxic T Lymphocytes (CTL) do not recognize epitopes on the infectious agents themselves. Rather, CTL detect fragments of antigens derived from infectious agents that are displayed on the surface of infected cells. As a result antigens are visible to CTL only after they have been processed by the infected cell and thus displayed on the surface of the cell

The antigen processing and display system on the surface of cells has been well established. CTL recognize short peptide antigens, which arc displayed on the surface in noncovalent association with class I major histocompatibility complex molecules (MHC). These class I peptides are in turn derived from the degradation of cytosolic proteins

### Summary of the invention

Disclosed herein are expression cassettes, for example, for use in vaccine vectors, which encode one or more embedded housekeeping epitopes, and methods for designing and testing such expression cassettes. Housekeeping epitopes can be liberated from the translation product of such cassettes through proteolytic processing by the immunoproteasome of professional antigen presenting cells (pAPC). In one embodiment, sequences flanking the housekeeping epitope(s) can be altered to promote cleavage by the immunoproteasome at the desired location(s), Housekeeping epitopes, their uses, and identification are described in U.S. Patent No. 6,861,234.

Examples of housekeeping epitopes are disclosed in U.S. Application No. 2003/0220239.

In other embodiments, the housekeeping epitope(s) can be flanked by arbitrary sequences or by sequences incorporating residues known to be favored in immunoproteasome cleavage sites. As used herein the term "arbitrary sequences" refers to sequences chosen without reference to the native sequence context of the epitope, their ability to promote processing, or immunological function. In further embodiments of the invention multiple epitopes can be arrayed head-to-tail. These arrays can be made up entirely of housekeeping epitopes. Likewise, the arrays can include alternating housekeeping and immune epitopes. Alternatively, the arrays can include housekeeping epitopes flanked by immune epitopes, whether complete or distally truncated. Further, the arrays can be of any other similar arrangement. There is no restriction on placing a housekeeping epitope at the terminal positions of the array. The vectors can additionally contain authentic protein coding sequences or segments thereof containing epitope clusters at a source of immune epitopes. The term "authentic" refers to natural protein sequences.

Epitope clusters and their uses are described in U.S. Patent application No. 2003/021545.

Embodiments can encompass screening the constructs to determine whether the housekeeping epitope is liberated. In constructs containing multiple housekeeping epitopes, embodiments can include screening to determine which epitopes are liberated. Also disclosed herein is a vector containing an embedded epitope that can be used to immunise HLA transgenic mice and the resultant CTL can be tested for their ability to recognize target cells presenting the mature epitope. In another embodiment, target cells expressing immunoproteasome can be transformed with the vector. The target cell may express immunoproteasome either constitutively, because of treatment with interferon or through genetic manipulation, for example. CTL that recognize the mature epitope can be tested for their ability to recognize these target cells. In yet another embodiment, the embedded epitope can be prepared as a synthetic peptide. The synthetic peptide then can be subjected to digestion by an immunoproteasome preparation *In vitro* and the resultant fragments can be analysed to determine the sites of cleavage. Such polypeptides, recombinant or synthetic, from which embedded epitopes can be successfully liberated, can also be incorporated into immunogenic compositions.

The invention disclosed herein relates to the identification of a polypeptide suitable for epitope liberation. One embodiment relates to a method of identifying a polypeptide suitable for epitope liberation inducing, for example, the steps of identifying an epitope of interest, providing a substrate polypeptide sequence including the epitope, wherein the substrate polypeptide permits processing by a proteasome; contacting the substrate polypeptide with a composition including the protaesome, under conditions that support processing of the substrate polypeptide by the proteasome; and assaying for liberation of the epitope.

The epitope can be embedded in the substrate polypeptide, and in some aspects the substrate polypeptide can include more than one epitope, for example. Also, the epitopes can be a housekeeping epitope.

It is disclosed that the substrate polypeptide can be a synthetic peptide. Optionally, the substrate polypeptide can be included in a formulation promoting protein transfer. Alternatively, the substrate polypeptide can be a fusion protein. The fusion protein can further include a protein domain possessing protein transfer activity. Further, the contacting step can include immunization with the substrate polypeptide.

In one aspect, the substrate polypeptide can be encoded by a polynucleotide. The contacting step can include immunization with a vector including the polynucleotide, for example. The immunization can be carried out in can HLA-transgenic mouse or any other suitable animal, for example. Alternatively, the contacting step can include transforming a cell with a vector including the polynucleotide. In some embodiments the transformed cell can be a target cell that is targeted by CTL for purposes of assaying for proper liberation of epitope.

The proteasome processing can take place intracellularly, either *in vitro* or *in vivo*. Further, the proteasome processing can take place in a cell-free system.

The assaying step can include a technique selected from the group including, but not limited to, mass spectrometry, N-terminal pool sequencing, HPLC, and the like. Also, the assaying step can include a T cell target recognition assay. The T cell target recognition assay can be selected from the group including, but not limited to, a cytolytic activity assay, a chromium release assay, a cytokine assay, an ELISPOT assay, tetramer analysis, and the like.

It is disclosed that the amino acid sequence of the substrate polypeptide including the epitope can be arbitrary. Also, the substrate polypeptide in which the epitope is embedded can be derived from an authentic sequence of a target-associated antigen. Further, the substrate polypeptide in which the epitope is embedded can be conformed to a preferred immune proteasome cleavage site flanking sequence.

It is disclosed that the substrate polypeptide can include an array of additional epitopes. Members of the array can be arranged head-to-tail, for example. The array can include more than one housekeeping epitope. The more than one housekeeping epitope can include copies of the same epitope, The array can include a housekeeping and an immune epitope, or alternating housekeeping and immune epitopes, for example. Also, the array can include a housekeeping epitope positioned between two immune epitopes in an epitope battery, The array can include multiple epitope batteries, so that there arc two immune epitopes between each housekeeping epitope in the interior of the array. Optionally, at least one of the epitopes can be truncated distally to its junction with an adjacent epitope. The truncated epitopes can be immune epitopes, for example. The truncated epitopes can have lengths selected from the group including, but not limited to, 9, 8, 7, 8, 5, 4 amino acids, and the like.

It is disclosed that the substrate polypeptide can include an array of epitopes and epitope clusters. Members of the array can be arranged head-to-tail, for example.

It is disclosed that the proteasome can be an immune proteasome.

Also described are vectors including a housekeeping epitope expression cassette. The housekeeping epitope(s) can be derived from a target-associated antigen, and the housekeeping epitope can be liberatable, that is capable of liberation, from a translation product of the cassette by immunoprateasome processing.

The expression cassette can encode an array of two or more epitopes or at least one epitope and at least one epitope cluster. The members of the array can be arranged head-to-tail, for example. Also, the members of the array can be arranged head-to-tail separated by spacing sequences, for example. Further, the array can include a plurality of housekeeping epitopes. The plurality of housekeeping epitopes can include more than one copy of the same epitopes or single copies of distinct epitopes, for example. The array can include at least one housekeeping epitope and at least one immune epitopes. Also, the array can include alternating housekeeping and immune epitopes. Further, the array includes a housekeeping epitope sandwiched between two immune epitopes so that there are two immune topes between each housekeeping epitope in the interior of the array. The immune epitopes can be truncated distally to their junction with the adjacent housekeeping epitope.

It is described herein that the expression cassette further encodes an authentic protein sequence, or segment thereof, including at least one immune epitope. Optionally, the segment can include at least one epitope cluster. The housekeeping epitope expression cassette and the authentic sequence including at least one immune epitope can be encoded in a single reading frame or transcribed as a single mRNA species, for example. Also, the housekeeping epitope expression. cassette and the authentic sequence including at least one immune epitope may not be transcribed as a single mRNA species.

In yet another aspect, the vector can include a DNA molecule or an RNA molecule. The vector can encode, SEQ ID NO. 17, or SEQ ID NO. 20.

Also, the vector can include SEQ ID NO. 21.

Also, the vector can encode SEQ ID NO. 18.

It is described herein that the target-associated antigen can be an antigen derived from or associated with a tumor or an intracellular parasite, and the intracellular parasite can be, for example, a virus, a bacterium, a protozoan, or the like.

Also described herein are vectors including a housekeeping epitope identified according to any of the methods disclosed herein, claimed or otherwise. For example, described is a vector encoding a substrate polypeptide that includes a housekeeping epitope by any of the methods described herein.

It is described herein that the housekeeping epitope can be liberated from the cassette translation product by immune proteasome processing

Also described herein are methods of activating a T cell. The methods can include, for example, the steps of contacting a vector including a housekeeping epitope expression cassette with an APC. The housekeeping epitope can be derived from a target-associated antigen, for example, and the housekeeping epitope can be liberatable from a translation product of the cassette by immunoproteasome processing. The methods can further include contacting the APC with a T cell. The contacting of the vector with the APC can occur *In vitro* or in vivo.

Also described herein is a substrate polypeptide including a housekeeping epitope wherein the housekeeping epitope can be liberated by immunoproteasome processing in a pAPC.

Also described herein is a method of activating a T cell comprising contacting a substrate polypeptide including a housekeeping epitope with an APC wherein the housekeeping epitope can be liberated by immunoproteasome processing and contacting the APC with a. T cell.

### Brief Description of the Drawing

Figure 1. An illustrative drawing depicting pMA2M.
Figure 2. Assay results showing the % of specific lysis of ELAGIGILTV pulsed and unpulsed T2 target cells by mock immunized CTL.
Figure 3. Assay results showing the % of specific lysis of ELAGIGILTV pulsed and impulsed T2 target cells by pVAXM3 immunized CTL.
Figure 4. Assay results showing the % of specific lysis of ELAGIGILTV pulsed and impulsed T2 target cells by pVAXM2 immunized CTL.
Figure 5. Assay results showing the % of specific lysis of ELAGIGILTV pulsed and unpulsed T2 target cells by pVAXM1 immunized CTL.
Figure 6. illustrates a sequence of SEQ ID NO. 22 from which the NY-ESO-1₁₅₇₋₁₆₅ epitope is liberated by immunoproteasomal processing.
Figure 7. Shows the differential processing by immunoproteasome and housekeeping proteasome of the SLLM-WITQC epitope (SEQ ID NO. 12) in its native context where the cleavage following the C is more efficiently produced by housekeeping than immunoproteasome,
Figure 8. 8A: Shows the results of the human immunoproteasome digest of SEQ ID NO. 31. 8B: Shows the comparative results of mouse versus human immunoproteasome digestion of SEQ ID NO. 31.
Figure 9. Shows the differential processing of SSX-2₃₁₋₆₈ by housekeeping and immunoproteasome.

### Detailed Descritption of the Preferred Embodiment

### Definitions

Unless otherwise clear from the context of the use of a term herein, the following listed terms shall generally have the indicated meanings for purposes of this description.

PROFESSIONAL ANTIGEN-PRESENTING CELL (pAPC)-acell that possesses Tcell costimulatory molecules and is able to induce a T cell response. Well characterized pAPCs include dendritic cells, B cells, and macrophages.

PERIPHERAL CELL - a cell that is not a pAPC.

HOUSEKEEPING PROTEASOME -a proteasome normally active in peripheral cells, and generally not present or not strongly active in pAPCs.

IMMUNOPROTEASOME - a proteasome normally active in pAPCs; the immunoproteasome is also active in some peripheral cells in infected tissues or following exposure to interferon.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments; epitopes according to this definition include but are not necessarily limited to peptides presented on the surface of cells, the peptides being non-covalently bound to the binding cleft of class I MHC, such that they can interact with T cell receptors (TCR). Epitopes presented by class I MHC may be in immature or mature form. "Mature" refers to an MHC epitope in distinction to any precursor ("Immature") that may include or consist essentially of a housekeeping epitope, but also includes other sequences in a primary translation product that are removed by processing, including without limitation, alone or in any combination, proteasomal digestion, N-terminal trimming, or the action of exogenous enzymatic activities. Thus, a mature epitope may be provided embedded in a somewhat longer polypeptide, the immunological potential of which is due, at least in part, to the embedded epitope; or in its ultimate form that can bind in the MHC binding cleft to be recognized by TCR, respectively.

MHC EPITOPE - a polypeptide having a known or predicted binding affinity for a mammalian class I or class II major histocompatibility complex (MHC) molecule.

HOUSEKEEPING EPITOPE- in a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions. Exemplary housekeeping epitopes are provide in U.S. Application No. 10/117,937, filed on April 4, 2002; and U.S. Provisional Application Nos. 60/282,211, filed on April 6, 2001; 60/337,017, filed on November 7, 2001; 60/363210 filed 3/7/02; and 60/409,123, filed on September 5, 2002; all of which are entitled EPITOPE SEQUENCES.

IMMUNE EPITOPE- In a preferred embodiment, an immune epitope is defined as a polypeptide fragment that is an MHC epitope, and that is displayed on a cell in which immunoproteasomes are predominantly active. In another preferred embodiment, an immune epitope is defined as a polypeptide containing an immune epitope according to the foregoing definition, that is flanked by one to several additional amino acids, in another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL -a cell to be targeted by the vaccines and methods of the invention. Examples of target cells according to this definition include but are not necessarily limited to: a neoplastic cell and a cell harboring an intracellular parasite, such as, for example, a virus, a bacterium, or a protozoan. Target cells can also include cells that are targeted by CTL as a part of assays to determine or confirm proper epitope liberation and processing by a cell expressing immunoproteasome, to determine T cell specify or immunogenicity for a desired epitope. Such cells may be transformed to express the substrate or liberation sequence, or the cells can simply be pulsed with peptide/epitope.

TARGET-ASSOCIATED ANTIGEN (TAA) -a protein or polypeptide present in a target cell.

TUMOR-ASSOCIATED ANTIGENS (TuAA)- a TAA, wherein the target cell is a neoplastic cell.

HLA EPITOPE -a polypeptide having a known or predicted binding affinity for a human class I or class 11 HLA complex molecule.

ANTIBODY-natural immunoglobulin (Ig), poly- or monoclonal, or any molecule composed in whole or in part of an lg binding domain, whether derived biochemically or by use of recombinant DNA. Examples include *Inter alia*, F (ab), single chain Fv, and if, variable region-phage coat protein fusions.

ENCODE -an open-ended term such that a nucleic acid encoding a particular amino acid sequence can consist of codons specifying that (poly)peptide, but can also comprise additional sequences either translatable, or for the control of transcription, translation, or replication, or to facilitate manipulation of some host nucleic add construct.

SUBSTANTIAL SIMILARITY -this term is used to refer to sequences that differ from a reference sequence in an inconsequential way as judged by examination of the sequence. Nucleic add sequences encoding the same amino add sequence are substantially similar despite differences in degenerate positions or modest differences in length or composition of any non-coding regions. Amino acid sequences differing only by conservative substitution or minor length variations are substantially similar. Additionally, amino add sequences comprising housekeeping epitopes that differ in the number of N-terminal flanking residues, or immune epitopes and epitope clusters that differ in the number of flanking residues at either terminus, arc substantially similar. Nucleic acids that encode substantially similar amino acid sequences are themselves also substantially similar.

FUNCTIONAL SIMILARITY-this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino add substitutions (and thus do not meet the substantial similarity definition). Pairs of antibodies, or TCRs, that recognize the epitope can be functionally similar to each other despite whatever structural differences exist. In testing for functional similarity of immunogenicity one would generally immunize with the "altered" antigen and test the ability of the elicited response (Ab, CTL, cytokine production, etc.) to recognize the target antigen. Accordingly, two sequences may be designed to differ in certain respects while retaining the same function. Such designed sequence variants are among the embodiments of the present invention.

EXPRESSION CASSETTE-a polynucleotide sequence encoding a polypeptide, operably linked to a promoter and other transcription and translation control elements, including but not limited to enhancers, termination codons, internal ribosome entry sites, and polyadenylation sites. The cassette can also include sequences that facilitate moving it from one host molecule to another.

EMBEDDED EPITOPE -an epitope contained within a longer polypeptide, also can include an epitope in which either the N- terminus or the C-terminus is embedded such that the epitope is not in an interior position,

MATURE EPITOPE -a peptide with no additional sequence beyond that present when the epitope is bound in the MHC peptide-binding cleft.

EPITOPE CLUSTER - a polypeptide, or a nucleic acid sequence encoding it, that is a segment of a native protein sequence comprising two or more known or predicted epitopes with binding affinity for a shared MHC restriction element, wherein the density of epitopes within the cluster is greater than the density of all known, or predicted epitopes with binding affinity for the shared MHC restriction element within the compete protein sequence, and as disclosed in U.S. Patent Application No. 09/561,571 entitled EPITOPE CLUSTERS.

Substrate or liberation sequence-a designed or engineered sequence comprising or encoding a housekeeping epitope (according to the first of the definitions offered above) embedded in a larger sequence that provides a context allowing the housekeeping epitope to be liberated by immunoproteasomal processing, directly or in combination with N-terminal trimming or other processes.

Degradation, of cytosolic proteins takes place via the ubiquity-dependent multi-catalytic multi-subunit protease system known as the proteasome. The proteasome degrades cytosolic proteins generating fragments that can then be translocated from the cytosol into the endoplasmic reticulum (ER) for loading onto class I MHC. Such protein fragments shall be referred to as class I peptides. The peptide loaded MHC are subsequently transported to the cell surface where they can be detected by CTL.

The multi-catalytic activity of the proteasome is the result of its multi-subunit structure. Subunits are expressed from different genes and assembled post-translationally into the proteasome complex. A key feature of the proteasome is its bimodal activity, which enables it to exert its protease, or cleavage function, with two discrete kinds of cleavage patterns. This bimodal action of the proteasome is extremely fundamental to understanding how CTL are targeted to recognize peripheral cells in the body and how this targeting requires synchronization between the immune system and the targeted cells.

The housekeeping proteasome is constitutively active in all peripheral cells and tissues of the body. The first mode of operation for the housekeeping proteasome is to degrade cellular protein, recycling it into amino acids. Proteasome function is therefore a necessary activity for cell life. As a corollary to its housekeeping protease activity, however, class I peptides generated by the housekeeping proteasome are presented on all of the peripheral cells of the body.

The proteasome's second mode of function is highly exclusive and occurs specifically in pAPCs or as a consequence of a cellular response to interferons (IFNs). In its second mode of activity the proteasome incorporates unique subunits, which replace the catalytic subunits of the constitutive housekeeping proteasome. This "modified" proteasome has been called the immunoproteasome, owing to its expression in pAPC and as a consequence of induction by IFN in body cells.

APC define the repertoire of CTL that recirculate through the body and are potentially active as killer cells. CTL are activated by interacting with class I peptide presented on the surface of a pAPC. Activated CTL are induced to proliferate and caused to recirculate through the body in search of diseased cells, This is why the CTL response in the body is defined specifically by the class I peptides produced by the pAPC. It is important to remember that pAPCs express the immunoproteasome, and that as a consequence of the bimodal activity of the proteasome, the cleavage pattern of proteins (and the resultant class I peptides produced) are different from those in peripheral body cells which express housekeeping proteasome. The differential proteasome activity in pAPC and peripheral body cells, therefore, is important to consider during natural infection and with therapeutic CTL vaccination strategies.

All cells of the body are capable of producing IFN In the event that they are infected by a pathogen such as a virus. IFN production in turn results in the expression of the immunoproteasome in the infected cell. Viral antigens are thereby processed by the immunoproteasome of the Infected cell and the consequent peptides are displayed with class I MHC on the cell surface. At the same time, pAPC are sequestering virus antigens and are processing class I peptides with their immunoproteasome activity, which is normal for the pAPC cell type. The CTL response in the body is being stimulated specifically by the class I peptides produced by the pAPC. Fortunately, the infected cell is also producing class I peptides from the immunoproteasome, rather than the normal housekeeping proteasome. Thus, virus-related class I peptides are being produced that enable detection by the ensuing CTL response. The CTL immune response is induced by pAPC, which normally produce different class I peptides compared to peripheral body cells, owing to different proteasome activity. Therefore, during infection there is epitope synchronization betweenthe infected cell and the immune system.

This is not the case with tumors and chronic viruses, which block the interferon system. For tumors there is no infection in the tumor cell to induce the immunoproteasome expression, and chronic virus infection either directly or indirectly blocks immunoproteasome expression. In both cases the diseased cell maintains its display of class I peptides derived from housekeeping proteasome activity and avoids effective surveillance by CTL.

In the case of therapeutic vaccination to eradicate tumors or chronic Infections, the bimodal function of the proteasome and its differential activity in APC and peripheral cells of the body is significant Upon vaccination with protein antigen, and before a CTL response can occur, the antigen must be acquired and processed into peptides that are subsequently presented on class I MHC on the pAPC surface. The activated CTL recirculate in search of cells with similar class I peptide on the surface. Cells with this peptide will be subjected to destruction by the cytolytic activity of the CTL. If the targeted diseased cell does not express the immunoproteasome, which is present in the pAPC, then the epitopes are not synchronized and CTL fail to find the desired peptide target on the surface of the diseased cell.

Preferably, therapeutic vaccine design takes into account the class 1 peptide that, is actually present on the target tissue. That is, effective antigens used to stimulate CTL to attack diseased tissue are those that are naturally processed and presented on the surface of the diseased tissue. For tumors and chronic infection this generally means that the CTL epitopes are those that have been processed by the housekeeping proteasome. In order to generate an effective therapeutic vaccine, CTL epitopes arc identified based on the knowledge that such epitopes are, in fact, produced by the housekeeping proteasome system. Once identified, these epitopes, embodied as peptides, can be used to successfully immunize or induce therapeutic CTL responses against housekeeping proteasome expressing target cells in the host.

However, in the case of DNA vaccines, there can be an additional consideration. The immunization with DNA requires that APCs take up the DNA. and express the encoded proteins or peptides, It is possible to encode a discrete class I peptide on the DNA. By immunizing with this construct, APCs can be caused to express a housekeeping epitope, which is then displayed on class I MHC on the surface of the cell for stimulating an appropriate CTL response. Constructs for generation of proper termini of housekeeping epitopes have been, described in U.S. Patent application No. 09/561,572 entitled EXPRESSION VECTORS ENCODING EPITOPES OF TARGET-ASSOCIATED ANTIGENS, filed on April 28, 2000.

Disclosed herein are expression cassettes that encode one or more embedded housekeeping epitopes, and methods for designing and testing such expression cassettes. The expression cassettes and constructs can encode epitopes, including housekeeping epitope, derived from antigens that are associated with targets. Housekeeping epitopes can be liberated from the translation product(s) of the cassettes. It is described that the housekeeping epitope(s) can be flanked by arbitrary sequences or by sequences Incorporating residues known to be favored in immunoproteasome cleavage sites. It is described that multiple epitopes can be arrayed head-to-tell. It is described that these arrays can be made up entirely of housekeeping epitopes. Likewise, the arrays can include alternating housekeeping and Immune epitopes. Alternatively, the arrays can include housekeeping epitopes flanked by immune epitopes, whether complete or distally truncated. It is described that each housekeeping epitope can be flanked on either side by an immune epitope, such that an array of such arrangements has two immune epitopes between each housekeeping epitope. Further, the arrays can be of any other similar arrangement. There is no restriction on placing a housekeeping epitope at the terminal positions of the array. The vectors can additionally contain authentic protein coding sequences or segments thereof containing epitopes clusters as a source of immune epitopes.

Several disclosures make reference to polyepitopes orstring-of-bead arrays. See, for example, WO0119408A1, March 22, 2001; WO9955730A2, November 4,1999; WO0040261 A2, July 13, 2000; WO9603144A1, February 8, 1996; EP1181314A1, February 27,2002; W00123577A.3, April 5; US6074817, June 13,2000; US5965381, October 1.2, 1999; WO9741440A1, November 6, 1997; US6130066, October 10, 2000; US6004777, December 21, 1999; US5990091, November23,1999; WO9840501A1, September 177,1998; WO9840500A1, September 17,1998; WO0118035A2, March 15, 2001; WO02068654A2, September 6, 2002; WO0189281A2, November 29, 2001; WO0158478A, August 16, 2001; EP1118860A1, July 25, 2001; WO0111040A1, February 15, 2001; WO0073438A1, December 7, 2000; WO0071158A1, November 30, 2000; WO0066727A1, November 9, 2000; WO0052451A1, September 8. 2000; WO0052157A1, September 8, 2000; WO0029008A2, May 25, 2000; WO0006723A1, February 10, 2000. Additional disclosures, include Palmowski MJ, et al -J Immunol 2002; 168(9):4391-8; Fang ZY, et al - Virology 2001 ;291 (2):272-84; Firat H, et al - J Gene Med 2002;4(1):38-45; Smith SG, et al - Clin Cancer Res 2001;7(12):4253-61; Vonderheide RH, et a1- Clin Cancer Res 2001; 7(11):3343-8; Firat H, et al- Eur J Immunol 2001;31(10):3084-74; Le TT, et el Vaccine 2001;19(32):4669.75; Fayolle C, et al - J virol 2001;75(16):7330-8; Smith. SG - Curr Opin Mol Ther 1999,1(1);10-5); Firat H, et al - Eur J Immunol 1999;29(10):3112.-21; Mateo L, et al -J Immmol 1999;163(7):4058-63; Heemskerk MH, et al - Cell Immunol 1999;195(1): 10-7; Woodberry T, et al - J Virol 1999;73(7):5320-5; Hanke T, et al - Vaccine 1998;16(4):426-35; Thomson SA, et al - J Immunol 1998,160(4):1717-23; Toes RE, et al -Proc Natl Acad Sci USA. 1997;94(26):14660-5; Thomson SA, et al -J Immunol 1996;157(2):822-6; Thomson SA, et al - Proc Natl Acad sci USA 199S;92(13):5M5-9; Street MD, et al - Immunology 2002;106(4):526-36; Hirano K, et al Histochem Cell Biol 2002;117(1):41-53; Ward SM, et al virus Genes 2001;23(I):97-104; Liu WJ, et al - Virology 2000;273(2):374-82; Gariglio P, et al - Arch Med Res 1998;29(4): 279-84; Suhrbler A - Immunol Cell Biol 1997;75(4);402-8; Fomsgaard A, at al - Vaccine 1999;18(7-8):681-91; An LL, et al - J Virol 1997;71 (3):2292-302; Whitton JL, et al - J Virol 1993;67(1):348-52; Ripalti A, et al - J Clin Microbiol 1994;32 (2):358-63; and Gilbert, S.C., et al., Nat. Biotech. 15:1280-1284, 1997.

One important feature that the disclosures In the preceding paragraph all share is their lack of appreciation for the desirability of regenerating housekeeping epitopes when the construct is expressed in a pAPC. This understanding was not apparent until the present invention. Embodiments of the invention include sequences, that when processed by an immune proteasome, liberate or generate a housekeeping epitope. Embodiments also can liberate or generate such epitopes in immunogenically effective amounts. Accordingly, while the preceding references contain disclosures relating to polyepitope arrays, none is enabling of the technology necessary to provide or select a polyepitope capable of liberating a housekeeping epitopes by action of an immunoproteasome in a pAPC. In contrast, embodiments are based upon a recognition of the desirability of achieving this result. Accordingly, embodiments include any nucleic acid construct that encodes a polypeptide containing at least one housekeeping epitopes provided in a context that promotes its generation via immunoproteasomal activity, whether the housekeeping epitope is embedded in a string-of-beads array or some other arrangement. Some embodiments include uses of one or more of the nucleic acid constructs or their products that are specifically disclosed in any one or more of the above-listed references. Such uses include, for example, screening a polyepitope for proper liberation context of a housekeeping epitope and/or an immune epitope, designing an effective immunogen capable of causing presentation of a housekeeping epitope and/or an immune epitope on a pAPC, immunizing a patient, and the like. Alternative embodiments include use of only a subset of such nucleic acid constructs or a single such construct, while specifically excluding one or more other such constructs, for any of the purposes disclosed herein.

Embodiments also include methods, uses, therapies, and compositions directed to verious types of targets. Such targets can Include, for example, neoplastic cells such as those listed below, for example; and cells infected with any virus, bacterium, protozoan, fungus, or other agents, examples of which are listed below, in Tables 1-5, or which are disclosed In any of the references listed above. Alternative embodiments include the use of only a subset of such neoplastic cells and infected cells listed below, in Tables 1-5, or in any of the references disclosed herein, or a single one of the neoplastic cells or infected cells, while specifically excluding one or more other such neoplastic cells or infected cells, for any of the purposes disclosed herein. The following are examples of neoplastic cells that can be targeted: human sarcomas and carcinomas, e.g., fibrosarcoma, myxosarcoma, liposarcoma, chondrosarcoma, osteogenic sarcoma, chordoma, angiosancoma, endotheliosarcoma, lymphanglosarcoma, lymphangioendothellosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, chonocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngloma, ependymoma, pinealoma, hemangloblastoma, acoustic neuroma, oligodendroglioma, meningioma, melanoma, neuroblastoma, retinoblastoma; leukemias, e.g., acute lymphocytic leukemia and acute myelocytic leukemia (mycloblastic, promyelocytic, myelomonocytic, monocytic and crythrolaukemia); chronic leukemia (chronic myelocytic (granulocytic) leukemia and chronic lymphocytic leukemia); and polycythemia vera, lymphoma (Hodgkin's disease and non Hodgkin's disease), multiple myeloma, Waldenstrom's macroglobulinemia, heavy chain disease, hepatocellular cancer, brain cancer, stomach cancer, liver cancer, and the like. Examples of infectious agents that infect the target cells can include the following: adenovirus, cytomegalovirus, Epstem-Barr virus, herpes simplex virus 1 herpes simplex virus 2, human herpesvirus 6, varicella-zoster virus, hepatitis B virus, hepatitis D virus, papilloma Virus, parvovirus B19, polyomavirus BK, polyomavirus JC, hepatitis C virus, measles virus, rubella virus, human immunodeficiency virus (HIV), human Tcell leukemia virus I, human T cell leukemia virus II, *Chlamydia, Listeria, Salmonella, Legionella, Brucella, Coxiella, Rickettsia, Mycobacterium, Leishmania, Trypanasoma, Toxoplasma, Plasmodium,* and the like. Exemplary infectious agents and neoplastic cells are also included in Tables 1-5 below.

Furthermore the targets can include neoplastic cells described in or cells infected by agents that are described in any of the following references: Jäger, E. et al., "Granulocyte-macrophage-colony-stimulating factor enhances immune responses to melanoma-associated peptides in vivo," Int. J Cancer, 67:54-62 (1996); Kündig, T.M., Althage, A., Hengartner, H. & Zinkemagel, R.M., "A skin test to assess CD8+ cytotoxic T cell activity," Proc. Natl. Acad Sci. USA, 89: 7757-76 (1992); Bachmann, M.F. & Kundig, T.M., "In vitro vs, in vivo assays for the assessment of T- and B-cell function," Curr. Opin. Immunol., 6:320-326 (1994), Kundig et al., "On the role of antigen in maintaining cytotoxic T cell memory." Proceedings of the National Academy of Sciences of the United States of America, 93:9716-23 (1996); Steinmann, R.M., "The dendritic cells system and its role in immunogenicity," Annual Review of Immunology 9:271-96 (1991); Inaba, K. et al., "Identification of proliferating dendritic cell precursors in mouse blood," Journal of Experimental Medicine, 175: 1157-67 (1992); Young, J.W. & Inaba, K., "Dendritic cells as adjuvants for class I major histocompatibility complex-restricted anti-tumor immunity," Journal of Experimental Medicine, 183:7-1. (1996); Kuby, Janis, Immunology, Second Edition, Chapter 15, W.H. Freeman and Company (1991); Austenst, E., Stahl, T., and de Gruyter, Walter, Insulin Pump Therapy, Chapter 3, Berlin, New York (1990); Remington, The Science and Practice of Pharmacy, Nineteenth Edition, Chapters 86-88; Cleland, Jeffery L. and Lanker, Robert (Editor), "Formulation and delivery of proteins and peptides," American Chemical Society (ACS Symposium Series, No. 567) (1994); Dickinson, Becton, which is fixed usingTegadenn transparent dressing Tegaderm™ 1624, 3M, St. Paul, MN 55144, USA; Santus, Giancario and Baker, Richard, "Osmotic drug delivery: A. review of the patent literature," Journal of Controlled Release, 35:121 (1995); Rammensee, U.S. Patent No. 5,747,269, issued May 05, 1998; Magruder, U.S. Patent No. 5,059,423, issued October 22, 1991; Sandbrook, U.S. Patent No. 4,552,651, issued November 25, 1985; Eckenhoff et al., U.S. Patent No.3 3,987,790, issued October 26, 1976; Theeuwes, U.S. Patent No. 4,455,145, issued June 19, 1984, Roth et al. U.S. Patent No. 4,929,233, issued May 29 1990; van der Bruggen et al., U.S. Patent No. 5,554,506, issued September 10, 1996; Pfreundschuh, U.S. Patent No. 5,698,396, issued December 16, 1997; Magruder, U.S, Patent. No. 5,110,596, issued May 5, 1992; Eckenhoff, U.S. Patent. No. 4,619,652, issued October 28, 1986; Higuchi et al., U.S. Patent No. 3,995,631, issued December07, 1976; Maruyama, U.S. Patent No, 5,017,381, issued May 21, 1991; Eckenhoff, U.S. Patent No. 4,963,141, issued October 16, 1990; van der Bruggen et al., U.S. Patent No. 5,558,995, issued September 24, 1996; Stolzenberg et al. U.S. Patent No. 3,604,417, issued September 14, 1971; Wong et al., US. Patent No. 5,110,597, issued May 05, 1992; Eckenhoff, U.S. Patent No. 4,753,651, issued June 28, 1988; Theeuwes, U.S. Patent No. 4,203,440, issued May 20, 1980; Wong et al. U.S. Patent No. 5,023,088, issued June 11, 1991; Wong et al., U.S. Patent. No. 4,976,966, issued December 11, 1990; Van den Eynde et al., U.S. Patent No. 5,648,226, issued July 15, 1997; Baker et al., U.S. Patent No. 4,838,862, issued June 13, 1989; Magruder, U.S. Patent No. 5,135,523, issued August 04, 1992; Higuchi et al., U.S. Patent No. 3,732,865, issued May 15, 1975, ; Theeuwes, U.S. Patent No. 4,286,067, issued August, 25 1981; Theeuwes et al., U.S. Patent No. 5,030,216, issued July 09, 1991; Boon et al., U.S. Patent No. 5,405,940, issued April 11, 1995; Faste, U.S. Patent No. 4,898,582, issued February 06, 1990; Eckenhoff, U.S. Patent No. 5,137,727, issued August 11, 1992; Higuchi et al., U.S. Patent No. 3,760,804, issued September 25, 1973; Eckenhoff et al., U.S. Patent No. 4,300,558, issued November 12, 1981; Magruder et al., U.S. Patent No. 5,034,229, issued July 23, 1991 Boon et al., U.S. Patent No. 5,487,974, issued January 30, 1996; Kam et al., U.S. Patent No. 5,135,498, issued August 04, 1992; Magruder et al., U.S. Patent No. 5,174,999, issued December 29, 1992; Higuchi, U.S. Patent No. 3,760,805, September 25, 1973; Michaels, U.S. Patent No. 4,304,232, issued December 08, 1981; Magruder et al., U.S. Patent No. 5,037,420, issued October 15, 1991; Wolfel et al., U.S. Patent No. 5,530,096, issued June 25, 1996; Athadye et al., U.S. Patent No. 5,169,390, issued December 08, 1992; Balaban et al., U.S. Patent No. 5,209,746, issued May 11, 1993; Higuchi, U.S. Patent No. 3,929,132, issued December 30,1975; Michaels, U.S, Patent No. 4,340,054, issued July 20,1982; Magruder et al., U.S. Patent No. 0,057,318, issued October 15, 1991; Wolfel et al., U.S. Patent No. 5,519,117, issued May 21, 1996; Athadye et al., U.S. Patent. No. 5,257,987, issued November 02, 1993; Linkwitz et al., U.S. Patent No. 5,221,278, issued June 22,1993; Nakano et al., U.S. Patent No. 3,995,632, issued December 07,1976; Michaels, U.S. Patent No. 4,367,741, issued January 11, 1983; Eckenhoff, U.S. Patent No. 4,865,598, issued September 12, 1989; Eat al., U.S. Patent No. 5,774,316., issued April 28, 1998; Eckenhoff, U.S. Patent No. 4,340,048, issued July 20, 1982; Wong, U.S. Patent No. 5,223,265, issued June 29, 1993; Higuchi et al., U.S. Patent No. 4,034,756, issued July 12, 1977; Michaels, U.S. Patent No. 4,450,198, issued May 22, 1984; Eckenhoff et al., U.S. Patent No. 4,865,845, issued September 12, 1989; Melief et al., U.S. Patent No. 5.554,724, issued September to, 1996; Eckenhoff et al., U.S. Patent No. 4,474,575, issued October 02, 1984; Theeuwes, U.S. Patent No. 3,760,984, issued September 25, 1983; Eckenhoff, U.S. Patent No. 4,350,271, issued September 21, 1982; Eckenhoff et al., U.S. Patent No. 4,855,141, issued August 08, 1989; Zingerman, U.S. Patent No. 4,872,873, issued October 10, 1989; Townsend at al., U.S. Patent No. 5,585,461, issued December 17, 1996; Carulli. J.P. et al., J. Cellular Biochem Suppl., 30/31:286-96 (1998); Türeci, Ö., Sahin, U., and Pfreundschuh, M., "Serological analysis of human tumor antigens; molecular definition and implications," Molecular Medicine Today, 3:342 (1997); Rammensee et al., MHC Ligands and Peptide Motifs Landes Bioscience Austin, TX, 224-27, (1997); Parker et al., "Scheme for ranking potential HLA-A2 binding peptides based on independent binding of individual peptide side-chains," J. Immunol, 152:163-175; Kido & Ohshita, Anal. Biochem., 230:41-47 (1995); Yamada et al., J. Biochem, (Tokyo), 95:1155-60 (1984); Kawashima et al., Kidney Int., 54:275.8 (1998); Nakabayshi & Ikezawa, Biochem. Int. 16:1119.-25 (1988); Kanaseki & Ohkuma, J. Biochem. (Tokyo), 110:541-7 (1991); Wattiaux et al., J. Cell Biol., 78:349-68 (1978); Lisman et al., Biochem, J., 178:79-87 (1979); Dean, B., Arch. Blochem. Blophys., 227:154-63 (1983); Overdijk et al., Adv. Exp. Med. Biol., 101:601-10 (1978); Stromhaug et al., Biochem. J., 335:217-24 (1998); Escola et al., J. Biol. Chem., 271:27360-05 (1996); Hammond et al., Am. J. Physiol.,267:F516-27 (1994); Williams & Smith, Arch. Biochem. Biophys., 305:298-306 (1993); Marsh, M., Methods Cell Biol., 31:319.34 (1989); Schmid & Mellman, Prog. Clin. Biol. Res., 270:35-49 (1988); Falk, K. et at., Nature, 351:290, (1991); Ausubel et al., Short Protocols in Molecular Biology, Third Edition, Unit 11.2 (1997); hypertext transfer protocol address 134.2.96.221/scripts/hlaserver.dil/EpPredict.htm; Levy, Morel, S. et al., Immunity 12:107-117 (2000); Seipelt et Virus Research, 62:159-68, 1999; Storkus et al., U.S. Patent No. 5,989,565, issued November 23, 1999; Morton, U.S. Patent No. 5,993,828, issued November 30, 1999; Virus Research 62:159-168, (1999); U.S. Patent Application No. 2003/0215425 Miura et al., WO 99/01283, January 14, 1999; Simard et al., U.S. Patent 6,861,234, U.S. Patent Application, U.S. Patent US 6,861,234.

Also disclosed herein are methods, uses, therapies, and compositions relating to a particular antigen, whether the antigen is derived from, for example, a target cell or an infective agent, such as those mentioned above. Some preferred embodiments employ the antigens listed herein, in Tables 1-5, or in the list below, alone, as subsets, orin any combination. For example, some embodiments exclude use of one or more ofthose antigens. Other embodiments may exclude any combination or all of those antigens. Several examples of such antigens include MelanA (MART-1), gp100 (Pmel 17), tyrosinase, TRP-1, TRP-2, MAGE-1, MAGE-3, BAGE, GAGE-1, GAGE-2, CEA, RAGE, NY-ESO, SCP-1, Hom/Mel-40, FRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23HL PSA, TAG-72.4, CAM 17,1, NuMa, K-ras, β-Catenin, CDK4, Mum-1, p16, as well as any of those set forth in the above mentioned references. Other antigens are included in Tables 1-4 below.

Also disclosed herein are methods, uses, compositions, and therapies relating to epitopes, including, for example those epitopes listed in Tables 1-5. These epitopes can be useful to flank housekeeping epitopes in screening vectors, for example. Some embodiments include one or more epitopes from Tables 1-5, while other embodiments specifically exclude one or more of such epitopes or combinations thereof.

**Table 1**

| Virus | Protein | AA Position | T cell epitope MHC ligand (Antigen) | MHC molecule |
|---|---|---|---|---|
| Adenovirus 3 | E3 9Kd | 30-38 | LIVIGILIL | HLA-A *0201 |
| | | | (SEQ. ID NO.:44) | |
| Adenovirus 5 | EIA | 234-243 | SGPSNTPPEI | H2-Db |
| | | | (SEQ. ID NO.:45) | |
| Adenovirus 5 | EIB | 192-200 | VNIRNCCYI | H2-Db |
| | | | (SEQ. ID NO.: 46) | |
| Adenovirus 5 | EIA | 234-243 | SGPSNIPPEI (T>I) | H2-Db |
| | | | (SEQ. ID NO.:47) | |
| CSFV | NS polyprotein | 2276-2284 | ENALLVALF | SLA,haplotype d/d |
| | | | (SEQ. ID NO.:48) | |
| Dengue virus 4 | NS3 | 500-508 | TPEGIIPTL | HLA-B*3501 |
| | | | (SEQ. ID NO.:49) | |
| EBV | LMP-2 | 426-434 | CLGGLLTMV | HLA-A*0201 |
| | | | (SEQ. ID NO.:50) | |
| EBV | EBNA.-1 | 480-484 | NIAEGLRAL | HLA-A*0201 |
| | | | (SEQ. ID NO.:51) | |
| EBV | EBNA.1. | 519-527 | NLRRGTALA | HLA-A*0201 |
| | | | (SEQ. ID NO.:52) | |
| EBV | EBNA-1 | 525-533 | ALAIPQCRL | HLA-A*0201 |
| | | | (SEQ. ID NO.:53) | |
| EBV | EBNA-1 | 575-582 | VLKDAIKDL | HLA-A*0201 |
| | | | (SEQ. ID NO.:54) | |
| EBV | EBNA-1 | 562-570 | FMVFLQTHI | HLA-A*0201 |
| | | | (SEQ. ID NO.:55) | |
| EBV | EBNA-2 | 15-23 | HLIVDTDSL | HLA-A*0201 |
| | | | (SEQ. ID NO.:56) | |
| EBV | EBNA-2 | 22-30 | SLGNPSLSV | HLA-A*0201 |
| | | | (SEQ. ID NO.:57) | |
| EBV | EBNA-2 | 126-134 | PLASAMRML | HLA-A*0201 |
| | | | (SEQ. ID NO.:58) | |
| EBV | EBNA-2 | 132-140 | RMLWMANYI | HLA-A*020 |
| | | | (SEQ. ID NO.:59) | |
| EBV | EBNA-2 | 133-141 | MLWMANYIV | HLA-A*0201 |
| | | | (SEQ. ID NO.:60) | |
| EBV | EBNA-2 | 151-159 | ILPQGPQTA | HLA-A*0201 |
| | | | (SEQ. ID NO.:61) | |
| EBV | EBNA-2 | 171-179 | PLRPTAPTI | HLA-A*0201 |
| | | | (SEQ. ID NO.:62) | |
| EBV | EBNA-2 | 205-213 | PLPPATLTV | HLA-A*0201 |
| | | | (SEQ. ID NO.:63) | |
| EBV | EBNA-2 | 246-254 | RMHLPVLHV | HLA-A*0201 |
| | | | (SEQ. ID NO.:64) | |
| EBV | EBNA-2 | 287-295 | PMPLPPSQL | HLA-A*0201 |
| | | | (SEQ. ID NO.:65) | |
| EBV | EBNA-2 | 294..302 | QLPPPAAPA | HLA-A*0201 |
| | | | (SEQ. ID NO.:66) | |
| EBV | EBNA-2 | 381-389 | SMPELSPVL | HLA-A *020 |
| | | | (SEQ. ID NO.:67) | |
| EBV | EBNA-2 | 453-461 | DLDESWDYI | HLA-A*0201 |
| | | | (SEQ. ID NO.:68) | |
| EBV | BZLFI | 43-51 | PLPCVLWPV | HLA-A*0201 |
| | | | (SEQ. ID NO.:69) | |
| EBV | BZLF1 | 167-175 | SLEECDSEL | HLA-A*0201 |
| | | | (SEQ. ID NO.:70) | |
| EBV | BZLFI | 176-184 | EIKRYKNRV | HLA-A*0201 |
| | | | (SEQ. ID NO.:71) | |
| EDV | BZLF1 | 195-203 | QLLQHYREV | HLA-A*0201 |
| | | | (SEQ. ID NO.:72) | |
| EBV | BZLFI | 196-204 | LLQHYREVA | HLA-A*0201 |
| | | | (SFQ. ID NO.:73) | |
| EBV | BZLFI | 217-225 | LLKQMCPSL | HLA-A*0201 |
| | | | (SEQ. ID NO.:74) | |
| EBV | BZLFI | 229-237 | SIIPRTPDV | HLA-A*0201 |
| | | | (SEQ. ID NO.:75) | |
| EBV | ETNA-6 | 284-293 | LLDFVRFMGV | HLA-A*0201 |
| | | | (SEQ ID NO.:76) | |
| EBV | EBNA-3 | 464-472 | SVRDRLARL | HLA-A*0203 |
| | | | (SEQ. ID NO:77) | |
| EBV | EBNA-4 | 416-424 | IVTDFSVIK | HLA-A*1101 |
| | | | (SEQ. ID NO.:78) | |
| EBV | EBNA-4 | 399-408 | AVFDRKSDAK | HLA-A*0201 |
| | | | (SEQ. ID NO.:79) | |
| EBV | EBNA-3 | 246-253 | RYSIFFDY | HLA-A24 |
| | | | (SEQ ID NO.: 80) | |
| EBV | EBNA-6 | 881-889 | QPRAPIRPI | HLA-B7 |
| | | | (SEQ. ID NO.:81) | |
| EBV | EBNA-3 | 379-387 | RPPIFIRRI. | HLA-B7 |
| | | | (SEQ ID NO.:82) | |
| EBV | EBNA-1 | 426-434 | EPDVPPGAI | HLA-B7 |
| | | | (SEQ. ID NO.:83) | |
| EBV | EDNA-1 | 228-236 | IPQCRLTPL | HLA-B7 |
| | | | (SEQ ID NO.:84) | |
| EBV | EDNA-1 | 546-554 | GPGPQPGPL | HLA-B7 |
| | | | (SEQ ID NO.:85) | |
| EBV | EBNA-1 | 550-558 | QPGPLRESI | HLA-B7 |
| | | | (SEQ, ID NO.:86) | |
| EBV | EBNA-1 | 72-80 | R.PQKRPSCI | HLA-B7 |
| | | | (SEQ ID NO.:87) | |
| EBV | EBNA-2 | 224-232 | PPTPLLTVL | HLA-B7 |
| | | | (SEQ. ID NO.:88) | |
| EBV | EBNA-2 | 241-249 | TFPSPPRMHL | HLA-B7 |
| | | | (SEQ. ID NO.:89) (SEQ. ID NO.:89) | |
| EBV | EBNA-2 | 244-252 | PPRMHLPVL | HLA-B7 |
| | | | (SEQ. ID NO.: 90) | |
| EBV | EBNA-2 | 254-262 | VPDQSMHPL | HLA-B7 |
| | | | (SEQ. ID NO,: 91) | |
| EBV | EBNA-2 | 446-454 | PPSIDPADL | HLA-B7 |
| | | | (SEQ, ID NO.:92) | |
| EBV | BZLFI | 44-52 | LPCVEWPVL | HLA-B7 |
| | | | (SEQ. ID NO.:93) | |
| EBV | BZLFI | 222-231 | CPSLDVDSII | HLA-B7 |
| | | | (SEQ. ID NO.:94) | |
| EBV | BZLFI | 234-242 | TPDVLHEDL | HLA-B7 |
| | | | (SEQ. ID NO.:95) | |
| EBV | ETNA-3 | 339-347 | FLIIGRAYGL | HLA-B8 |
| | | | (SEQ. ID NO.:96) | |
| EBV | EBNA-3 | 26-34 | QAKWRLQTL | HLA-B8 |
| | | | (SEQ. ID NO.:97) | |
| EBV | EBNA-3 | 325-333 | AYPLHEQHG | HLA-B8 |
| | | | (SEQ. ID NO.:98) | |
| EBV | EBNA-3 | 158-166 | YIKSFVSDA | HLA-B8 |
| | | | (SEQ. ID NO.:99) | |
| EBV | LMP-2 | 236-244 | RRRWRRLTV | HLA-B*2704 |
| | | | (SEQ. ID NO.: 100) | |
| EBV | EBNA-6 | 258-266 | RRIYDLIEL | HLA-B*2705 |
| | | | (SEQ. ID NO.:101) | |
| EBV | EBNA-3 | 458-466 | YPLHEQHGM | HLA-B*3501 |
| | | | (SEQ. U) NO.: 102) | |
| EBV | EBNA-3 | 458-466 | YPLHEQHGM | HLA-B*3503 |
| | | | (SEQ.IDNO.:103) | |
| HBV | NS3 | 389-397 | HSKKKCDEL | HLA-B8 |
| | | | (SEQ. ID NO.: 104) | |
| HCV | env E | 44-51 | ASRCWVAM | HLA-B*3501 |
| | | | (SEQ. ID NO.: 105)"° | |
| HCV | core protein | 27-35 | GQIVGGVYL | HLA-B*40012 |
| | | | (SEQ. ID NO.: 106) | |
| HCV | NSI | 77-85 | PPLTDFDQGW | HLA-B*5301 |
| | | | (SEQ. ID NO.: 107) | |
| HCV | core protein | 18-27 | LMGYIPLVGA | H2-Dd |
| | | | (SEQ. ID NO.: 108) | |
| HCV | core protein | 16-25 | ADLMGY1 PLV | H2-Dd |
| | | | (SEQ. ID NO.: 109) | |
| HCV | NS5 | 409-424 | MSYSWTGALVTPCAEE | H2-Dd |
| | | | (SEQ. ID NO.: 110) | |
| HCV | NS1 | 205-213 | KHPDATYSR | Papa-A06 |
| | | | (SEQ. ID NO.:111) | |
| HCV-1 | NS3 | 400-409 | KLVALGINAV | HLA-A*0201 |
| | | | (SEQ. ID NO.: 112) | |
| HCV-1 | NS3 | 440-448 | GDFDSVIDC | Patr-B16 |
| | | | (SEQ. ID NO.: 113) | |
| HCV-1 | env E | 118-126 | GNASRCWVA | Patr-B16 |
| | | | (SEQ. ID NO.: 114) | |
| HCV-1 | NSI | 159-167 | TRPPLGNWF | Patr-B13 |
| | | | (SEQ. ID NO.: 115) | |
| HCV-1 | NS3 | 351-359 | VPHPNIEEV | Patr-B13 |
| | | | (SEQ. ID NO.: 116) | |
| HCV-1 | NS3 | 438-446 | YTGDFDSVI | Patr-B01 |
| | | | (SEQ. ID NO.:117) | |
| HCV-1 | NS4 | 328-335 | SWAIKWEY | Patr-A11 |
| | | | (SEQ. ID NO.:118) | |
| HCV-1 | NSI | 205-213 | KHPDATYSR | Patr-A04 |
| | | | (SEQ. ID NO.:119) | |
| HCV-1 | NS3 | 440-448 | GDFDSVIDC | Patr-A04 |
| | | | (SEQ. ID NO.:120) | |
| HIV | gp41 | 583-591 | RYLKDQQLL | HLA A24 |
| | | | (SEQ. ID NO.:121) | |
| HIV | gagp24 | 267-275 | IVGLNKIVR | HLA-A*3302 |
| | | | (SEQ. ID NO.:122) | |
| HIV | gagp24 | 262-270 | EIYKRWIIL | HLA-B8 |
| | | | (SEQ. ID NO.:123) | |
| HIV | gagp24 | 261-269 | GEIYKRWII | HLA-B8 |
| | | | (SEQ. ID NO.:124) | |
| HIV | gagp17 | 93-101 | EIKDTKEAL | HLA-B8 |
| | | | (SEQ. ID NO.:125) | |
| HIV | gp41 | 586-593 | YLKDQQLL | HLA-B8 |
| | | | (SEQ. ID NO.:126) | |
| HIV | gagp24 | 267-277 | ILGLNKIVRMY | HLA-B*1501 |
| | | | (SEQ. ID NO.:127) | |
| HIV | gp41 | 584-592 | ERYLKDQQL | HLA-B14 |
| | | | (SEQ.IDNO.:128) | |
| HIV | nef | 115-125 | YHTQGYFPQWQ | HLA-B17 |
| | | | (SEQ. ID NO.:129) | |
| HIV | nef | 117-128 | TQGYFPQWQNYT | HLA-B17 |
| | | | (SEQ, ID NO.:130) | |
| HIV | gp120 | 314-322 | GRAFVTIGK | HL-A-B*2705 |
| | | | (SEQ. ID NO.: 131 | |
| HIV | gagp24 | 263-271 | KRWIILGLN | HLA-B*2702 |
| | | | (SEQ. ID NO.:132) | |
| HIV | nef | 72-82 | QVPLRPMTYK | HLA-B*3501 |
| | | | (SEQ. ID NO.:133) | |
| HIV | nef | 117-125 | TQGYFPQWQ | HLA-B*3701 |
| | | | (SEQ. ID NO.:134) | |
| HIV | gagp24 | 143-151 | HQAISPRTI, | HLA-Cw*0301 |
| | | | (SEQ. ID NO.:135) | |
| HIV | gagp24 | 140-151 | QMVHQAISPRTL | HLA-Cw*0301 |
| | | | (SEQ. ID NO.:136) | |
| HIV | gp120 | 431-440 | MYAPPIGGQI | H2-Kd |
| | | | (SEQ. ID NO.:137) | |
| HIV | gp160 | 318-327 | RGPGRAFVTI | H2-Dd |
| | | | (SEQ. ID NO.:138) | |
| HIV | gp120 | 17-29 | MPGRAFVTI | H2-Ld |
| | | | (SEQ. ID NO.:139) | |
| HIV-1 | RT | 476-484 | ILKEPVHGV | HLA-A*0201 |
| | | | (SEQ. ID NO.:140) | |
| HIV-1 | nef | 190-198 | AFHHVAREL | HLA-A*0201 |
| | | | (SEQ. ID NO.:141) | |
| HIV-1 | gp160 | 120-128 | KLTPLCVTL | HLA-A*0201 |
| | | | (SEQ. ID NO.:142) | |
| HIV-1 | gp]60 | 814-823 | SLLNATDIAV | HLA-A*0201 |
| | | | (SEQ. ID NO.:143) | |
| HIV-1 | RT | 179-187 | VIYQYMDDL | HLA-A*0201 |
| | | | (SEQ. ID NO.:144) | |
| HIV-1 | gagp 17 | 77-85 | SLYNTVATL | HLA-A*0201 |
| | | | (SEQ. ID NO.:145) | |
| HIV-1 | gp160 | 315-329 | RGPGRAFVT1 | HLA-A*0201 |
| | | | (SEQ. ID NO.:146) | |
| HIV-1 | gp41 | 768-778 | RLRDLLLIVTR | HLA-A3 |
| | | | (SEQ. ID NO.:147) | |
| HIV-1 | nef | 73-82 | QVPLRPMTYK | HLA-A3 |
| | | | (SEQ. ID NO.:148) | |
| HIV-1 | gp120 | 36-45 | TVYYGVPVWK | HLA-A3 |
| | | | (SEQ. ID NO.:149) | |
| HIV-1 | gagp17 | 20-29 | RLRPGGKKK | HLA-A3 |
| | | | (SEQ. 1D NO.:150) | |
| HIV-1 | gp120 | 38-46 | VYYGVPVWK | HLA-A3 |
| | | | (SEQ.1D NO.:151) | |
| HIV-1 | nef | 74-82 | VPLRPMTYK | HLA-a*1101 |
| | | | (SEQ. ID NO.:152) | |
| HIV-1 | gagp24 | 325-333 | AIFQSSMTK | HLA-A*1101 |
| | | | (SEQ. ID NO.:153) | |
| HIV-1 | nef | 73-82 | QVPLRPMTYK | HLA-A*1101 |
| | | | (SEG. ID NO.:154) | |
| HIV-1 | nef | 83-94 | AAVDLSHFLKEK | HLA-A*1101 |
| | | | (SEQ. ID NO.:155) | |
| HIV-1 | gagp24 | 349-359 | ACQGVGGPGGHK | HLA-A*1101 |
| | | | (SEQ. ID NO.:156) | |
| HIV-1 | gagp24 | 203-212 | ETINEEAAEW | HLA-A25 |
| | | | (SEQ. ID NO.:157) | |
| HIV-1 | nef | 128-137 | TPGPGVRYPL | HLA-B7 |
| | | | (SEQ. ID NO:158) | |
| HIV-1 | gagp 17 | 24-31 | GGKKKYKL | HLA-B8 |
| | | | (SEQ. ID. NO.:159) | |
| HIV-1 | gpl20 | 2-10 | RVKEKYQHL | HLA-B8 |
| | | | (SEQ. ID. NO.:160) | |
| HIV-1 | gagp24 | 298-306 | DRFYKTLRA | HLA-B 14 |
| | | | (SEQ. ID NO.:161) | |
| HIV-1 | NEF | 132-147 | GVRYPLTFGWCYKLVP | HLA-B18 |
| | | | (SEQ. ID NO.:162) | |
| HIV-1 | gagp24 | 205-24 | KRWIILGLNK | HLA-B*2705 |
| | | | (SEQ. ID NO.:163) | |
| HIV-1 | nef | 190-198 | AFHHVAREL | HLA-B*5201 |
| | | | (SEQ. ID NO.:164) | |
| EBV | EBNA-6 | 335-343 | KEHVIQNAF | HLA-B44 |
| | | | (SEQ. ID NO.:165) | |
| EBV | EBNA-8 | 130-139 | EENLLDFVRF | HLA-B*4403 |
| | | | (SEQ. ID NO.: 166) | |
| EBV | EBNA-2 | 42-51 | DTPLIPLTIF | HLA-B51 |
| | | | (SEQ. ID NO.:167) | |
| EBV | EBNA-6 | 213-222 | QNGALAINTF | HLA-1362 |
| | | | (SEQ. ID NO.:168) | |
| EBV | EBNA-3 | 603-611 | RLRAEAGVK | HLA-A3 |
| | | | (SEQ. ID NO.:169) | |
| HBV | sAg | 348-357 | GLSPTVWLSV | HLA-A*0201 |
| | | | (SEQ. ID NO.: 170) | |
| HBV | SAg | 335-343 | WLSLLVPFV | HLA-A*0201 |
| | | | (SEQ. ID NO.:171) | |
| HBV | cAg | 18-27 | FLPSDFFPSV | HLA-A*0201 |
| | | | (SEQ. ID NO.: 172) | |
| HBV | cAg | 18-27 | FLPSDFFPSV | HLA-A*0202 |
| | | | (SEQ. ID NO.:173) | |
| HBV | cAg | 18-27 | FLPSDFFPSV | HLA-A*0205 |
| | | | (SEQ. ID NO.:174) | |
| HBV | cAg | 18-27 | FLPSDFFPSV | HLA-A*0206 |
| | | | (SEQ. ID NO.:175) | |
| HBV | pol | 575-583 | FLLSLOIHL | HLA-A*0201 |
| | | | (SEQ. ID NO:176) | |
| HBV | pol | 816-824 | SLYADSPSV | HLA-A*0201 |
| | | | (SEQ. ID NO.:177) | |
| HBV | pol | 455-463 | GLSRYVARL | HLA-A*0201 |
| | | | (SEQ. ID NO.:178) | |
| HBV | env | 338-347 | LLVPFVQWFV | HLA-A*0201 |
| | | | (SEQ. ID NO.:179) | |
| HBV | pol | 642-650 | ALMPLYACI | HLA-A*0201 |
| | | | (SEQ. ID NO.:180) | |
| HBV | env | 378-387 | LLPIFFCLWV | HLA-A*0201 |
| | | | (SEQ. ID NO.:181) | |
| HBV | pol | 538-546 | YMDDVVLGA | HLA-A*0201 |
| | | | (SEQ. ID NO.:182) | |
| HBV | env | 250-258 | LLLCLIFLL | HLA-A*0201 |
| | | | (SEQ. ID NO.:183) | |
| HBV | env | 260-269 | LLDYQGMLPV | HLA-A*0201 |
| | | | (SEQ. ID NO.:184) | |
| HBV | env | 370-379 | SIVSPFIPLL | HLA-A*0201 |
| | | | (SEQ. ID NO.:185) | |
| HBV | env | 183-191 | FLLTMILTI | HLA-A*0201 |
| | | | (SEQ. ID NO.: 186) | |
| HBV | cAg | 88-96 | YVMVNMGLK | HLA-A*1101 |
| | | | (SEQ. ID NO.:187) | |
| HBV | cAg | 141-151 | STLPETTVVRR | HLA-A*3101 |
| | | | (SEQ. ID NO.: 188) | |
| HBV | can | 141-151 | STLPETTVVRR | HLA-A*6801 |
| | | | (SEQ. ID NO.:189) | |
| HBV | cAg | 18-27 | FLPSDFFPSV | HLA-A*6801 |
| | | | (SEQ. ID NO.: 190) | |
| HBV | sAg | 28-39 | IPQSLDSWWTSL | H2-Ld |
| | | | (SEQ. ID NO.:191) | |
| HBV | cAg | 93-100 | MGLKFRQL | H2-Kb |
| | | | (SEQ. ID NO.:192) | |
| HBV | preS | 141-149 | STBXQSGXQ | HLA-A*0201 |
| | | | (SEQ.ID NO.: 193) | |
| HCMV | gp B | 618-628 | FIAGNSAYEYV | HLA-A*0201 |
| | | | (SEQ. ID NO.: 194) | |
| HCMV | E1 | 978-989 | SDEEFAIVAYTL | HLA-B18 |
| | | | (SEQ. ID NO.: 195) | |
| HCMV | pp65 | 397-411 | DDVWTSGSDSDEELV | HLA-b35 |
| | | | (SEQ. ID NO.: 196) | |
| HCMV | pp65 | 123-131 | IPSINVHHY | HLA-B*3501 |
| | | | (SEQ. ID NO.:197) | |
| HCMV | pp65 | 495-504 | NLVPMVATVO | HLA-A*0201 |
| | | | (SEQ. ID NO.: 198) | |
| HCMV | pp65 | 415-429 | RKTPRVTOGGAMAGA | HLA-B7 |
| | | | (SEQ. ID NO.:199) | |
| HCV | MP | 17-25 | DLMGYIPLV | HLA-A*0201 |
| | | | (SEQ. ID NO.:200) | |
| HCV | MP | 63-72 | LLALLSCLTV | HLA-A*0201 |
| | | | (SEQ. ID NO.: 201) | |
| HCV | MP | 105-112 | ILHTPGCV | HLA-A*0201 |
| | | | (SEQ. ID NO.: 202) | |
| HCV | env E | 66-75 | QLRRHIDLLV | HLA-A*0201 |
| | | | (SEQ. ID NO.:203) | |
| HCV | end E | 88-96 | DLCGSVFLV | HLA-A*0201 |
| | | | (SEQ. ID NO.:204) | |
| HCV | env E | 172-180 | SMVGNWAKV | HLA-A*0201 |
| | | | (SEQ. ID NO.:205) | |
| HCV | NSI | 308-316 | HLHQNIVDV | HLA-A*0201 |
| | | | (SEQ. ID NO.:206) | |
| HCV | NSI | 340-348 | FLLLADARV | HLA-A*0201 |
| | | | (SEQ. ID NO.:207) | |
| HCV | NS2 | 234-246 | GLRDLAVAVEPVV | HLA-A*0201 |
| | | | (SEQ. ID NO.:208) | |
| HCV | NSI | 18-28 | SLLAPGAKQNV | HLA-A*0201 |
| | | | (SEQ. ID NO.:209) | |
| HCV | NSI | 19-28 | LLAPGAKQNV | HLA-A*0201 |
| | | | (SEQ.ID NO.:210) | |
| HCV | NS4 | 199-201 | LLFNILGGWV | HLA-A*0201 |
| | | | (SEQ. ID NO. :211) | |
| HCV | NS3 | 579-587 | YLVAYQATV | HLA-A*0201 |
| | | | (SEQ. ID NO.:212) | |
| HCV | core protein | 34-43 | YLLPRRGPRL | HLA-A*0201 |
| | | | (SEQ. ID NO.:213) | HLA-A*0201 |
| HCV | MP | 63-72 | LLALLSCLTI | HLA-A*0201 |
| | | | (SEQ. ID NO.:214) | |
| HCV | NS4 | 174-182 | SLMAFTAAV | HLA-A*0201 |
| | | | (SEQ. ID NO.:215) | |
| HCV | NS3 | 67-75 | CINGVCWTV | HLA-A*0201 |
| | | | (SEQ. ID NO.:216) | |
| HCV | NS3 | 163-171 | LLCPAGHAV | HLA-A*0201 |
| | | | (SEQ. ID NO.:217) | |
| HCV | NS5 | 239-247 | ILDSFDPLV | HLA-A*0201 |
| | | | (SEQ. ID NO.:218) | |
| HCV | NS4A | 236-244 | ILAGYGAGV | HLA-A*0201 |
| | | | (SEQ. ID NO.:219) | |
| HCV | NS5 | 714-722 | GLQDCTMLV | HLA-A*0201 |
| | | | (SEQ. ID NO.:220) | |
| HCV | NS3 | 281-290 | TGAPVTYSTY | HLA-A*0201 |
| | | | (SEQ. ID NO.:221) | |
| HCV | NS4A | 149-157 | HMWNFISGI | HLA-A*0201 |
| | | | (SEQ. ID NO.:222) | |
| HCV | NS5 | 575-583 | RVCEKMALY | HLA-A*0201-A3 |
| | | | (SEQ. ID NO.:223) | |
| HCV | NS1 | 238-246 | TINYTIFK | HLA-A*1101 |
| | | | (SEQ. ID NO.:224) | |
| HCV | NS2 | 109-116 | YISWCLWW | HLA-A23 |
| | | | (SEQ. ID NO.:225) | |
| HCV | core protein | 40-48 | GPRLGVRAT | HLA-B7 |
| | | | (SEQ. ID NO.:226) | |
| HIV-1 | gp120 | 380-388 | SFNCGGEFF | HLA-Cw*0401 |
| | | | (SEQ. ID NO.:227) | |
| HIV-1 | RT | 206-214 | TEMEKBGKI | H2-Kk |
| | | | (SEQ. ID NO.:228) | |
| HIV-1 | p17 | 18-26 | KIRLRPGGK | HLA-A*0301 |
| | | | (SEQ. ID NO.:229) | |
| HIV-1 | P17 | 20-29 | RLRPGGKKKY | HLA-A*0301 |
| | | | (SEQ. ID NO.:230) | |
| HIV-1 | RT | 325-333 | AIFQSSMTK | HLA-A*0301 |
| | | | (SEQ. ID NO.:231) | |
| HIV-1 | p17 | 84-92 | TLYCVHQRI | HLA-A11 |
| | | | (SEQ. ID NO.:232) | |
| HIV-1 | RT | 508-517 | IYQEPFKNLK | HLA-A11 |
| | | | (SEQ. ID NO.:233) | |
| HIV-1 | p17 | 28-36 | KYKLKHIVW | HLA-A24 |
| | | | (SEQ. ID NO.:234) | |
| HIV-1 | gp120 | 53-62 | LFCASDAKAY | HLA-A24 |
| | | | (SEQ. ID NO.:235) | |
| HIV-1 | gagp24 | 145-155 | QAISPRTLNAW | HLA-A25 |
| | | | (SEQ. ID NO.236) | |
| HIV-1 | gagp24 | 167-175 | EVIMFSAL | HLA-A26 |
| | | | (SEQ. ID NO.:237) | |
| HIV-1 | RT | 593-603 | ETFYVDGAANR | HLA-A26 |
| | | | (SEQ. ID NO.:238) | |
| HIV-1 | gp41 | 775-785 | RLRDLLLIVTR | HLA-A31 |
| | | | (SEQ. ID NO.:239) | |
| HIV-1 | RT | 559-568 | PIQKETWETW | HLA-A32 |
| | | | (SEQ. ID NO.:240) | |
| HIV-1 | gp120 | 419-427 | RIKQIINMW | HLA-A32 |
| | | | (SEQ. ID NO.:241) | |
| HIV-1 | RT | 71-79 | ITLWQRPLV | HLA-A*6802 |
| | | | (SEQ. ID NO.:242) | |
| HIV-1 | RT | 85-93 | DTVLEEMNL | HLA-A*6802 |
| | | | (SEQ. ID NO.:243) | |
| HIV-1 | RT | 71-79 | ITLWQRPLV | HLA-A*7401 |
| | | | (SEQ. ID NO.:244) | |
| HIV-1 | gag p24 | 148-156 | SPRTLNAWV | HLA-B7 |
| | | | (SEQ. ID NO.:245) | |
| HIV-1 | gagp24 | 179-187 | ATPQDLNTM | HLA-B7 |
| | | | (SEQ. ID NO.:246) | |
| HIV-1 | gp120 | 303-312 | RPNNNTRKSI | HLA-B7 |
| | | | (SEQ. ID NO.:247) | |
| HIV-1 | gp41 | 843-851 | HIRRIRQGL | HLA-B7 |
| | | | (SEQ. ID NO.:248) | |
| HIV-1 | p17 | 74-82 | ELRSLYNTY | HLA-B8 |
| | | | (SEQ. ID NO.:249) | |
| HIV-1 | nef | 13-20 | WPTVRERM | HLA-B8 |
| | | | (SEQ. ID NO.:250) | |
| HIV-1 | nef | 90-97 | FLKEKGGL | HLA-B8 |
| | | | (SEQ. ID NO.:251) | |
| HIV-1 | gag p24 | 183-191 | DLNTMLNTV | HLA-B14 |
| | | | (SEQ. ID NO.:252) | |
| HIV-1 | P17 | 18-27 | KIRLRPGGKK | HLA-B27 |
| | | | (SEQ. ID NO.:253) | |
| HIV-1 | p17 | 19-27 | IRLRPGGKK | HLA-B27 |
| | | | (SEQ. ID NO.: 254) | |
| HIV-1 | gp41 | 791-799 | GRRGWEALKY | HLA-B27 |
| | | | (SEQ. ID NO.:255) | |
| HIV-1 | nef | 73-82 | QVPLRPMTYK | HLA-B27 |
| | | | (SEQ. ID NO.:256) | |
| HIV-1 | GP41 | 590-597 | RYLKDQQL | HLA-B27 |
| | | | (SEQ. ID NO.:257) | |
| HIV-1 | nef | 105-114 | RRQDILDLWI | HLA-B*2705 |
| | | | (SEQ. ID NO.:258) | |
| HIV-1 | nef | 134-141 | RYPLTFGW | HLA-B*2705 |
| | | | (SEQ. ID NO.:259) | |
| HIV-1 | p17 | 36-44 | WASRELERF | HLA-B35 |
| | | | (SEQ. ID NO.:260) | |
| HIV-1 | GAG P24 | 262-270 | TVLDVGDAY | HLA-B35 |
| | | | (SEQ. ID NO.:261) | |
| HIV-1 | gp120 | 42-52 | VPVWKEATTTL | HLA-B35 |
| | | | (SEQ. ID NO.:262) | |
| HIV-1 | P17 | 36-44 | NSSKVSQNY | HLA-B35 |
| | | | (SEQ. ID NO.:263) | |
| HIV-1 | gag p24 | 254-262 | PIPVGDIY | HLA-835 |
| | | | (SEQ. ID NO.:264) | |
| HIV-1 | RT | 342-350 | HPDIVIYQY | HLA-B35 |
| | | | (SEQ. ID NO.:265) | |
| HIV-1 | gp41 | 611-619 | TAVPWNASW | HLA-B35 |
| | | | (SEQ. ID NO.:266) | |
| HIV-1 | gag | 245-253 | NPVPVGNIY | HLA-B35 |
| | | | (SEQ. ID NO.:267) | |
| HIV-1 | net | 120-128 | YFPDWQNYT | HLA-B37 |
| | | | (SEQ. ID NO.:268) | |
| HIV-1 | gag p24 | 193-201 | GHQAAMQML | HLA-B42 |
| | | | (SEQ. ID NO.:269) | |
| HIV-1 | p17 | 20-29 | RLRPGGKKKY | HLA-B42 |
| | | | (SEQ. ID NO.:270) | |
| HIV-1 | RT | 438-446 | YPGIKVRQL | HLA-B42 |
| | | | (SEQ. ID NO.:271) | |
| HIV-1 | RT | 591-600 | GAETFYVDGA | HLA-B45 |
| | | | (SEQ. ID NO.:272) | |
| HIV-1 | gag p24 | 325-333 | NANPDCKTI | HLA-B51 |
| | | | (SEQ. ID NO.:273) | |
| HIV-1 | gag p24 | 275-282 | RMYSPTSI | HLA-B52 |
| | | | (SEQ. ID NO.:274) | |
| HIV-1 | gyp120 | 42-51 | VPVWKEATTT | HLA-B*5501 |
| | | | (SEQ. ID NO.:275) | |
| HIV-1 | gag p24 | 147-155 | ISPRTLNAW | HLA-B57 |
| | | | (SEQ. ID NO.:276) | |
| HIV-1 | gag p24 | 240-249 | TSTLOEQIGW | HLA-B57 |
| | | | (SEQ. ID NO.:277) | |
| HIV-1 | gag p24 | 162-172 | KAFSPEVIPMF | HLA-B57 |
| | | | (SEQ. ID NO.:278) | |
| HIV-1 | gag p24 | 311-319 | QASQEVKNW | HLA-B57 |
| | | | (SEQ. ID NO.:279) | |
| HIV-1 | gag p24 | 311-319 | QASQDVKNW | HLA-B57 |
| | | | (SEQ. ID NO.:280) | |
| HIV-1 | nef | 116-125 | HTQGYFPDWQ | HLA-B57 |
| | | | (SEQ. ID NO.:281) | |
| HIV-1 | nef | 120-128 | YFPDWQNYT | HLA-B57 |
| | | | (SEQ. ID NO.:282) | |
| HIV-1 | gag p24 | 240-249 | TSTLQEQIGW | HLA-B58 |
| | | | (SEQ. ID NO.:283) | |
| HIV-1 | p17 | 20-29 | RLRPGGKKKY | HLA-B62 |
| | | | (SEQ. ID NO.:284) | |
| HIV-1 p24 | p24 | 268-277 | LGLNKJVRMY | HLA-B62 |
| | | | (SEQ. ID NO.:285) | |
| HIV-1 | RT | 415-426 | LVGKLNWASQIY | HLA-B62 |
| | | | (SEQ. ID NO.:286) | |
| HIV-1 | RT | 476-485 | ILKEPVHGVY | HLA-B62 |
| | | | (SEQ. ID NO.:287) | |
| HIV-1 | nef | 117-127 | TQGYFPDWQNY | HLA-B62 |
| | | | (SEQ. ID NO.:288) | |
| HIV-1 | net | 84-91 | AVDLSHFL | HLA-B62 |
| | | | (SEQ. ID NO.:289) | |
| HIV-1 | gag p24 | 168-175 | VIPMFSAL | HLA-Cw*0102 |
| | | | (SEQ. ID NO.:290) | |
| HIV-1 | gp120 | 376-384 | FNCGGEFFY | HLA-A29 |
| | | | (SEQ. ID NO.:291 | |
| HIV-1 | gp120 | 375-383 | SFNCGGEFF | HLA-B15 |
| | | | (SEQ. ID NO.:292) | |
| HIV-1 | nef | 136-145 | PLTFGWCYKL | HLA-A*0201 |
| | | | (SEQ. ID NO.:293) | |
| HIV-1 | nef | 180-189 | VLEWRFDSRL | HLA-A*0201 |
| | | | (SEQ. ID NO.:294) | |
| HIV-1 | nef | 68-77 | FPVTPQVPLR | HLA-B7 |
| | | | (SEQ. ID NO.:295) | |
| HIV-1 | nef | 128-137 | TPGPGVRYPL | HLA-B7 |
| | | | (SEQ. ID NO.:296) | |
| HIV-1 | gag p24 | 308-316 | QASQEVKNW | HLA-Cw*0401 |
| | | | (SEQ. ID NO.:297) | |
| HIV-1 IIIB | RT | 273-282 | VPLDEDFRKY | HLA-B35 |
| | | | (SEQ. ID NO.:298) | |
| HIV-1 IIIB | RT | 25-33 | NPDIVFYQY | HLA-B35 |
| | | | (SEQ. ID NO.:299) | |
| HIV-1 IIIB | gp41 | 557-565 | RAIEAQAHL | HLA-B51 |
| | | | (SEQ. ID NO.:300) | |
| HIV-1 IIIB | RT | 231-238 | TAFTIPSI | HLA-B51 |
| | | | (SEQ. ID NO.:301) | |
| HIV-1 IIIB | p24 | 215-223 | VHPVHAGPIA | HLA-B*5501 |
| | | | (SEQ. ID NO.:302) | |
| HIV-1 IIIB | gp120 | 156-165 | NCSFNISTSI | HLA-Cw8 |
| | | | (SEQ. ID NO.:303) | |
| HIV-1 IIIB | gp120 | 241-249 | CTNVSTVQC | HLA-Cw8 |
| | | | (SEQ. ID NO.:304) | |
| HIV-1 5F2 | gp120 | 312-320 | IGPGRAFHT | H2-Dd |
| | | | (SEQ. ID NO.:305) | |
| HIV-1 5F2 | pol | 25-33 | NPDIVIYQY | HLA-B*3501 |
| | | | (SEQ. ID NO.:306) | |
| HIV-1 5F2 | pol | 432-441 | EPIVGAETFY | HLA-B*3501 |
| | | | (SEQ. ID NO.:307) | |
| HIV-1 5F2 | pol | 432-440 | EPIVGAETF | HLA-B*3501 |
| | | | (SEQ. ID NO.:308) | |
| HIV-1 5F2 | pol | 6-14 | SPAHFQSSM | HLA-B*3501 |
| | | | (SEQ. ID NO.:309) | |
| HIV-1 5F2 | pol | 59-68 | VPLDKDFRKY | HLA-B*3501 |
| | | | (SEQ. ID NO.:310) | |
| HIV-1 5F2 | pol | 6-14 | IPLTEEAEL | HLA-B*3501 |
| | | | (SEQ. ID NO.:311) | |
| HIV-1 5F2 | nef | 69-79 | RPQVPLRMTY | HLA-B*3501 |
| | | | (SEQ. ID NO.:312) | |
| HIV-1 5F2 | nef | 66-74 | FPVRPQVPL | HLA-B*3501 |
| | | | (SEQ. ID NO.:313) | |
| HIV-1 5F2 | env | 10-18 | DPNPQEVVL | HLA-B*3501 |
| | | | (SEQ. ID NO.:314) | |
| HIV-1 5F2 | env | 7-15 | RPIVSTQLL | HLA-B*3501 |
| | | | (SEQ. ID NO.:315) | |
| HIV-1 5F2 | pol | 6-14 | IPLTEEAEL | HLA-B51 |
| | | | (SEQ. ID NO.:316) | |
| HIV-1 5F2 | env | 10-18 | DPNPQEVVL | HLA-B51 |
| | | | (SEQ. ID NO.:317) | |
| HIV-1 5F2 | gagp24 | 199-207 | AMQMLKETI | H2-Kd |
| | | | (SEQ. ID NO.:318) | |
| HIV-2 | gagp24 | 182-190 | TPYDrNQML, | HLA-B*5301 |
| | | | (SEQ. ID NO.:319) | |
| HIV-2 | gag | 260-269 | RRWIQLGLQKV | HLA-B*2703 |
| | | | (SEQ. ID NO.:320) | |
| HIV-1 5F2 | gp41 | 593-607 | GIWGCSGKLICTTAV | HLA-B17 |
| | | | (SEQ. ID NO.:321) | |
| HIV-1 5F2 | gp41 | 753-767 | ALIWEDLRSLCLFSY | HLA-B22 |
| | | | (SEQ. ID NO.:322) | |
| HPV 6b | E7 | 21-30 | GLHCYEQLV | HLA-A*0201 |
| | | | (SEQ. ID NO.:323) | |
| HPV 6b | E7 | 47-55 | PLKQHFQIV | HLA-A*0201 |
| | | | (SEQ. ID NO.:324) | |
| HIV11 | E7 | 4-12 | RLVTLKDIV | HLA-A*0201 |
| | | | (SEQ. ID NO.:325) | |
| HPV16 | E7 | 86-94 | TLGIVCPIC | HLA-A*0201 |
| | | | (SEQ. ID NO.:326) | |
| HPV16 | E7 | 85-93 | GTLGIVCPI | HLA-A*0201 |
| | | | (SEQ. ID NO.:327) | |
| HPV16 | E7 | 12-20 | MLDLQPETT | HLA-A*0201 |
| | | | (SEQ. ID NO.:328) | |
| HPV16 | E7 | 11-20 | YMLDLQPETT | HLA-A*0201 |
| | | | (SEQ. ID NO.:329) | |
| HPV-16 | E6 | 15-22 | RPRKLPQL | HLA-B7 |
| | | | (SEQ. ID NO.:330) | |
| HPV16 | E6 | 49-57 | RAHYNIVTF | HW-Db |
| | | | (SEQ. ID NO.:331) | |
| HSV | gpB | 498-505 | SSIEFARL | H2-Kb |
| | | | (SEQ. ID NO.:332) | |
| HSV-1 | gpC | 480-488 | GIGIGVLAA | HLA-A*0201 |
| | | | (SEQ. ID NO.:333) | |
| HSV-1 | ICP27 | 448-456 | DYATLGVGV | H2-Kd |
| | | | (SEQ. ID NO.:334) | |
| Virus | Protein | | T cell epitope MHC ligand | MHC molecule |
| | | | (Antigen) | |
| HSV-1 | ICP27 | 322-332 | LYRTFAGNPRA | H2-Kd |
| | | | (SEQ. ID NO.:335) | |
| HSV-1 | UI.39 | 822-829 | QTFDFGRL | H2-Kd |
| | | | (SEQ. ID NO.:336) | |
| HSV-2 | gpC | 446-454 | GAGIGVAVL | HLA-A*0201 |
| | | | (SEQ. ID NO.:337) | |
| HLTV-1 | TAX | 11-19 | LLFGYPVYV | HLA-A*0201 |
| | | | (SEQ. ID NO.:338) | |
| Influenza | MP | 58-66 | GILGFVFTL | HLA-A*0201 |
| | | | (SEQ. ID NO.:339) | |
| Influenza | MP | 59-68 | ILGFVFTLTV | HLA-A*0201 |
| | | | (SEQ. ID NO.:340) | |
| Influenza | NP | 265-273 | ILROSVAHK | HLA-A3 |
| | | | (SEQ. ID NO.:341) | |
| Influenza | NP | 91-99 | KTGGPIYKR | HLA-A*6801 |
| | | | (SEQ. ID NO.:342) | |
| Influenza | NP | 380-388 | ELRSRYWAI | HLA-B8 |
| | | | (SEQ. ID NO.:343) | |
| Influenza | NP | 381-388 | LRSRYWAI | HLA-B*2702 |
| | | | (SEQ. ID NO.:344) | |
| Influenza | NP | 339-347 | EDLRVLSFI | HLA-B*3701 |
| | | | (SEQ. ID NO.: 345) | |
| Influenza | NSI | 158-166 | GEISPLPSL | HLA-B44 |
| | | | (SEQ. ID NO..:346) | |
| Influenza | NP | 338-346 | FEDLRVLSF | HLA-B44 |
| | | | (SEQ. ID NO.: 347) | |
| Influenza | NSI | 158-166 | GEISPLPSL | HLA-B*4402 |
| | | | (SEQ. ID NO.:348) | |
| Influenza | NP | 338-346 | FEDLRVLSP | HLA-B*4402 |
| | | | (SEQ. ID NO.:349) | |
| Influenza | PBI | 591-599 | VSDGGPKLY | HLA-A1 |
| | | | (SEQ. ID NO.: 3 50) | |
| Influenza A | NP | 44-52 | CTELKLSDY | HLA-A1 |
| | | | (SEQ. ID NO.:351) | |
| Influenza | NSI | 122-130 | AIMDKNIIL | HLA-A*0201 |
| | | | (SEQ. ID NO.:352) | |
| Influenza A | NSI | 123-132 | MDKNIILKA | HLA-A*0201 |
| | | | SEQ. ID NO.:353) | |
| Influenza A | NP | 383:391 | SRYWAIRTR | HLA-B*2705 |
| | | | (SEQ. ID NO.:354) | |
| Influenza A | NP | 147-155 | TYQRTRALV | 112-Kd |
| | | | (SEQ. ID NO.: 355) | |
| Influenza A | HA | 210-219 | TYVSVSTSTL | H2-Kd |
| | | | (SEQ. ID NO.:356) | |
| Influenza A | HA | 518-526 | IYSTVASSL | H2-Kd |
| | | | (SEQ. ID NO.:357) | |
| Influenza A. | HA | 259-266 | FEANGNLI | H2-Kk |
| | | | (SEQ. ID NO.:358) | |
| Influenza A | HA | 10-18 | IEGGWTGMI | H2-Kk |
| | | | (SEQ. ID NO.:359) | |
| Influenza A | NP | 50-57 | SDYEGRLI | H2-Kk |
| | | | (SEQ. ID NO.:360) | |
| Influenza a | NSI | 152-160 | EEGAIVGEI | H2-Kk |
| | | | (SEQ. ID NO,:361) | |
| Influenza A34 | NP | 336-374 | ASNENMETM | H2Db |
| | | | (SEQ. ID N'O.:362) | |
| Influenza A68 | NP | 366-374 | ASNENMDAM | H2Db |
| | | | (SEQ. ID NO.:363) | |
| Influenza B | NP | 85-94 | KLGEFYNQMM | HLA-A*0201 |
| | | | (SEQ. ID NO.:364) | |
| Influenza B | NP | 85-94 | KAGEFYNQMM | HLA-A*0201 |
| | | | (SEQ. ID NO.:365) | |
| Influenza JAP | HA | 204-212 | LYQNVGTYV | H2-Kd |
| | | | (SEQ. ID NO.:366) | |
| Influenza JAP | HA | 210-219 | TYVSVGTSTL | H2-Kd |
| | | | (SEQ. ID No.:367) | |
| Influenza JAP | HA | 523-531 | VYQILATYA | H2-Kd |
| | | | (SEQ. ID NO.:368) | |
| Influenza JAP | HA | 529-537 | IYATVAGSL | H2-Kd |
| | | | (SEQ. ID NO.:369) | |
| Influenza JAP | HA | 210-219 | TYVSVCYfSTI(L>I) | H2-Kd |
| | | | (SEQ. ID NO.:370) | |
| Influenza JAP | HA | 255-262. | FESTGNLI | H2-Kk |
| | | | (SEQ. ID NO.: 371) | |
| JHMV | cAg | 318-326 | APTAGAFFF | H2-Ld |
| | | | (SEQ. ID NO.:372) | |
| LCMV | NP | 118-126 | RPQASGVYM | H2-Ld |
| | | | (SEQ. ID NO.:373) | |
| LCMV | NP | 396-404 | FQPQNGQFI | H2-Db |
| | | | (SEQ. ID NO.:374) | |
| LCMV | GP | 276-286 | SGVENPGGYCL | H2-Db |
| | | | (SEQ. ID NO.:375) | |
| LCMV | GP | 33-42 | KAVYNFATCG | H2-Db |
| | | | (SEQ. ID NO.:376) | |
| MCMV | pp89 | 168-176 | YPHFMPTNL | H2-Ld |
| | | | (SEQ. ID NO.:377) | |
| MHV | spike protein | 510-518 | CLSWNGPHL | H2-Db |
| | | | (SEQ. ID NO,:378) | |
| MMTV | env gp 36 | 474-482 | SFAVATTAL | H2-Kd |
| | | | (SEQ. ID NO.:379) | |
| MMTV | gag p27 | 425-433 | SYETFISRL | H2-Kd |
| | | | (SEQ. ID NO.:380) | |
| MMTV | env gp73 | 544-551 | ANYDFICV | H2-Kb |
| | | | (SEQ. ID NO.: 381) | |
| MuLV | env p15E | 574-581 | KSPWFTTL | H2-Kb |
| | | | (SEQ. ID NO.: 382) ° | |
| | env gp70 | 189-196 | sswDFrrv | H2-Kb |
| | | | (SEQ. ID NO.:383) | |
| MuLV | gag 75K | 75-83 | CCLCLTVFL | H2-Db |
| | | | (SEQ. ID NO.:384) | |
| MuLV | env gp70 | 423-431 | SPSYVYHQP | H2Ld |
| | | | (SEQ. ID NO.:385) | |
| MV | F protein | 437-447 | SRRYPDAVYLH | HLA-B*2705 |
| | | | (SEQ. ID NO.:386) | |
| Mv | F protein | 438-446 | RRYPDAVYL | HLA-B*2705 |
| | | | | |
| | | | (SEQ. ID NO.:387) | |
| Mv | NP | 281-289 | YPALGLHEF | H2-Ld |
| | | | (SEQ. ID NO..388) | |
| Mv | HA | 343-351 | DPVIDRLYL | H2-Ld |
| | | | (SEQ. ID NO.:389) | |
| MV | HA | 544-552 | SPGRSFSYF | H2-Ld |
| | | | (SEQ. ID NO.:390) | |
| Pollovirus | VPI | 111-118 | TYKDTVQL | H2-Kd |
| | | | (SEQ. ID NO.:391) | |
| Pollovirus | VPI | 208-217 | FYDGFSKVPL | H2-Kd |
| | | | (SEQ. ID) NO.: 392) | |
| Pseudorabies Virus gp | G111 | 455-463 | IAGIGILAI | HLA-A*020 |
| | | | (SEQ. ID NO.:393) | |
| Rabiesvirus | NS | 197-205 | VEAEIAHQI | H2-Kk |
| | | | (SEQ. ID NO.:394) | |
| Rotavirus | VP7 | 33-40 | IIYRFLLI | H2-Kb |
| | | | (SEQ. ID NO.:395) | |
| Rotavirus | VP6 | 376-384 | VGPVFPPGM | H2-Kb |
| | | | (SEQ. ID NO.:396) | |
| Rota vims | VP3 | 585-593 | YSGYIFRDL | H2-Kb |
| | | | (SEQ. ID NO.:397) | |
| RSV | M2 | 82-90 | SYIGSINNI | H2-Kb |
| | | | (SEQ, ID NO.:398) | |
| SIV | gagp11c | 179-190 | EGCTPYDTNQML | Mamu-A*01 |
| | | | (SEQ. ID NO.:399) | |
| SV | NP | 324-332 | FAPGNYPAL | H2-Db |
| | | | (SEQ. ID NO.:400) | |
| SV | NP | 324-332 | FAPCTNYPAL | H2-Kb |
| | | | (SEQ. ID NO.:401) | |
| SV40 | T | 404-411 | VVYDFLKC | H2-Kb |
| | | | (SEQ. ID NO.: 402) | |
| SV40 | T | 206-215 | SAINNYAQKL | H2-Db |
| | | | (SEQ. ID NO.:403) | |
| SV40 | T | 223-231 | CKGVNKEYL | H2-Db |
| | | | (SEQ. ID NO.:404) | |
| SV40 | T | 489-497 | QGINNLDNI, | H2-Db |
| | | | (SEQ. ID NO.:405) | |
| SV40 | T | 492-500 (501) | NNLDNLRDY(L) | H2-Db |
| | | | (SEQ. ID NO.:406) | |
| SV40 | T | 560-568 | SEFLLEKRI | H2-Kk |
| | | | (SEQ. ID NO.:407) | |
| VSV | NP | 52-59 | RGYVYQGL | H2-Kb |
| | | | (SEQ. ID NO.:408) | |

**Table2**

| **HLA-A1** | **Position (Antigen)** | **Source** |
|---|---|---|
| T cell epitopes | EADPTGHSY | MAGE-1 161-169 |
| | (SEQ. ID NO.:409) | |
| | VSDGGPNLY | Influenza A PB 1591-599 |
| | (SEQ. ID NO.:410) | |
| | CTELKLSDY | Influenza A NP 44-52 |
| | (SEQ. ID NO.:411) | |
| | EVDPIGHLY | MAGE-3168-176 |
| | (SEQ. ID NO.:412) | |
| HLA-A201 | MLLSYPLLLG | Calreticulin signal sequence 1-10 |
| | (SEQ. ID NO,: 413) | |
| | STBXQSGXQ | HBV PRE-S PROTEIN 141-149 |
| | (SEQ. ID NO.:414) | |
| | YMDGTMSQV | Tyrosinase 369-377 |
| | (SEQ. ID NO.:41 5) | |
| | ILKEPVHGV | HIV-.IRT 476-484 |
| | (SEQ. ID NO.:416) | |
| | LLGFYFTLTV | Influenza MP 59-68 |
| | (SEQ. ID NO.:417) | |
| | LLFGYPYYVV | HTVL-1 tax 11-19 |
| | (SEQ. ID NO.:418) | |
| | GLSPTVWLSV | HBV sAg 348-357 |
| | (SEQ. ID NO.:419) | |
| | WLSLLVPFV | HBY sAg 335-343 |
| | (SEQ. ID NO.:420) | |
| | FLPSDFFPSV | HBV cAg 18-27 |
| | SEQ. ID.:421) | |
| | CLG0LITMV | B8V LMP-2 426-434 |
| | (SEQ. ID NO.:422) | |
| | FLAGNSAVEYV | HCMV gp 618-628B |
| | (SEQ. ID NO.:423) | |
| | KLGEFYNQMM | Influenza BNP 85-94 |
| | (SEQ. ID NO.:424) | |
| | KLVALGMAV | HCV-1 NS3 400..409 |
| | (SEQ. ID NO.425) | |
| | DLMGYDILV | HCV-1 MP 17-.25 |
| | (SEQ. ID NO.:426) | |
| | RLVTLKDIV | HCV 11 EZ 4-12 |
| | (SEQ. ID NO.:427) | |
| | MLLAVLYCL | Tyrosinase. 1-9 |
| | (SEQ. ID NO.:428) | |
| | AAGIGILTV | Melan A\Mart-.127.-35 |
| | (SEQ. ID NO.:429) | |
| | YLEPGPYTA | Pmel 17/gp 100 480-488 |
| | (SEQ. ID NO.:430) | |
| | ILDGTATLRL | Pmel17/ gp 100 457-466 |
| | (SEQ. ID NO.:431) | |
| | LLDGTATLRL | Pmel gp100 457-466 |
| | (SEQ. ID NO.:432) | |
| | ITDQVPFSV | Pmel gp 100 209-217 |
| | (SEQ. ID NO,:433) | |
| | KTWGQYWQV | Pmel gp 100 154-162 |
| | (SEQ. ID NO.:434) | |
| | TITDQVPFSV | Pmel gp 100 208-217 |
| | (SEQ. ID NO.:435) | |
| | AFHIIVAREL | HIV-Inef 190-198 |
| | (SEQ. ID NO.:436) | |
| | YLNKIQNSL | P. falciparum CSP 334-342 |
| | (SEQ. ID NO.:437) | |
| | MMRKLA11.SV | P. falciparum CSP 1-10 |
| | (SEQ. ID NO,:438) | |
| | KAGEFYMQMM. | Influenza BNP 85-94 |
| | (SEQ. NO.:439) | |
| | NIAEGLRAL | EBNA-1 480-48 |
| | (SEQ. NO 440) | |
| | NLRRGTALA | EBNA-1 519-527 |
| | (SEQ. NO 441) | |
| | ALAIPQCRL | EBNA-1 525-533 |
| | (SEQ. ID NO.:442) | |
| | VLKDAIKDL | EBNA-1 575-582 |
| | (SEQ. ID NO.:443) | |
| | FMVFLQTHI | EBNA-1 562-570) |
| | (SEQ. ID NO.:444) | |
| | HLIVDTDS, | EBNA-2 15-23 |
| | (SEQ. ID NO.:445) | |
| | SLGNPSLSV | EBNA-2 22-30 |
| | (SEQ. ID NO.:446) | |
| | PLASAMRML | EBNA-2 126-134 |
| | (SEQ. ID NO.:447) | |
| | RMLWMANYI | EBNA-2 132-140 |
| | (SEQ. ID NO.:448) | |
| | MLWMANYIV | EBNA-2 133-141 |
| | (SEQ. ID NO.:449) | |
| | ILPQGPQIA | EBNA-2151-159 |
| | (SEQ. ID NO.:450) | |
| | PLRPTAPTTI | EBNA-2171-179 |
| | (SEQ. ID NO.:451) | |
| | PLRPTAPTTV | EBNA-2 205-213 |
| | (SEQ. ID NO.:452) | |
| | R M H L P V L H V | EBNA-2 246-254 |
| | (SEQ- ID NO.:453) | |
| | PMPLPPSQL | EBNA-2 287-295 |
| | (SEQ. ID NO.:454) | |
| | QLPPPAAPA | EBNA-2 294-302 |
| | (SEQ. ID NO.:455) | |
| | SMPELSPVL | EBNA-2 381-389 |
| | (SEQ. ID NO.:456) | |
| | DLDESWDYI | EBNA-2 453-46 |
| | (SEQ. ID NO.:457) | |
| | PLPCVLWPW | BZLF1 43-51 |
| | (SEQ. ID NO.:458) | |
| | SLEECDSEL | BZLF1167-175 |
| | (SEQ, ID NO.:459) | |
| | EIKRYKNRV | BZLFH176-184 |
| | (SEQ. ID NO.:460) | |
| | QLLQFIY1-LEV | BZLF1 195-203 |
| | (SEQ.ID NO.:461) | |
| | LLQHYREVA | BZLFI 196-204 |
| | (SEQ. ID NO.:462) | |
| | LLKQMCPSL | BZLFI 217-225 |
| | (SEQ. ID NO.:463) | |
| | SUPRTPDV | BZLFI 229-237 |
| | (SEQ.ID NO.:464) | |
| | AIMDKNHL | Influenza ANSI 122-130 |
| | (SEQ. ID NO.:465) | |
| | IMDKNIILKA | Influenza A NSI 123-132 |
| | (SEQ.ID NO.:466) | |
| | LLALLSCLTV | HCV MP 63-72 |
| | (SEQ.ID NO.:467) | |
| | ILHTPGCV | HCV MP 105-112 |
| | (SEQ.ID NO.:468) | |
| | QLRRHIDLLV | HCV env E 66-75 |
| | (SEQ, ID NO.:469) | |
| | DLCGSVFLV | HCV en E 88-96 |
| | (SEQ. ID NO.:470) | |
| | SMVGNWAKV | HCV env E 172-180 |
| | (SEQ. ID NO.:471) | |
| | HLHQNIVDV | HCV NSI 308-316 |
| | (SEQ.ID NO.:472) | |
| | FLLLADAR V | HCV NSI 340-348 |
| | (SEQ.ID NO.:473) | |
| | GLRDLAVAVEPV | HCV NSI234-246 |
| | (SEQ. ID NO.:474) | |
| | SLLAPGAKQNV | HCV NSI 18-28 |
| | (SEQ.ID NO.:475) | |
| | LLAPGAKQNV | HCV NSI19-28 |
| | (SEQ. ID NO.:476) | |
| | FLLSLGIHL | HBV pol 575-583 |
| | (SEQ. ID NO.:477) | |
| | SLYADSPSV | B16-B24 |
| | (SEQ. ID NO.:478) | |
| | GLSRYVARL | HBV POL 455 463 |
| | (SEQ. ID NO.:479) | |
| | KIFGSLAFL | HER-2 369-377 |
| | (SEQ. ID NO.:480) | |
| | ELVSEFSRM | HER-2 971-979 |
| | (SEQ. ID NO.:481) | |
| | KLTPLCVTL | HIV-1 gp 160 120-128 |
| | (SEQ. ID NO.:482) | |
| | SLLNATDIAV | HIV-I GP 160 814-823 |
| | (SEQ. ID.NO.:483) | |
| | VLYRYGSFSV | PmeI gp100 476-485 |
| | (SEQ. ID NO.:484) | |
| | YIGEVLVSV | Non-filament forming class 1 myosin family (HA.-2)** |
| | (SEQ. ID NO.:485) | |
| | LLFNILGGWV | HCV NS4 192-201 |
| | (SEQ. ID NO.486) | |
| | LLVPFVQWPW | HBV env 338-347 |
| | (SEQ. ID NO. :487) | |
| | ALMPLYACI | HBV pol 642-G.Sb |
| | (SEQ. ID NO.:4S8) | |
| | YLVAYQATV | HCV NS3 579-587 |
| | (SEQ. ID NO.:489) | |
| | TLGFVCPIC | HIPV 16 E7 86-94 |
| | (SEQ. ID NO.:490) | |
| | YLLPRRGPRL | HCV core protein 34-43 |
| | (SEQ.ID NO.:491) | |
| | LLPIFFCLWV | HBV env 378-387 |
| | (SEQ. ID NO.:492) | |
| | YMDDVVLGA | HBV Pol 538-546 |
| | (set. ID NO.:493) | |
| | GTLGIVCPI | HPV16 E7 85-93 |
| | (SEQ. ID NO. :494) | |
| | LLALLSCLTI | HCV MP 63-72 |
| | (SEQ. ID NO,:495) | |
| | MLDLQPETT | HPV 1 E712-20 |
| | (SEQ. ID NO.:496) | |
| | SLMAFTAAV | HCV NS4174-182 |
| | (SEQ. ID NO.:497) | |
| | CINGVCWTV | HCV NS3 67-75 |
| | (SEQ. ID NO.:498) | |
| | VMNILLQYVV | Glutamic acid decarboxylase 114-123 |
| | (SEQ. ID NO.:499) | |
| | ILTVILGVL | Man A/Mart-32, 40 |
| | (SEQ.ID NO.:500) | |
| | FLWGPRALV | MAGE-3 271-279 |
| | (SEQ.ID NO.:501) | |
| | LLCPAGHAV | HCV NS3 163-171 |
| | (SEQ. ID NO.:502) | |
| | ILDSFDPLV | HCV NSS 239-247 |
| | (SEQ. ID NO.:503) | |
| | LLLCLIFLL | HBV env 250-258 |
| | (SEQ. ID NO.:504) | |
| | LIDYQGMLPV | HBV env 260-269 |
| | (SEQ. ID NO.:505) | |
| | SIVSPFIPLL | HBV env 370-379 |
| | (SEQ. ID NO.:506) | |
| | FLLTRILTI | HBV env 183-191 |
| | (SEQ. ID NO.:507) | |
| | HLGNVKYLV | P. faciparum TRAP 3-11 |
| | (SEQ. ID NO.:508) | |
| | GIAGGLALL | P. faciparum TRAP 500-508 |
| | (SEQ. ID NO.:509) | |
| | ILAGYGAGV | HCV NS S4A 236-244 |
| | (SEQ. ID NO.:510) | |
| | GLQDCTMLV | HCV NS5 714-722 |
| | (SEQ. ID NO.:511) | |
| | TGAPVTYSTY | HCV NS3 281-290 |
| | (SEQ. ID NO.:512) | |
| | VIYQYMDDLV | HIV-IRT 179-187 |
| | (SEQ. ID NO.:513) | |
| | VLPDVFIRCV | N-acetylglucosaminyltransferase V |
| | | Gnt-V intron |
| | (SEQ. ID NO.:514) | |
| | VLPDVFIRC | N-acetylglucosaminyltransferase V |
| | | Gnt-V intron |
| | (SEQ. ID NO.:515) | |
| | AVGIGIAVV | Human CD9 |
| | (SEQ. ID NO.:516) | |
| | LWLGLLAV | Human glutamyltransferase |
| | (SEQ. ID NO.:517) | |
| | ALGLGLLPV | Human G protein coupled receptor |
| | (SEQ. ID NO.:518) | |
| | 164-172 | |
| | GIGIGVLAA | HSV-I gp C 480-488 |
| | (SEQ. ID NO.:519) | |
| | GAGIGVAVL | HSV-2 gp C 446-454 |
| | (SEQ. ID NO.:520) | |
| | IAGIGILAI | Pseudorabies gpGIN 455-463 |
| | (SEQ. ID NO.:521) | |
| | LIVIGILIL | Adenovirus 3 E3 9kD 30-38 |
| | (SEQ. ID NO.:522) | |
| | LAGIGLIAA | S. Lincolnensis ImrA |
| | (SEQ. ID NO.:523) | |
| | VDGIGILTI | Yeast ysa-1 77-85 |
| | (SEQ. ID NO.:524) | |
| | GAGIGVLTA | B. polymyxa, βendoxylanase 149-157 |
| | (SEQ. ID NO.:525) | |
| | 157 | |
| | AAGIGIIQI | E. coli methionine synthase 590-598 |
| | (SEQ. ID NO.:526) | |
| | QAGIGILLA | E. coli hypothetical protein 4-12 |
| | (SEQ. ID NO.:527) | |
| | KARDPHSGHFV | CDK4wl 22.32 |
| | (SEQ. ID NO.:528) | |
| | KACDPI-ISGIIFV | CDK4-R24C 22-32 |
| | (SEQ. ID NO.:529) | |
| | ACDPFISGHFV | CDK4-R24C 23-32 |
| | (SEQ. ID NO.:530) | |
| | SLYNTVATL | HIV-I gag p 17 77-85 |
| | (SEQ. ID NO.:531) | |
| | ELVSEFSRV | HER-2, m>V substituted 971-979 |
| | (SEQ. ID NO.:532) | |
| | RGPGRAFVTI | HIV-1 I gp 160 315-329 |
| | (SEQ. ID NO.:533) | |
| | HMWNFISGI | HCVNS4A 149-157 |
| | (SEQ. ID NO.:534) | |
| | NLVPMVATVQ | HCMV pp65 495-504 |
| | (SEQ. ID NO.:535) | |
| | GLHCYEQLV | HPV 6b E7 21-30 |
| | (SEQ. ID NO.:536) | |
| | PLKQHFQIV | HPV 6b E7 47-55 |
| | (SEQ. ID NO.:537) | |
| | LLDFYRFMGV | EBNA-6 284-293 |
| | (SEQ. ID NO.:538) | |
| | AIMEKNIML | Influenza Alaska NS 1 122-130 |
| | (SEQ. ID NO.:539) | |
| | YLKTIQNSL | P. falciparum cp36 CSP |
| | (SEQ. ID NO.:540) | |
| | YLNKIQNSL | P. falclparum cp39 CSP |
| | (SEQ. ID NO.: 541) | |
| | YMLDLQPETT | HPV 16 E7 11-20* |
| | (SEQ. ID NO.:542) | |
| | LLMGTLGIV | HPV16 E7 82-90** |
| | (SEQ. ID NO.:543) | |
| | TLGIVCPI | HPV 16 E7 86-93 |
| | (SEQ. ID NO.:544) | |
| | TLTSCNTSV | HIV-1 gp120 197-205 |
| | (SEQ. ID NO.:545) | |
| | KLPQLCTEL | HPV 16 E6 18-26 |
| | (SEQ. ID NO.:546) | |
| | TIHDIILEC | HPV16 E6 29-37 |
| | (SEQ. ID NO.:547) | |
| | LGIVCPICS | HPV16 E7 87-95 |
| | (SEQ. ID NO.:548) | |
| | VILGVLLLI | Melan A/Mart-1 35-43 |
| | (SEQ. ID NO.:549) | |
| | ALMDKSLHV | Melan A/Mart-1 56-64 |
| | (SEQ. ID NO.:550) | |
| | GILTVILGV | Melan A/Mart-1 31-39 |
| | (SEQ. ID NO.:551) | |
| T cell epitopes, | MINAYLDKL | P. Falciparum STARP 523-531 |
| | (SEQ. ID NO.:552) | |
| | AAGIGILTV | Melan A/Mart- 127-35 |
| | (SEQ. ID NO.:553) | |
| | FLPSDFFPSV | HBV cAg 18-27 |
| | (SEQ. ID NO.:554) | |
| Motif unknown | SVRDRLARL | EBNA-3 464-472 |
| T cell epitopes | (SEQ. ID NO.:555) | |
| T cell epitopes | AAGIGILTV | Melan A/Mart-1 27-35 |
| | (SEQ. ID NO.:556) | |
| | FAYDGKDYI | Human MHC I-ot 140-148 |
| | (SEQ. ID NO.:557) | |
| T cell epitopes | AAGIGILTV | Melan A/Mart-1 27-35 |
| | (SEQ. ID NO.:558) | |
| | FLPSDFFPSV | HBV cAg 18-27 |
| | (SEQ. ID NO.:559) | |
| Motif unknown | AAGMILTV | Meland A/Mart-1 27-35 |
| T cell epitopes | (SEQ. ID NO.:560) | |
| | FLPSDFFPSV | HBV cAg 18-27 |
| | (SEQ. ID NO.:561) | |
| | AAGIGILTV | Melan A/Mart-1 27-35 |
| | (SEQ. ID NO.:562) | |
| | ALLAVGATK | Pmell7 gp 100 17-25 |
| | (SEQ. ID NO.:563) | |
| T cell epitopes | RLRDLLLIVTR | HIV-1 gp41 768-778 |
| | (SEQ. ID NO.: 564) | |
| | QVPLRPMTYK | HIV-1 nef 73-82 |
| | (SEQ. ID NO.:565) | |
| | TVYYGVPVWK | HIV-1 gp120-36-45 |
| | (SEQ. ID NO.:566) | |
| | RLRPGGKKK | HIV- 1 gag p 17 20-29 |
| | (SEQ. ID NO.:567) | |
| | ILRGSVAHK | Influenza NP 265-273 |
| | (SEQ. ID NO.:668) | |
| | RLRAEAGVK | EBNA-3 603-611 |
| | (SEQ. ID NO.:569) | |
| | RLRDLLLIVTR | HIV-1 gp41 770-780 |
| | (SEQ. ID NO.:570) | |
| | VYYGVPVWK | HIV-1 GP 120 38-46 |
| | (SEQ. ID NO.:571) | |
| | RVCEKMALY | HCV NS5 575-583 |
| | (SEQ. ID NO.:572) | |
| Motif unknown | KIFSEVTLK | Unknown; muta melanoma peptide ted |
| | | (p I 83L) 175-183 |
| T cell epitope | (SEQ. ID NO.:573) | |
| | YVNVNMGLK* | HBV cAg 88-96 |
| | (SEQ. ID NO.:574) | |
| T cell epitopes | IVTDFSVIK | EBNA-4 416-424 |
| | (SEQ. ID NO.:575) | |
| | ELNEALELK | P53 343-351 |
| | (SEQ. ID NO.:576) | |
| | VPLRPMTYK | HIV- 1 NEF 74-82 |
| | (SEQ. ID NO.:577) | |
| | AIFQSSMTK | HIV- I gag p24 325-333 |
| | (SEQ. ID NO.:578) | |
| | QVPLRPMTYK | HIV-1 nef 73-82 |
| | (SEQ. ID NO.:579) | |
| | TRNYTIFK HCV | NSI 238-246 |
| | (SEQ. ID NO.:580) | |
| | AAVDLSHFLKEK | HIV-1 nef 83-94 |
| | (SEQ. ID NO.:581) | |
| | ACQGVGGPGGHK | HIV-1 II 1B p24 349.-359 |
| | (SEQ. ID NO.:581) | |
| HLA-A24 | SYLDSGIHF* | β-catenin, mutated (proto-onocogen) |
| | | 29-37 |
| | (SEQ. ID NO.:582) | |
| T cell epitopes | RYLKDQQLL | HIV GP 41 583-591 |
| | (SEQ. ID NO.:583) | |
| | AYGLDPYIL | P 15 melanoma Ag 10-18 |
| | (SEQ. ID NO.: 5 84) | |
| | AFLPWHRLFL | Tyrosinase 206-215 |
| | (SEQ. ID NO.:585) | |
| | AFLPWHRLF | Tyrosinase 206-214 |
| | (SEQ. ID NO.:586) | |
| | RYSIFFDY | Ebna-3 246-253 |
| | (SEQ. ID NO.:587) | |
| T cell epitope | ETINEEAAEW | HIV-1 gag p24 203-212 |
| | (SEQ. ID NO.:588) | |
| T cell epitopes | STLPETTVVRR | HBV cAg 141 -151 |
| | (SEQ. ID NO.:589) | |
| | MSLQRQFLR | ORF 3P-gp75 294-321 (bp) |
| | (SEQ. ID NO.:590) | |
| | LLPGGRPYR | TRP (tyrosinase rel.) 197-205 |
| | (SEQ. ID NO.:591) | |
| T cell epitope | IVGLNKIVR | HIV gag p24 267-267-275 |
| | (SEQ. ID NO.:592) | |
| | AAGIGILTV | Melan A/Mart- 127 35 |
| | (SEQ. ID NO.:593) | |

Table 3 sets forth additional antigens useful in the invention that are available from the Ludwig Cancer Institute. The Table refers to patents in which the identified antigens can be found. TRA refers to the tumor-related antigen and the LUD No. refers to the Ludwig Institute number.

**Table 3**

| TRA | LUD No. | Patent No. | Date Patent Issued | Peptide (Antigen) | HLA |
|---|---|---|---|---|---|
| MAGE-4 | 5293 | 5,405,940 | 11 April 1995 | EVDPASNTY | HLA-AI |
| | | | | (SEQ. ID NO.:532) | |
| MAGE-41 | 5293 | 5,405,940 | 11 April 1995 | EVDPTSNTY | HLA-A1 |
| | | | | (SEQ ID NO.:533) | |
| MAGE-5 | 5293 | 5,405,940 | 11 April 1995 | EADPTSNTY | HLA-A 1 |
| | | | | (SEQ ID NO:534) | |
| MAGE-51 | 5293 | 5,405,940 | 11 April 1995 | EADPTSNTY | HLA-A 1 |
| | | | | (SEQ ID NO:534) | |
| MAGE-6 | 5294 | 5,405,940 | 11 April 1995 | EVDPIGHVY | HLA-A1 |
| | | | | (SEQ ID NO:535) | |
| | 5299.2 | 5,487,974 | 30 January 1996 | MLLAVLYCLL | HLA-A2 |
| | | | | (SEQ ID NO:536) | |
| | 5360 | 5,530,096 | 25 June 1996 | MLLAVLYCL | HLA-B44 |
| | | | | (SEQ ID NO:537) | |
| Tyrosinase | 5360.1 | 5,519,11 | 21 May 1996 | SEIWRDIDFA | HLA-B44 |
| | | | | (SEQ ID NO:538) | |
| | | | | SEIWRDIDF | |
| | | | | (SEQ ID NO:539) | |
| Tyrosinase | 5431 | 5,774,316 | 28 April 1998 | XEIWRDIDF | HLA-B44 |
| | | | | (SEQ ID NO:540) | |
| MAGE-2 | 5340 | 5,554,724 | 10 September 1996 | STLVEVTLGEV | HLA-A2 |
| | | | | (SEQ ID NO:541) | |
| | | | | LVEVTLGEV | |
| | | | | (SEQ ID NO:542) | |
| | | | | VIFSKASEYL | |
| | | | | (SEQ ID NO:543) | |
| | | | | IIVL-AIIAI | |
| | | | | (SEQ ID NO:544) | |
| (Continued) | | | | KIWEELSMLEV | |
| | | | | (SEQ ID NO:.545) | |
| | | | | LIETSYVKV | |
| | | | | (SEQ ID NO:546) | |
| | 5327 | 5,585,461 | 17 December 1996 | FLWGPRALV | HLA-A2 |
| | | | | (SEQ ID NO: 547) | |
| | | | | TLVEVTLGEV | |
| | | | | (SEQ ID NO:548) | |
| | | | | ALVETSYVKV | |
| | | | | (SEQ ID NO:549) | |
| MAGE-3 | 5344 | 5,554,506 | 10 September 1996 | KIWEELSVL | HLA-A2 |
| | | | | (SEQ ID NO:550) | |
| MAGE-3 | 5393 | 5,405,940 | 11 April 1995 | EVDPIGHLY | HLA-A1 |
| | | | | (SEQ ID NO:551) | |
| MAGE | 5293 | 5,405,940 | 11 April 1995 | EXDX5Y | HLA-A1 |
| | | | | (SEQ. ID NO.:552) | |
| | | | | (but not EADPTGHSY) | |
| | | | | (SEQ. ID NO.:553) | |
| | | | | E (A/V) D X5 Y | |
| | | | | (SEQ. ID NO.:554) | |
| | | | | E(A/V)DPX4Y | |
| | | | | (SEQ. ID NO.:555) | |
| | | | | E (A/V) D P (I/A/T) X3 Y | |
| | | | | (SEQ. ID NO.:556) | |
| | | | | E (A/V) D P (I/A/T) (G/S) X2 Y | |
| | | | | (SEQ. ID NO.:557) | |
| | | | | E (A/V) D P (I/A/T) (G/S) (H/N) X Y | |
| | | | | E (A/V) DP (I/A/T) (G/S) (H/N) (L/T/V) Y | |
| | | | | (SEQ. ID NO.:559) | |
| MAGE-1 | 5361 | 5,558.995 | 24 September 1996 | ELHSAYGEPRKLLTQD | HLA-C |
| | | | | (SEQ ID NO:560) | Clone 10 |
| | | | | EHSAYGEPRKLL | |
| | | | | (SEQ ID NO:561) | |
| | | | | SAYGEPRKL | |
| | | | | (SEQ ID NO:562) | |
| MAGE-1 | 5253.4 | TBA | TBA | EADPTGHSY | HLA-A 1 |
| | | | | (SEQ ID NO:563) | |
| BAGE | 5310.1 | TBA | TBA | MAARAVFLALSAQLLQARLMKE | HLA-C |
| | | | | (SEQ ID NO:564) | Clone 10 |
| | | | | MAARAVFLALSAQLLQ | HLA-C |
| | | | | (SEQ ID NO:565) | Clone 10 |
| | | | | AARAVFLAL | HLA-C |
| | | | | (SEQ ID NO:566) | Clone 10 |
| | | | | | |
| GAGE | 5323.2 | 5,648,226 | 15 July 1997 | YRPRPRRY | HLA-CW6 |
| | | | | (SEQ. ID NO:567) | |

**Table 4**

| **Source** | **Protein** | **AA Position** | **MHC molecules** | **T cell epitope MHC ligand (Antigen)** | **Ref.** |
|---|---|---|---|---|---|
| synthetic peptides | synthetic peptides | synthetic peptides | HLA-A2 | ALFAAAAAV | Parker, et al., "Scheme for ranking potential HLA-A2 binding peptides based on independent binding of individual peptides sidechains," J. Immunol. 152: 163-175 |
| | | | " | GIFGGVGGV | " |
| | | | " | GLDKGGGV | " |
| | | | " | GLFGGFGGV | " |
| | | | " | GLFGGGAGV | " |
| | | | " | GLFGGGEGV | " |
| | | | " | GLFGGGFGV | " |
| | | | " | GLFGGGGGL | " |
| | | | " | GLFGGGGGV | " |
| | | | " | GLFGGGVGV | " |
| | | | " | GLFGGVGGV | " |
| | | | " | GLFGGVGKV | " |
| | | | " | GLFKGVGGV | " |
| | | | " | GLGGGGFGV | " |
| | | | " | GLLGGGVGV | " |
| | | | " | GLYGGGGGV | " |
| | | | " | GMFGGGGGV | " |
| | | | " | GMFGGVGGV | " |
| | | | " | GQFGGVGGV | " |
| | | | " | GVFGGVGGV | " |
| | | | " | KLFGGGGGV | " |
| | | | " | KLFGGVGGV | " |
| | | | " | AILGFVFTL | " |
| | | | " | GAIGFVFTL | " |
| | | | " | GALGFVFTL | " |
| | | | " | GELGFVFTL | " |
| | | | " | GIAGFVFTL | " |
| | | | " | GIEGFVFTL | " |
| | | | " | GILAFVFTL | " |
| | | | " | GILGAVFTL | " |
| | | | " | GILGEVFTL | " |
| | | | " | GILFGAFTL | " |
| | | | " | GILGFEFTL | " |
| | | | " | GILGFKFTL | " |
| | | | " | GILGFVATL | " |
| | | | " | GILGFVETL | " |
| | | | " | GILGFVFAL | " |
| | | | " | GILGFVFEL | " |
| | | | " | GILGFVFKL | " |
| | | | " | GILGFVFTA | " |
| | | | " | GILGFVFTL | " |
| | | | " | GILGFVFVL | " |
| | | | " | GILGFVKTL | " |
| | | | " | GILGKVFTL | " |
| | | | " | GILKFVFTL | " |
| | | | " | GILPFVFTL | " |
| | | | " | GIVGFVFTL | " |
| | | | " | GKLGFVFTL | " |
| | | | " | GLLGFVFTL | " |
| | | | " | GQLGFVFTL | " |
| | | | " | KALGFVFTL | " |
| | | | " | KILGFVFTL | " |
| | | | " | KILGKVFRL | " |
| | | | " | AILLGVFML | " |
| | | | " | AIYKRWIIL | " |
| | | | " | ALFFFDIDL | " |
| | | | " | ATVELLSEL | " |
| | | | " | CLFGYPVYV | " |
| | | | " | FIFPNYTIV | " |
| | | | " | IISLWDSQL | " |
| | | | " | ILASLFAAV | " |
| | | | " | ILESLFAAV | " |
| | | | " | KLGEFFNQM | " |
| | | | " | KLGEFYNQM | " |
| | | | " | LLFGYPVYV | " |
| | | | " | LLWKGEGAV | " |
| | | | " | LMFGYPVYV | " |
| | | | " | LNFGYPVYV | " |
| | | | " | LQFGYPVYV | " |
| | | | " | NIVAKRFKV | " |
| | | | " | NLPMVATV | " |
| | | | " | QMLLAIARL | " |
| | | | " | QMWQARLTV | " |
| | | | " | RLLQTGIHV | " |
| | | | " | RLVNGSLAL | " |
| | | | " | SLYNTVATL | " |
| | | | " | TLNAWVKVV | " |
| | | | " | WLYRETCNL | " |
| | | | " | YLFKRMIDL | " |
| | | | " | GAFGGVGGV | " |
| | | | " | GAFGGVGGY | " |
| | | | " | GEFGGVGGV | " |
| | | | " | GGFGGVGGV | " |
| | | | " | GIFGGGGGV | " |
| | | | " | GIGGFGGGL | " |
| | | | " | GIGGGGGGL | " |
| | | | " | GLDGGGGGV | |
| | | | " | GLDGKGGGV | " |
| | | | " | GLDKKGGGV | " |
| | | | " | GLFGGGFGF | " |
| | | | " | GLFGGGFGG | " |
| | | | " | GLFGGGFGN | " |
| | | | " | GLFGGGFGS | " |
| | | | " | GLFGGGGGI | " |
| | | | " | GLFGGGGGM | " |
| | | | " | GLFGGGGGT | " |
| | | | " | GLFGGGGGY | " |
| | | | " | GLGFGGGGV | " |
| | | | " | GLGGFGGGV | " |
| | | | " | GLGGGFGGV | " |
| | | | " | GLGGGGGFV | " |
| | | | " | GLGGGGGGY | " |
| | | | " | GLGGGVGGV | " |
| | | | " | GLLGGGGGV | " |
| | | | " | GLPGGGGGV | " |
| | | | " | GNFGGVGGV | " |
| | | | " | GSFGGVGGV | " |
| | | | " | GTFGGVGGV | " |
| | | | " | AGNSAYEYV | " |
| | | | " | GLFPGQFAY | " |
| | | | " | HILLGVFML | " |
| | | | " | ILESLFFRAV | " |
| | | | " | KKKYKLKHI | " |
| | | | " | MLASIDLKY | " |
| | | | " | MLERELVRK | " |
| | | | " | KLFGFVFTV | " |
| | | | " | ILDKKVEKV | " |
| | | | " | ILKEPVHGV | " |
| | | | " | ALFAAAAAY | |
| | | | " | GIGFGGGGL | " |
| | | | " | GKTGGVGGV | " |
| | | | " | GLFGGGGGK | " |
| | | | " | EILGFVFTL | " |
| | | | " | GIKGFVFTL | " |
| | | | " | GQLGFVFTK | " |
| | | | " | ILGFVFTLT | " |
| | | | " | KILGFVFTK | " |
| | | | " | KKLGFVFTL | " |
| | | | " | KLFEKVYNY | " |
| | | | " | LRFGYPVYV | " |
| Human | HSP60 | 140-148 | HLA-B27 | IRRGVMLAV | Rammensee et al. 1997 |
| | | | | | 160 |
| " | " | 369-377 | " | KRIQEHEQ | " |
| | " | 469-477 | " | KRTLKIPAM | " |
| Yersinia | HSP60 | 35-43 | " | GRNVVLDKS | " |
| " | " | 117-125 | " | KRGIDKAVI | " |
| " | " | 420-428 | " | IRAASAITA | " |
| | HSP 60 | 284-292 | HLA-B*2705 | RRKAMFEDI | 169 |
| P.falciparum | LSA-1 | 1850-1857 | HLA-B3501 | KPKDELDY | 170 |
| Influenza NP | | 379-387 | HLA-B*4402 | LELRSRYWA | 183 |
| | Tum-P35B | 4-13 | HLA-D^{d} | GPPHSNNFGY | 230 |
| Rotavirus | VP7 | 33-40 | | IIYRFLLI | 262 |
| | OGDH (F108Y) | 104-112 | H2-L^{d} | QLSPYPFDL | 253 |
| | TRP-2 | 181-188 | p287 | VYDFFVWL | 284 |
| | DEAD box | 547-554 | p287 | SNFVFAGI | 283 |
| | p 68 | | | | |
| | Vector "artefact" | | p287 | SVVEFSSL | 260 |
| | Epiopemimic of tumor Ag | | p287 | AHYLFRNL | 278 |
| | | | " | THYLFRNL | |
| | Epitope mimic of H-3 miHAg^{■} | | " | LIVIYNTL | 279 |
| | | | " | LIYEFNTL | " |
| | | | " | IPYIYNTL | " |
| | | | | IIYIYHRL | " |
| | | | " | LIYIFNTL | |
| | HBV cAg | 93-100 | " | MGLKFRQL | 280 |
| Human | autoantigen LA | 51-58 | " | IMIKFRNRL | 281 |
| Mouse | UTY protein | | H2D^{b} | WMHHNMDLI | 303 |
| Mouse | p53 | 232-240 | " | KYMCNSSCM | 302 |
| MURINE | MDM2 | 441-449 | " | GRPKNGCIV | 277 |
| | Epitope mimic of natural | | " | AQHPNAELL | 278 |
| | MuLV gag75K | 75-83 | " | CCLCLTVFL | 301 |
| P. Falciparum | CSP | 375-383 | p290 | YENDIEKK | 315 |
| " | " | 371-379 | " | DELDYENDI | 315 |
| HIV | -IRT | 206-214 | " | TEMEKEGKI | 316 |
| Rabies | NS | 197-205 | | VEAEIAHQI | 309,310 |
| Influenza A | NSI | 152-160 | " | EEGAIVGEI | 304 |
| Murine | SMCY | | p291 | TENSGKDI | 317 |
| | MHC class I leader | 3-11 | p293 | AMAPRTLLL | 318 |
| | ND1 alpha | 1-12 | p293 | FFINILTLLVP | 323 |
| | ND Beta | 1-12 | p293 | FFINILTLLVP | 323 |
| | ND alpha | 1-17 | " | FFNILTLLVPILIAM | 324 |
| | ND Beta | 1-17 | " | FFINALTLLVPILIAM | " |
| | COI mitochondrial | 1-6 | " | FINRW | 325 |
| L. monocytogenes | LemA | 1-6 | " | IGWII | 326 |
| | SIV gag p11C | 179-190 | Mamu-A*01 | EGCTPYDINQML | 334 |
| | | | | | |
| | MAGE-3 | | HLA-A2 | ALSRKVAEL | 5,554,506 |
| | | | | IMPKAGLLI | " |
| | | | " | KIWEELSVL | " |
| | | | " | ALVETSYVKV | " |
| | | | " | | |
| | | | " | | " |
| | | | " | | " |
| | | | " | | " |
| | | | " | | " |
| | | | | | |
| | peptides which bind to MHCs | | HLA-A2 | | 5,989,565 |
| | | | " | | " |
| | | | " | | " |
| | | | " | | " |
| | | | " | | " |
| | | | " | | " |
| | | | " | | " |
| | | | " | | " |
| | | | " | | " |
| | | | " | | " |
| | | | " | | " |
| | | | | | |
| | Peptides from MAGE-1 | | HLA-C clone 10 | | 5,558,995 |
| | | | " | | " |
| | | | " | | " |
| | | | | | |
| | GAGE | | HLA-Cw6 | | 5,648,226 |
| | | | " | | " |
| | | | " | | " |
| | | | " | | " |
| | | | " | | " |
| | | | " | | " |
| | | | " | | " |
| | MAGE | | HLA-A1, primarily | Isolated nonapeptide having Glu at its N terminal, Tyr at its C-terminal, and Asp at the third residue from its N terminal, with the proviso that said isolated nonapeptide is not Glu Ala Asp Pro Thr Gly His Ser Tyr (SEQ ID NO: 1), and wherein aid isolated nonapeptide binds to a human leukocyte antigen molecule on a cell to form a complex, said complex provoking lysis of said | 5,405,940 |
| | | | | cell by a cytolytic T cell specific to said complex | |
| | | | " | | " |
| | | | " | | " |
| | | | " | | " |
| | | | " | | " |
| | | | " | | " |
| | | | " | | " |
| | | | " | | " |
| | | | " | | " |
| | | | " | | " |
| | | | " | | " |
| | | | " | | " |
| | | | " | | " |
| | | | " | | |
| | | | " | | " |
| | | | " | | " |
| | | | " | | " |
| | | | " | | " |
| | MAGE peptides | | HLA-A2 | | 5,554,724 |
| | " | | " | | " |
| | " | | " | | |
| | " | | " | | " |
| | " | | " | | " |
| | " | | " | | " |
| | " | | " | | |
| | " | | " | llelleValLeuAlallelleAlalle | " |
| | " | | " | | " |
| | " | | " | | " |
| | " | | " | | " |
| | " | | " | | |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | | | | | " |
| | " | | " | | " |
| | " | | " | | " |
| | " | | " | | " |
| | " | | " | | " |
| | " | | | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | " | | " | | " |
| | Mage-3 peptides | | HLA-A2 | | 5,585,461 |
| | " | | " | | " |
| | " | | " | | " |
| | " | | " | | " |
| | " | | " | | " |
| | " | | " | | " |
| | | | | | |
| | Tyrosinase | | HLA-A2 | | 5,487,974 |
| | " | | " | | " |
| | | | | | |
| | Tyrosinase | | HLA-A2 | | 5,530,096 |
| | " | | " | | " |
| | Tyrosinase | | HLA-A2 and HLA-B44 | | 5,519,117 |
| | " | | " | | " |
| | " | | " | | " |
| | Melan | | | EAAGIGILTV | Jäger, E. et al. |
| | A/MART-1 | | | | Granulocyte-macrophage-colony-stimulating Factor Enhances Immune Responses To Melanoma-associated Peptides in vivo Int. J Cancer 67, 54-62 (1996) |
| | Tyrosinase | | | MLLAVLYCL | |
| | " | | | YMDGTMSQV | " |
| | gp 100/Pmel 17 | | | YLEPGPVTA | " |
| | " | | | LLDGTATLRL | " |
| | Influenza matrix | | | GILGFVFTL | " |
| | MARGE-1 | | | EADPTGHSY | " |
| | " | | | | |
| | MAGE.-1 | | HLA-A1 | EADPTGHSY | DIRECTLY FROM DAVID'S LIST |
| | BAGE | | HLA-C | MAARAVFLALSAQL LQARLMKE | " |
| | " | | | MAARAVFLALSAQL LQ | " |
| | " | | | AARAVFLAL | " |
| Influenza | PR8 NP | 147-154 | K^{d} | IYQIURALV | Falk et al., Allele-specific motifs revealed by sequencing of self-peptides eluted from MHC molecules |
| SELF | P815 | | " | SYFPETHI | " |
| PEPTIDE | " | | " | | " |
| Influenza | Jap HA 523-549 | | " | IVATVAGSL | " |
| | " | | " | VYQILAIYA | " |
| | " | | " | IYSTVASSL | " |
| | JAP HA 202-221 | | " | LYQNVGTYV | " |
| | HLA-A24 | | " | RYLENQKRT | " |
| | HLA-Cw3 | | " | RYLKNGKET | " |
| | P815 | | " | KYQAVTTTL | " |
| Plasmodium berghen | CSP | | " | SYIPSAEKI | " |
| Plasmodium yoelli | CSP | | " | SYVPSAFQI | " |
| Vesicular stomatitis viruse | NP 52-59 | | K^{b} | RGYVYQGL | " |
| Ovalbumin | | | " | SIINFEKL | " |
| Sandal Virus | NP 321-332 | | " | APGNYPAL | " |
| | | | | VPYGSFKHV | Morel et al., Processing of some antigens by the standard proteasome but not by the Immunoproteaso me results in poor presentation by dendritic cells, Immunity, vol.12: 107-117,2000. |
| | | | MOTIFS | | |
| Iinfluenza | PR8 NP | | K^{d} restricted peptide motif | TYQRTRALV | 5,747,269 |
| self peptide | P815 | | " | SYFPEITHI | " |
| influenza | JAP HA | | " | IYATVAGSL | " |
| influenza | JAP HA | | " | VYQILAIYA | " |
| influenza | FR8 HA | | " | IYSTVASSL | " |
| Iinfluenza | JAP HA | | " | LYQNVGTYV | " |
| | | | HLA-A24 | RYLENGKETL | " |
| | | | HLA-Cw3 | RYLKNGKETL | " |
| | P815 tumour antigen | | " | KY4AVTTTL | " |
| Plasmodium berghei | CSP | | " | SYILPSAEKI | " |
| Plasmodium yoeli | CSP | | " | SYVPSAEQI | " |
| influenza | NP | | D^{b} - restricted peptides motif | ASNENMETM | " |
| adenovirus | E1A | | " | SGPSNTPPEI | " |
| lymphocytic choriomenin gitis | | | " | SGVENPGGYCL | " |
| simian virus | 40 T | | " | SAINNY ... | " |
| HIV | reverse transcriptase | | HLA-A2.1-restricted peptide motif | ILKEPVHGV | " |
| | influenza matrix protein | | " | GILGFVFRL | " |
| influenza | influenza matrix protein | | " | ILG FV FTLTV | " |

| | | | **MOTIFS** | | |
|---|---|---|---|---|---|
| HIV | Gag protein | | | FLQSRPEPT | " |
| HIV | Gag protein | | | AMQMLKE | " |
| HIV | Gag protein | | | PIAPGQMRE | " |
| HIV | Gag protein | | | QMKDCTERQ | " |
| | | | HLA-A*0205-restricted peptide motif | VYGVIQK | " |

**Tablet 5**

| |
|---|
| VSV-NP peptide (49-62) |
| LCMV-NP peptide (118-132) |
| LCMV glycoprotein peptide. 33-41 |
| ISNQLTLDSNTKYFHKLN |
| ISNQLTLDSNTKYFHKL |
| ISNALTLDSNTKYFHK |
| VDTFLEDVKNLYHSEA |
| KPRAIWDPVHGFMY |
| KQTISPDYRNMI |
| Y[DFIMDPKEKDKV |
| NIQLINDQEVARFD |
| LLSFVRDLNQYRADI |
| LPKPPKPVSKMRMATPL |
| LPKPPKPVSKMRMATPLLMQALP |
| LPKPPKPVSKMRMATPLLMQALPM |
| PKPPKPVSKMRMATPL |
| PKPPKPVSKMRMATPLLMQA |
| KPPKPVSKMRMATPLLMQ |
| KPPKPVSKMRMATPLLMQALPM |
| VDDTQFVRFDSDAASQ |
| ATKYGNMTEDHVMHLLQNA |
| VFLLLLADKVPETSLS |
| LNKILLDEQAQWK |
| GPPKLDIRKEEKQIMUDIFH |
| GPPKLDIRKEEKQIMIDIFHP |
| GFKAIRPDKKSNPIIRTV |
| YANILLDRRVPQTDMTF |
| NLFLKSDGRIKYTLNKNSLK |
| IPDNLFLKSDGRIKYTLNKN |
| IPDNLFLKSDGRIKYTLNK |
| IPDNLFLKSDGRIKYTLN |
| IPDNLFLKSDGRIKYTL |
| NLFLKSDGRIKYTLNK |
| NLFLKSDGRIKYTLNK |
| VTTLNSDLKYNALDLTN |
| VGSDWRFLRGYHC1YA |

Also described herein are methods, uses, therapies and compositions rotated to epitopes with specificity for MHC, including, for example, those listed in Tables 6-10. Also described herein are the MHCs listed in Tables 6-10, including combinations of the same, while other embodiments specifically exclude any one or more of the MHCs or combinations thereof. Tables 8-10 include frequencies for the listed HLA. antigens.

**Table 6**

| **Class I MHC Molecules** |
|---|
| **Class I** |
| **Human** |
| HLA-A1 |
| HLA-A*0101 |
| HLA-A*0201 |
| HIA-A*0202 |
| HLA-A*0203 |
| HLA-A *0204 |
| HLA-A *0205 |
| HLA-A *0206 |
| HLA-A *0207 |
| HLA-A *0209 |
| HLA-A *0214 |
| HLA-A3 |
| HLA-A*0301 |
| HLA-A*1101 |
| HLA-A23 |
| HLA-A24 |
| HLA-A25 |
| HLA-A*2902 |
| HLA-A*3101 |
| HIA-A *3302 |
| HLA-A*6801 |
| HLA-A*6901 |
| HLA-B7 |
| HLA-B*0702 |
| HLA- B'0703 |
| HLA-B*0704 |
| HLA-B *0705 |
| HLA-B8 |
| HLA-B13 |
| HLA-B14 |
| HLA-B-1501 (B62) |
| HLA-B17 |
| HLA-B18 |
| HLA-B22 |
| HLA-B27 |
| HLA-B*2702 |
| BLA-B*2704 |
| HLA-B*2705 |
| HLA-B*2709 |
| HLA-B35 |
| HLA-B*3501 |
| HLA-B*3502 |
| HLA-B*3701 |
| HLA-B*3801 |
| HLA-B*39011 |
| HLA-B*3902 |
| HLA-B40 |
| HLA-B*40012 (B60) |
| HIA-13*4006 (B61) |
| HLA-B44 |
| HLA-B*4402 |
| HLA-B*4403 |
| HLA-B*4501 |
| HLA-B*4601 |
| HLA-B51 |
| HLA-B*5101 |
| HLA-B*5102 |
| HLA-B*5103 |
| HLA-B*5201 |
| HLA-B*5301 |
| HLA-B*5401 |
| HLA-B*5501 |
| HLA-B*5502 |
| HLA-B*5601 |
| HLA-B*6801 |
| HLA-B*6701 |
| HLA-B*7301 |
| HLA-B*7801 |
| HLA-Cw*0102 |
| HLA-Cw*030 |
| HLA-Cw*0304 |
| HLA-Cw*0401 |
| HLA-Cw*0601 |
| HLA-Cw*0602 |
| HLA-Cw*0702 |
| HLA-Cw8 |
| HLA-Cw*1601 M |
| HLA-G |

| **Murine** |
|---|
| H2-K^{d} |
| H2-D^{d} |
| H2-L^{d} |
| H2-K^{b} |
| H2-D^{b} |
| H2-K^{k} |
| H2-K^{kml} |
| |
| Qa-1**^{a}** Qa-2 |
| H2-M3 |

| Rat |
|---|
| RT1.Aⁿ |
| RT1.A^{l} |

| **Bovine** |
|---|
| Bota-Al |
| Bota-A20 |

| **Chicken** |
|---|
| B-F4 |
| B-F12 |
| B-F15 |
| B-F19 |

| **Chimpanzee** |
|---|
| Patr-A*04 |
| Patr-A*11 |
| Patr-B*01 |
| Patr-B*13 |
| Patr-B*16 |

| **Baboon** |
|---|
| Papa..A*06 |

| **Macaque** |
|---|
| Mamu-A*01 |

| **Swine** |
|---|
| SLA (haplotype d/d) |

| **Virus homolog** |
|---|
| hCMV class I homolog UL18 |

**Table 7**

| **Class I MHC Molecules** |
|---|
| **Class 1** |
| **Human** |
| HLA-A1 |
| HLA-A*0101 |
| HLA-A*0201 |
| HLA-A*0202 |
| HLA-A*0204 |
| HLA-A*0205 |
| HLA-A*0206 |
| HLA-A *0207 |
| HLA-A*0214 |
| HLA-A3 |
| HLA-A*1101 |
| HLA-A24 |
| HLA-A*2902 |
| HLA-A*3101 |
| HLA-A*3302 |
| HLA-A*6801 |
| HLA-A*6901 |
| HLA-B7 |
| HLA-B*0702 |
| HLA-B*0703 |
| HLA-B*0704 |
| HLA-B*0705 |
| HLA-B8 |
| HLA-B14 |
| HLA-B*1501 (B62) |
| HLA-B27 |
| HLA-B*2702 |
| HLA-B*2705 |
| HLA-B35 |
| HLA-B*3501 |
| HLA-B*3502 |
| HLA-B*3701 |
| HLA-B*3801 |
| HLA-B*39011 |
| HLA-B*3902 |
| HLA-B40 |
| HLA-B*40012(B60) |
| HLA-B*4006(B61) |
| HLA-B44 |
| HLA.B*4402 |
| HLA-B*4403 |
| HLA-B*4601 |
| HLA-B51 |
| HLA-B*5101 |
| HLA-B*5102 |
| HLA-B*5103 |
| HLA-B*5201 |
| HLA-B*5301 |
| HLA-B*5401 |
| HLA-B*5501 |
| HLA-B*5502 |
| HLA-B*5601 |
| HLA-B*5801 |
| HLA-B*6701 |
| HLA-B*7301 |
| HLA-B*7801 |
| HLA-Cw*0102 |
| HLA-Cw*0301 |
| HLA-Cw*0304 |
| HLA-Cw*0401 |
| HLA-Cw*0601 |
| HLA-Cw*0802 |
| HLA-Cw*0702 |
| HLA-G |

| **Murine** |
|---|
| H2-K^{d} |
| H2-D^{d} |
| H2-L^{d} |
| H2-K^{b} |
| H2-D^{b} |
| H2-K^{k} |
| H2-K^{kml} |
| Qa-2 |

| **Rat** |
|---|
| RT1.A^{a} |
| RT1.A¹ |

| **Bovine** |
|---|
| Bota-A11 |
| Bota-A20 |

| **Chicken** |
|---|
| B-F4 |
| B-F12 |
| B-F15 |
| B-F19 |

| **Virus homolog** |
|---|
| hCMV class I homolog UL 18 |

**Table 8**

| Estimated gene frequencies of HLA-A antigens | | | | | | | | | | |
|---|---|---|---|---|---|---|---|---|---|---|
| Antigen | CAU | | AFR | | ASI | | LAT | | NAT | |
| | Gf^{a} | SE^{b} | Gf | SE | Gf | SE | Gf | SE | Gf | SE |
| A1 | 15.1843 | 0.0489 | 5.7256 | 0.0771 | 4.4818 | 0.0846 | 7.4007 | 0.0978 | 12.0316 | 0.2533 |
| A2 | 28.6535 | 0.0619 | 18.8849 | 0.1317 | 24.6352 | 0.1794 | 28.1198 | 0.1700 | 29.3408 | 0.3585 |
| A3 | 13.3890 | 0.0463 | 8.4406 | 0.0925 | 2.6454 | 0.0655 | 8.0789 | 0.1019 | 11.0293 | 0.2437 |
| A28 | 4.4652 | 0.0280 | 9.9269 | 0.0997 | 1.7657 | 0.0537 | 8.9446 | 0.1067 | 5.3856 | 0.1750 |
| A36 | 0.0221 | 0.0020 | 1.8836 | 0.0448 | 0.0148 | 0.0049 | 0.1584 | 0.0148 | 0.1545 | 0.0303 |
| A23 | 1.8287 | 0.0181 | 10.2086 | 0.1010 | 0.3256 | 0.0231 | 2.9269 | 0.0628 | 1.9903 | 0.1080 |
| A24 | 9.3251 | 0.0395 | 2.9668 | 0.0560 | 22.0391 | 0.1722 | 13.2610 | 0.1271 | 12.6613 | 0.2590 |
| A9 unsplit | 0.0809 | 0.0038 | 0.0367 | 0.0063 | 0.0858 | 0.0119 | 0.0537 | 0.0086 | 0.0356 | 0.0145 |
| A9 total | 11.2347 | 0.0429 | 13.2121 | 0.1128 | 22.4505 | 0.1733 | 16.2416 | 0.1382 | 14.6872 | 0.2756 |
| A25 | 2.1157 | 0.0195 | 0.4329 | 0.0216 | 0.0990 | 0.0128 | 1.1937 | 0.0404 | 1.4520 | 0.0924 |
| A26 | 3.8795 | 0.0262 | 2.8284 | 0.0547 | 4.6628 | 0.0862 | 3.2612 | 0.0682 | 2.4292 | 0.1191 |
| A34 | 0.1508 | 0.0052 | 3.5228 | 0.0810 | 1.3529 | 0.0470 | 0.4928 | 0.0260 | 0.3150 | 0.0432 |
| A43 | 0.0018 | 0.0006 | 0.0334 | 0.0060 | 0.0231 | 0.0062 | 0.0055 | 0.0028 | 0.0059 | 0.0059 |
| A66 | 0.0173 | 0.0018 | 0.2233 | 0.0155 | 0.0478 | 0.0089 | 0.0399 | 0.0074 | 0.0534 | 0.0178 |
| A10 unsplit | 0.0790 | 0.0038 | 0.0939 | 0.0101 | 0.1255 | 0.0144 | 0.0647 | 0.0094 | 0.0298 | 0.0133 |
| A10 total | 6.2441 | 0.0328 | 7.1349 | 0.0850 | 6.3111 | 0.0993 | 5.0578 | 0.0816 | 4.2853 | 0.1565 |
| A29 | 3.5796 | 0.0252 | 3.2071 | 0.0582 | 1.1233 | 0.0429 | 4.5156 | 0.0774 | 3.4345 | 0.1410 |
| A30 | 2.5067 | 0.0212 | 13.0969 | 0.1129 | 2.2025 | 0.0598 | 4.4873 | 0.0772 | 2.5314 | 0.1215 |
| A31 | 2.7386 | 0.0221 | 1.6556 | 0.0420 | 3.6005 | 0.0761 | 4.8328 | 0.0800 | 6.0881 | 0.1855 |
| A32 | 3.6956 | 0.0256 | 1.5384 | 0.0405 | 1.0331 | 0.0411 | 2.7064 | 0.0604 | 2.5521 | 0.1220 |
| A33 | 1.2080 | 0.0148 | 6.5607 | 0.0822 | 9.270 | 0.1191 | 2.6593 | 0.0599 | 1,0754 | 0.0796 |
| A74 | 0.0277 | 0.0022 | 1.9949 | 0.0461 | 0.0561 | 0.0096 | 0.2027 | 0.0167 | 0.1068 | 0.0252 |
| A19 unsplit | 0.0567 | 0.0032 | 0.2057 | 0.0149 | 0.0990 | 0.0128 | 0.1211 | 0.0129 | 0.0475 | 0.0168 |
| A19 total | 13.8129 | 0.0468 | 28.2593 | 0.1504 | 17.3846 | 0.1555 | 19.5252 | 0.1481 | 15.8358 | 0.2832 |
| AX | 0.8204 | 0.0297 | 4.9506 | 0.0963 | 2.9916 | 0.1177 | 1.6332 | 0.0979 | 1.8454 | 0.1925 |
| ^{a}Gene frequency | | | | | | | | | | |
| ^{b}Standard error. | | | | | | | | | | |

**Table 9**

| Estimated gene frequencies for HLA-B antigens | | | | | | | | | | |
|---|---|---|---|---|---|---|---|---|---|---|
| Antigen | CAU | | Afar | | ASI | | LAT | | NAT | |
| | Gf^{a} | SE^{b} | Gf | SE | Gf | SE | Gf | SE | Gf | SE |
| B7 | 12.1782 | 0.0445 | 10.5960 | 0.1024 | 4.2691 | 0.0827 | 6.4477 | 0.0918 | 10.9845 | 0.2432 |
| B8 | 9.4077 | 0.0397 | 3.8315 | 0.0634 | 1.3322 | 0.0467 | 3.8225 | 0.0715 | 8.5789 | 0.2176 |
| B13 | 2.3061 | 0.0203 | 0.8103 | 0.0295 | 4.9222 | 0.0886 | 1.2699 | 0.0416 | 1.7495 | 0.1013 |
| B14 | 4.3481 | 0.0277 | 3.0331 | 0.0566 | 0.5004 | 0.0287 | 5.4166 | 0.0846 | 2.9823 | 0.1316 |
| B18 | 4.7980 | 0.0290 | 32057 | 0.0582 | 1.1246 | 0.0429 | 4.2349 | 0.0752 | 3.3422 | 0.1391 |
| B27 | 4.383 | 0.0278 | 1.2918 | 0.0372 | 2.2355 | 0.0603 | 2.3724 | 0.0567 | 5.1970 | 0.1721 |
| B35 | 9.6614 | 0.0402 | 8.5172 | 0.0927 | 8.1203 | 0.1122 | 14.6516 | 0.1329 | 10.1198 | 0.2345 |
| B37 | 1.4032 | 0.0159 | 0.5916 | 0.0252 | 1.2327 | 0.0449 | 0.7807 | 0.0327 | 0.9755 | 0.0759 |
| B41 | 0.9211 | 0.0129 | 0.8183 | 0.0296 | 0.1303 | 0.0147 | 1.2818 | 0.0418 | 0.4766 | 0.0531 |
| B42 | 0.0608 | 0.0033 | 5.6991 | 0,0768 | 0.0841 | 0,0118 | 0.5866 | 0.0284 | 0.2856 | 0.0411 |
| B46 | 0.0099 | 0.0013 | 0.0151 | 0.0040 | 4.9292 | 0.0886 | 0.0234 | 0.0057 | 0.0238 | 0.0119 |
| B47 | 0.2069 | 0.0061 | 0.1305 | 0.0119 | 0.0956 | 0.0126 | 0.1832 | 0.0159 | 0.2139 | 0.0356 |
| B49 | 0.0865 | 0.0040 | 0.1316 | 0.0119 | 2.0276 | 0.0575 | 1.5915 | 0.0466 | 1.0267 | 0.0778 |
| B53 | 0.4620 | 0.0092 | 10.9529 | 0.1039 | 0.4315 | 0.0266 | 1.6982 | 0.0481 | 1.0804 | 0.0798 |
| B59 | 0.0020 | 0.0006 | 0.0032 | 0.0019 | 0.4277 | 0.0265 | 0.0055 | 0.0028 | 0^{c} | |
| B67 | 0.0040 | 0.0009 | 0.0086 | 0.0030 | 0.2276 | 0.0194 | 0.0055 | 0.0028 | 0.0059 | 0.0059 |
| B70 | 0.3270 | 0.0077 | 7.3571 | 0.0866 | 0.8901 | 0.0382 | 1.9266 | 0.0512 | 0.6901 | 0.0639 |
| B73 | 0.0108 | 0.0014 | 0.0032 | 0.0019 | 0.0132 | 0.0047 | 0.0261 | 0.0060 | 0^{c} | |
| B51 | 5.4215 | 0.0307 | 2.5980 | 0.0525 | 7.4751 | 0.1080 | 6.8147 | 0.0943 | 6.9077 | 0.1968 |
| B52 | 0.9658 | 0.0132 | 1.3712 | 0.0383 | 3.5121 | 0.0752 | 2.2447 | 0.0552 | 0.6960 | 0.0641 |
| 85 unsplit | 0.1565 | 0.0053 | 0.1522 | 0.0128 | 0.1288 | 0.0146 | 0.1546 | 0.0146 | 0.1307 | 0.0278 |
| 85 total | 6.5438 | 0.0435 | 4.1214 | 0.0747 | 11.1160 | 0.1504 | 9.2141 | 0.1324 | 7.7344 | 0.2784 |
| B44 | 13.4838 | 0.0465 | 7.0137 | 0.0847 | 5.6807 | 0.0948 | 9.9253 | 0.1121 | 11.8424 | 0.2511 |
| B45 | 0.5771 | 0.0102 | 4.8069 | 0.0708 | 0.1816 | 0.0173 | 1.8812 | 0.0506 | 0.7603 | 0.0670 |
| B12 unsplit | 0.0788 | 0.0038 | 0.0280 | 0.0055 | 0.0049 | 0.0029 | 0.0193 | 0.0051 | 0.0654 | 0.0197 |
| B12 total | 14.1440 | 0.0474 | 11.8486 | 0.1072 | 5.8673 | 0.0963 | 11.8258 | 0.1210 | 12.6281 | 0.2584 |
| | | | | | | | | | 6.9421 | |
| B62 | 5.9117 | 0.0320 | 1.5267 | 0.0404 | 9.2249 | 0.1190 | 4.1825 | 0.0747 | | 0.1973 |
| | | | | | | | | | 0.3738 | |
| B63 | 0.4302 | 0.0088 | 1.8865 | 0.0448 | 0.4438 | 0.0270 | 0.8083 | 0.0333 | | 0.0471 |
| | | | | | | | | | 0.0356 | |
| B75 | 0.0104 | 0.0014 | 0.0226 | 0.0049 | 1.9673 | 0.0566 | 0.1101 | 0.0123 | | 0.0145 |
| | | | | | | | | | 0 | |
| B76 | 0.0026 | 0.0007 | 0.0065 | 0.0026 | 0.0874 | 0.0120 | 0.0055 | 0.0028 | | |
| | | | | | | | | | 0^{c} | |
| B77 | 0.0057 | 0.0010 | 0.0119 | 0.0036 | 0.0517 | 0.0098 | 0.0083 | 0.0034 | | 0.0059 |
| | | | | | | | | | 0.0059 | |
| B15 unsplit | 0.1305 | 0.0049 | 0.0691 | 0.0086 | 0.4301 | 0.0266 | 0.1820 | 0.0158 | | 0.0206 |
| | | | | | | | | | 0.0715 | |
| B15 total | 6.4910 | 0.0334 | 3.5232 | 0.0608 | 12.2112 | 0.1344 | 5.2967 | 0.0835 | | 0.2035 |
| | | | | | | | | | 7.4290 | |
| B38 | 2.4413 | 0.0209 | 0.3323 | 0.0189 | 3.2818 | 0.0728 | 1.9662 | 0.0517 | 1.1017 | 0.0806 |
| B39 | 1.9614 | 0.0188 | 1.2893 | 0.0371 | 2.0352 | 0.0576 | 6.3040 | 0.0909 | 4.5527 | 0.1615 |
| B16 unsplit | 0.0638 | 0.0034 | 0.0237 | 0.0051 | 0.0644 | 0.0103 | 0.1226 | 0.0130 | 0.0593 | 0.0188 |
| B16 total | 4.4667 | 0.0280 | 1.6453 | 0.0419 | 5.3814 | 0.0921 | 8.3917 | 0.1036 | 5.7137 | 0.1797 |
| B57 | 3.5955 | 0.0252 | 5.6746 | 0.0766 | 2.5782 | 0.0647 | 2.1800 | 0.0544 | 2.7265 | 0.1260 |
| B58 | 0.7152 | 0.0114 | 5.9546 | 0.0784 | 4.0189 | 0.0803 | 1,2481 | 0.0413 | 0.9398 | 0.0745 |
| B17 unsplit | 0.2845 | 0.0072 | 0.3248 ' | 0.0197 | 0.3751 | 0.0248 | 0.1446 | 0.0141 | 0.2674 | 0.0398 |
| B17 total | 4.5952 | 0.0284 | 11.9540 | 0.1076 | .9722 | 0.1041 | 3.5727 | 0.0691 | 3.9338 | 0.1503 |
| B49 | 1.6452 | 0.0172 | 2.6286 | 0.0528 | 0.2440 | 0.0200 | 2.3353 | 0.0562 | 1.5462 | 0.0953 |
| B50 | 1.0580 | 0.0138 | 0.8636 | 0.0304 | 0.4421 | 0.0270 | 1.8883 | 0.0507 | 0.7862 | 0.0681 |
| B21 unsplit | 0.0702 | 0.0036 | 0.0270 | 0.0054 | 0.0132 | 0.0047 | 0.0771 | 0.0103 | 0.0356 | 0.0145 |
| B21 total | 2.7733 | 0.0222 | 3.5192 | 0.0608 | 0.6993 | 0.0339 | 4.3007 | 0.0755 | 2.3680 | 0.1174 |
| B54 | 0.0124 | 0.0015 | 0.0183 | 0.0044 | 2.6873 | 0.0660 | 0.0289 | 0.0063 | 0.0534 | 0.0178 |
| B55 | 1.9046 | 0.0185 | 0.4895 | 0.0229 | 2.2444 | 0.0604 | 0.9515 | 0.0361 | 1.4054 | 0.0909 |
| B56 | 0.5527 | 0.0100 | 0.2686 | 0.0170 | 0.8260 | 0.0368 | 0.3596 | 0.0222 | 0.3387 | 0.0448 |
| B22 unsplit | 0.1682 | 0.0055 | 0.0496 | 0.0073 | 0.2730 | 0.0212 | 0.0372 | 0.0071 | 0.1246 | 0.0272 |
| B22 total | 2.0852 | 0.0217 | 0.8261 | 0.0297 | 6.0307 | 0.0971 | 1.3771 | 0.0433 | 1.9221 | 0.1060 |
| B60 | 5.2222 | 0.0302 | 1.5299 | 0.0404 | 8.3254 | 0.1135 | 2.2538 | 0.0553 | 5.7218 | 0.1801 |
| 861 | 1.1918 | 0.0147 | 0.4709 | 0.0225 | 6.2072 | 0.0989 | 4.6691 | 0.0788 | 2.6023 | 0.1231 |
| B40 unsplit | 0.2696 | 0.0070 | 0.0388 | 0.0065 | 0.3205 | 0.0230 | 0.2473 | 0.0184 | 0.2271 | 0.0367 |
| B40 total | 6.6834 | 0.0338 | 2.0396 | 0.0465 | 14.8531 | 0.1462 | 7.1702 | 0.0963 | 8.5512 | 0.2168 |
| BX | 1.0922 | 0.0252 | 3.5258 | 0.0802 | 3.8749 | 0.0988 | 2.5266 | 0.0807 | 1.9867 | 0.1634 |
| ^{a}Gene frequency. ^{b}Standard error. ^{c}The observed gene count was zero. | | | | | | | | | | |

**Table 10.**

| Estimated gene frequencies of HLA-DR antigens | | | | | | | | | | |
|---|---|---|---|---|---|---|---|---|---|---|
| Antigen | CAU | | AFR | | ASI | | LAT | | NAT | |
| | Gf^{a} | SE^{b} | Gf | SE | Gf | SE | Gf | SE | Gf | SE |
| DR1 | 10.2279 | 0.0413 | 6.8200 | 0.0832 | 3.4628 | 0.0747 | 7.9859 | 0.1013 | 8.2512 | 0.2139 |
| DR2 | 15.2408 | 0.0491 | 16.2373 | 0.1222 | 18.6162 | 0.1608 | 11.2389 | 0.1182 | 15.3932 | 0.2818 |
| DR3 | 10.8708 | 0.0424 | 13.3080 | 0.1124 | 4.7223 | 0.0867 | 7.8998 | 0.1008 | 10.2549 | 0.2361 |
| DR4 | 16.7589 | 0.0511 | 5.7084 | 0.0765 | 15.4623 | 0.1490 | 20.6373 | 0.1520 | 19.8264 | 0.3123 |
| DR6 | 14.3937 | 0.0479 | 18.6117 | 0.1291 | 13.4471 | 0.1404 | 17.0265 | 0.1411 | 14.8021 | 0.2772 |
| DR7 | 13.2807 | 0.0463 | 10.1317 | 0.0997 | 6.9270 | 0.1040 | 10.6726 | 0.1155 | 10.4219 | 0.2378 |
| DRB | 2.8820 | 0.0227 | 62673 | 0.0800 | 6.5413 | 0.1013 | 9.7731 | 0.1110 | 6.0059 | 0.1844 |
| DR9 | 1.0616 | 0.0139 | 2.9646 | 0.0559 | 9.7527 | 0.1218 | 1.0712 | 0.0383 | 2.8662 | 0.1291 |
| DR10 | 1.4790 | 0.0163 | 2.0397 | 0.0465 | 2.2304 | 0.0602 | 1.8044 | 0.0495 | 1.0898 | 0.0801 |
| DR11 | 9.3180 | 0.0396 | 10.6151 | 0.1018 | 4.7375 | 0.0869 | 7.0411 | 0.0955 | 5.3152 | 0.1740 |
| DR12 | 1.9070 | 0.0185 | 4.1152 | 0.0655 | 10.1365 | 0.1239 | 1.7244 | 0.0484 | 2.0132 | 0.1086 |
| DR5 unsplit | 1.2199 | 0.0149 | 2.2957 | 0.0493 | 1.4118 | 0.0480 | 1.8225 | 0.0498 | 1.6769 | 0.0992 |
| DR5 total | 12.4449 | 0.0045 | 17.0260 | 0.1243 | 16.2858 | 0.1316 | 10.5880 | 0.1148 | 9.0052 | 0.2218 |
| DRX | 1.3598 | 0.0342 | 0.8853 | 0.0760 | 2.5521 | 0.1089 | 1.4023 | 0.0930 | 2.0834 | 0.2037 |
| ^{a}Gene frequency. | | | | | | | | | | |
| ^{b}Standard error. | | | | | | | | | | |

It can be desirable to express housekeeping peptides in the context of a larger protein. Processing can be detected even when a small number of amino acids are present beyond the terminus of an epitope. Small peptide hormones are usually proteolytically processed from longer transition products, often In the size range of approximately 60-120 amino acids. This fact has led some to assume that this is the minimum size that can be efficiency translated. In some embodiments, the housekeeping peptide can be embedded in a translation produce of at least about 60 amino acids, in others 70, 80, 90 amino acids, and in still others 100,110 or 120 amino acids, for example. In other embodiments the housekeeping peptide can be embedded in a translation product of at least, about 50, 30, or 15 amino acids.

Due to differential proteasomal processing, the immunoprotcasome of the pAPC produces peptides that are different from those produced by the housekeeping proteasome In peripheral body cells. Thus, in expressing a housekeeping peptide in the context of a larger protein, it is preferably expressed in the pAPC in a context other than its full-length native sequence, because, as a housekeeping epitope, it is generally only efficiently processed from the native protein by the housekeeping proteasome, which is not active in the pAPC. In order to encode the housekeeping epitope in a DNA sequence encoding a larger polypeptide, it is useful to find flanking areas on either side of the sequence encoding the epitope that permit appropriate cleavage by the immunoproteasome in order to liberate that housekeeping epitope. Such a sequence promoting appropriate processing is referred to hereinafter as having substrate or liberation sequence function. Altering flanking amino acid residues at the N-terminus and C-terminus of the desired housekeeping epitope: can facilitate appropriate cleavage and generation of the housekeeping epitope in the pAPC. Sequences embedding housekeeping epitopes can be designed *de novo* and screened to determine which can be successfully processed by immunoproteasomes to liberate housekeeping epitopes.

Alternatively, another strategy is very effective for identifying sequences allowing production of housekeeping epitopes in APC. A contiguous sequence of amino acids can be generated from head to tall arrangement, of one or more housekeeping epitopes. A construct expressing this sequence is used to immunize an animal, and the resulting T cell response is evaluated to determine its specificity to one or more of the epitopes in the array. These immune responses indicate housekeeping epitopes that are processed in the pAPC effectively. The necessary flanking areas around this epitopes are thereby defined. The use of flanking regions of about 4-6 amino acids on either side of the desired peptide can provide the necessary information to facilitate proteasome processing of the housekeeping epitope by the immunoproteasome. Therefore, a substrate or liberation sequence of approximately 16-22 amino acids can be inserted into, or fused to, any protein sequence effectively to result in that housekeeping epitope being produced in an APC. In some embodiments, a broader context of a substrate sequence can also influence processing. In such embodiments, comparisons of a liberaton sequence in a variety of contexts can be useful in further optimising a particular substrate sequence. In alternate embodiments the whole head-to-tail array of epitopes, or just the epitopes immediately adjacent, to the correctly processed housekeeping epitope can be similarly transferred from a test construct to a vaccine vector,

It is also described herein that, the housekeeping epitopes can be embedded between known immune epitopes, or segments of such, thereby providing an appropriate context for processing. The abutment of housekeeping and immune epitopes can generate the necessary context to enable the immunoproteasome to liberate the housekeeping epitope, or a larger fragment, preferably including a correct C-terminus. It can be useful to screen constructs to verify that the desired epitope is produced. The abutment of housekeeping epitopes can generate a site cleavable by the immunoproteasome. Some embodiments of the invention, employ known epitopes to flank: housekeeping epitopes in test substrates; in others, screening as described below is used, whether the flanking regions are arbitrary sequences or mutants of the natural flanking sequence, and whether or not knowledge of proteasomal cleavage preferences are used in designing the substrates.

Cleavage at the mature N-terminus of the epitope, while advantageous, is not required, since a variety of N-terminal trimming activities exist in the cell that can generate the mature N-terminus of the epitope subsequent to proteasomal processing. It is preferred that such N-terminal extension be less than about 25 amino acids in length and it is further preferred that the extension have few or no proline residues. Preferably, in screening, consideration is given not only to cleavage at the ends of the epitope (or at least at its C-terminus), but consideration also can be given to ensure limited cleavage within the epitope.

Shotgun approaches can be used in designing test substrates and can increase the efficiency of screening, in one embodiment multiple epitopes can be assembled one after the other, with Individual epitopes possibly appearing more than once. The substrate can be screened to determine which epitopes can be produced. In the case where a particular epitope is of concern, a substrate can be designed in which it appears in multiple different contexts. When a single epitope appearing in more than one context is liberated from the substrate additional secondary test substrates, in which individual instances of the epitope are removed, disabled, or are unique, can be used to determine which are being liberated and truly confer substrate or liberation sequence function.

Several readily practicable screens exist A preferred *in vitro* screen utilizes proteasomal digestion analysis, using purifled immunoproteasomes, to determine the desired housekeeping epitope can be liberated from a synthetic peptide embodying the sequence in question. The position of the cleavages obtained can be determined by techniques such as mass spectrometry, HPLC, and N-terminal pool sequencing; as described in greater detail In U.S. Patent 6,861,234 and US Patent Application No. US 2003/0220239.

Alternatively, *in vivo* and cell-based screens such as immunization or target sensitization can be employed. For immunization a nucleic acid construct capable of expressing the sequence In question is used. Harvested CTL can be tested for their ability to recognize target cells presenting the housekeeping epitope in question. Such targets cells are most readily obtained by pulsing cells expressing the appropriate MHC molecule with synthetic peptide embodying the mature housekeeping epitope. Alternatively, immunization can be carried out using cells known to express housekeeping proteasome and the antigen from which the housekeeping epitope is derived, either endogenously or through genetic engineering. To use target sensitization as a screen, CTL, or preferably a CTL clone, that recognizes the housekeeping epitope can be used, in this case it is the target cell that expresses the embedded housekeeping epitope (instead of the pAPC during immunization) and it must express immunoproteasome. Generally, the cell or target cell can be transformed with an appropriate nucleic acid construct to confer expression of the embedded housekeeping epitope. Loading with a synthetic peptide embodying the embedded epitope using peptide loaded liposomes, or complexed with cationic lipid protein transfer reagents such as BIOPORTER™ (Gene Therapy Systems, San Diego, CA), represents an alternative.

Once sequences with substrate or liberation sequence function are identified they can be encoded in nucleic acid vectors, chemically synthesized, or produced recombinantly. In any of these forms they can be incorporated into immunogenic compositions. Such compositions can be used *in vitro* in vaccine development or in the generation or expansion of CTL to be used in adoptive immunotherapy. *In vivo* they can be used to induce, amplify or sustain and active immune response. The uptake of polypeptides for processing and presentation can be greatly enhanced by packaging with cationic lipid, the addition of a tract of cationic amino acids such as poly-L-lysine (Ryser, H-J, et al., J. Cell Physiol. 113-167-178, 1982; Shen, W.C. & Ryser, H.J., Proc. Natl. Aced. Sci. USA 75:1872-1876, 1978), the incorporation into branched structures with importation signals (Sheldon, K. et al., Proc. Natl. Aced. Sci. USA 92: 2056-2060,1995), or mixture with or fusion to polypeptides with protein transfer function including peptide carriers such as pep-1 (Moms, M.C., et al., Nat. Biotech. 19:1173-1176, 2001), the PreS2 translocation motif of hepatitis 13 virus surface antigen, VP22 of herpes viruses, and HIV-TAT protein (Oess, S. & Hildt, E., Gene Ther. 7:750.-758,2000; Ford, K.G., et al., Gene Ther. 8:1-4, 2001; Hung, C.F. et al.,J. Virol. 76:2676-2682, 2002; Oliveira, S.C., a;. Hum. Gene Ther. 12:1353-1359, 2001; Normand, N. et al., J. Biol. Chem. 276: 15042. 15050, 2001; Schwartz, J.J. & Zhang, S., Curr. Opin Mo/. Ther. 2:162.-167,2000; Elliot G., 7 Hare, P, Cell 88:223..233,1997), among other methodologies. Particularly for fusion proteins the immunogen can be produced in culture and the purified protein administered or, in the alternative, the nucleic acid vector can be administered so that the immunogen is produced and secreted by cells transformed *in vivo.* In either scenario the transport function of the fusion protein facilitates uptake by pAPC.

### EXAMPLES

### Example1.

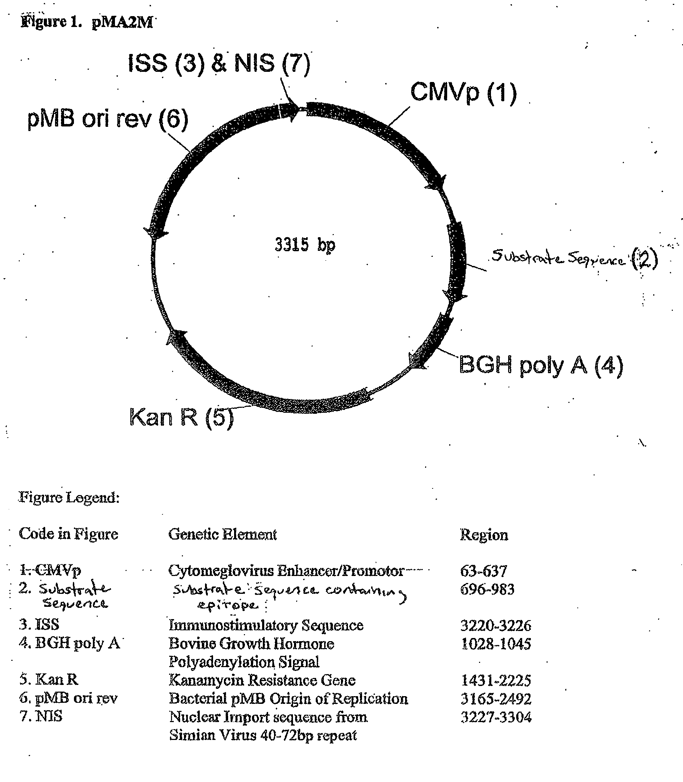

A recombinant DNA plasmid vaccine, pMA2M, which encodes one polypeptide with an HLA A2-specific CTL epitope ELAGIGILTV (SEQ ID NO.1) from melan-A (26-35A27L), and a portion (amino acids 31.-96) of melan-A (SEQ ID NO. 2) including the epitope clusters at amino acids 31-48 and 56-69, was constructed. These clusters were previously disclosed in U.S. Patent. Application No. 09/561,571 entitled EPITOPE CLUSTERS. Flanking the defined melan-A CTL epitope are short amino acid sequences derived from human tyrosinase (SEQ ID NO, 3) to facilitate liberation of the melan-A housekeeping epitope by processing by the inmunoproteasome. In addition, these amino acid sequences represent potential CTL epitopes themselves. The CDNA sequence for the polypeptide in the plasmid is under the control of promoter/enhancer sequence from cytomegalovirus (CMVp) (see Figure 1), which allows efficient transcription of messenger for the polypeptide upon uptake by APCs. The bovine growth hormone polyadenylation signal (BGH poly A) at the 3' end of the encoding sequence provides a signal for polyadenylation of the messenger to increase its stability as well as for translation out of nucleus into the cytoplasm for translation. To facilitate plasmid transport into the nucleus after uptake, a nuclear import sequence (NIS) from simian virus 40 (SV40) has been inserted in the plasmid backbone. The plasmid carries two copies of a CpG immunostimulatory motif, one in the NIS sequence and one in the plasmid backbone. Lastly, two prokaryotic genetic elements in the plasmid are responsible for amplification in *E.coli*, the kan-amycin resistance gene (Kan R) and the pMB1 bacterial origin of replication.

### SUBSTRATE or LIBERATION sequence

The amino acid sequence of the encoded polypeptide (94 amino acid residues in length) (SEQ ID NO. 4) containing a 28 amino acid substrate or liberation sequence at its N-terminus (SEQ ID NO. 5) is given below:

MLLAVLYCL- ELAGIGILTV- YMDGTMSOV- GILTVILGVLLLIGCWYCRRRNGYRALMDKSLHVGTQCAL-TRRCPQEGFDHRDSKVSLQEK NCEPV

The first 9 amino acid residues are derived from tyrosinase₁₋₉ (SEQ ID NO.), the next ten constitute melan-A (26-35A27L) (SEQ ID NO. 1), and amino acid residues 20 to 29 are derived from tyrosinase 369-377 (SEQ ID NO. 7). These two tyrosinase nonamer sequences both represent potential HLA A2-specific CTL, epitopes. Amino acid residues 10-19 constitute melan-A (26-35A27L) an analog of an HLA A2-speciflc CTL epitope from melan-A, EAAGIGILTV (SEQ ID NO. 8), with an elevated potency in inducing CTL responses during *in vitro* immunization of human PBMC *and in vino* immunization in mice. The segment of melan-A constituting the rest of the polypeptide (amino add residues 30 to 94) contain a number of predicted HLA A2-specific epitopes, including the epitope clusters cited above, and thus can be useful in generating a response to immune epitopes as described at length in the patent applications 'Epitope Syn-chronization in Antigen Presenting Cells' and'Epitope Clusters'. This regionwas also included to overcome any difficulties that can be associated with the expression of shorter sequences. A drawing of pMA2M is shown in Figure 1.

### Plasmid construction

A pair of long complementary oligonucleotides was synthesized which encoded the first 30 amino acid residues. In addition, upon annealing, these oligonucleotides generated the cohensive ends of *Afl* II at the 5' end and that of *EcoR* I at the 3' end. The melan A₃₁-₉₆ region was amplified with PCR using oligonucleotides carrying restriction sites for *EcoR* I at the 5' end and *Not* I at the 3' end. The PCR product was digested with *EcoR* I and *Not* I and ligated into the vector backbone, described in Example 1, that had been digested with *AFL* II and *Not* I, along with the annealed oligonucleotides encoding the amino terminal region in a three-fragment ligation. The entire coding sequence was verified by DNA sequencing. The sequence of the entire insert, from the *Afl* II site at the 5' end to the *Not* I site at the 3' end is disclosed as SEQ ID NO, 9, Nucleotides 12-293 encode the polypeptide.

### Example2

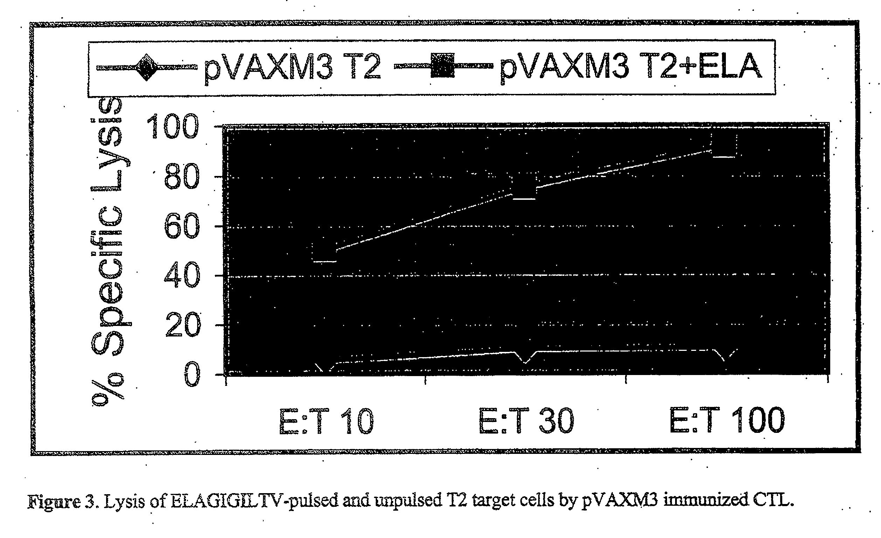

Three vectors containing melan-A (26-35A27L) (SEQ ID NO.1) as an embedded housekeeping epitope were tested for their ability to induce a CTL response to this epitope in HLA-A2 transgenic HHD mice (Pascolo et al. J. Exp. Med.185:2043-2051, 1997). One of the vectors was pMA2M described above (called pVAXM3 in Figure 3). In pVAXM2 the same basic group of 3 epitopes was repeated several times with the flanking epitopes truncated by differing degrees In the various repeats of the array. Specifically the cassette consisted of:

M-Tyr(5-9)-ELA-Tyr(369-373)-Tyr(4-9)-ELA-Tyr(369-374)-Tyr(3-9)-ELA-Tyr(369-375)-Tyr(2-9)-ELA (SEQ ID NO, 10)

where ELA represents melan-A (26-35A27L) (SEQ ID NO.1). This cassette was inserted in the same plasmid backbone as used for pVAXM3. The third, pVAXMI is identical to pVAXM2 except that the epitope array is followed by an IRES (internal ribosome entry site for encephalomyocarditis virus) linked to a reading frame encoding melan-A 31-70.

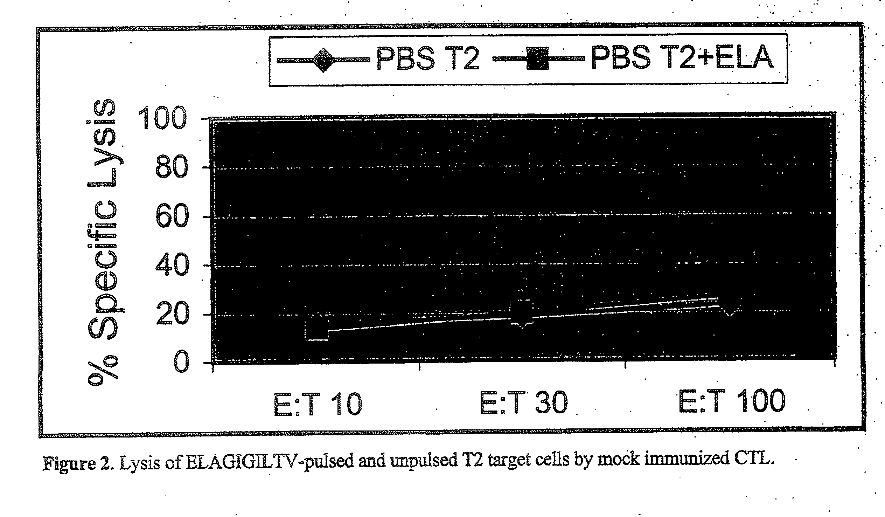

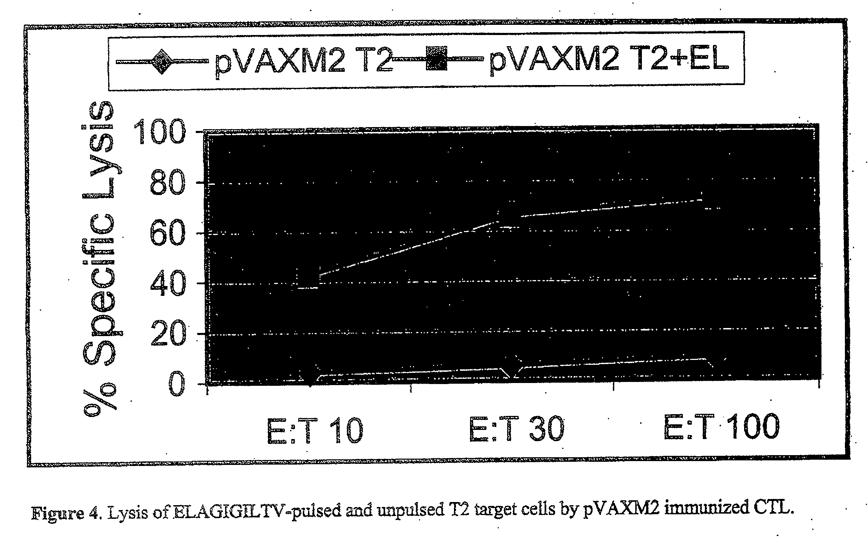

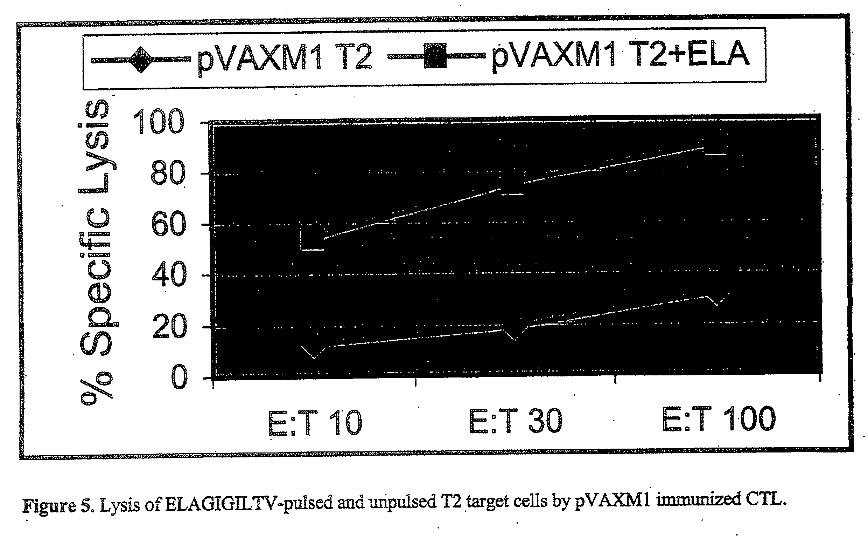

Four groups of three HHD A2.1 mice were injected intranodally in surgically exposed inguinal lymph nodes with 25 >1 of 1 mg/ml plasmid DNA. in PBS on days 0,3. and 6, each group receiving one of the three vectors or PBS alone. On day 14 the spleens were harvested and restimulated *in vitro* one time with 3-day LPS blasts pulsed with peptide (melan-A (26-35A27L)(SEQ ID NO. 1)). The *in vitro* cultures were supplemented with RatT-Stim (Collaborative Biomedical Products) on the 3^{rd} day and assayed for cytolytic activity on the 7^{th} day using a standard ⁵¹Cr-ralease assay. Figures 2 to 5 show % specific lysis obtained using the cells immunized with PBS, pVAXM1, pVAXM2, and pVAXM3, respectively on T2 target cells and T2 target cells pulsed with melan-A (26-35A27L) (ELA) (SEQ ID NO.1). All three vectors generated strong CTL responses. These data indicated that the plasmids have been taken up by APCs, the encoded polypeptide has been synthesized and proteolytically processed to produce the decamer epitope in question (that is, it had substrate or liberation sequence function), and that the epitopes became HLA-A2 bound for presentation. Also, an isolated variant of pVAXM2, that terminates after the 55^{th} amino acid, worked similarly well as the full length version (data not shown). Whether other potential epitopes within the expression cassette can also be produced and be active in inducing CTL responses can be determined by testing for CTL activity against target cells pulsed with corresponding synthetic peptides.

### Example 3

### An NY-ESO-1 (SEQ ID NO.11) SUBSTRATE/LIBERATION Sequence

Six other epitope arrays were tested leading to the identification of a substrate/liberation sequence for the housekeeping epitope NY-ESO-1 ₁₅₇₋₁₆₅ (SEQ ID NO. 12). The component epitopes of the arrays were:
SSX-2₄₁₋₄₉: KASEKI FYV (SEQ ID NO. 13) Array element A
NY-ESO-1 ₁₅₇₋₁₆₅: SLLMWITQC (SEQ ID) NO. 12) Array element B
NY-ESO-1₁₆₃₋₁₇₁: TQCFLPVFL (SEO ID NO. 14) Array element C
PSMA₂₈₈₋₂₉₇: GLPSIPVHPI (SEQ ID NO.15) Array element D
TYR₄₋₉: AVLYCL (SEQ ID NO. 16) Array element E
The six arrays had the following arrangements of elements after starting with an initiator methionine:
pVAX-PC-A: B-A-D-D-A-B-A-A.
pVAX-PC-B: D-A-B-A-A-D-B-A
pVAX-PC-C: E-A-D-B-A-B-E-A-A
pVAX-BC-A: B-A-C-B-A-A-GA
pVAX-BC-B: C-A-B-C-A-A-B-A
pVAX-BC-C: E-A-A-B-C-B-A-A

These arrays were inserted into the same vector backbone described in the examples above. The plasmid vectors were used to immunize mice essentially as described in Example 2 and the resulting CTL were tested for their ability to specifically lyse target cells pulsed with the peptide NY-ESO-1 157-165, corresponding to element B above. Both pVAX-PC-A and pVAX-BC-A were found to induce specific lytic activity. Comparing the contexts of the epitope (element B) in the various arrays, and particularly between pVAX-PGA and pVAX-BC-A, between pVAX-PC-A and pVAX-PC-B, and between pVAX-BGA and pVAX-BC-C, it was concluded that it was the first occurrence of the epitope In pVAX-PC-A and pVAX-BC-A that was being correctly processed and presented. In otherwords an initiator methionine followed by elements B-A constitute a substrate/liberation sequence for the presentation of element B. On this basis a new expression cassette for use as a vaccine was constructed encoding the following elements:
An Initiatormethionine,
NY-ESO-1₁₅₇₋₁₆₅ (bold) --- a housekeeping epitope,
SSX2₄₁₋₄₉ (*italic*) -providing appropriate context for processing, and
NY-ESO-1₇₇₋₁₈₀-to avoid "short sequence" problems and provide immune epitopes,
Thus the construct encodes the amino acid sequence:
M-SLLMW[TQC-*KASEKIFYV*-RCGARGPESRLLEFYLAMPFATPMEAELARRSLAQDAPPLPVPGVLLKEFT-VSGNILTIRLTA ADHRQLQLSISSCLQQLSLLMWHCFLPVFLAQPPSGQRR (SEQ ID NO. 17) and MSLLMWITQCK-ASEKITYV (SEQ ID NO. 18) constitutes the liberation or substrate sequence. A. polynucleotide encoding SEQ ID NO. 17 (SEQ ID NO. 19: nucleotides 12-380) was inserted into the same plasmid backbone as used for pMA2M generating the plasmid pN157.

### Example 4

A construct similar to pN157 containing the whole epitope array from PVAX-PC-A was also made and designated pBPL. Thus the encoded amino add sequence in pBPL is:
M-SLLMWTQC-*KASEKIFYV*-GLPSIPVHPI-GLPSIPVHPI-*KASEKIFYV*-SLLMWITQC-*KASEKIFYV*- *KASE-KIFYV*- RCGARGPESRLEFYLAMPFATPMEAELARRSLAQDAPPLPVPGVLLKEFTVSGNLLTIRLTA ADHRQLQLSI-SSCLQQLSLLMWITQCFL,PYFLAQPPSGQRR (SEQ ID NO. 20).
SEQ ID NO. 21 is the polynucleotide encoding SEQ ID NO. 20 used in pBPL.

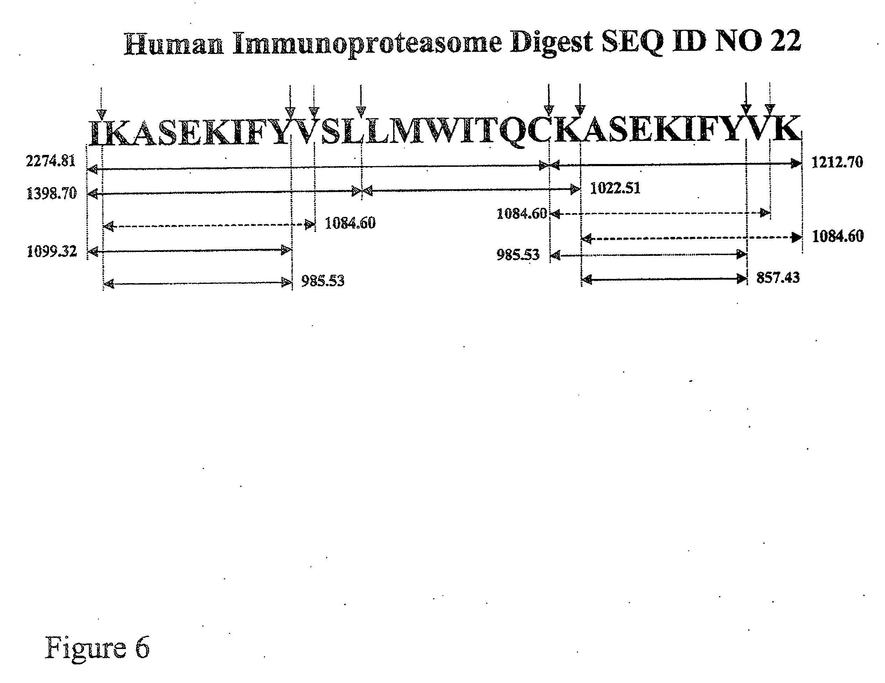

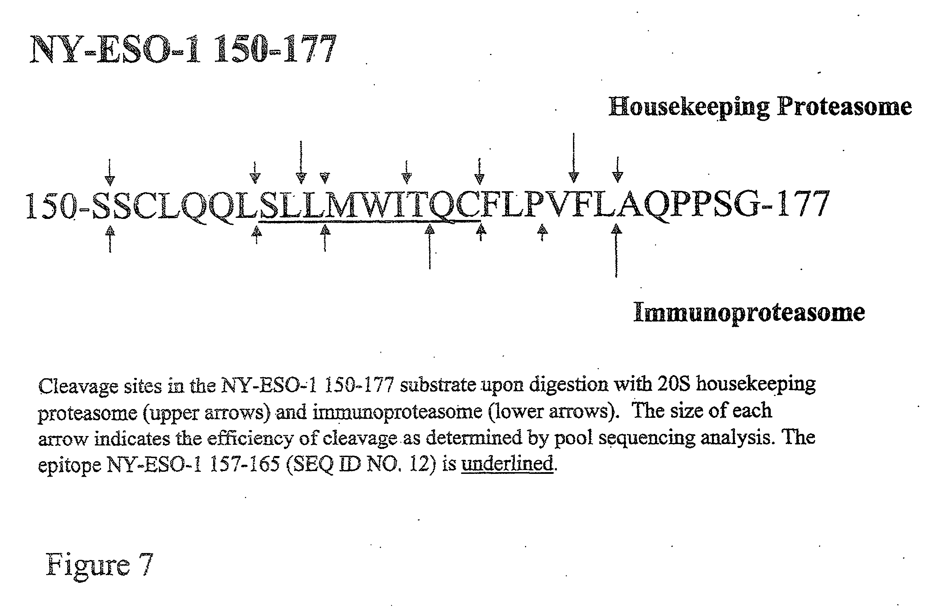

A portion of SEQ ID NO. 20, *IKASEKIFYV*SLLMWITQC*KASEKIFYVK* (SEQ ID NO. 22) was made as a synthetic peptide and subjected to *In vitro* proteasomal digestion analysis with human immunoproteasome, utilizing both mass spectrometry and N-terminal pool sequencing. The identification of a cleavage after the C residue indicates that this segment of the construct can function as a substrate or liberation sequence for NY-ESO-1 157-165 (SEQ ID NO. 12) epitope (see Figure 6). Figure 7 shows the differential processing of the SLLMWJTQC epitope (SEQ ID NO. 12) in its native context where the cleavage following the C is more efficiently produced by housekeeping than immunoproteasome. The immunoproteasome also produces a major cleavage internal to the epitope, between the T and the Q when the epitope is in its native context, but not in the context of SEQ ID NO. 22 (compare fig. 6 and 7).

### Example_5

Screening of further epitope arrays led to the identification of constructs promoting the expression of the epitope SSX-2₄₁₋₄₉ (SEQ ID NO. 13). In addition to some of the array elements defined in Example 3, the following additional elements were also used:
SSX-4₅₇₋₆₅:VMTKLGFKV (SEQ ID NO. 23) Array element F.
PSMA-₇₃₀₋₇₃₉: RQIYVAAFTV (SEQ ID NO. 24) Array element G.

A construct, denoted CTLA02, encoding an initiator methionine and the array F-A-G-D-C-F-G-A, was found to successfully immunize HLA-A2 transgenic mice to generate a CTL response recognizing the peptide SSX-2₄₁-₄₉ (SEQ ID NO. 13).

As described above, it can be desirable to combine a sequence with substrate or liberation sequence function with one that can be processed into immune epitopes. Thus SSX-2₁₅₋₁₈₃ (SEQ ID NO. 25) was combined with all or part of the array as follows:
CTLSI: F-A-G-D-C-F-G-A- SSX-2₁₅₋₁₈₃ (SEQ ID NO. 26)
CTLS2: SSX-2₁₅₋₁₈₃ - F-A-G-D-C-F-G-A (SBQ ID NO. 27)
CTLS3: F-A-G-D- SSX-2₁₅₋₁₈₃ (SEQ ID NO. 28)
CTLS4: SSX-2₁₅₋₁₈₃ -G-F-G-A (SEQ ID NO. 29).

All of the constructs except CTLS3 were able to induce CTL recognizing the peptide SSX-2₄₁₋₄₉ (SEQ ID NO. 13). CTLS3 was the only one of these four constructs which did not include the second element A from CTLA02 suggesting that it was this second occurrence of the element that provided substrate or liberation sequence function. In CTLS2 and CTLS4 the A element is at the C-terminal end of the array, as In CTLA02. In CTLS1 the A element is immediately followed by the SSX-2₁₅₋₁₈₃ segment which begins with an alanine, a residue often found after proteasomal cleavage sites (Toes, R.E.M., et al., J. Exp. Med. 194:1-12, 2001). SEQ ID NO. 30 is the polynucleotide sequence encoding SEQ ID NO. 26 used in CTLS1, also called pCBP.

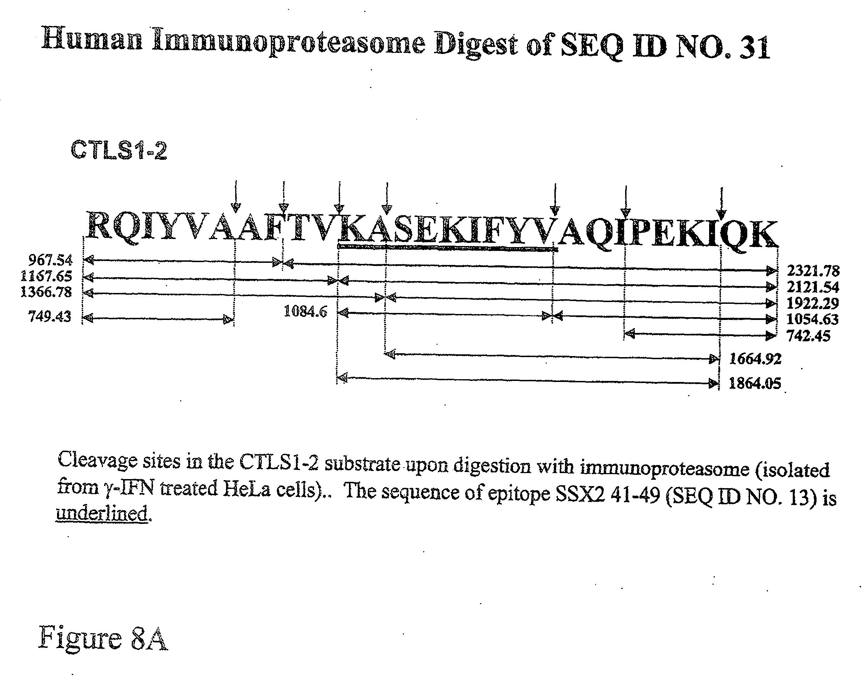

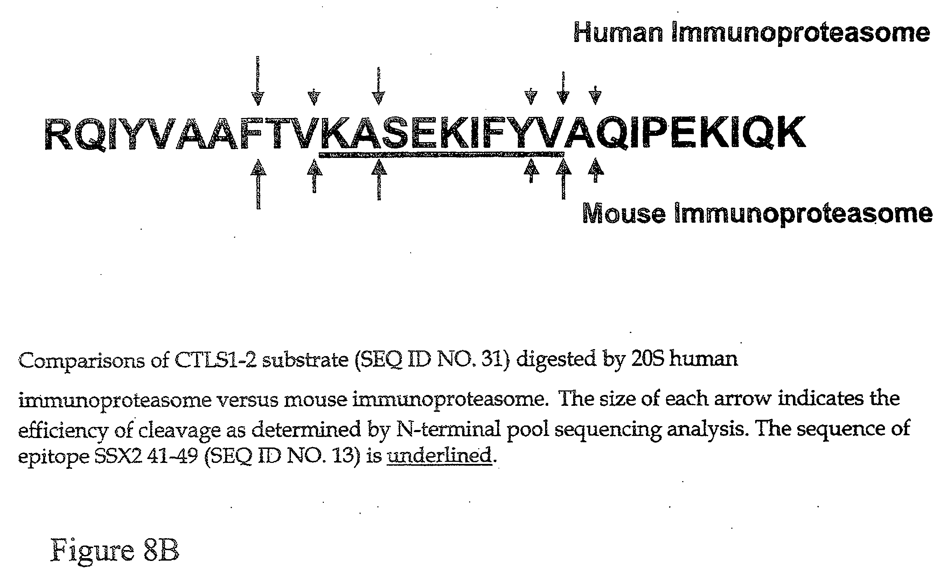

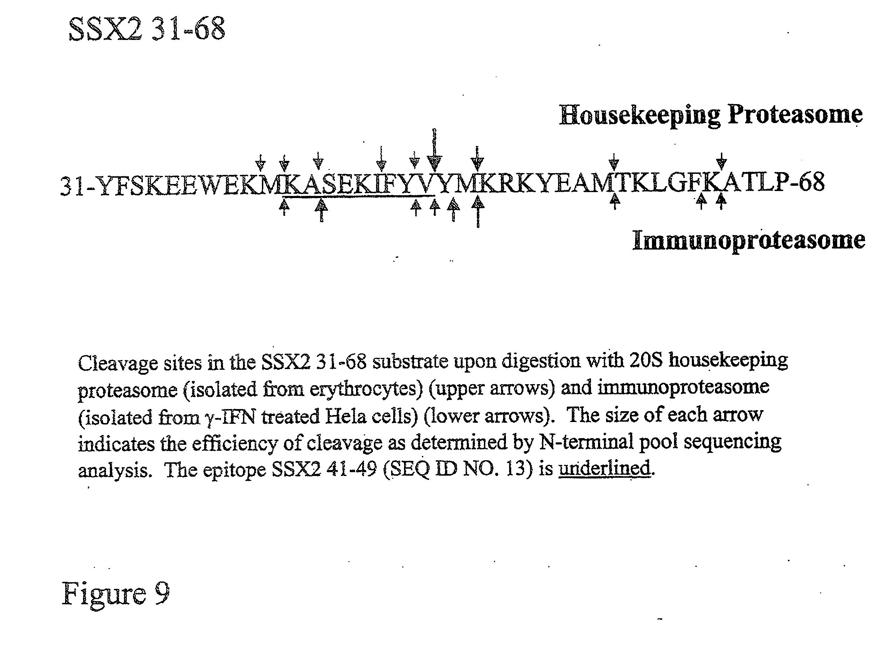

A portion of CTLS1 (SEQ ID NO. 26), encompassing array elements F-A-SSX-2₁₅₋₂₃ with the sequence RQI-YVAAFTV-*KASEKIFV*-AQIPEKIQK (SEQ ID NO. 31), was made as a synthetic peptide and subjected to *in vitro* proteasomal digestion analysis with human immunoproteasome, utilizing both mass spectrometry and N-terminal pool sequencing. The observation that the C-terminus of the SSX-2₄₁₋₄₉ epitope (SEQ ID NO.13) was generated (see Figure 8) provided further evidence In support of substrate or liberation sequence function. The data in Figure 9 showed the differential processing of the SSX-2₄₁₋₄₉ epitope, *KASEKlFYV*(SEQ ID NO.13), in its native context, where the cleavage following the V was the predominant cleavage produced by housekeeping proteasome, while the immunoproteasome had several major cleavage sites elsewhere in the sequence. By moving this epitope Into the context provided by SEQ ID NO. 31 the desired cleavage became a major one and its relative frequency compared to other immunoproteasome cleavages was Increased (compare figs. 8 and 9). The data In figure 8B also showed the similarity in specificity of mouse and human immunoproteasome lending support to the usefulness of the transgente mouse model to predict human antigen processing.

### Example 6

Screening also revealed substrate or liberation sequence function for a tyrosinase epitope, Tyr₂₀₇₋₂₁₅ (SEQ ID NO. 32), as part of an array consisting of the sequence [Tyr₁₋₁₇- Tyr_{207-215]4}, [MLLAVLYCLLWSFQTSA-FLPWHRLFL]₄, (SEQ ID NO. 33). The same vector backbone described above was used to express this array. This array differs from those of the other examples in that the Tyr₁₋₁₇ segment, which was inducled as a source of immune epitopes, is used as a repeated element of the array. This is in contrast with the pattern shown in the other examples where sequence included as a source of immune epitopes and/or length occurred a single time at the beginning or end of the array, the remainder of which was made up of individual epitopes or shorter sequences.

### Plasmid constriction

The polynucleotide encoding SEQ ID NO. 33 was generated by assembly of annealed synthetic oligonucleotides. Four pairs of complementary oligonucleotides were synthesized which span the entire coding sequence with cohesive ends of the restriction sites of AfI II and EcoR I at either terminus. Each complementary pair of oligonucleotides were first annealed, the resultant DNA fragments were ligated stepwise, and the assembled DNA fragment was inserted into the same vector backbone described above pre-digested with AfI II/Vector. I. The construct was called CTLT2/pMEL and SEQ ID NO. 34 is the polynucleotide sequence used to encode SEQ ID NO. 33.

### Example 7

### Administration of a DNA-plasmid formulation of a immunotherapeutic for melanoma to humans.

An MA2M melanoma vaccine with a sequence as described in Example 1 above, was formulated in 1% Benzyl alcohol, 1% ethyl alcohol, 0.5mM EDTA, citrate-phosphate, pH 7.6. Aliquots of 200, 400, and 600 µg DNA/ml were prepared for loading into MINIMED 407C infusion pumps. The catheter of a SILHOUETTE infusion set was placed into an inguinal lymph node visualized by ultrasound imaging. The pump and infusion set assembly was originally designed forthe delivery of insulin to diabetics. The usual 17mm catheter was substituted with a 31 mm catheter for this application. The infusion set was kept patent for 4 days (approximately 96 hours) with an infusion rate of about 25 µl/hour resulting in a total infused volume of approximately 2.4 ml. Thus the total administered dose per infusion was approximately 500, and 1000 µg; and can be 1500 µg, respectively, for the three concentrations described above. Following an infusion, subjects were given a 10 day rest period before starting a subsequent infusion. Given the continued residency of plasmid DNA in the lymph node after administration and the usual kinetics of CTL response following disappearance of antigen. this schedule will be sufficient to maintain the immunologic CTL response.

### Example 8

SEQ ID NO. 22 is made as a synthetic peptide and packaged with a cationic lipid protein transfer reagent. The composition is infused directly into the inguinal lymph node (see example 7) at a rate of 200 to 600 µg of peptide per day for seven days, followed by seven days rest An initial treatment, of 3-8 cycles are conducted.

### Example 9

A fusion protein is made by adding SEQ ID NO. 34 to the 3' end of a nucleotide sequence encoding herpes simplex virus 1 VP22 (SEQ ID NO. 42) in an appropriate mammalian expression vector; the vector used above is suitable. The vector is used to transform HEK 293 cells and 48 to 72 hours later the cells are pelleted, lysed and a soluble extract prepared. The fusion protein is purified by affinity chromatagraphy using an anti-VP22 monoclonal antibody. The purified fusion protein is administered intranodally at a rate of 10 to 100 µg per day for seven days, followed by seven days rest. An initial treatment of 3-8 cycles are conducted.

Further, the present invention can utilize various aspects of the following: U.S. Patent Nos.6,994,851. 6,977,074; 6,709,844.

**Table 11**

| Partial listing of SEQ ID NOS. | | |
|---|---|---|
| 1 | ELAGIGILTV | melan-A 26-35 (A27L) |
| 2 | Melan-A protein | Accession number: NP 005502 |
| 3 | Tyrosinase protein | Accession number P14679 |
| 4 | MLLAVLYCLELAGIGILVYMDGTMSQVGILT | pMA2M expression product |
| | VILGVLLLIGCWYCRRRNGYRALMDKSLHVG | |
| | TQCALTRRCPQEGFDHRDSKVSLQEKNCEPV | |
| 5 | MLLAVLYCLELAGIGILTVYMDGTMSQV | Liberation or substrate sequence for SEQ ID NO.1 from pMA2M |
| 6 | MLLAVLYCL | tyrosinase 1-9 |
| 7 | YMDGTMSQV | tyrosinase 369-377 |
| 8 | EAAGIGILTV | melan-A 26-35 |
| 9 | | pMA2M insert |
| 10 | MVLYCLELAGIGILTVYMDGTAVLYCLELAGI | Epitope array from pVAXM2 and |
| | GILTVYMDGTMLAVLYCLELAGIGILTVYMD GTMSLLAVLYCLELAGIGILTV | pVAXM1 |
| 11 | NY-ESO-1 protein. | Accession number: P78358 |
| 12 | SLLMWITQC | NY-ESO-1 157-165 |
| 13 | KASEKIFYV | SSX-241-49 |
| 14 | TQCFLPVFL | NY-ESO-1 163-171 |
| 15 | GLPSIPVHPI | PSMA 288-297 |
| 16 | AVLYCL | tyrosinase 4-9 |
| 17 | | pN157 expression product |
| 18 | MSLLMWITQCKASEKIFYV | liberation or substrate sequence for SEQ ID NO. 12 from pN157 |
| 19 | | Insert for pN 157 |
| 20 | | pBPL expression product |
| 21 | | pBPL insert coding region |
| 22 | IKASEKIFYVSLLMWKTQCKASEKIFYVK | Substrate in Fig. 6 |
| 23 | VMTKLGFKY | SSX-4₅₇₋₆₅ |
| 24 | RQIYVAAFTV | PSMA₇₃₀₋₇₃₉ |
| 25 | | SSX-2₁₅₋₁₈₃ |
| 26 | | CTLS1/pCBP expression product |
| 27 | | CTLS2 expression product |
| 28 | | CTLS3 expression product |
| 29 | | CTLS4 expression product |
| 30 | | pCBP insert coding region |
| 31 | RQIYVAAFTVKASEKIFYVAQIPEKIQKIQK | Fig.8 substrate/ CTLS1-2 |
| 32 | FLPWHRLFL | TYR.₂₀₇₋₂₁₅ |
| 33 | | CTLT2/pMBL expression product |
| | WSFQTSAFLPWI-IRLFLMLLAVLYCLLWSFQT SAFLPWHRLFL | |
| 34 | | CTLT2/pMEL insert coding region |
| | | |
| 35 | MELAN-A cDNA | Accession number: NM 005511 |
| 36 | Tyrosinase cDNA | Accession number NM 000372 |
| 37 | NY-ESO-1 DNA | Accession number: U87459 |
| 38 | PSMA protein | Accession number: NP 004467 |
| 39 | PSMA cDNA | Accession number NM 004476 |
| 40 | SSX-2 protein | Accession number NP 003138 |
| 41 | SSX-2 cDNA | Accession number: NM 003147 |
| 42 | | From accession number: D10879 Herpes Simplex virus 1 UL49 coding sequence (VP22) |
| 43 | | Accession number P10233 Herpes Simplex virus 1 UL49/VP22 protein sequence |

### Melan-A mRNA sequences

LOCUS NM_005511 1524 bp mRNA PRI 14-OCT-2001

### DEFINITION Homo sapiens melan-A (MLANA), mRNA.

ACCESSION NM_005511

VERSION NM_005511.1 01:5031912

### (SEQ ID NO. 2)

/transladon-'MPREDAHFIYGYPKKGHGHSYTTAEEAAGtGILGVILGVLILIGCWYCRRRNGY RALMDKSLH-VGTQCALTRRCPQEGFDHRDSKVSLQEKNCEPVVPNAPPAYEKLSAEQSPPP YSP'

### (SEQ ID NO. 35)

### ORIGIN

### Tyrosinase mRNA sequence

LOCUS NM_000372 1964 bp mRNA PRI 31 -OCT-2000

### DEFINITION Homo sapiens tyrosinase (oculocutaneous albinism IA) (TYR), mRNA.

ACCESSION NM_000372

VERSION NM_000372.1 GI:4507752

### (SEQ ID NO. 3)

### (SEQ ID NO. 36)

### ORIGIN

### NY-ESO-1 mRNA sequence

LOCUS HSU87459 752 bp mRNA PRI 22-DEC-1999 DEFINITION Human autoimmunogenic cancer/testis antigen NY-ESO-1 mRNA, complete cds.

ACCESSION U87459

VERSION U87459.1 GI-1890098

### (SEQ ID NO. 11)

### (SEQ ID NO. 37)

### ORIGIN

### PSMA cDNA sequence

LOCUS NM_004476 2653 bp mRNA PRI 01-NOV-2000

### DEFINITION Homo sapiens folate hydrolese (prostate-specific membrane antigen) 1 (FOLH1), mRNA.

ACCESSION NM_004476

VERSION NM_004476.1 GI:4758397

### (SEQ ID NO. 38)

### (SEQ ID NO. 39)

### ORIGIN

NM 003147 **Homo sapiens synovial sarcoma, X breakpoint 2 (SSX2), mRNA** LOCUS NM_003147 766 bp mRNA PRI 14-MAR-2001 DEFINITION Homo sapiens synovial sarcoma, X breakpoint 2 (SSX2), mRNA.

ACCESSION NM_003147

VERSION NM_003147.1 GI:10337b82

SEQ ID NO. 40

**SEQ ID NO 41**

## Claims

1. A vector encoding a polypeptide comprising an initiator methionine followed by elements B-A, wherein B is the sequence of SEQ ID No. 12 and A is the sequence of SEQ ID No. 13.

2. Vector of claim 1, wherein the vector comprises a coding sequence consisting of the sequence of SEQ ID No. 21.

3. Vector of claim 1 comprising a sequence encoding SEQ ID No. 17.

4. Vector of claim 1, wherein the vector comprises a sequence encoding the sequence m-(B-A-D-D-A-B-A-A), wherein m is an initiator methionine, B is the sequence according to SEQ ID No. 12, A is the sequence according to SEQ ID No. 13 and D is the sequence according to SEQ ID No. 15.

5. Vector of claim 1, wherein the vector comprises a sequence encoding SEQ ID No. 20.

6. Vector of claim 1 comprising a sequence encoding SEQ ID No. 22.

7. Vector of claim 1, wherein the vector comprises a sequence encoding the sequence m-(B-A-C-B-A-A-C-A), wherein m is an initiator methionine, B is the sequence according to SEQ ID No. 12, A is the sequence according to SEQ ID No. 13 and C is the sequence according to SEQ ID No. 14.

8. A polypeptide encoded by the vector according to any one of the preceding claims, wherein the polypeptide comprises an initiator methionine followed by elements B-A, wherein B is the sequence of SEQ ID No. 12 and A is the sequence of SEQ ID No. 13.

## Patentansprüche

1. Vektor kodierend für ein Polypeptid, das ein Initiatormethionin gefolgt von den Elementen B-A umfasst, wobei B die Sequenz gemäß SEQ ID Nr. 12 und A die Sequenz gemäß SEQ ID Nr. 13 ist.

2. Vektor nach Anspruch 1, wobei der Vektor eine kodierende Sequenz umfasst, die aus der Sequenz gemäß SEQ ID Nr. 21 besteht.

3. Vektor nach Anspruch 1 umfassend eine Sequenz, die für SEQ ID Nr. 17 kodiert.

4. Vektor nach Anspruch 1, wobei der Vektor eine Sequenz umfasst, die für die Sequenz m-(B-A-D-D-A-B-A-A) kodiert, wobei m ein Initiatormethionin ist, B die Sequenz gemäß SEQ ID Nr. 12 ist, A die Sequenz gemäß SEQ ID Nr. 13 ist und D die Sequenz gemäß SEQ ID Nr. 15 ist.

5. Vektor nach Anspruch 1, wobei der Vektor eine Sequenz umfasst, die für SEQ ID Nr. 20 kodiert.

6. Vektor nach Anspruch 1, umfassend eine Sequenz, die für SEQ ID Nr. 22 kodiert.

7. Vektor nach Anspruch 1, wobei der Vektor eine Sequenz umfasst, die für die Sequenz m-(B-A-C-B-A-A-C-A) kodiert, wobei m ein Initiatormethionin ist, B die Sequenz gemäß SEQ ID Nr. 12 ist, A die Sequenz gemäß SEQ ID Nr. 13 ist und C die Sequenz gemäß SEQ ID Nr. 14 ist.

8. Polypeptid, das durch den Vektor nach einem der vorhergehenden Ansprüche kodiert wird, wobei das Polypeptid ein Initiatormethionin gefolgt von den Elementen B-A umfasst, wobei B die Sequenz gemäß SEQ ID Nr. 12 ist und A die Sequenz gemäß SEQ ID Nr. 13 ist.

## Revendications

1. Vecteur codant un polypeptide comprenant une méthionine initiatrice suivie par des éléments B-A, où B est la séquence de SEQ ID N° 12 et A est la séquence de SEQ ID N° 13.

2. Vecteur de la revendication 1, le vecteur comprenant une séquence de codage constituée par la séquence de SEQ ID N° 21.

3. Vecteur de la revendication 1, comprenant une séquence codant SEQ ID N° 17.

4. Vecteur de la revendication 1, le vecteur comprenant une séquence codant la séquence m-(B-A-D-D-A-B-A-A), où m est une méthionine initiatrice, B est la séquence selon SEQ ID N° 12, A est la séquence selon SEQ ID N° 13 et D est la séquence selon SEQ ID N° 15.

5. Vecteur de la revendication 1, le vecteur comprenant une séquence codant SEQ ID N° 20.

6. Vecteur de la revendication 1, comprenant une séquence codant SEQ ID N° 22.

7. Vecteur de la revendication 1, le vecteur comprenant une séquence codant la séquence m-(B-A-C-B-A-A-C-A), où m est une méthionine initiatrice, B est la séquence selon SEQ ID N° 12, A est la séquence selon SEQ ID N° 13 et C est la séquence selon SEQ ID N° 14.

8. Polypeptide codé par le vecteur selon l'une quelconque des revendications précédentes, le polypeptide comprenant une méthionine initiatrice suivie par des éléments B-A, où B est la séquence de SEQ ID N° 12 et A est la séquence de SEQ ID N° 13.
